# EUROPEAN PATENT APPLICATION

(11) **EP 4 424 717 A1**
(43) Date of publication of application: **04.09.2024**
(21) Application number: 22887168.7
(22) Date of filing: 28.10.2022
(51) Int. Cl.: C08B 37/00, C07H 1/00, C07H 15/203, C07H 19/04

(54) **NOVEL OLIGOSACCHARIDE, MANUFACTURING INTERMEDIATE FOR NOVEL OLIGOSACCHARIDE, AND METHOD FOR MANUFACTURING THESE**

(30) Priority: 29.10.2021 JP 2021178383; 17.01.2022 JP 2022005353
(71) Applicant: Daiichi Sankyo Company, Limited, Tokyo 103-8426 (JP)
(72) Inventor: UEDA Tsuyoshi, Tokyo 103-8426 (JP); ITOH Ryusei, Tokyo 103-8426 (JP); NAKAMURA Tatsuya, Tokyo 103-8426 (JP); SUZUKI Keisuke, Tokyo 103-8426 (JP); NAKANE Satoshi, Tokyo 103-8426 (JP); YANG ZEKUN, Tokyo 103-8426 (JP)
(74) Representative: Marks & Clerk LLP
(86) International application number: PCT/JP2022/040346
(87) International publication number: WO 2023/074843

(57) **Abstract**

The objective of the invention is to provide a novel oligosaccharide, which can be used for producing a biantennary glycan having an α2,6-sialic acid structure at a non-reducing end, a method for production thereof, production intermediates thereof and a method for producing the intermediates, as well as a novel oligosaccharide, which is a biantennary glycan having an α2,6-sialic acid structure at a non-reducing end, a method for producing the oligosaccharide, intermediates thereof, and a method for producing the intermediates. Provided are a novel oligosaccharide represented by Formula A-13 or D-13: 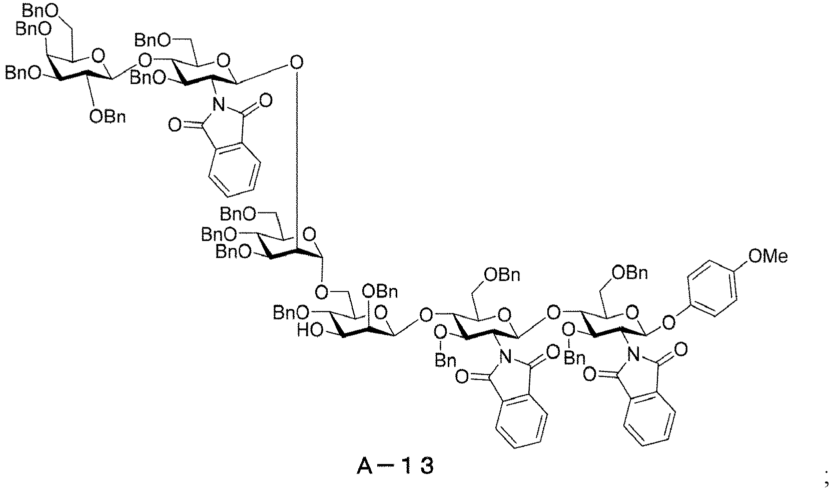 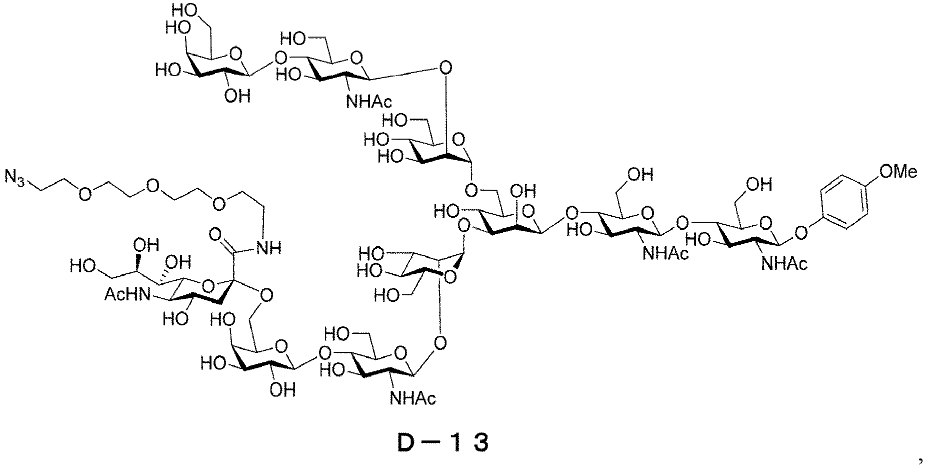 a method for producing the oligosaccharide represented by Formula A-13 or Formula D-13 shown in, for example, Figure 1 or Figure 3, intermediates thereof and a method for producing the intermediates.

## Description

### FIELD OF INVENTION

The present invention relates to a novel oligosaccharide, which is a biantennary glycan having an α2,6-sialic acid structure at a non-reducing end, a method for producing the oligosaccharide, each intermediate thereof, and a method for producing the intermediate.

### BACKGROUND OF THE INVENTION

Addition of a glycan to a protein (glycosylation) is known to significantly affect the function and structure of the protein. Especially, N-linked glycans are deeply involved in physiological activities of proteins. Among the N-linked glycans, a biantennary N-glycan with an α2,6-sialic acid structure at a non-reducing end has been reported to be a structure optimal for increasing antibody-dependent cellular cytotoxic activity (ADCC activity) and complement-dependent cytotoxic activity (CDC activity) (Non-Patent Literature 1).

For the development and commercialization of pharmaceuticals using glycans, it is desirable to be able to stably produce large quantities of highly pure glycans at an industrially applicable price. The synthesis of α2,6-sialyl glycan reported so far can be roughly classified into two methods: 1) semi-chemical synthesis by natural extract isolation and purification or main backbone precursor chemical synthesis in combination with enzymatic chemical conversion and 2) pure chemical synthesis.

For example, it has been reported that as the semi-chemical synthesis, an enzymatic process and a chemical process can be combined to obtain an N-linked glycan from yolks of chicken eggs (Non-Patent Literature 2). Such an approach can be used to synthesize a target glycan with fewer steps than pure chemical synthesis. On the other hand, a large amount of egg yolks must be provided, and special techniques and purification equipment are often required for subsequent isolation and purification from egg yolks and purification of water-soluble unprotected glycans after chemical conversion (Patent Literatures 1 to 4).

Meanwhile, examples of the pure-chemical synthesis of the α2,6-sialyl glycan include the following reported cases:
(1) total synthesis of α2,6-sialyl moiety-containing complex glycan having 11 sugars (Non-Patent Literature 3);
(2) total synthesis of α2,6-sialyl moiety-containing immunoglobulin G peptide having 13 sugars (Non-Patent Literature 4);
(3) total synthesis of core fucose-containing α2,6-sialyl N-linked glycan having 12 sugars (Non-Patent Literature 5); and
(4) total synthesis of position 3-fluorinated α2,6-sialyl N-linked glycan having 10 sugars (Non-Patent Literature 6).
(5) total synthesis of asymmetrically deuterated α2,6-sialyl biantennary oligosaccharide chain with 11 sugars and tetraantennary oligosaccharide chain having 17 sugars (Non-Patent Literature 7)

For pure chemical synthesis of glycan, if a robust method for production thereof is established, the flexibility of the production quantity is considered to be extremely high by producing the glycan from monosaccharides, similar to the case of a common compound with low molecular weight. Further, since sugars modified with a protecting group may be converted, most of operations for purification may be the same as those for non-aqueous compounds, and the complexity of the operations and man-hour for the operations are expected to be significantly reduced compared to those for the semi-chemical synthesis. Furthermore, established methods for the chemical synthesis may be modified to facilitate synthesis of a wide variety of unnatural glycans.

Meanwhile, in view of the aforementioned cases reported in the past, big problems regarding the synthesis include the following two problems: 1) Among the step of converting sugar moieties that are difficult to be converted and the step of binding them, for example, for the construction of β-mannoside and α-sialyl moieties, there is a step with low selectivity and low yield; and 2) in both the steps of converting the sugar moieties and binding them, chromatography purification on silica gel column, which is not suitable for scale-up, is often used, and thus precise operations for preparative chromatography purification , which are performed to remove isomers and impurities generated as byproducts in a reaction, must be required in many of the steps.

As described above, the pure chemical synthesis of glycan has potential advantages in mass synthesis over the semi-chemical synthesis. However, it is difficult to say that sufficient technical development has been achieved in terms of yield, selectivity, efficiency, and cost, and there are very few cases of the pure chemical synthesis, which have been actually adopted as a method for the mass synthesis. Further, it is very difficult to achieve the mass synthesis of a biantennary glycan (α2,6-sialyl glycan) having different glycan units linked to mannose at positions 3 and 6 as branching points, either in the production from a natural extract, the semi-chemical synthesis, or the total chemical synthesis, due to its structure.

In a sugar derivative protected with phthalimide group(s), it is sometimes necessary to benzylate a plurality of hydroxyl groups simultaneously. In this case, the reaction should be carried out while suppressing ring-opening of the phthalimide group(s). However, the ring-opening reaction of the phthalimide group(s) proceeds readily under strongly basic conditions, due to the presence of a trace amount of hydroxide ions. Accordingly, under the NaH/DMAc condition used in conventional benzylation reactions, the yield varies greatly depending on the amount of sodium hydroxide in NaH. Further, NaH/DMAc is not easy to be used for the mass synthesis due to hazardous reagent combination and risk of explosion. Thus, there is a need for a method for benzylating a plurality of hydroxyl groups simultaneously under milder conditions while suppressing ring-opening of the phthalimide group(s).

In the sugar derivative protected with the phthalimide group(s), deacylation is sometimes necessary to be performed. However, if a trace amount of water is present in the system, the phthalimide group(s) readily undergoes a ring-opening reaction under the basic condition. Thus, it is necessary to strictly control water content in the system, but it is difficult to completely suppress the ring-opening reaction even at the level of water content on the order of ppm. Therefore, there is a need for a method for achieving the deacylation in high yield while suppressing the ring-opening of the phthalimide group(s).

In the chemical synthesis of an oligosaccharide chain having hydroxyl group(s), etc., it is necessary to make rational use of a method for selectively protecting and deprotecting such hydroxyl groups in order to efficiently obtain a compound of interest. In particular, a 2-naphthylmethyl group has been widely used as a common protecting group for a hydroxyl group. Meanwhile, as a method for the deprotection of the 2-naphthylmethyl group, a method in which 2,3-dichloro-5,6-dicyano-p-benzoquinone is used in dichloromethane-water is known, and provides a deprotected product of interest in moderate to high yield for a wide range of substrates. However, in general, dichloromethane and water are immiscible, and 2,3-dichloro-5,6-dicyano-p-benzoquinone and its byproduct 2,3-dichloro-5,6-dicyano-p-benzohydroquinone are almost insoluble in dichloromethane-water, which affect stirring properties, and thus make it difficult to apply this method to the mass synthesis. In addition, it has been reported that the deprotected product of interest cannot always be obtained in satisfactory yield, depending on the substrate to be used (Non-Patent Literature 8). In addition, an increase in the number of benzyl groups in the substrate tends to result in decreasing the reaction yield (Non-Patent Literature 9), and thus it has been desired to develop a milder and more efficient method applicable to complex substrates. As a means for achieving such an object, improved conditions using β-pinene as an additive have been reported (Non-Patent Literature 10), but even using this method, the yield remains to be moderate for complex substrates having a plurality of benzyl groups (Non-Patent Literature 11). In view of the above background, it has been desired to develop de-2-naphthylmethylation reaction, in which a protecting group of the 2-naphthylmethyl group can be deprotected under milder conditions to obtain a deprotected product in high yield.

Further, methods of liquid-phase and solid-phase synthesis are known as methods for chemical synthesis of oligosaccharide chains. For the method of the liquid-phase synthesis, a common approach for an organic synthesis can be used, and thus it is easy to track and scale up its reaction, whereas post-processing and purification are required in every step, causing disadvantages of taking time and effort. In addition, the method for the solid-phase synthesis has an advantage in that it can be automated and can achieve a quick production, but is not suitable for industrial mass synthesis, because its scale-up is limited due to the limitation of equipment; an excessive amount of glycosyl donor should be used in the sugar elongation reaction due to low reactivity; and it has a disadvantage of being difficult to check the progress of the reaction during the reaction (Patent Literature 5). To solve these problems, several methods have been developed for production of an oligosaccharide using a substrate, to which a molecular structure (tag), which can induce, for example, specific precipitation, distribution, adsorption, etc., in a certain environment, is attached (Patent Literature 6 and Non-Patent Literatures 12, 13, and 14). These methods have both the advantages in the method for the liquid-phase synthesis and solid-phase synthesis, specifically, reactions for the methods can be performed in a homogeneous system, which makes it easy to analyze the reaction, and remnants of reagents, etc., can be separated by utilizing the feature of the tag. For example, a method for separating an untagged compound by adsorbing it onto octadecylmodified silica gel in a post-reaction solution using a branched long-chain alkane as a hydrophobic tag, is known (Non-Patent Literature 14). However, since it is necessary to use the tag in any of the methods, a desorption step is required and the size of the substrate site becomes larger than that of the tag as an oligomerization proceeds, thereby, the physical properties of the substrate gradually became dominant, causing the disadvantage of reducing the function of the tag. Therefore, there is a need for a method for purifying oligosaccharide more efficiently in a method for producing the oligosaccharide.

In the glycosylation reaction, if a -NHAc group is present in a reaction substrate, interacting it with a Lewis acid causes a significant decrease in reactivity in the glycosylation reaction of interest, and an excess amount of glycosyl donor is often required to complete the reaction. Therefore, when an oligosaccharide chain containing acetylglucosamine is synthesized, a method has been used in which a Troc group (Non-Patent Literatures 5, 6 and 7), phthalimide group, or sulfonyl group (Non-Patent Literature 4) is used as a temporary protecting group of a nitrogen in glucosamine during the glycosylation reaction, followed by conducting deprotection and then N-acetylation. However, for the Troc group, a deprotection condition with zinc/AcOH or a reaction with an excess amount of lithium hydroxide for a long time is required, and degradation of the substrate in a complex glycan occurs under the deprotection condition. In the deprotection of the phthalimide group, there is a problem, that is, amidation of a sialic acid ester moiety proceeds as a side reaction due to using an excess amount of ethylenediamine. In order to avoid this problem, two-step processes are required, in which an ester moiety is selectively hydrolyzed in advance, followed by conducting deprotection. In addition, for the sulfonyl group, the deprotection is carried out under a reaction condition using metallic sodium, which is difficult to be scaled up. Hence, in the method for producing an oligosaccharide chain containing acetylglucosamine, there is a need for developing a protecting group that can be easily replaced with an acetyl group without reducing the reactivity of the glycosylation reaction.

Recently, polyethylene glycol has been frequently used as a biocompatible water-soluble substructure in the development of pharmaceuticals, etc. To develop such pharmaceuticals, the structure of the polyethylene glycol is desirable to be more uniform and highly pure. However, many impurities are includedin commercially available reagents, and thus it is required to conduct strict purification thereof by distillation or complicated purification thereof on column. Further, if a compound having a polyethylene glycol structure contains an azide structure, operations for the distillation that require heating cannot be carried out due to concerns about its explosion. Very recently, a method for the purification using a metal complex with MgCh has been reported (Non-Patent Literature 15). However, in this method, a substance of interest is adsorbed onto an excess amount of MgCl₂, causing a large amount of its loss to a filtrate, and thus the effect of the purification is expected to be smaller than that of isolation by crystallization. Therefore, it has been desired to achieve a method for purifying the above compound having the polyethylene glycol structure .

In the synthesis of an oligosaccharide chain, it becomes difficult to isolate and purify its intermediate as a crystal, as the molecular weight thereof increases. In particular, there have been no reports on the crystallization of an intermediate of a protected tri- or moresaccharide with a molecular weight greater than 1000. Therefore, a big problem is to remove, from the substance of interest, its analogues having similar structures, such as a trace amount of isomers produced in the reaction and remaining impurities derived from raw materials. In the past, purification on silica gel column, which was used in each step of the synthesis to remove these analogues, was a major obstacle for achieving an efficient mass synthesis. In view of the above background, it has been desired to develop a method for crystallization and purification , which can efficiently remove impurities having similar structures from intermediates in the synthesis of an oligosaccharide chain.

### CITATION LIST

### Patent Literature

Patent Literature 1: WO 2011/027868
Patent Literature 2: WO 96/02255
Patent Literature 3: WO 2014/208742
Patent Literature 4: WO 2017/110984
Patent Literature 5: WO 2002/16384
Patent Literature 6: Japanese Patent No. 6001267

### Non-Patent Literature

Non-Patent Literature 1: Proc. Natl. Acad. Sci. U.S.A.2015, 112, 10611-10616
Non-Patent Literature 2: Beilstein J. Org. Chem. 2018, 14, 416-429
Non-Patent Literature 3: TetrahedronLett. 1986, 27, 5739-5742
Non-Patent Literature 4: J. Am. Chem. Soc. 2009, 131, 16669-16671
Non-Patent Literature 5: J. Org. Chem. 2016, 81, 10600-10616
Non-Patent Literature 6: J. Am. Chem. Soc. 2019, 141, 6484-6488
Non-Patent Literature 7: Angew. Chem. Int. Ed. 2021, 60, 24686-24693
Non-Patent Literature 8: Org. Lett. 2002, 4, 4551-4554
Non-Patent Literature 9: J. Am. Chem. Soc. 2018, 140, 4632-4638
Non-Patent Literature 10: J. Org. Chem., 2017, 82, 3926-3934
Non-Patent Literature 11: Angew. Chem. Int. Ed. 2021, 60, 19287-19296
Non-Patent Literature 12: The Society of Synthetic Organic Chemistry, Japan Journal, 2002, 60(5), 494-495
Non-Patent Literature 13: Org. Biomol. Chem. 2018, 16, 4720-4727
Non-Patent Literature 14: J. AM. CHEM. SOC. 2005, 127, 7296-7297
Non-Patent Literature 15: Org. Process. Res. Dev. 2021, 25, 10, 2270-2276

### SUMMARY OF THE INVENTION

### Objectives ofthe invention

One of objectives of the present invention is to provide a novel oligosaccharide, which can be used for producing a biantennary glycan having an α2,6-sialic acid structure at a non-reducing end, a method for producing the oligosaccharide, intermediate(s) thereof, and a method for producing the intermediate. Another objective of the present invention is to provide a novel oligosaccharide, which is a biantennary glycan having an α2,6-sialic acid structure at a non-reducing end, a method for producing the oligosaccharide, intermediate(s) thereof, and a method for producing the intermediate.

### Means for achieving the objectives

The present inventors have conducted intensive study to achieve the above-mentioned objectives and, as a result, have found a novel oligosaccharide represented by A-13 below, a novel method for efficiently producing the oligosaccharide, intermediates thereof and a method for producing the intermediates, as well as a novel oligosaccharide, which is a biantennary glycan having an α2,6-sialic acid structure at a non-reducing end and is represented by D-13 below, a method for efficiently producing the oligosaccharide, intermediates thereof, and a method for producing the intermediate, thereby the present invention has been achieved.

In particular, the present inventions relate to, but are not limited to, the following embodiments.

[1] A method for producing an oligosaccharide represented by Formula A-13: 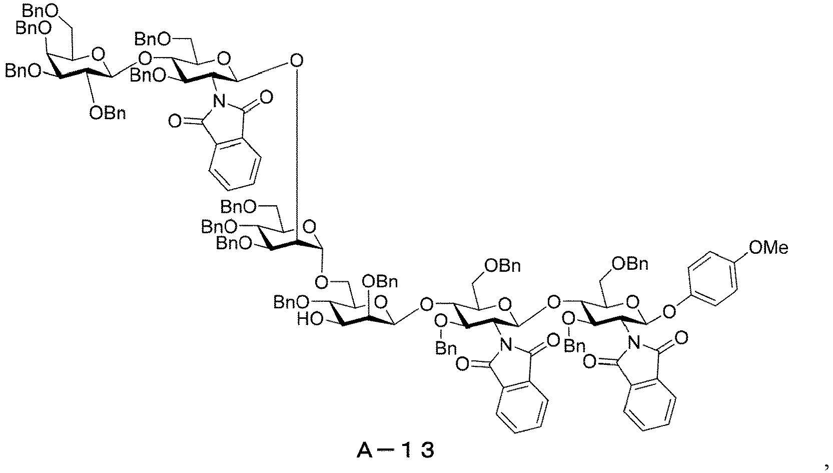 wherein the method comprises steps of:
   (Step I-1) producing a compound represented by Formula A-7: 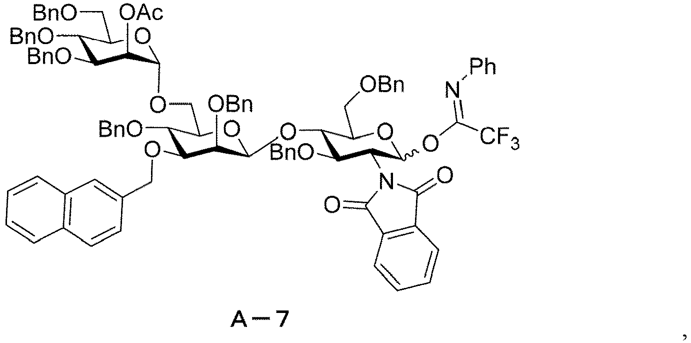 wherein the step comprising a step of:
      connecting a compound represented by Formula A-3: 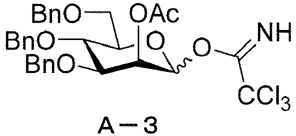 to a compound represented by Formula A-4: 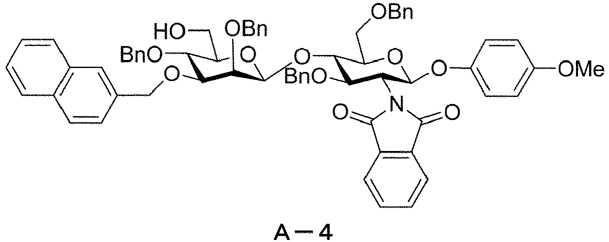 via α-1,6-glycosidic linkage to give a compound represented by Formula A-5: 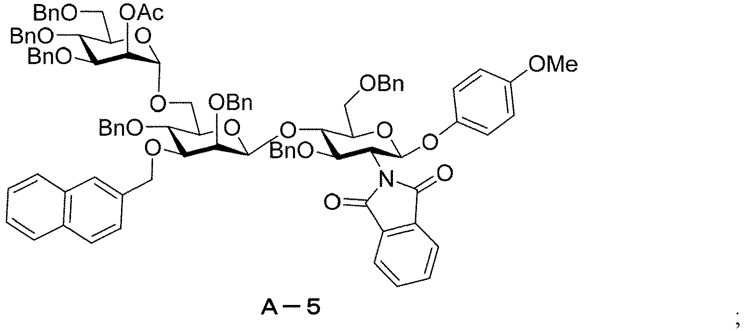
   (Step I-2) producing a compound represented by Formula A-10: 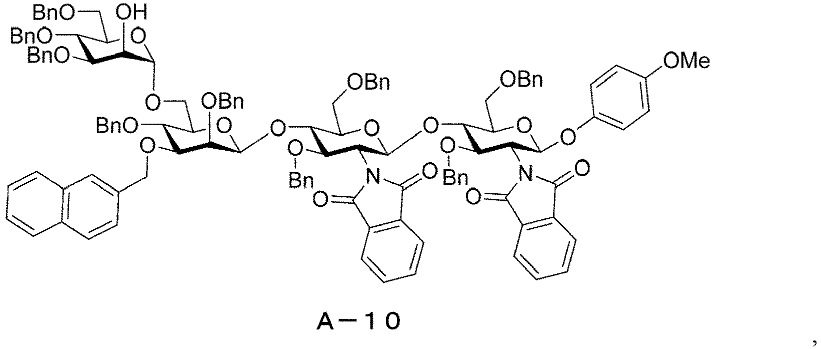 wherein the step comprising a step of:
      connecting the compound represented by Formula A-7 to a compound represented by Formula A-8: 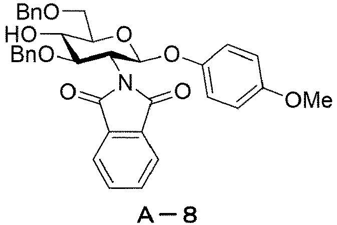 via β-1,4-glycosidic linkage to give a compound represented by Formula A-9: 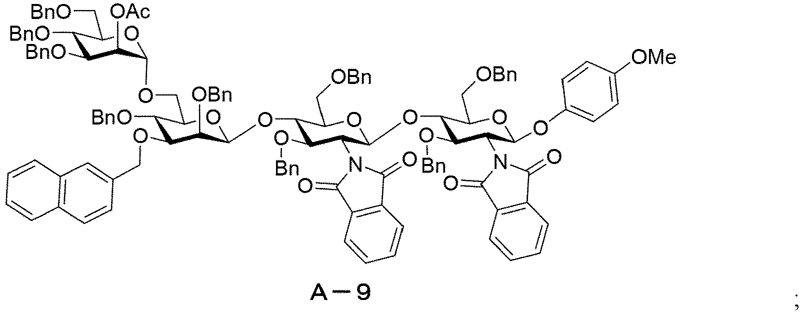 and
   (Step I-3) producing the oligosaccharide represented by Formula A-13, wherein the step comprising a step of:
      connecting the compound represented by Formula A-10 to a compound represented by Formula A-11: 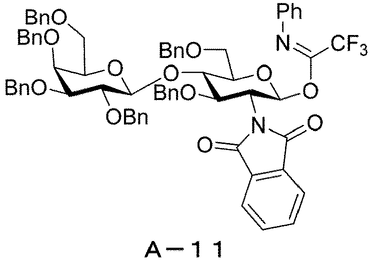 via β-1,2-glycosidic linkage to give a compound represented by Formula A-12: 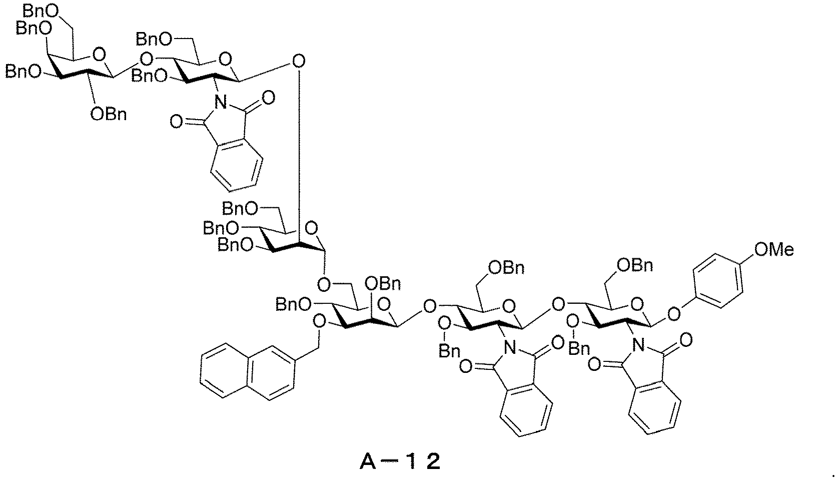
[2] The method according to [1], wherein Step I-2 comprises reacting the compound represented by Formula A-9 with a strong base in the presence of an alkyl ester of perfluorocarboxylic acid to give the compound represented by Formula A-10.
[3] The method according to [2], wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.
[4] The method according to [2] or [3], wherein the strong base is selected from the group consisting of sodium, lithium, and potassium salts of metal amides; sodium, lithium, potassium, cesium, and barium salts of C1-C20 alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and a combination thereof.
[5] The method according to [2] or [3], wherein the strong base is potassium tert-butoxide, sodium tert-butoxide, lithium tert-butoxide, or LHMDS (lithium hexamethyldisilazide).
[6] The method according to any one of [2] to [5], wherein the reaction of Step I-2 is carried out in a C1-C10 alcohol solvent alone or in a mixture of a C1-C10 alcohol solvent and an amide-based solvent, an ether-based solvent, an ester-based solvent, aromatic solvent, a halogen-based solvent, a hydrocarbon-based solvent, or a nitrile-based solvent.
[7] The method according to any one of [1] to [6], wherein Step I-3 comprises reacting the compound represented by Formula A-12 with DDQ (2,3-dichloro-5,6-dicyano-p-benzoquinone) in a mixed solvent of fluorous alcohol and water to remove a 2-naphthylmethyl group in the compound represented by Formula A-12 to give the oligosaccharide represented by Formula A-13.
[8] The method according to [7], wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and a combination thereof.
[9] The method according to [7] or [8], wherein the reaction of Step I-3 is carried out at - 35°C to 70°C.
[10] The method according to [7] or [8], wherein the reaction of Step I-3 is carried out at - 30°C to -10°C.
[11] The method according to any one of [1] to [10], wherein the method comprises purifying the compound represented by Formula A-5 by
   after stopping the reaction of the compound represented by Formula A-4 with the compound represented by Formula A-3 in Step I-1, adding a hydrophobic carrier and water to a water-soluble organic solvent containing the resulting compound represented by Formula A-5 and contaminants to adsorb the compound represented by Formula A-5 onto the hydrophobic carrier;
   then filtering the resultant and washing the hydrophobic carrier with a mixture of the water-soluble organic solvent and the water to remove the contaminants;
   and then eluting the compound represented by Formula A-5 from the hydrophobic carrier using an organic solvent.
[12] The method according to any one of [1] to [11], wherein the method comprises purifying the compound represented by Formula A-9 by
   after stopping the reaction of the compound represented by Formula A-7 with the compound represented by Formula A-8 in Step I-2, adding a hydrophobic carrier and water to a water-soluble organic solvent containing the resulting compound represented by Formula A-9 and contaminants to adsorb the compound represented by Formula A-9 onto the hydrophobic carrier;
   then filtering the resultant and washing the hydrophobic carrier with a mixture of the water-soluble organic solvent and the water to remove the contaminants;
   and then eluting the compound represented by Formula A-9 from the hydrophobic carrier using an organic solvent.
[13] The method according to any one of [1] to [12], wherein the method comprises purifying the compound represented by Formula A-12 by
   after stopping the reaction of the compound represented by Formula A-10 with the compound represented by Formula A-11 in Step I-3, adding a hydrophobic carrier and water to a water-soluble organic solvent containing the resulting compound represented by Formula A-12 and contaminants to adsorb the compound represented by Formula A-12 onto the hydrophobic carrier;
   then filtering the resultant and washing the hydrophobic carrier with a mixture of the water-soluble organic solvent and the water to remove the contaminants;
   and then eluting the compound represented by Formula A-12 from the hydrophobic carrier using an organic solvent.
[14] The method according to [11], wherein the contaminants include a sugar compound other than the compound represented by Formula A-5, and/or a compound derived from a reaction reagent to obtain the compound to be purified.
[15] The method according to [12], wherein the contaminants include a sugar compound other than the compound represented by Formula A-9, and/or a compound derived from a reaction reagent to obtain the compound to be purified.
[16] The method according to [13], wherein the contaminants include a sugar compound other than the compound represented by Formula A-12, and/or a compound derived from a reaction reagent to obtain the compound to be purified.
[17] The method according to any one of [11] to [16], wherein the hydrophobic carrier is a resin used for packing reversed-phase partition chromatography.
[18] The method according to [17], wherein the resin packed for reversed-phase partition chromatography is selected from the group consisting of apoly(styrene/divinylbenzene) polymer gel resin, a polystyrene-divinylbenzene resin, a polyhydroxymethacrylate resin, styrene-vinylbenzene copolymer resin, a polyvinyl alcohol resin, a polystyrene resin, polymethacrylate resin, a chemically bonded silica gel resin, and a combination thereof.
[19] The method according to [18], wherein the chemically bonded silica gel resin is selected from the group consisting of (1) a resin obtained by reacting silica gel with a silane coupling agent, (2) a resin obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) a resin obtained by chemically bonding a docosyl or triacontyl group to silica gel, and (4) a combination of the resins (1) to (3).
[20] The method according to [18], wherein the chemically bonded silica gel resin is an octadecyl group-bonded silica gel resin (ODS resin).
[21] The method according to any one of [11] to [20], wherein the water-soluble organic solvent is a water-soluble alcohol-based solvent, a water-soluble nitrile-based solvent, a water-soluble ether-based solvent, a water-soluble ketone-based solvent, a water-soluble amide-based solvent, or a water-soluble sulfoxide-based solvent, or a mixed solvent containing at least one of the aforementioned water-soluble organic solvents.
[22] The method according to [21], wherein the water-soluble nitrile-based solvent is acetonitrile.
[23] The method according to any one of [11] to [22], wherein the organic solvent used for eluting the compound of interest from the hydrophobic carrier is a nitrile-based solvent, an ether-based solvent, an ester-based solvent, a ketone-based solvent, a halogen-based solvent, an aromatic solvent, or a mixed solvent containing at least one of the aforementioned solvents.
[24] The method according to any one of [1] to [23], wherein the compound represented by Formula A-11 is produced by steps comprising:
   (Step Y-1) a step of producing a compound represented by Formula B-4: 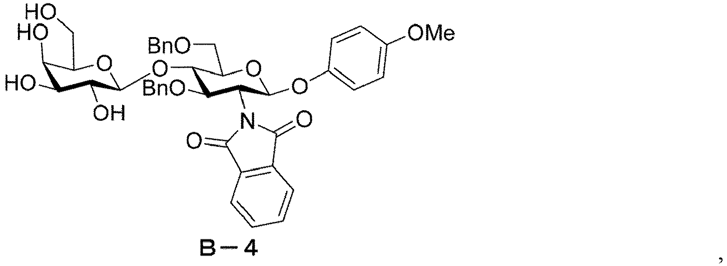 wherein the step comprising a step of:
      connecting a compound represented by Formula B-1: 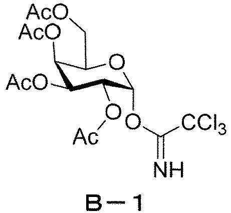 to a compound represented by Formula B-2: 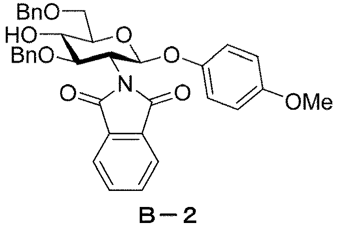 via β-1,4-glycosidic linkage to give a compound represented by Formula B-3: 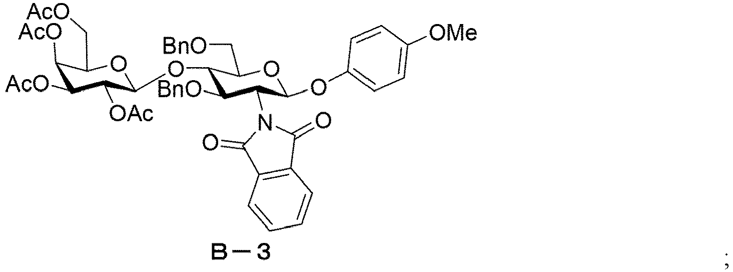 and
   (Step Y-2) a step of adding lithium tert-butoxide or lithium tert-amoxide to a solvent containing the compound represented by Formula B-4 and benzyl halide or benzyl sulfonate to protect each hydroxyl group present in the compound represented by Formula B-4 with a benzyl group to give a compound represented by Formula B-5: 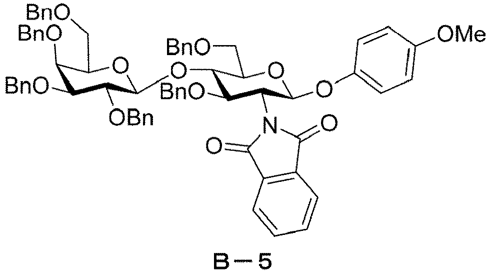
[25] The method according to [24], wherein the solvent containing the compound represented by Formula B-4 and benzyl halide or benzyl sulfonate is an amide-based solvent, an ether-based solvent, an aromatic solvent, or a hydrocarbon-based solvent, a urea-based solvent, or a mixed solvent containing at least one of the aforementioned solvents.
[26] The method according to [24] or [25], further comprising the step of purifying the compound represented by Formula B-5 by conducting ring-opening of a phthalimide group in the compound represented by Formula B-5, followed by forming a salt with cinchonidine to give a crystalline compound represented by Formula B-6: 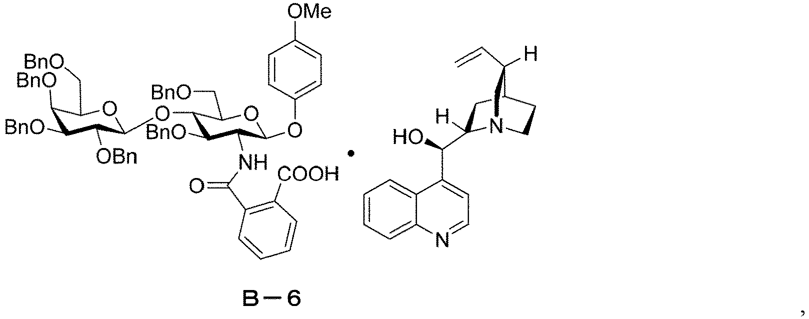 separating the crystalline compound represented by Formula B-6 from amorphous materials, then adding an acid aqueous solution and solvent to remove cinchonidine from the compound represented by Formula B-6 to give a compound represented by Formula B-7: 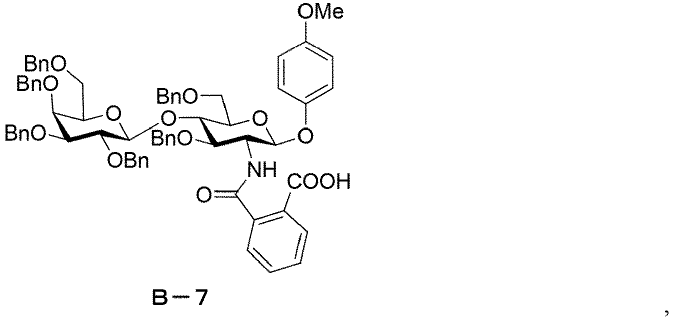 and then conducting ring-closing of the ring-opened phthalimide in the compound represented by Formula B-7.
[27] The method according to any one of [1] to [26], further comprising the step of purifying the compound represented by Formula A-13 by conducting ring-opening of phthalimide groups in the compound represented by Formula A-13, followed by forming a salt with (R)-(+)-1-(1-naphthyl)ethylamine to give a crystalline compound represented by Formula A-14: 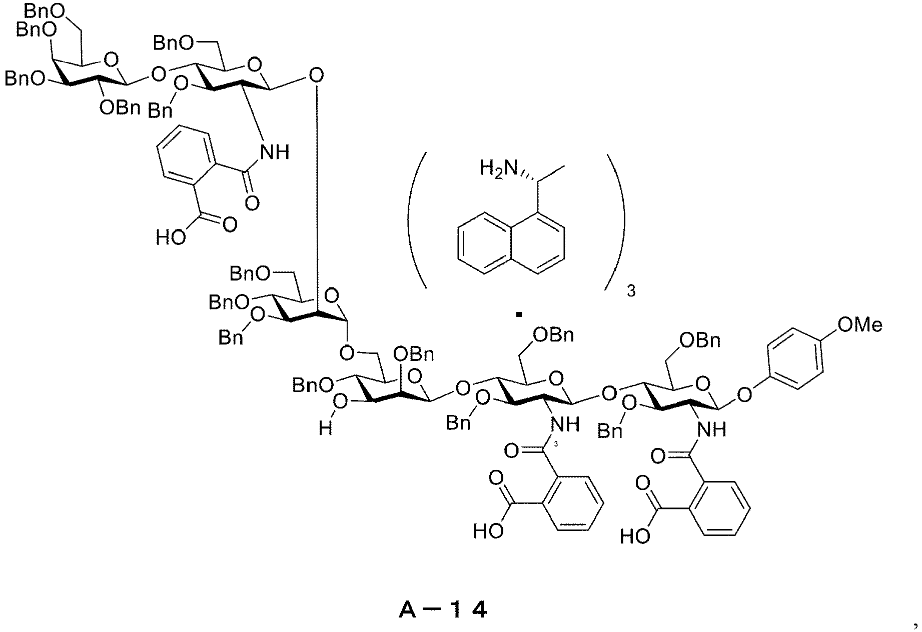 separating the crystalline compound represented by Formula A-14 from amorphous materials, then adding an acid aqueous solution and solvent to remove (R)-(+)-1-(1-naphthyl)ethylamine from the compound represented by Formula A-14 to give a compound represented by Formula A-15: 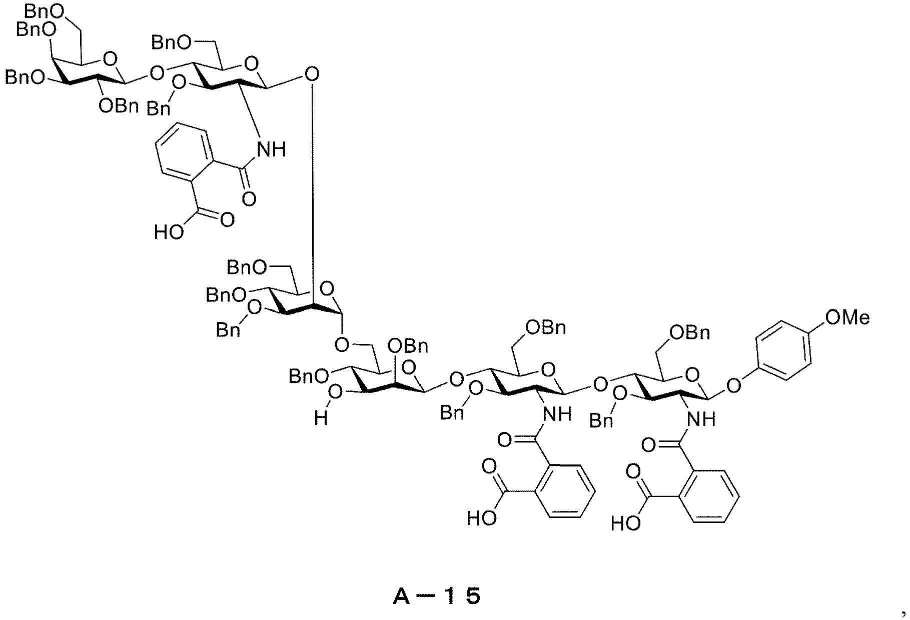 and then conducting ring-closing of the ring-opened phthalimides in the compound represented by Formula A-15.
[28] A method for producing an oligosaccharide represented by Formula D-13: 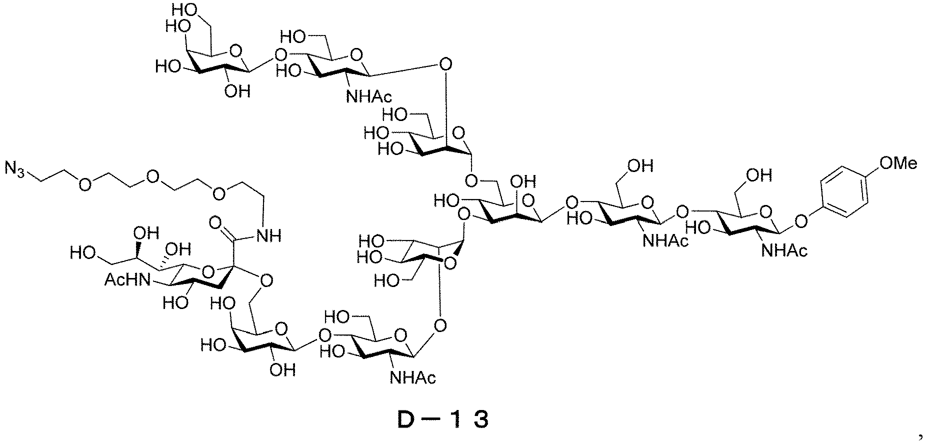 wherein the method comprises steps of:
   (Step II-1) producing a compound represented by Formula D-2: 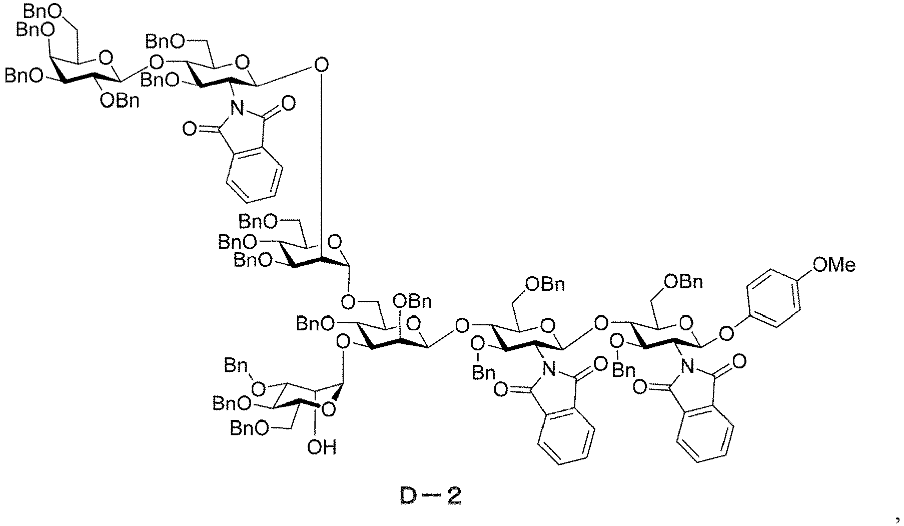 wherein the step comprising a step of:
      connecting an oligosaccharide represented by Formula A-13: 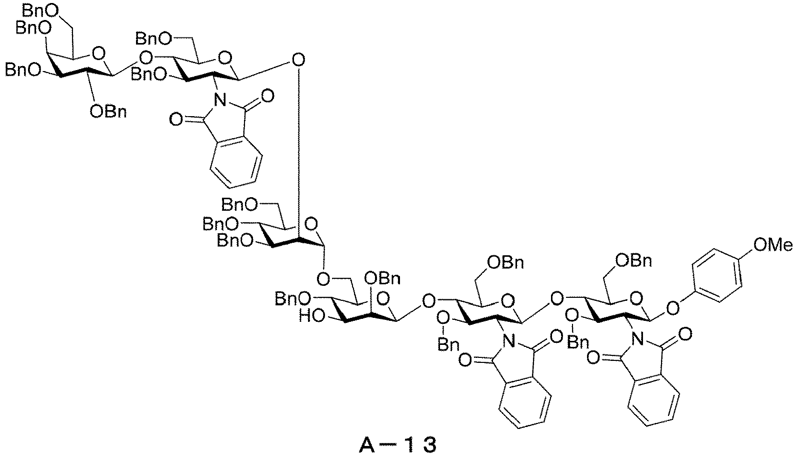 to a compound represented by Formula A-3: 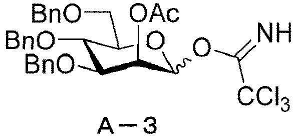 via α-1,3-glycosidic linkage to give a compound represented by Formula D-1: 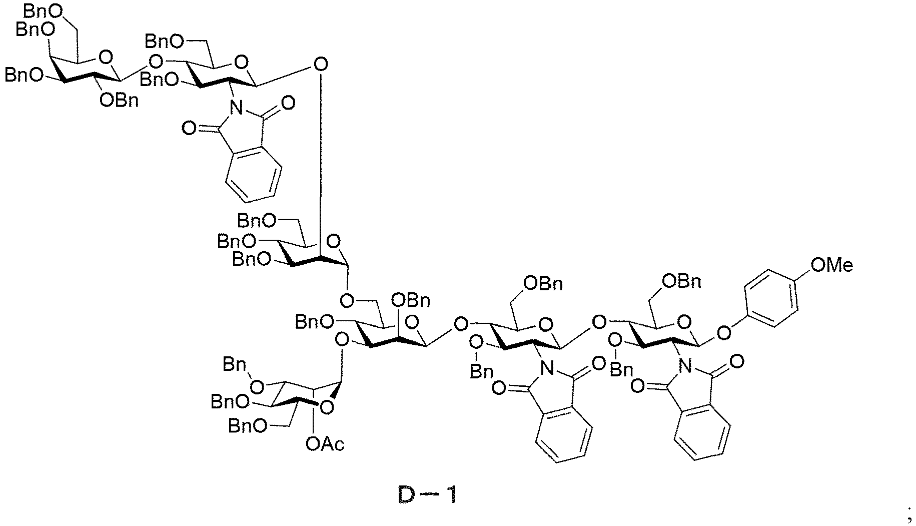
   (Step II-2) producing a compound represented by Formula D-5: 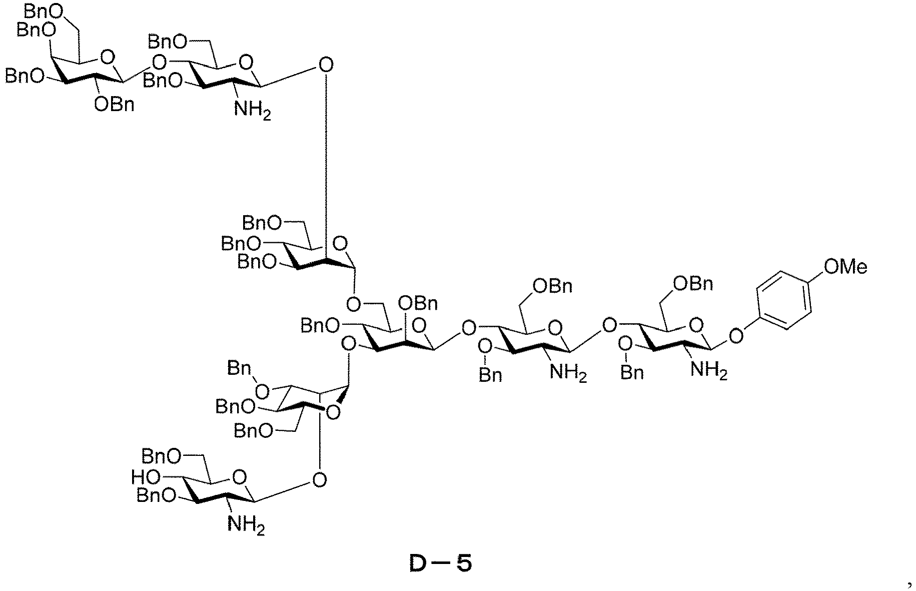 wherein the step comprising a step of
      connecting the compound represented by Formula D-2 to a compound represented by Formula D-3: 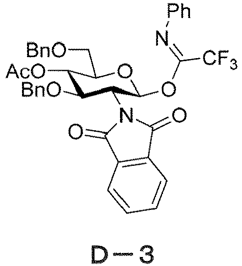 via β-1,2-glycosidic linkage to give a compound represented by Formula D-4: 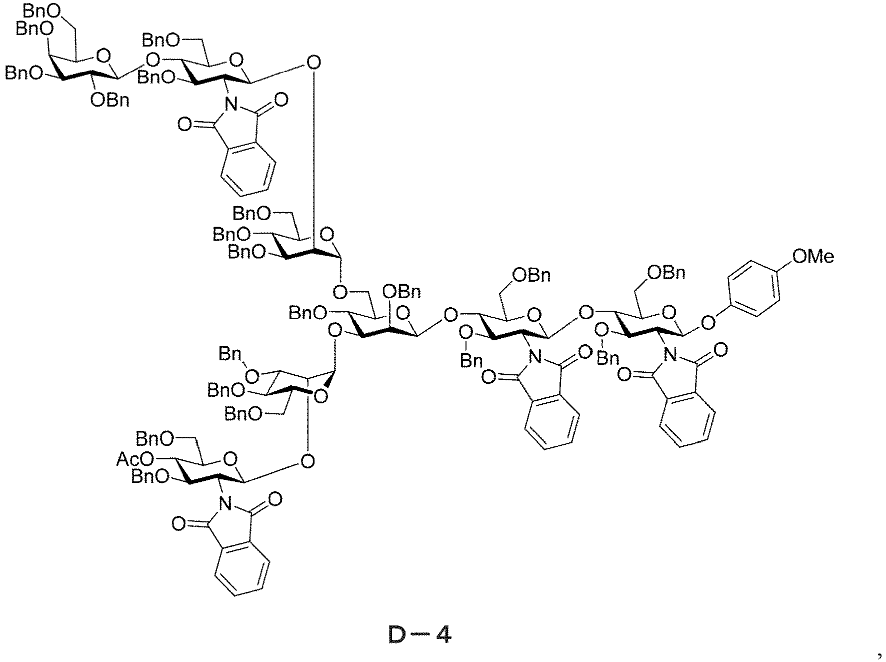 and then protecting the amino group in the compound represented by Formula D-5 with a protecting group selected from an aryloxycarbonyl (COOAr) group, an acetyl (Ac) group, a 2,2,2-trichloroethoxycarbonyl (Troc) group, or a phthalimide (Pht) group to give a compound represented by Formula D-6: 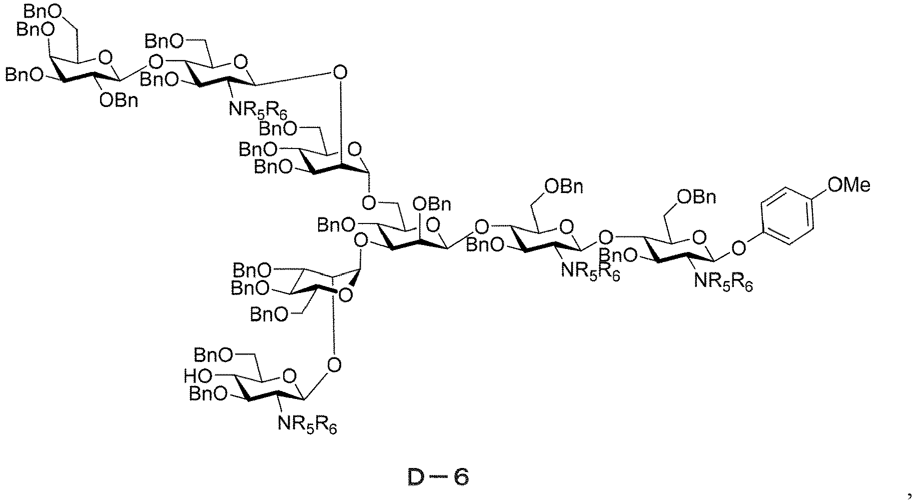
      wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
      or removing an acetyl (Ac) group on the compound represented by Formula D-4 to give the compound represented by Formula D-6 wherein R₅ and R₆ together with a nitrogen atom attached thereto form a phthalimide group;
   (Step II-3) producing a compound represented by Formula D-11: 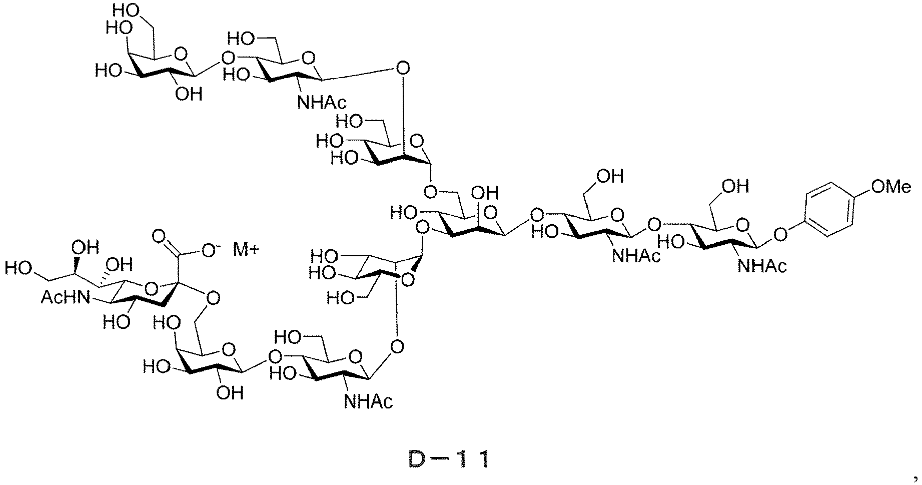
      wherein M⁺ is a sodium ion, a lithium ion, a potassium ion, or a protonated triethylamine cation,
      wherein the step comprising a step of
      connecting the compound represented by Formula D-6 to a compound represented by Formula D-7: 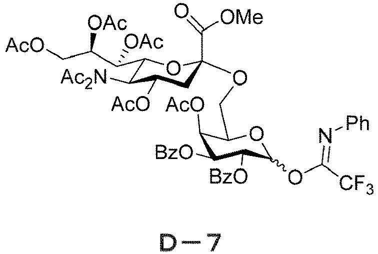 via β-1,4-glycosidic linkage to give a compound represented by Formula D-8: 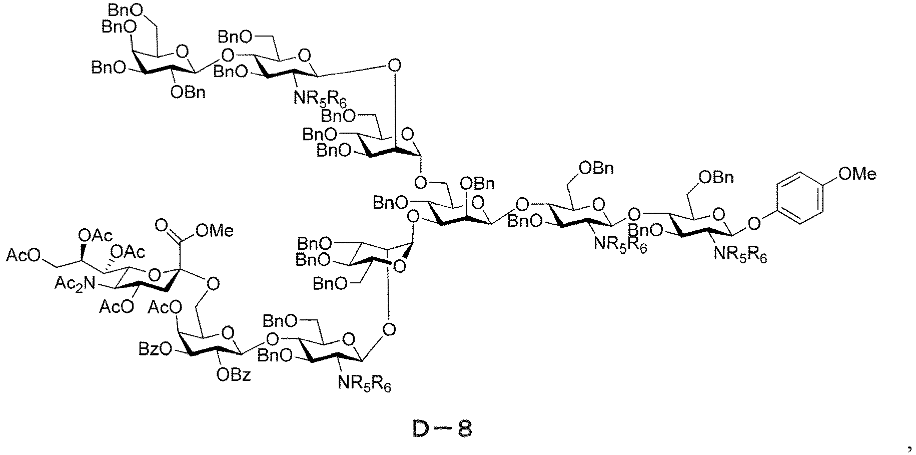
      wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
      and then removing each amino group-protecting group and alcohol acyl-based-protecting group on the compound represented by Formula D-8 to give a compound represented by Formula D-9: 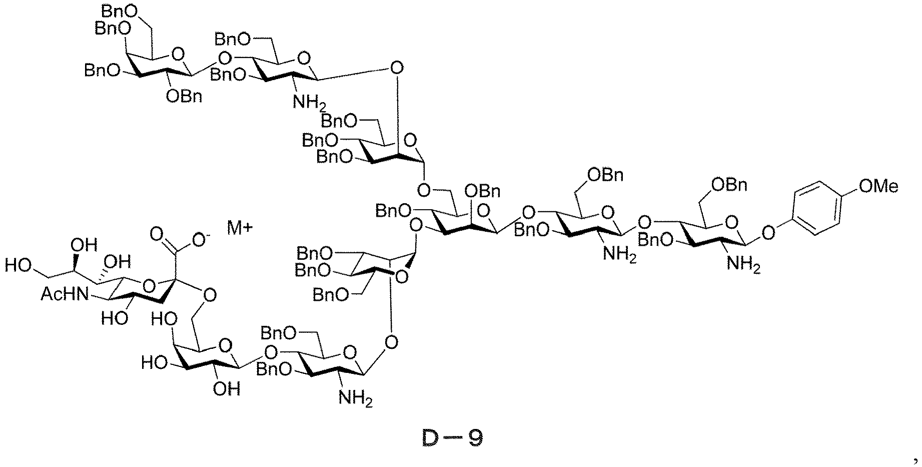
      wherein M⁺ is a sodium ion, a lithium ion, a potassium ion, or a protonated triethylamine cation; and
   (Step II-4) producing the oligosaccharide represented by Formula D-13 by reacting the compound represented by Formula D-11 with a compound represented by Formula D-12: 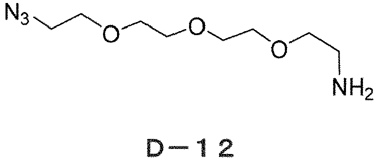
[29] The method according to [28], wherein Step II-1 comprises reacting the compound represented by Formula D-1 with a strong base in the presence of an alkyl ester of perfluorocarboxylic acid to give the compound represented by Formula D-2.
[30] The method according to [29], wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.
[31] The method according to [29] or [30], wherein the strong base is selected from the group consisting of sodium, lithium, and potassium salts of metal amides; sodium, lithium, potassium, cesium, and barium salts of C1-C20 alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and a combination thereof.
[32] The method according to [29] or [30], wherein the strong base is potassium tert-butoxide, sodium tert-butoxide, lithium tert-butoxide, or LHMDS (lithium hexamethyldisilazide).
[33] The method according to any one of [29] to [32], wherein the step of reacting the compound represented by Formula D-1 with a strong base in the presence of a trifluoroacetate to give the compound represented by Formula D-2 is carried out in a C1-C10 alcohol solvent alone or in a mixture of a C1-C10 alcohol solvent and an amide-based solvent, an ether-based solvent, an ester-based solvent, aromatic solvent, a halogen-based solvent, a hydrocarbon-based solvent, or a nitrile-based solvent.
[34] The method according to any one of [28] to [33], wherein in Step II-3, each amino group in the compound represented by Formula D-5 is protected with an aryloxycarbonyl (COOAr) group to give the compound represented by Formula D-6.
[35] The method according to any one of [28] to [34], wherein in Step II-3, the step of producing the compound represented by Formula D-6 from the compound represented by Formula D-5 is carried out in an aqueous solution containing sodium bicarbonate, potassium bicarbonate, disodium hydrogen phosphate, or dipotassium hydrogen phosphate.
[36] The method according to any one of [28] to [35], wherein the compound represented by Formula D-12 is obtained by a method for purification comprising steps of:
   adding, to a solution containing the crude compound represented by Formula D-12, a compound represented by Formula E-1: 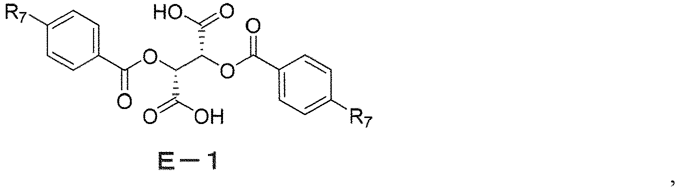 wherein R₇ is a hydrogen atom, a methyl group, or a methoxy group
   to give a crystallin compound represented by Formula E-2: 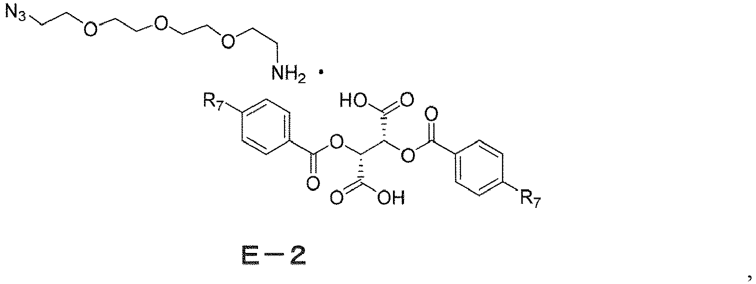
   wherein R₇ is a hydrogen atom, a methyl group, or a methoxy group; and
   isolating the crystalline compound and then extracting the compound represented by Formula D-12 from the crystalline compound isolated.
[37] The method according to [36], wherein the purified compound represented by Formula D-12 has a purity of 95% or higher as measured by HPLC.
[38] The method according to [37], wherein the purity is 98% or higher.
[39] The method according to any one of [28] to [38], wherein the method comprises purifying the compound represented by Formula D-1 by
   after stopping the reaction of the compound represented by Formula A-13 with the compound represented by Formula A-3 in Step II-1, adding a hydrophobic carrier and water to a water-soluble organic solvent containing the resulting compound represented by Formula D-1 and contaminants to adsorb the compound represented by Formula D-1 onto the hydrophobic carrier;
   then filtering the resultant and washing the hydrophobic carrier with a mixture of the water-soluble organic solvent and then water to remove the contaminants;
   and then eluting the compound represented by Formula D-1 from the hydrophobic carrier using an organic solvent.
[40] The method according to any one of [28] to [39], wherein in the method comprises purifying the compound represented by Formula D-5 by
   after stopping the reaction of the compound represented by Formula D-3 with the compound represented by Formula D-4 in Step II-2, adding a hydrophobic carrier and water to a water-soluble organic solvent containing the resulting compound represented by Formula D-5 and contaminants to adsorb the compound represented by Formula D-5 onto the hydrophobic carrier;
   then filtering the resultant and washing the hydrophobic carrier with a mixture of the water-soluble organic solvent and the water to remove the contaminants;
   and then eluting the compound represented by Formula D-5 from the hydrophobic carrier using an organic solvent.
[41] The method according to any one of [28] to [40], wherein the method comprises purifying the compound represented by Formula D-8 by
   after stopping the reaction of the compound represented by Formula D-6 with the compound represented by Formula D-7 in Step II-3, adding a hydrophobic carrier and water to a water-soluble organic solvent containing the resulting compound represented by Formula D-8 and contaminants to adsorb the compound represented by Formula D-8 onto the hydrophobic carrier;
   then filtering the resultant and washing the hydrophobic carrier with a mixture of the water-soluble organic solvent and the water to remove the contaminants;
   and then eluting the compound represented by Formula D-8 from the hydrophobic carrier using an organic solvent.
[42] The method according to any one of [28] to [41], wherein in Step II-3, the method comprises protecting each amino group on the Formula D-9 with an acetyl group to give a compound represented by Formula D-10: 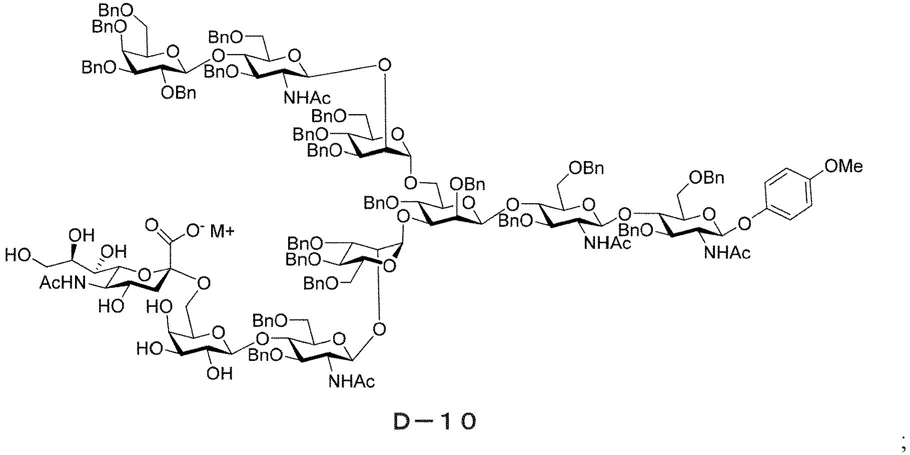 and purifying the compound represented by Formula D-10 by
   adding a hydrophobic carrier and water to a water-soluble organic solvent containing the resulting compound represented by Formula D-10 and contaminants to adsorb the compound represented by Formula D-10 onto the hydrophobic carrier;
   then filtering the resultant and washing the hydrophobic carrier with a mixture of the water-soluble organic solvent and the water to remove the contaminants;
   and then eluting the compound represented by Formula D-10 from the hydrophobic carrier using an organic solvent.
[43] The method according to [39], wherein the contaminants include a sugar compound other than the compound represented by Formula D-1, and/or a compound derived from a reaction reagent to obtain the compound to be purified.
[44] The method according to [40], wherein the contaminants include a sugar compound other than the compound represented by Formula D-5, and/or a compound derived from a reaction reagent to obtain the compound to be purified.
[45] The method according to [41], wherein the contaminants include a sugar compound other than the compound represented by Formula D-8, and/or a compound derived from a reaction reagent to obtain the compound to be purified.
[46] The method according to [42], wherein the contaminants include a sugar compound other than the compound represented by Formula C-10, and/or a compound derived from a reaction reagent to obtain the compound to be purified.
[47] The method according to any one of [39] to [46], wherein the hydrophobic carrier is a resin used for packing reversed-phase partition chromatography.
[48] The method according to [47], wherein the resin packed for reversed-phase partition chromatography is selected from the group consisting of a poly(styrene/divinylbenzene) polymer gel resin, a polystyrene-divinylbenzene resin, a polyhydroxymethacrylate resin, a styrene-vinylbenzene copolymer resin, a polyvinyl alcohol resin, polystyrene resin, a polymethacrylate resin, a chemically bonded silica gel resin, and a combination thereof.
[49] The method according to [48], wherein the chemically bonded silica gel resin is selected from the group consisting of (1) a resin obtained by reacting silica gel with a silane coupling agent, (2) a resin obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) a resin obtained by chemically bonding a docosyl or triacontyl group to silica gel, and (4) a combination of the resins (1) to (3).
[50] The method according to [48], wherein the chemically bonded silica gel resin is an octadecyl group-bonded silica gel resin (ODS resin).
[51] The method according to any one of [39] to [50], wherein the water-soluble organic solvent is a water-soluble alcohol-based solvent, a water-soluble nitrile-based solvent, a water-soluble ether-based solvent, a water-soluble ketone-based solvent, a water-soluble amide-based solvent, or a water-soluble sulfoxide-based solvent, or a mixed solvent containing at least one of the aforementioned water-soluble organic solvents.
[52] The method according to [51], wherein the water-soluble nitrile-based solvent is acetonitrile.
[53] The method according to any one of [39] to [52], wherein the organic solvent used for eluting the compound of interest from the hydrophobic carrier is a nitrile-based solvent, an ether-based solvent, an ester-based solvent, a ketone-based solvent, a halogen-based solvent, an aromatic solvent, or a mixed solvent containing at least one of the aforementioned solvents.
[54] The method according to any one of [28] to [53], further comprising the step of forming a salt of the compound represented by Formula D-5 with fumaric acid to produce a crystalline compound represented by Formula D-5-FMA: 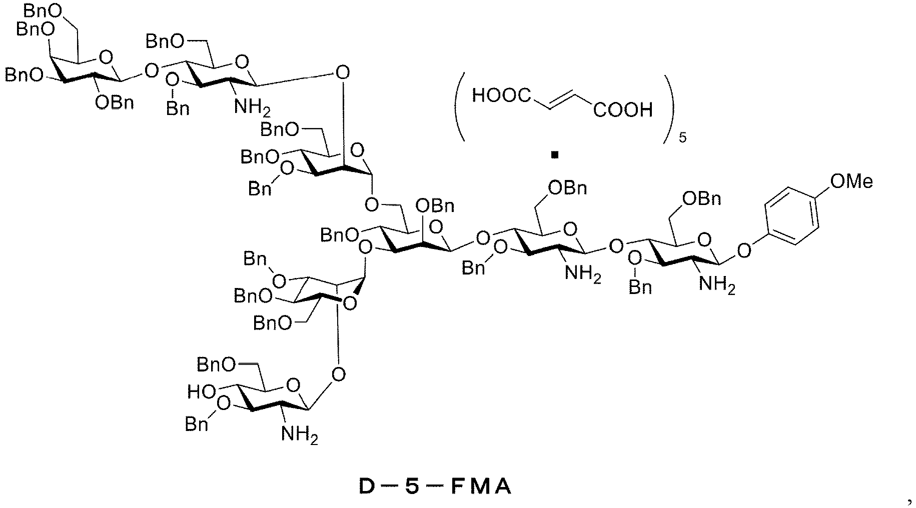 and then separating and purifying the crystalline compound represented by Formula D-5-FMA from non-crystalline materials.
[55] A method for producing a compound represented by Formula B-5: 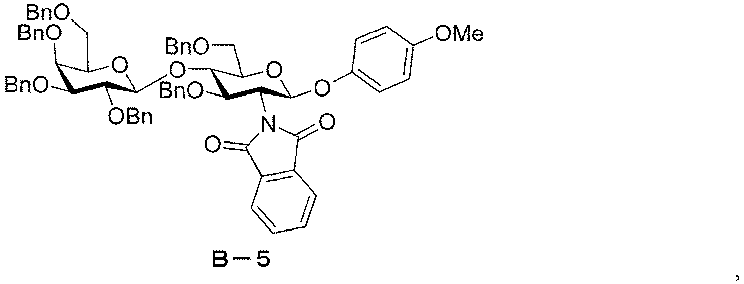 wherein the method comprises the step of adding lithium tert-butoxide or lithium tert-amoxide to a solvent containing a compound represented by Formula B-4: 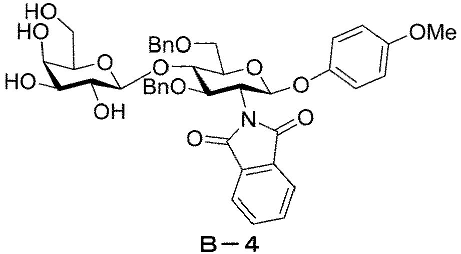 and benzyl halide or benzyl sulfonate to protect hydroxyl groups present in the compound represented by Formula B-4 with benzyl groups.
[56] The method according to [55], wherein the solvent is an amide-based solvent, an ether-based solvent, an aromatic solvent, a urea-based solvent, or a hydrocarbon-based solvent, or a mixed solvent containing at least one of the aforementioned solvents.
[57] A method for producing a compound represented by Formula A-10; 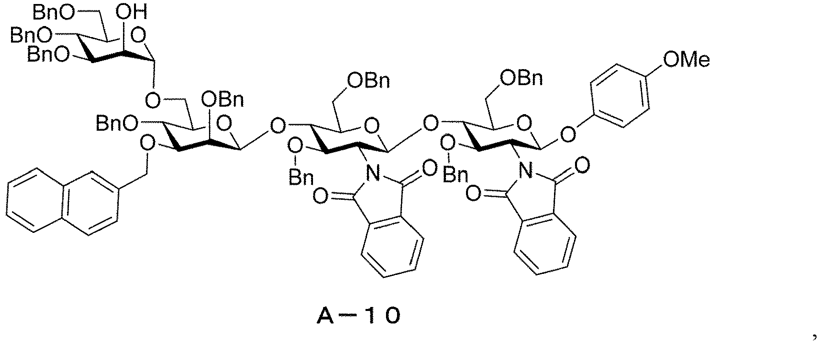 wherein the method comprises the step of reacting a compound represented by Formula A-9: 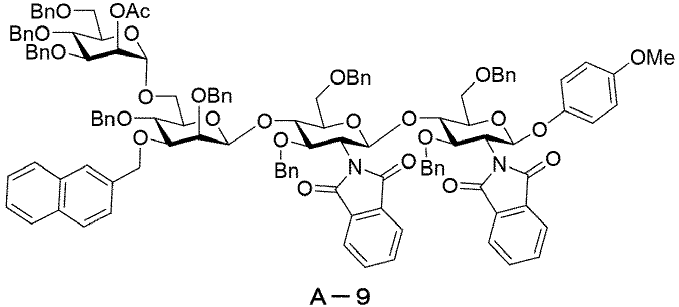 with a strong base in the presence of an alkyl ester of perfluorocarboxylic acid.
[58] The method according to [57], wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.
[59] The method according to [57] or [58], wherein the strong base is selected from the group consisting of sodium salts, lithium salts, potassium salts of metal amides; sodium, lithium, potassium, cesium, and barium salts of C1-C20 alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and a combination thereof.
[60] The method according to [57] or [58], wherein the strong base is potassium tert-butoxide, sodium tert-butoxide, lithium tert-butoxide, or LHMDS (lithium hexamethyldisilazide).
[61] The method according to any one of [57] to [60], wherein the reaction is carried out in a C1-C10 alcohol solvent alone or in a mixture of a C1-C10 alcohol solvent and an amide-based solvent, an ether-based solvent, an ester-based solvent, aromatic solvent, a halogen-based solvent, a hydrocarbon-based solvent, or a nitrile-based solvent.
[62] A method for producing an oligosaccharide represented by Formula A-13: 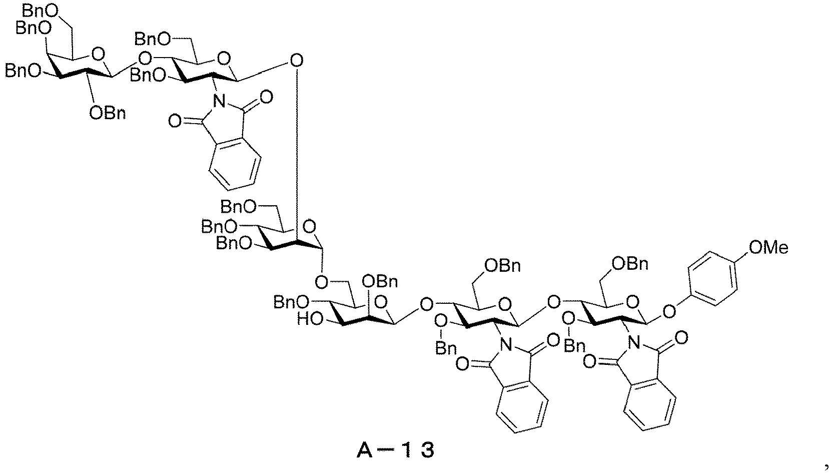 wherein the method comprises the step of reacting a compound represented by Formula A-12: 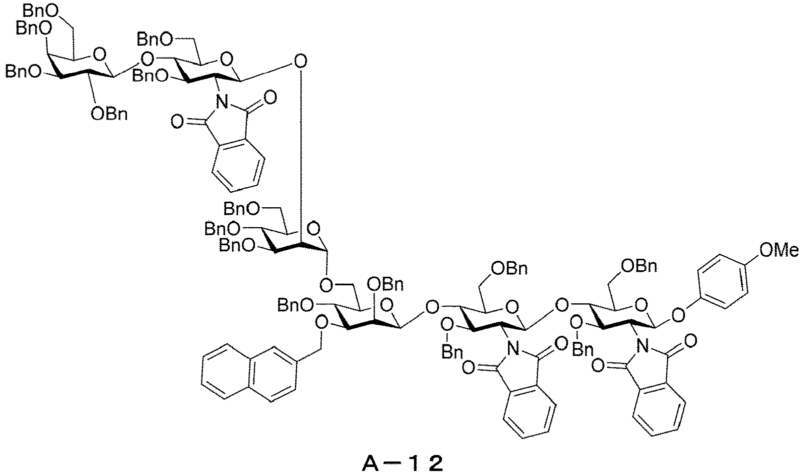 with DDQ (2,3-dichloro-5,6-dicyano-p-benzoquinone) in a mixed solvent of fluorous alcohol and water to remove a 2-naphthylmethyl group in the compound represented by Formula A-12.
[63] The method according to [62], wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and a combination thereof.
[64] The method according to [62] or [63], wherein the reaction is carried out at -35°C to 70°C.
[65] The method according to [62] or [63], wherein the reaction is carried out at -30°C to - 10°C.
[66] A method of purifying a compound represented by Formula A-5: 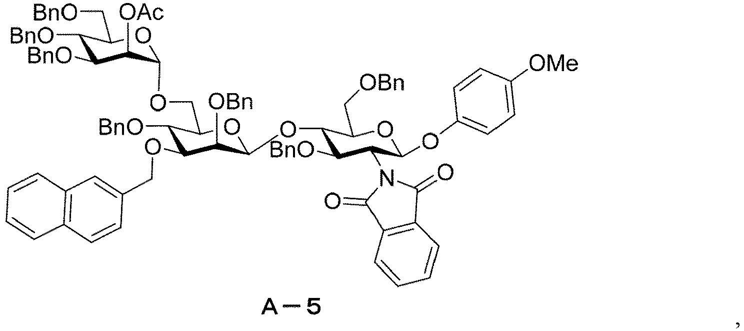 wherein the method comprises steps of:
   adding a hydrophobic carrier and water to a water-soluble organic solvent containing the compound represented by Formula A-5 and contaminants to adsorb the compound represented by Formula A-5 onto the hydrophobic carrier;
   then filtering the resultant and washing the hydrophobic carrier with a mixture of the water-soluble organic solvent and the water to remove the contaminants;
   and then eluting the compound represented by Formula A-5 from the hydrophobic carrier using an organic solvent to purify the compound represented by Formula A-5.
[67] A method of purifying a compound represented by Formula A-9: 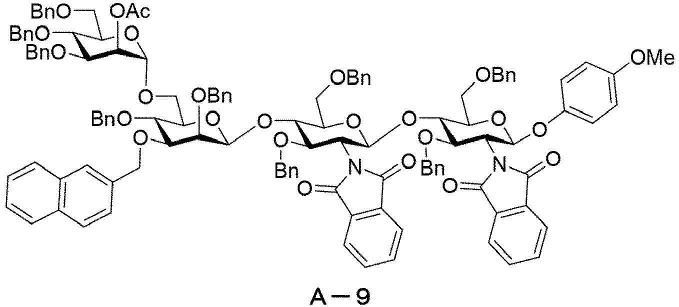 wherein the method comprises steps of:
   adding a hydrophobic carrier and water to a water-soluble organic solvent containing the compound represented by Formula A-9 and contaminants to adsorb the compound represented by Formula A-9 onto the hydrophobic carrier;
   then filtering the resultant and washing the hydrophobic carrier with a mixture of the water-soluble organic solvent and the water to remove the contaminants;
   and then eluting the compound represented by Formula A-9 from the hydrophobic carrier using an organic solvent to purify the compound represented by Formula A-9.
[68] A method of purifying a compound represented by Formula A-12: 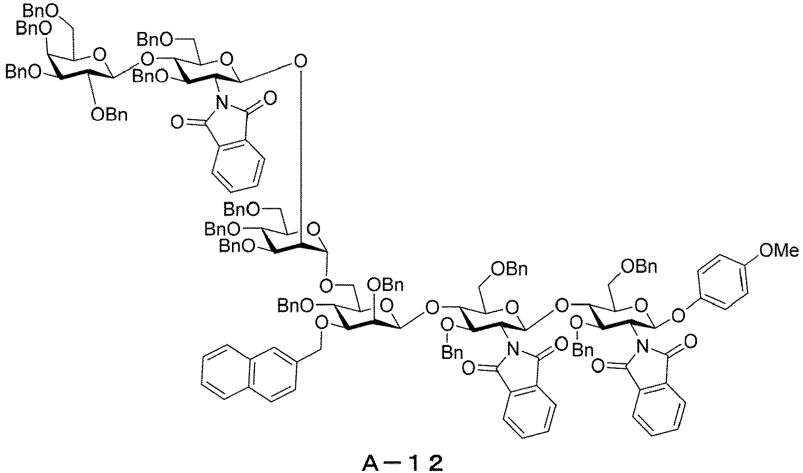 wherein the method comprises steps of:
   adding a hydrophobic carrier and water to a water-soluble organic solvent containing the compound represented by Formula A-12 and contaminants to adsorb the compound represented by Formula A-12 onto the hydrophobic carrier;
   then filtering the resulting material and washing the hydrophobic carrier with a mixture of the water-soluble organic solvent and the water to remove the contaminants;
   and then eluting the compound represented by Formula A-12 from the hydrophobic carrier using an organic solvent to purify the compound represented by Formula A-12.
[69] A method of purifying a compound represented by Formula D-1: 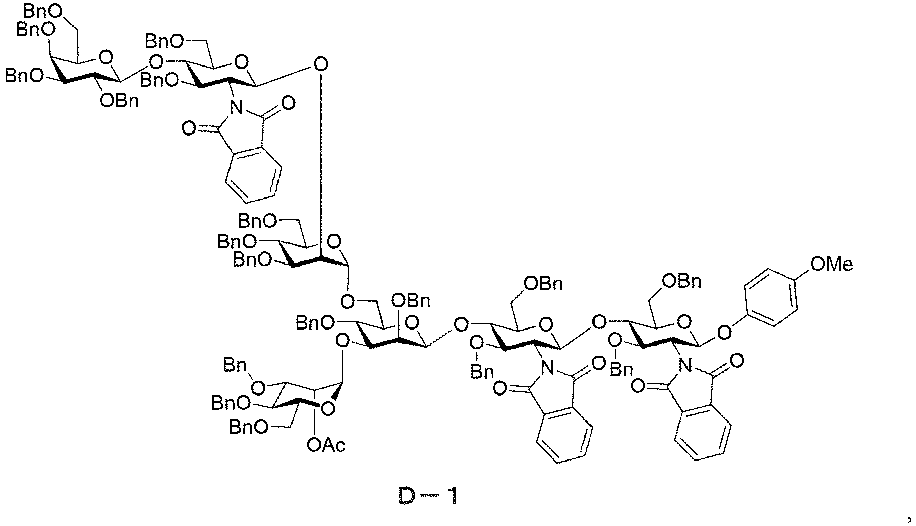 wherein the method comprises steps of:
   adding a hydrophobic carrier and water to a water-soluble organic solvent containing the compound represented by Formula D-1 and contaminants to adsorb the compound represented by Formula D-1 onto the hydrophobic carrier;
   then filtering the resulting material and washing the hydrophobic carrier with a mixture of the water-soluble organic solvent and the water to remove the contaminants;
   and then eluting the compound represented by Formula D-1 from the hydrophobic carrier using an organic solvent to purify the compound represented by Formula D-1.
[70] A method of purifying a compound represented by Formula D-5: 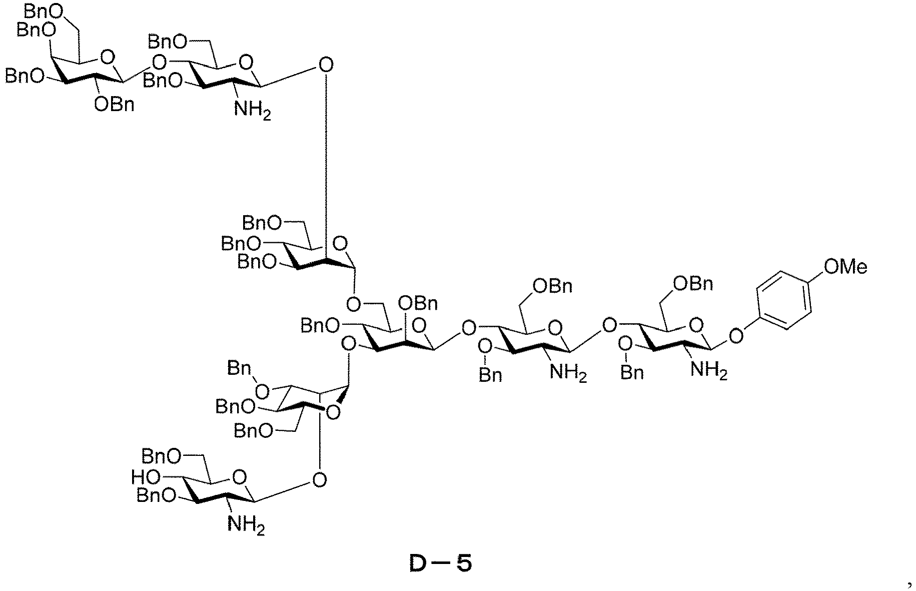 wherein the method comprises steps of:
   adding a hydrophobic carrier and water to a water-soluble organic solvent containing the compound represented by Formula D-5 and contaminants to adsorb the compound represented by Formula D-5 onto the hydrophobic carrier;
   then filtering the resulting material and washing the hydrophobic carrier with a mixture of the water-soluble organic solvent and the water to remove the contaminants;
   and then eluting the compound represented by Formula D-5 from the hydrophobic carrier using an organic solvent to purify the compound represented by Formula D-5.
[71] A method of purifying a compound represented by Formula D-8: 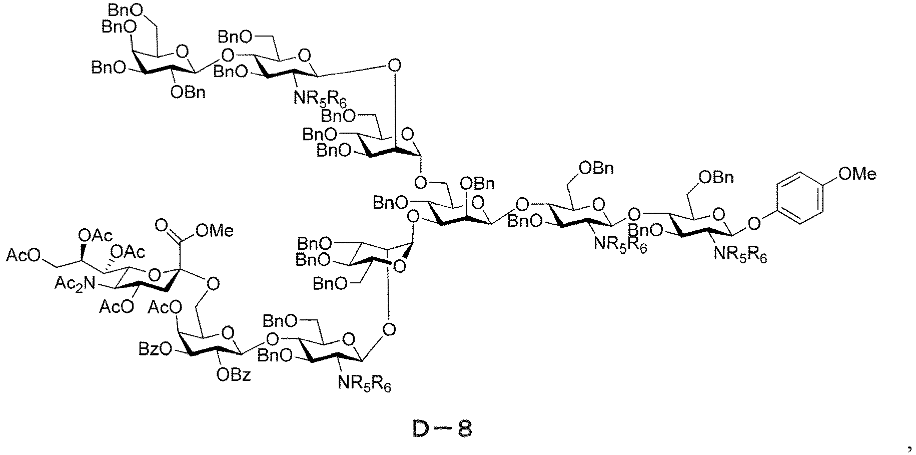
   wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
   wherein the method comprises steps of:
      adding a hydrophobic carrier and water to a water-soluble organic solvent containing the compound represented by Formula D-8 and contaminants to adsorb the compound represented by Formula D-8 onto the hydrophobic carrier;
      then filtering the resulting material and washing the hydrophobic carrier with a mixture of the water-soluble organic solvent and the water to remove the contaminants;
      and then eluting the compound represented by Formula D-8 from the hydrophobic carrier using an organic solvent to purify the compound represented by Formula D-8.
[72] A method of purifying a compound represented by Formula D-10: 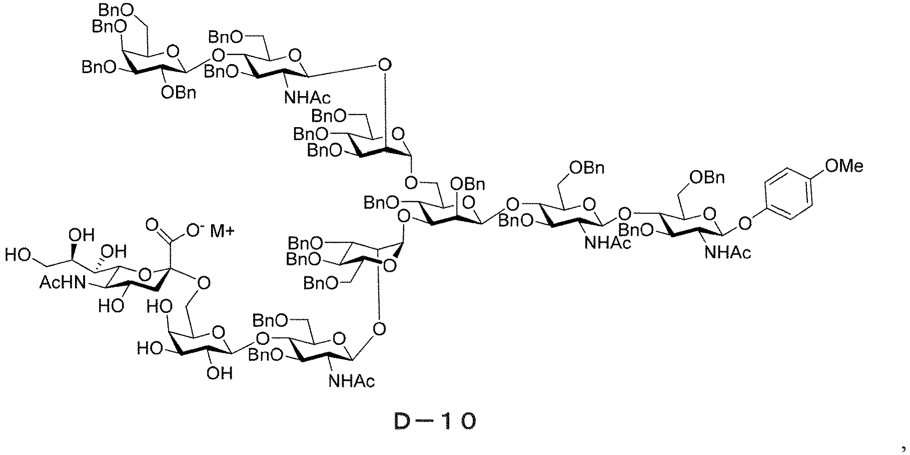 wherein the method comprises steps of:
   adding a hydrophobic carrier and water to a water-soluble organic solvent containing the compound represented by Formula D-10 and contaminants to adsorb the compound represented by Formula D-10 onto the hydrophobic carrier;
   then filtering the resulting material and washing the hydrophobic carrier with a mixture of the water-soluble organic solvent and the water to remove the contaminants;
   and then eluting the compound represented by Formula D-10 from the hydrophobic carrier using an organic solvent to purify the compound represented by Formula D-10.
[73] The method according to [69], wherein the contaminants include a sugar compound other than the compound represented by Formula D-1, and/or a compound derived from a reaction reagent to obtain the compound to be purified.
[74] The method according to [70], wherein the contaminants include a sugar compound other than the compound represented by Formula D-5, and/or a compound derived from a reaction reagent to obtain the compound to be purified.
[75] The method according to [71], wherein the contaminants include a sugar compound other than the compound represented by Formula D-8, and/or a compound derived from a reaction reagent to obtain the compound to be purified.
[76] The method according to [72], wherein the contaminants include a sugar compound other than the compound represented by Formula D-10, and/or a compound derived from a reaction reagent to obtain the compound to be purified.
[77] The method according to any one of [69] to [76], wherein the hydrophobic carrier is a resin used for packing reversed-phase partition chromatography.
[78] The method according to [77], wherein the resin packed for reversed-phase partition chromatography is selected from the group consisting of a poly(styrene/divinylbenzene) polymer gel resin, a polystyrene-divinylbenzene resin, a polyhydroxymethacrylate resin, a styrene-vinylbenzene copolymer resin, a polyvinyl alcohol resin, a polystyrene resin, a polymethacrylate resin, a chemically bonded silica gel resin, and a combination thereof.
[79] The method according to [78], wherein the chemically bonded silica gel resin is selected from the group consisting of (1) a resin obtained by reacting silica gel with a silane coupling agent, (2) a resin obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) a resin obtained by chemically bonding a docosyl or triacontyl group to silica gel, and (4) a combination of the resins (1) to (3).
[80] The method according to [79], wherein the chemically bonded silica gel resin is an octadecyl group-bonded silica gel resin (ODS resin).
[81] The method according to any one of [69] to [80], wherein the water-soluble organic solvent is a water-soluble alcohol-based solvent, a water-soluble nitrile-based solvent, a water-soluble ether-based solvent, a water-soluble ketone-based solvent, a water-soluble amide-based solvent, a water-soluble sulfoxide-based solvent, or a mixed solvent containing at least one of the aforementioned water-soluble organic solvents.
[82] The method according to [81], wherein the water-soluble nitrile-based solvent is acetonitrile.
[83] The method according to any one of [69] to [82], wherein the organic solvent used for eluting the compound of interest from the hydrophobic carrier is a nitrile-based solvent, an ether-based solvent, an ester-based solvent, a ketone-based solvent, a halogen-based solvent, an aromatic solvent, or a mixed solvent containing at least one of the aforementioned solvents.
[84] A method for producing a compound represented by Formula D-8: 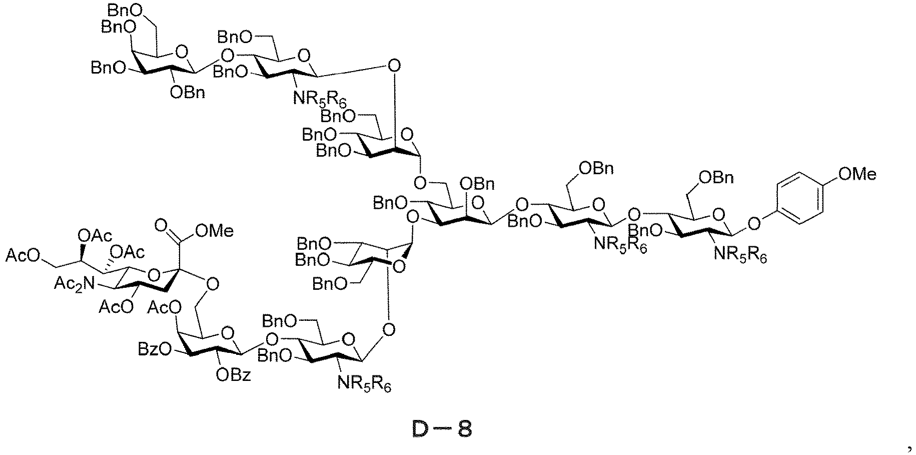
   wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
   wherein the method comprises steps of:
      producing a compound represented by Formula D-6: 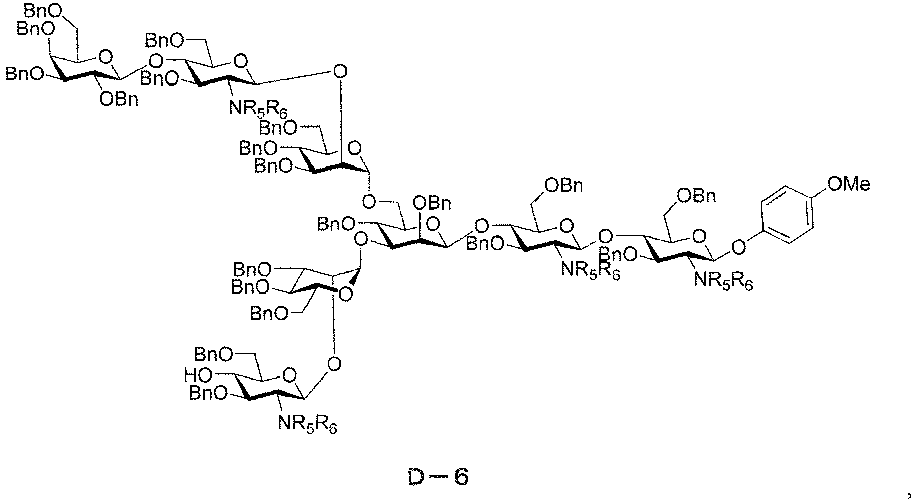
      wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group;
      and then connecting the compound represented by Formula D-6 to a compound represented by Formula D-7: 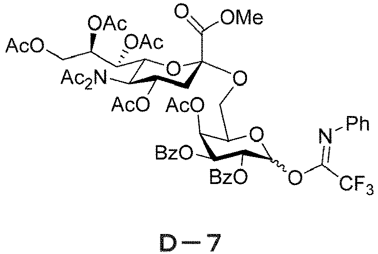 via β-1,4-glycosidic linkage to give the compound represented by Formula D-8.
[85] The method according to [84], wherein R₅ is an aryloxycarbonyl (COOAr) group.
[86] The method according to [84] or [85], wherein the method comprises the step of removing each amino group-protecting group and alcohol acyl-based-protecting group from the compound represented by Formula D-8 to give a compound represented by Formula D-9: 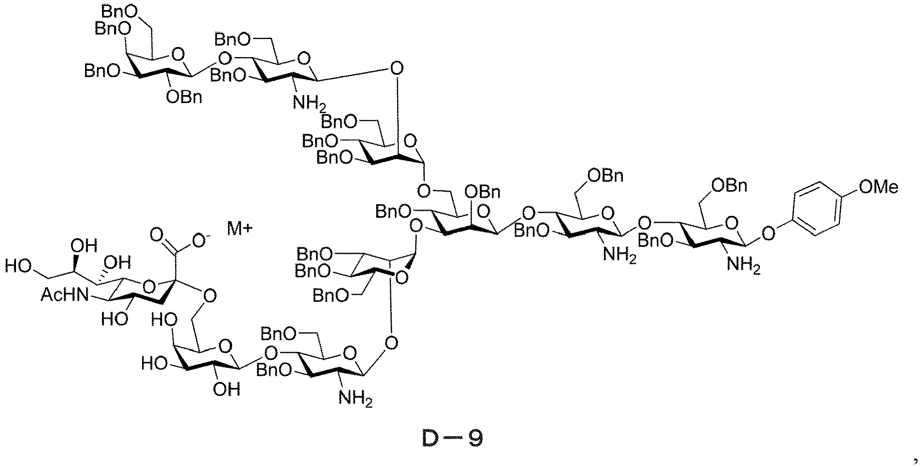 wherein M⁺ is a sodium ion, a lithium ion, a potassium ion, or a protonated triethylamine cation.
[87] A method of purifying a compound represented by Formula D-12: 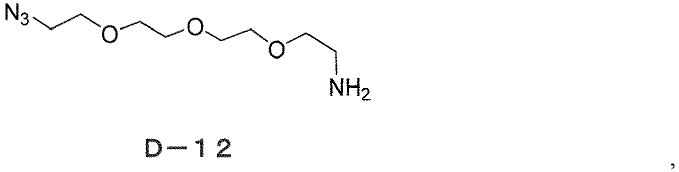 wherein the method comprises steps of:
   adding, to a solution containing the crude compound represented by Formula D-12, a compound represented by Formula E-1: 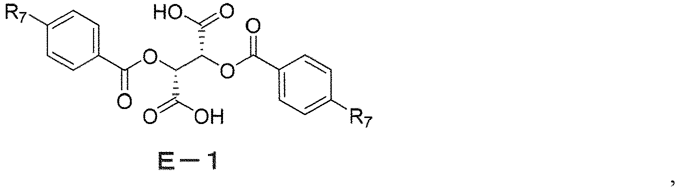
   wherein R₇ is a hydrogen atom, a methyl group, or a methoxy group
   to give a crystallin compound represented by Formula E-2: 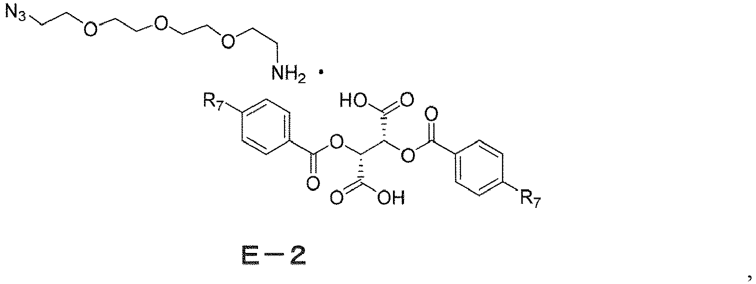
   wherein R₇ is a hydrogen atom, a methyl group, or a methoxy group; and
   isolating the crystalline compound and then extracting the compound represented by Formula D-12 from the crystalline compound isolated.
[88] An oligosaccharide represented by Formula A-13: 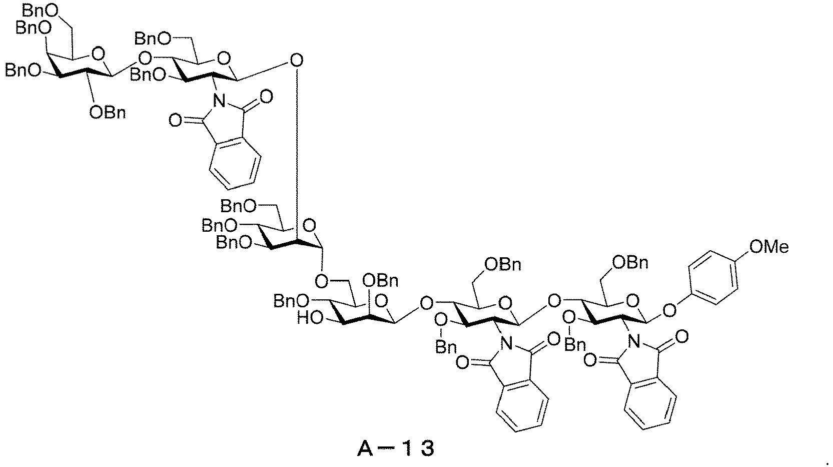
[89] A compound represented by Formula A-5: 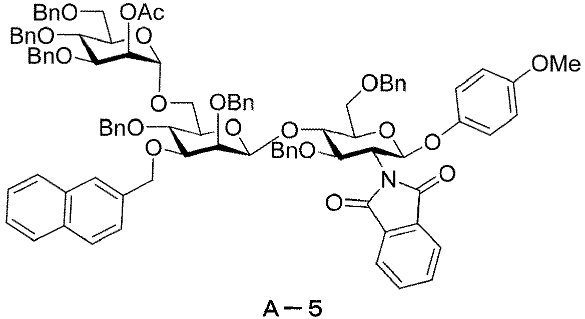
[90] A compound represented by Formula A-6: 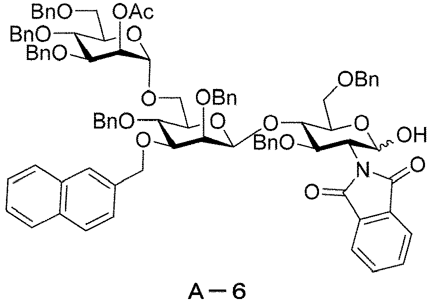
[91] A compound represented by Formula A-7: 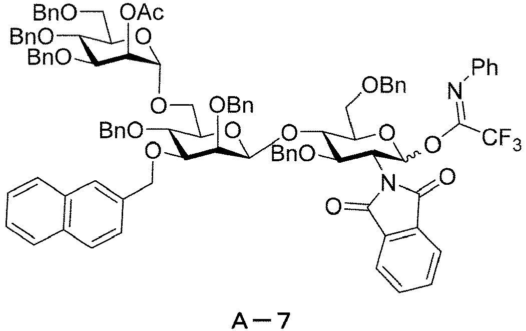
[92] A compound represented by Formula A-9: 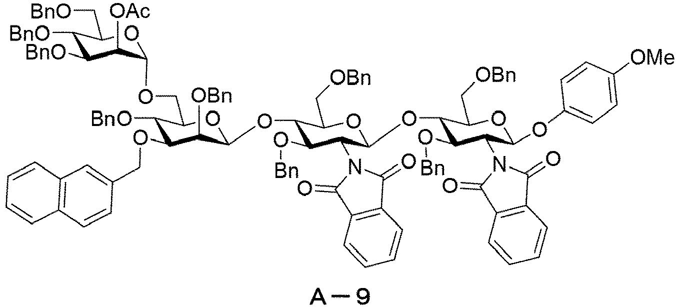
[93] A compound represented by Formula A-10: 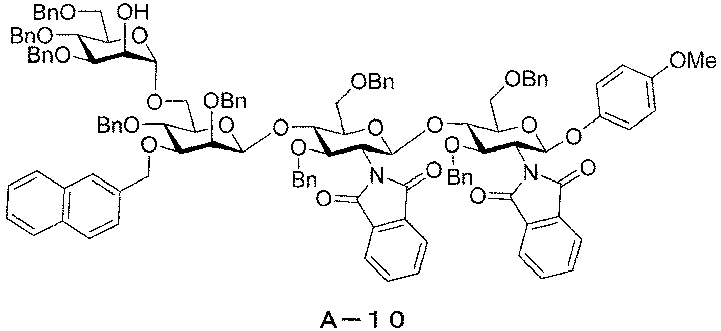
[94] A compound represented by Formula A-11: 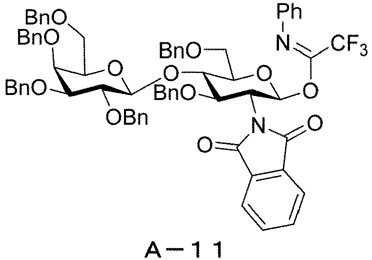
[95] A compound represented by Formula A-12: 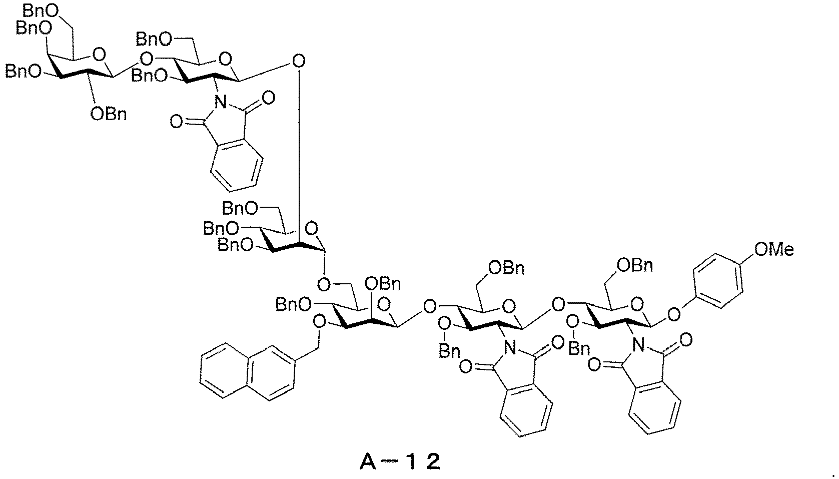
[96] A compound represented by Formula A-14: 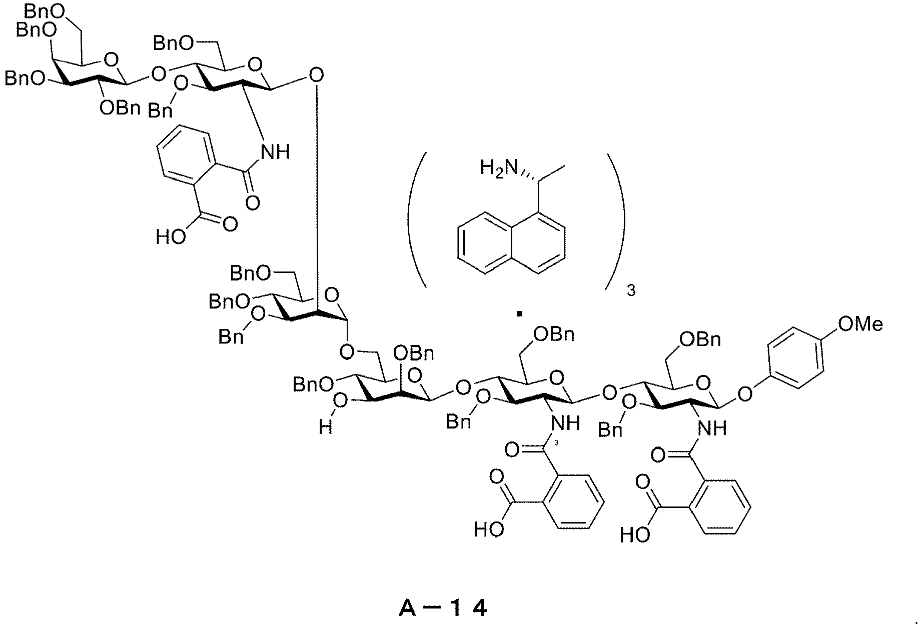
[97] A compound represented by Formula A-15: 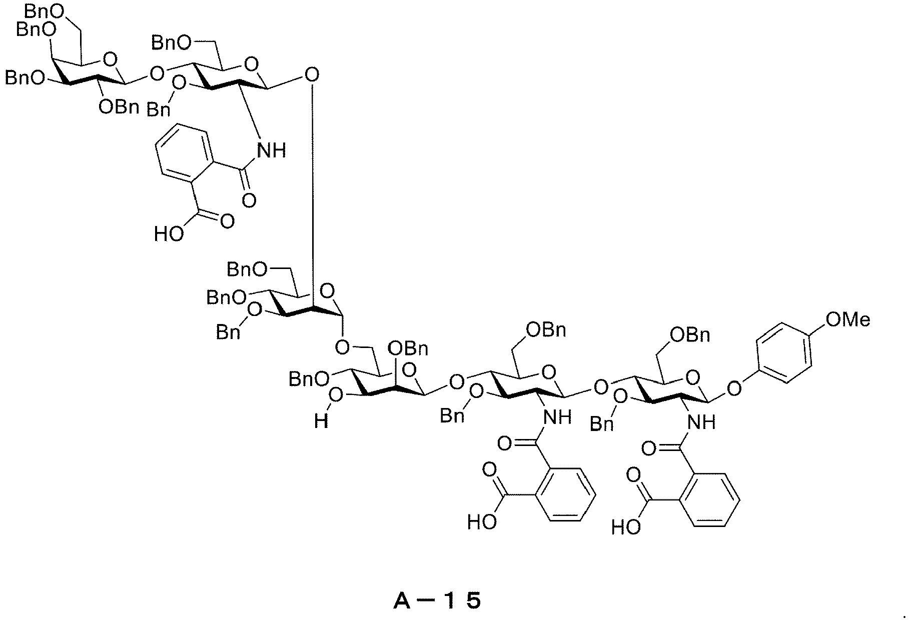
[98] A compound represented by Formula B-4: 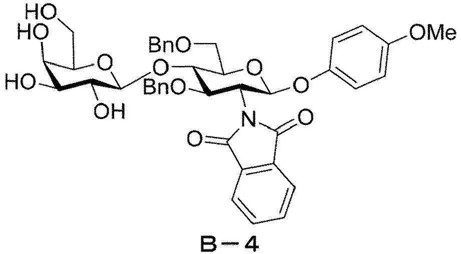
[99] A compound represented by Formula B-5: 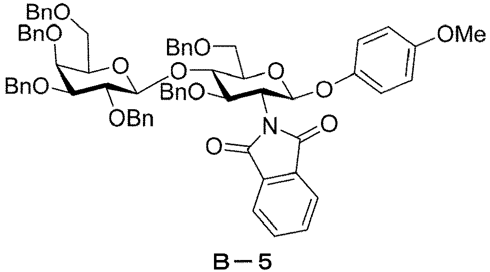
[100] A compound represented by Formula B-6: 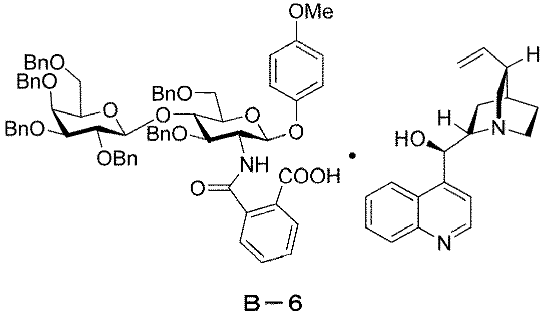
[101] A compound represented by Formula B-7: 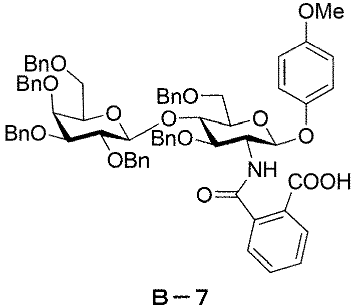
[102] A compound represented by Formula B-8: 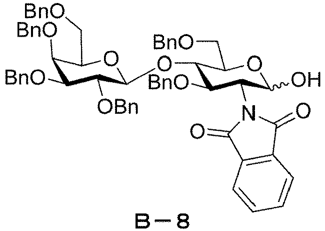
[103] A compound represented by Formula D-1: 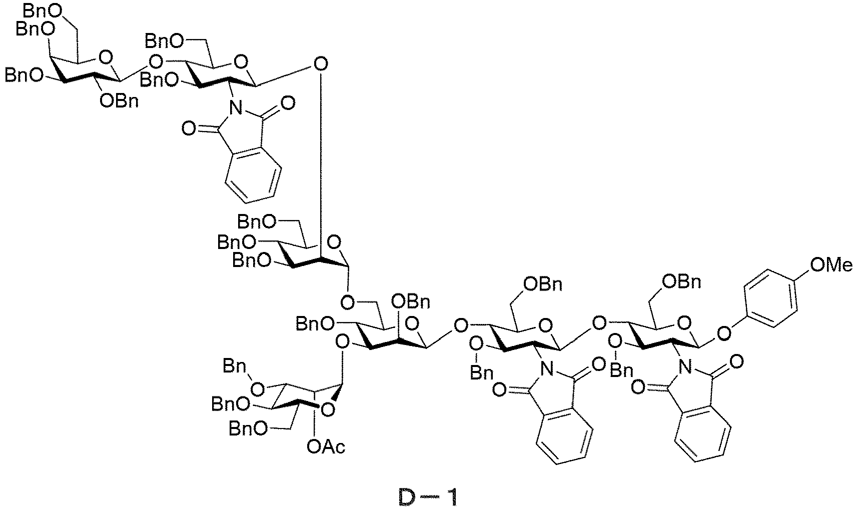
[104] A compound represented by Formula D-2: 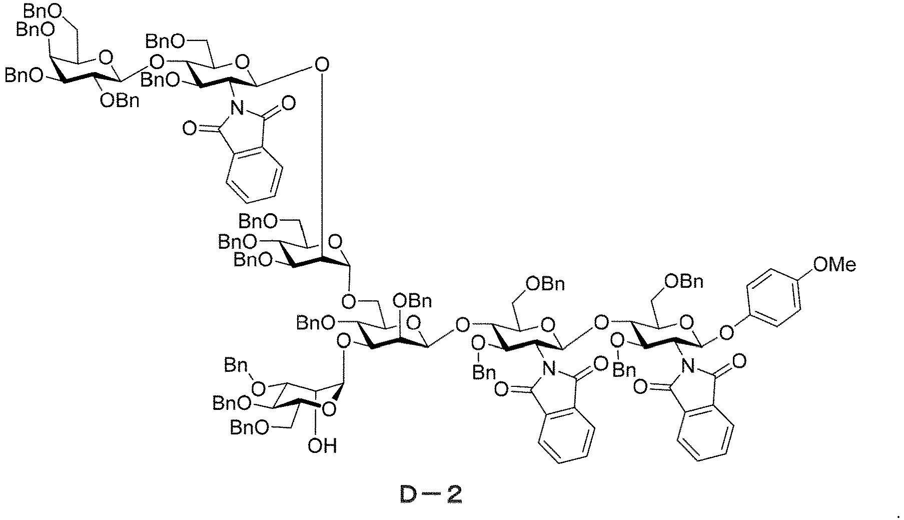
[105] A compound represented by Formula D-4: 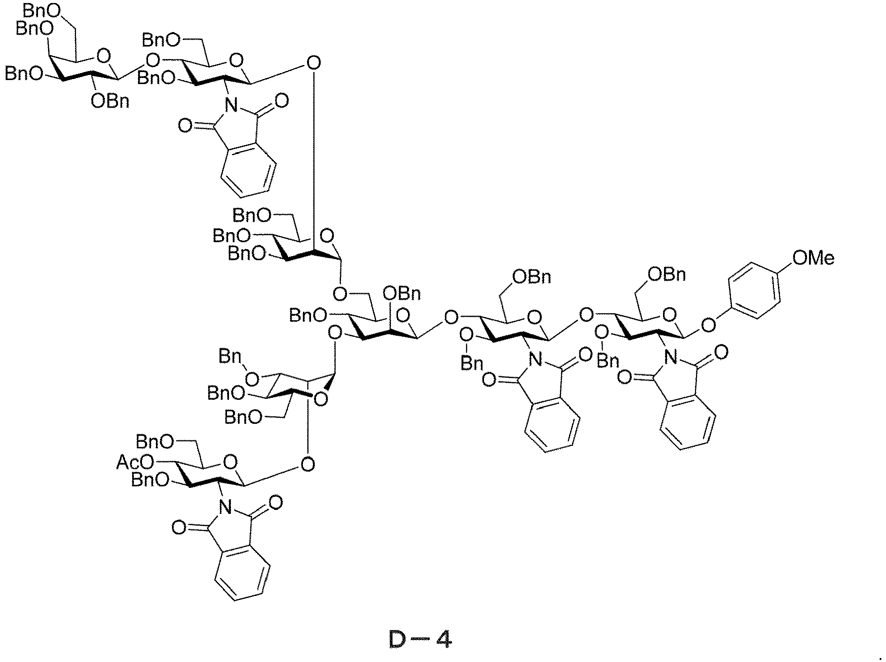
[106] A compound represented by Formula D-5: 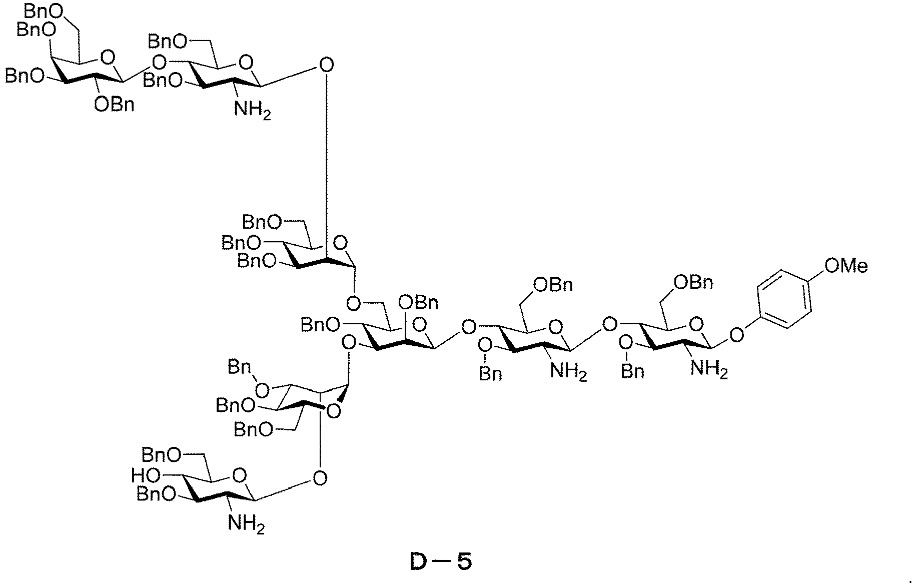
[107] A compound represented by Formula D-5-FMA: 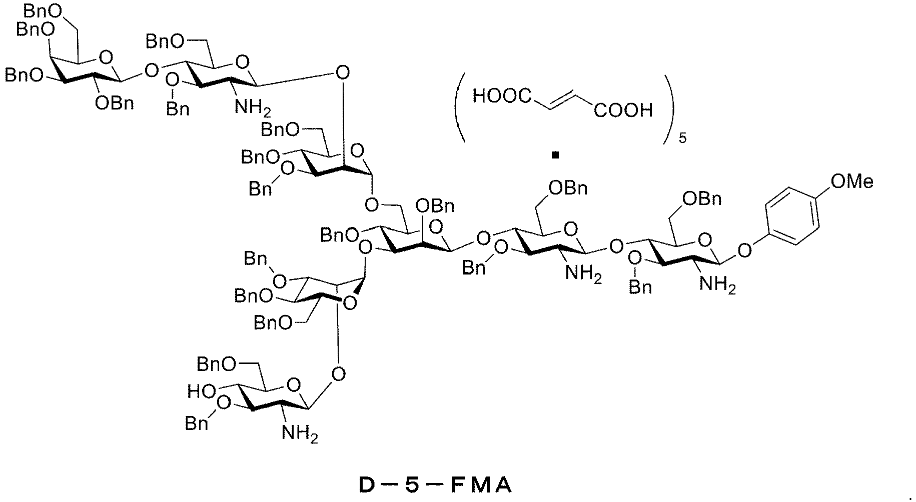
[108] A compound represented by Formula D-6: 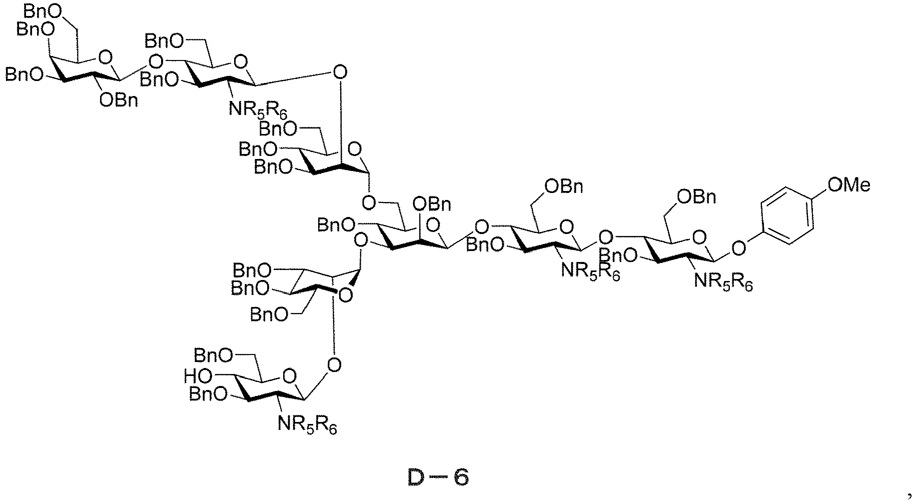 wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.
[109] A compound represented by Formula D-8: 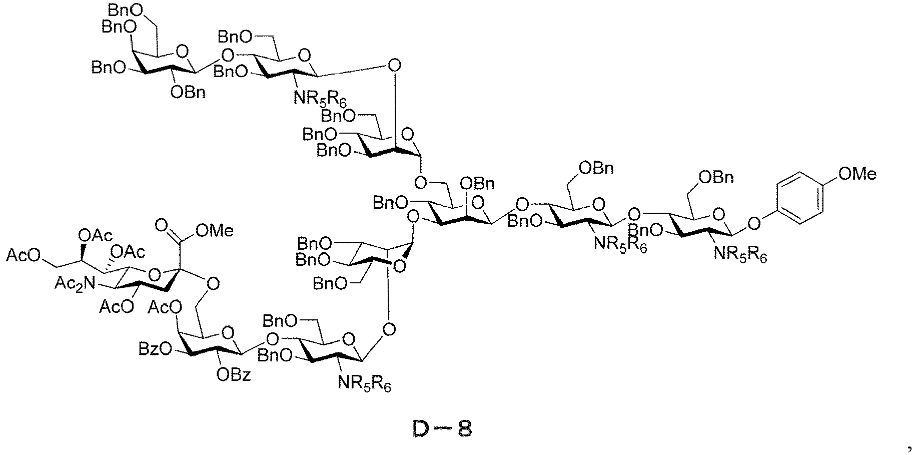 wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.
[110] A compound represented by Formula D-9: 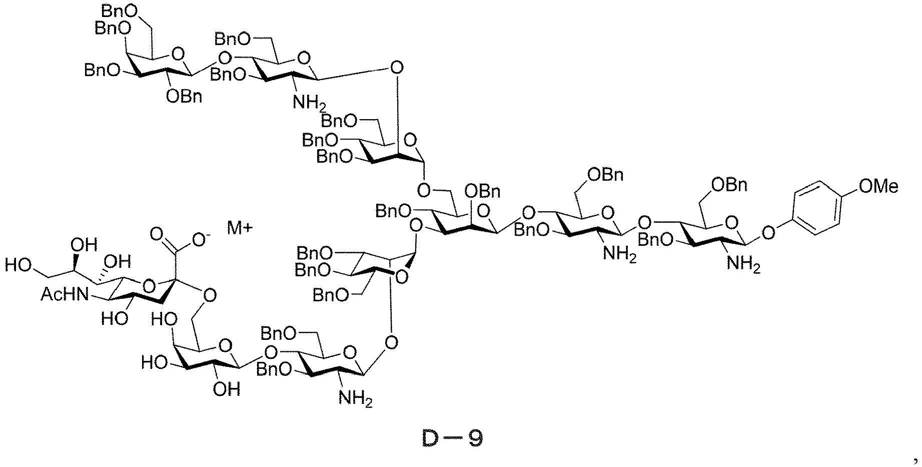 wherein M⁺ is a sodium ion, a lithium ion, a potassium ion, or a protonated triethylamine cation.
[111] A compound represented by Formula D-10: 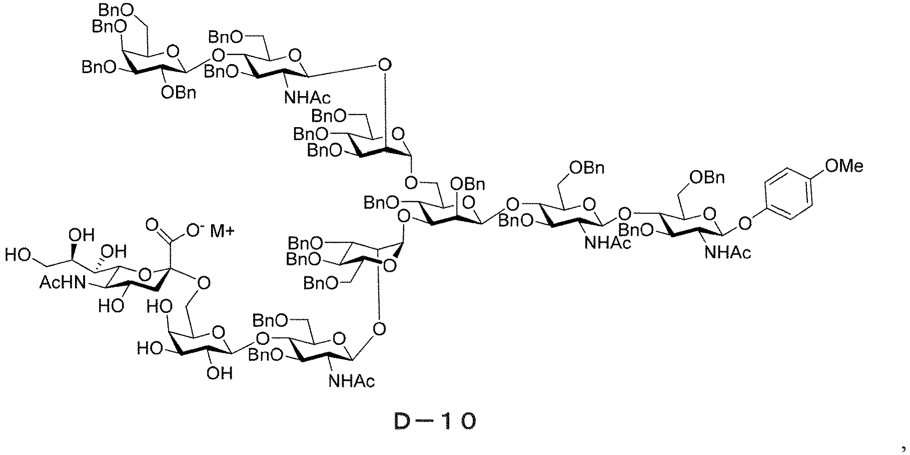 wherein M⁺ is a sodium ion, a lithium ion, a potassium ion, or a protonated triethylamine cation.
[112] A compound represented by Formula D-11: 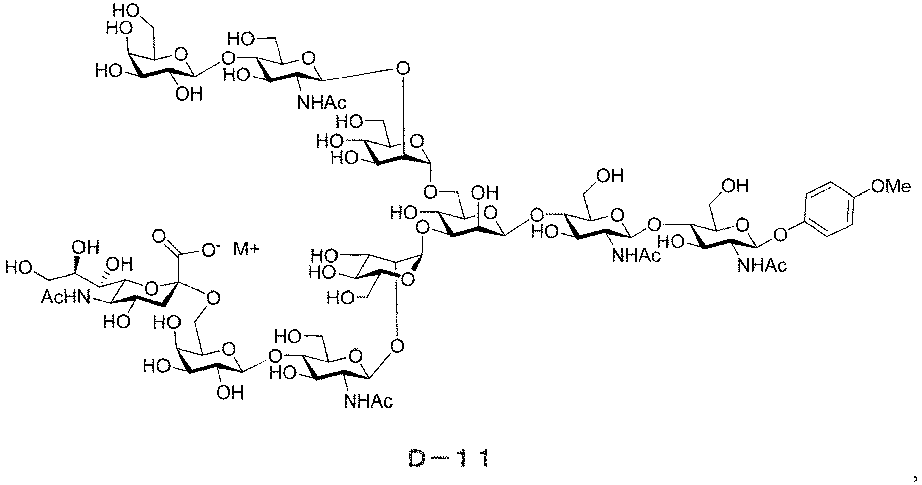 wherein M⁺ is a sodium ion, a lithium ion, a potassium ion, or a protonated triethylamine cation.
[113] A crystalline compound represented by Formula E-2: 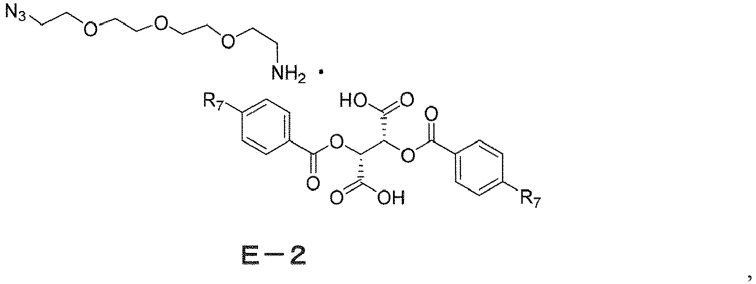 wherein R₇ is a hydrogen atom, a methyl group, or a methoxy group.
[114] A compound represented by Formula D-12: 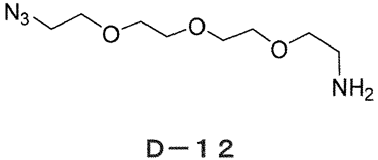 wherein the compound has a purity of 90% or higher as measured by HPLC.
[115] The compound according to [109], wherein the compound has a purity of 95% or higher.
[116] An oligosaccharide represented by Formula D-13:

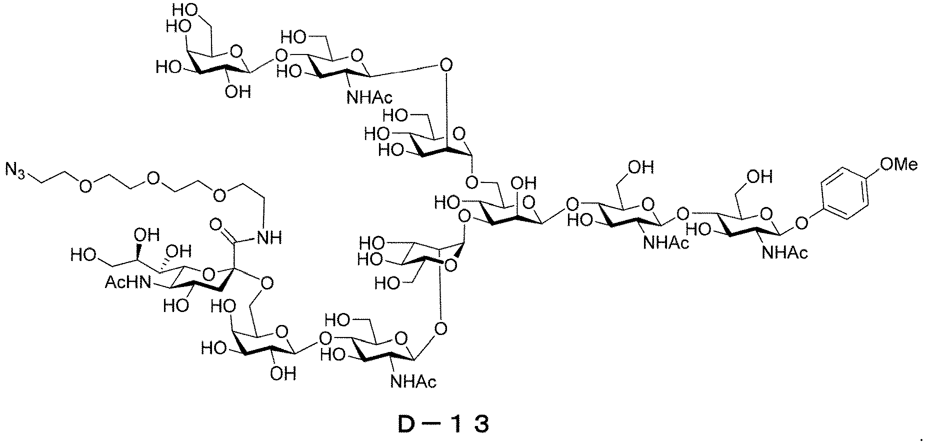

### ADVANTAGEOUS EFFECT OF INVENTION

The present invention provides the above oligosaccharide represented by Formula A-13, a novel method for production thereof, intermediates for production of the oligosaccharide and a method for the production thereof, as well as the above oligosaccharide represented by Formula D-13, a novel method for production thereof, intermediates for production of the oligosaccharide, and a method for the production thereof.

### BRIEF DESCRIPTION OF DRAWINGS

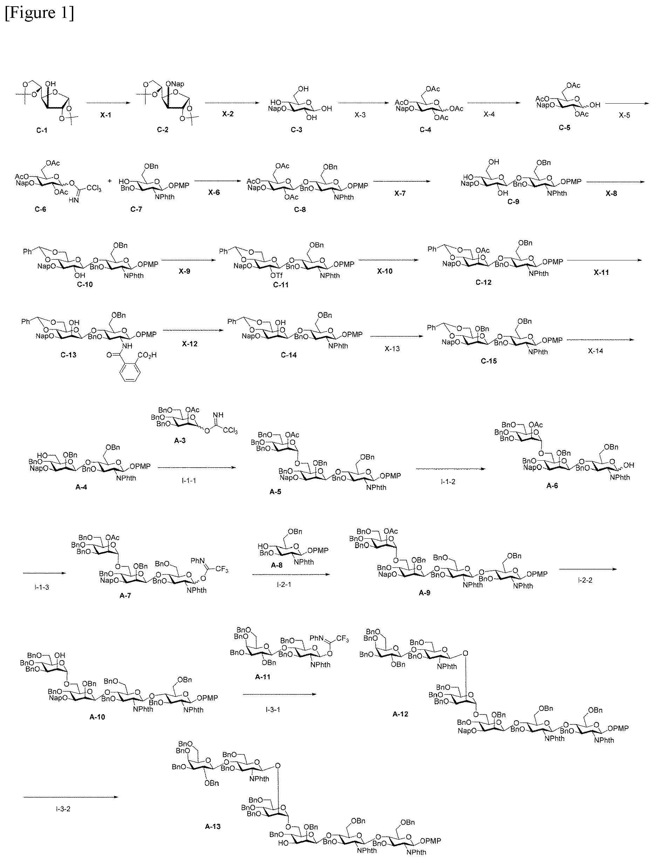

[Figure 1] Figure 1 shows a simplified example of a novel method for producing the above oligosaccharide represented by Formula A-13, which is provided as the present invention.

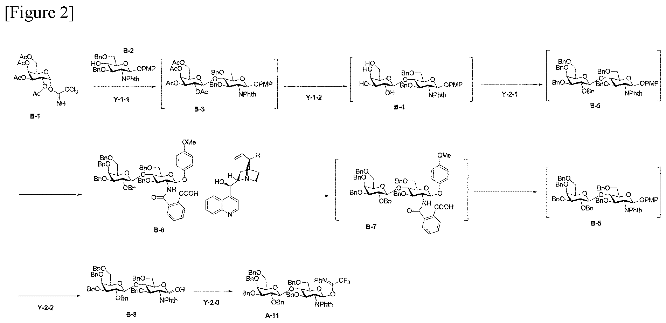

[Figure 2] Figure 2 shows a simplified example of a novel method for producing the above compound represented by Formula A-11, which is provided as the present invention.

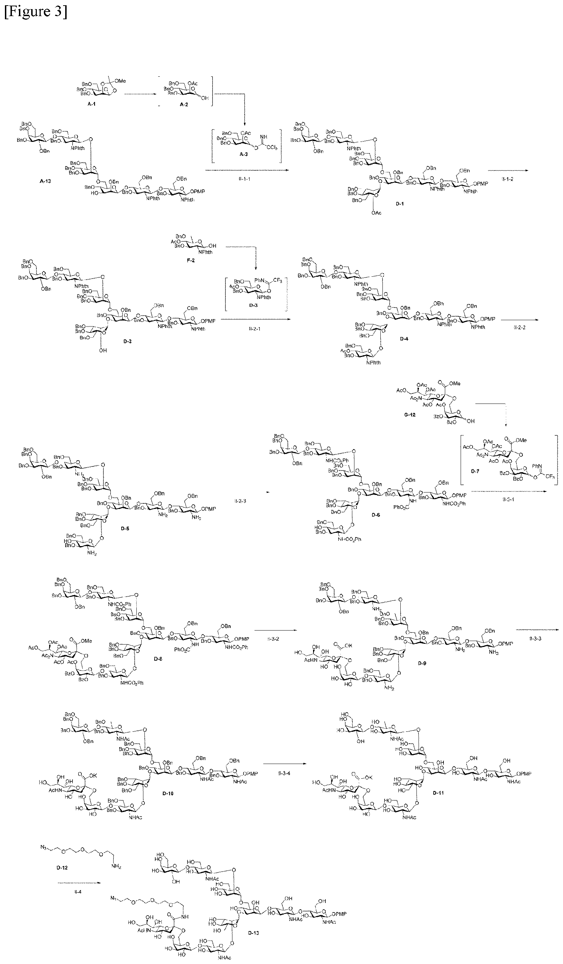

[Figure 3] Figure 3 shows a simplified example of a novel method for producing the above oligosaccharide represented by Formula D-13, which is provided as the present invention.

### DETAILED DESCRIPTION OF THE INVENTION

Preferable embodiments of the present invention are described below. These embodiments are examples of representative embodiments of the present invention., and the scope of the present invention should not be narrowly construed due to these embodiments.

### <1. Method for producing the oligosaccharide represented by Formula A-13>

In an embodiment of the present invention, provided is a novel oligosaccharide represented by Formula A-13 and a novel method for production thereof. In the present invention, the oligosaccharide represented by Formula A-13 means the following oligosaccharide.

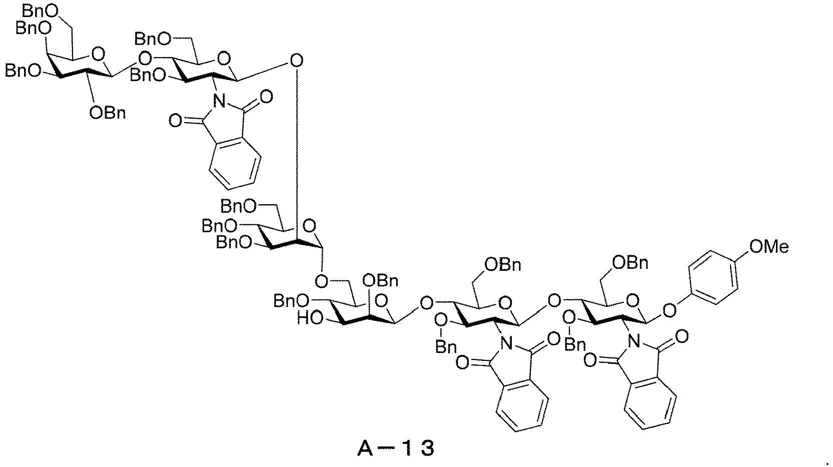

A new scheme for synthesis of the above oligosaccharide represented by Formula A-13 comprises the following Steps I-1 to I-3.

### <Step I-1>

Step I-1 is a step of producing a compound represented by Formula A-7: 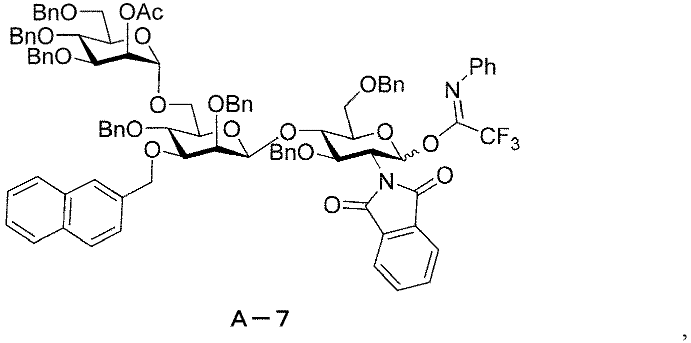 wherein the step comprising a step of:
connecting a compound represented by Formula A-3: 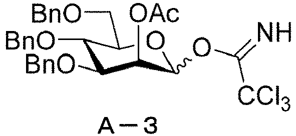 to a compound represented by Formula A-4: 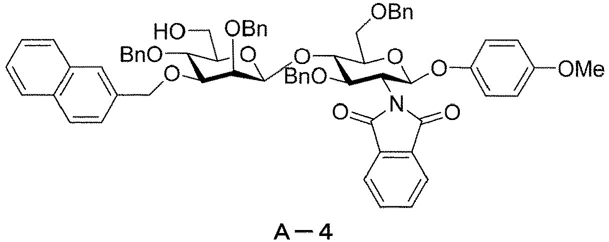 via α-1,6-glycosidic linkage to give a compound represented by Formula A-5: 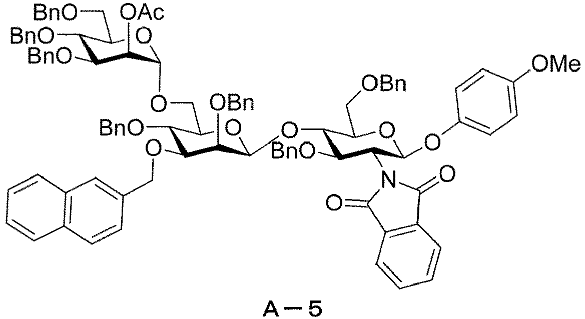 Step I-1 comprises the following Steps I-1-1 to I-1-3.

### <Step I-1-1>

Step I-1-1 is a step of connecting the compound represented by Formula A-3 to the compound represented by Formula A-4 via α-1,6-glycosidic linkage to give a compound represented by Formula A-5. This step may be performed by using or adapting a known method, and may be preferably performed, for example, by the procedure shown in Example 22. For example, the compound represented by Formula A-3 may be bound to the above compound represented by Formula A-4 via α-1,6-glycosidic linkage by sequentially adding Molecular Sieves 4A powder and trimethylsilyl trifluoromethanesulfonate (TMSOTf) in an organic solvent (e.g., toluene) to produce the above compound represented by Formula A-5. In addition, the compounds represented by Formula A-3 and Formula A-4, which are the starting materials, may be produced as follows.

### <Production of the compound represented by Formula A-3>

In one embodiment of the present invention, the compound represented by Formula A-3 may be produced by the following steps, but a method for production thereof is not limited to this method.

First, a compound (3,4,6-tri-O-benzyl-1,2-O-(1-methoxyethyliden)-β-D-mannopyranose) represented by Formula A-1: 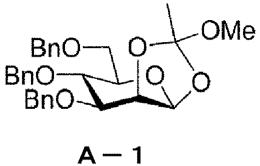 is added to, for example, water and p-TsOH·H₂O and then reacted with triethylamine to give a compound represented by Formula A-2: 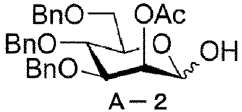 This step may be preferably performed by, for example, the procedure shown in Example 1.

Next, for example, trichloroacetonitrile and diazabicycloundecene (DBU) are added to the compound represented by Formula A-2 to produce the compound represented by Formula A-3. This step may be preferably performed by, for example, the procedure shown in Example 2.

### <Production of the compound represented by Formula A-4>

In one embodiment of the present invention, the compound represented by Formula A-4 may be produced by the following Steps X-1 to X-14 or the following Steps X-1 to X-8 + X-15 to X-16. The details of each step are illustrated below. Each step can be also carried out by using or adapting usual procedures for producing a monosaccharide or oligosaccharide.

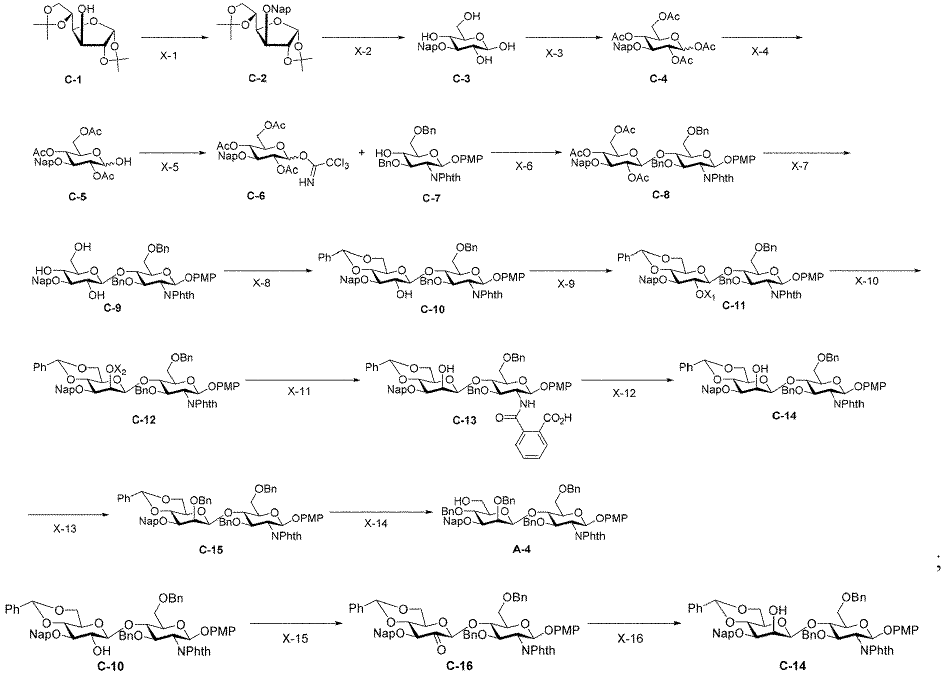

### <Step X-1>

Step X-1 is a step of protecting, with 2-naphthylmethyl (NAP) group, a hydroxyl group attached to carbon at position 3 of a compound represented by Formula C-1: 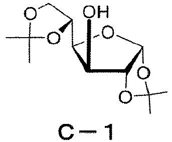 to produce a compound represented by Formula C-2: 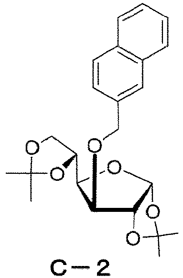 The compound represented by Formula C-1, which is the starting material for this step, may be produced by a known method, or a commercially available product thereof may be used. Examples of the commercially available compound represented by Formula C-1 include 1,2:5,6-di-O-isopropylidene-α-D-glucofuranose available by Sigma-Aldrich. This step may be performed by using or adapting a known method, and may be preferably performed, for example, by the procedure shown in Example 10.

### <Step X-2>

Step X-2 is a step of conducting acid hydrolysis of two isopropylidene moieties of the compound represented by Formula C-2 and forming a pyranose ring to produce a compound represented by Formula C-3: 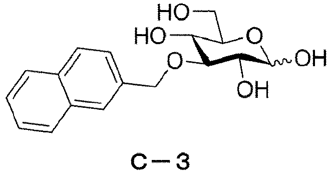 This step may be performed by using or adapting a known method, and may be preferably performed, for example, by the procedure shown in Example 11.

### <Step X-3>

Step X-3 is a step of protecting, with an acetyl group, hydroxyl groups on the compound represented by Formula C-3 to produce a compound represented by Formula C-4: 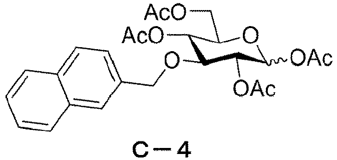 This step may be performed by using or adapting a known method, and may be preferably performed, for example, by the procedure shown in Example 12.

### <Step X-4>

Step X-4 is a step of selectively removing only the acetyl group in the acetyloxy group attached to the carbon at position 1 of the compound represented by Formula C-4 to produce a compound represented by Formula C-5: 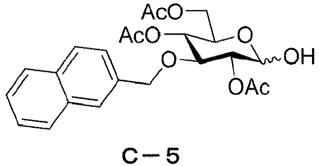 This step may be performed by using or adapting a known method, and may be preferably performed, for example, by the procedure shown in Example 12.

The above step of producing the compound represented by Formula C-5 from the compound represented by Formula C-3 may be performed in a one-pot approach, for example, as shown in Example 12.

### <Step X-5>

Step X-5 is a step of reacting the compound represented by Formula C-5 with trichloroacetonitrile to produce a compound represented by Formula C-6: 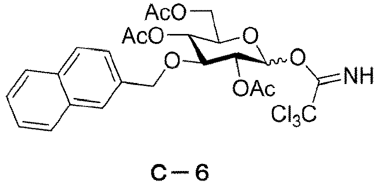 This step may be performed by using or adapting a known method, and may be preferably performed, for example, by the procedure shown in Example 13.

### <Step X-6>

Step X-6 is a step of reacting the compound represented by Formula C-6 with a compound represented by Formula C-7: 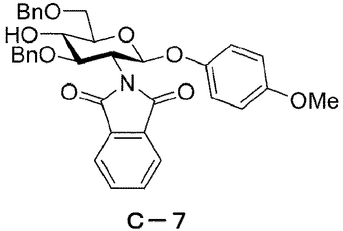 to produce a compound represented by Formula C-8: 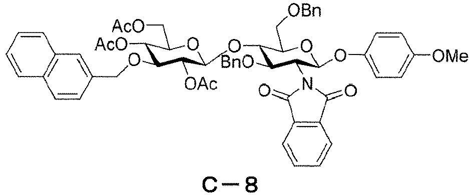 The compound represented by Formula C-7 may be produced by a known method, or a commercially available product thereof may be used. Examples of the commercially available compound represented by Formula C-7 include 4-methoxyphenyl 3,6-di-O-benzyl-2-deoxy-2-phthalimido-β-D-glucopyranoside, available by TOKYO CHEMICAL INDUSTRY CO., LTD. This step may be performed by using or adapting a known method, and may be preferably performed, for example, by the procedure shown in Example 14.

### <Step X-7>

Step X-7 is a step of removing each acetyl group in the compound represented by Formula C-8 to produce a compound represented by Formula C-9: 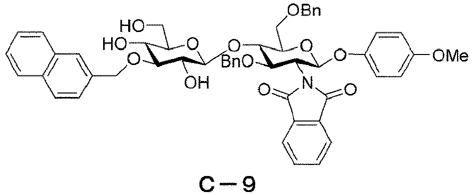 This step may be performed by using or adapting a known method, and may be preferably performed, for example, by the procedure shown in Example 15.

In an embodiment of the present invention, Step X-7 is a step of reacting the compound represented by Formula C-8 with a strong base in the presence of a trifluoroacetate to remove each acetyl group to give the compound represented by Formula C-9. A report (Org. Biomol. Chem., 2018, 16, 4720-4727) shows that the acetyl groups were removed using sodium methoxide in methanol, and in this case, the phthalimide ring may be opened at the same time as an undesired side reaction. In contrast, by using the approach of reaction with an alkoxide-based strong base in the presence of an alkyl ester of perfluorocarboxylic acid, the acetyl group(s) can be removed while suppressing the ring-opening of the phthalimide group.

The "alkyl ester of perfluorocarboxylic acid" used in the above step is not limited as long as the reaction proceeds, and include, for example, methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate. Methyl trifluoroacetate is preferably used.

The above "strong base" is not limited as long as the reaction proceeds, and is selected from the group consisting of, for example, sodium, lithium, and potassium salts of metal amides; sodium, lithium, potassium, cesium, and barium salts of C1-C20 alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and a combination thereof. Sodium, lithium, or potassium salt of C1-C20 alkoxide includes, for example, lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, potassium tert-butoxide, lithium tert-pentoxide, sodium tert-pentoxide, or potassium tert-pentoxide, and particularly preferably sodium tert-butoxide, lithium tert-butoxide, potassium tert-butoxide, or LHMDS (lithium hexamethyldisilazide).

The solvent in this step is not limited as long as the reaction proceeds, and for example, a C1-C10 alcohol solvent alone or a mixed solvent of a C1-C10 alcohol solvent and an amide-based solvent (e.g., dimethylformamide, dimethylacetamide), ether-based solvent (e.g., tetrahydrofuran, dimethoxyethane, cyclopentyl methyl ether), ester-based solvent (e.g., ethyl acetate), aromatic solvent (e.g., toluene), halogen-based solvent (e.g., dichloromethane), hydrocarbon-based solvent (e.g., hexane), or nitrile-based solvent (e.g., acetonitrile) may be used, and methanol or a mixed solvent system of methanol and tetrahydrofuran may be preferably used, but the solvent is not limited to any of them. The above "C1-C10 alcohol solvent" can be replaced by an alcohol having more carbon atoms, while C1-C5 alcohols (e.g., methanol, ethanol, propanol, butanol) may be preferably used due to their availability and convenience.

The reaction temperature in this step is not limited as long as the reaction proceeds, but may be, for example, from -20°C to 80°C, preferably from 0°C to 70°C, more preferably from 20°C to 65°C, and particularly preferably from 40°C to 60°C.

### <Step X-8>

Step X-8 is a step of selectively protecting, using benzaldehyde dimethyl acetal, the hydroxyl groups attached to the carbon atoms at positions 4 and 6 of D-glucopyranoside in the compound represented by Formula C-9 to produce a compound represented by Formula C-10: 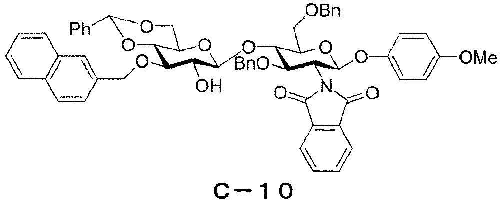 This step may be performed by using or adapting a known method, and may be preferably performed, for example, by the procedure shown in Example 16.

### <Step X-9>

Step X-9 is a step of reacting the compound represented by Formula C-10 with a compound that provides a leaving group selected from the group consisting of a trifluoromethanesulfonyloxy group, a nonafluorobutanesulfonyloxy group, a 2-nitrobenzenesulfonyloxy group, and a 4-nitrobenzenesulfonyloxy group to produce a compound represented by Formula C-11: 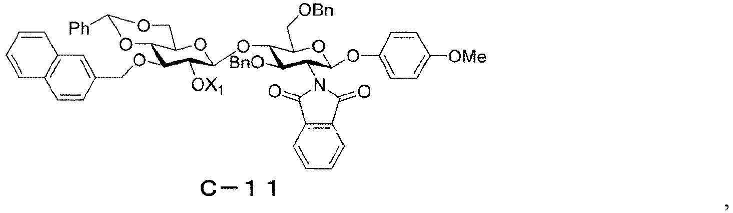 wherein X₁ represents a substituent selected from the group consisting of a trifluoromethanesulfonyl group, a nonafluorobutanesulfonyl group, a 2-nitrobenzenesulfonyl group, and a 4-nitrobenzenesulfonyl group. This step may be performed by using or adapting a known procedure for providing a leaving group, and may be preferably performed, for example, by the procedure shown in Example 17.

In this step, the "compound that provides a leaving group selected from the group consisting of a trifluoromethanesulfonyloxy group, a nonafluorobutanesulfonyloxy group, a 2-nitrobenzenesulfonyloxy group, and a 4-nitrobenzenesulfonyloxy group" includes trifluoromethanesulfonic anhydride, nonafluoro-1-butanesulfonyl fluoride, bis(nonafluoro-1-butanesulfonic acid)anhydride, 2-nitrobenzenesulfonyl chloride, or 4-nitrobenzenesulfonyl chloride, and preferably includes trifluoromethanesulfonic anhydride.

The solvent in this step is not limited as long as the reaction proceeds, and includes, for example, ethyl acetate, toluene, dichloromethane, acetonitrile, cyclopentyl methyl ether, or tert-butyl methyl ether, and preferably includes ethyl acetate, toluene, or dichloromethane.

The reaction temperature in this step is not limited as long as the reaction proceeds, but may be, for example, from -40°C to 60°C, preferably from -30°C to 40°C, and more preferably from -20°C to 10°C.

This step can be preferably performed in the presence of a base. The base used in this step is not particularly limited as long as the reaction proceeds, and includes, for example, 1-methylimidazole, pyridine, 4-dimethylaminopyridine, picoline, lutidine, or collidine, and preferably includes 1-methylimidazole.

### <Step X-10>

Step X-10 is a step of reacting the compound represented by Formula C-11 with cesium acetate or tetrabutylammonium acetate to produce a compound represented by Formula C-12: 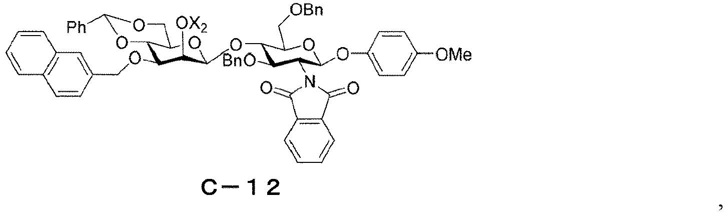 wherein X₂ is an acetyl group,
or a step of reacting the compound represented by Formula C-11 with tetrabutylammonium benzoate to produce a compound represented by Formula C-12: 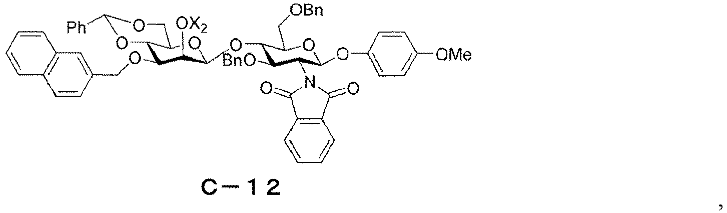 wherein X₂ is a benzoyl group. There is a known conversion reaction for the stereoinversion from glucose -> mannose, whereas there is no report of the conversion in the case where the protecting group of the hydroxyl group attached to the carbon at position 3 of D-glucopyranoside in a glucose-glucosamine disaccharide linked via a β-glycosidic linkage is a 2-naphthylmethyl (Nap) group. This method may be used to achieve the stereoinversion from glucose -> mannose, so that the disaccharide skeleton of mannose-glucosamine bound via the β-glycosidic linkage can be formed in high yield and with high selectivity.

The solvent in this step is not limited as long as the reaction proceeds, and includes, for example, dimethyl sulfoxide, dimethylformamide, dimethylacetamide, N-methylpyrrolidone, N,N-dimethylimidazolidinone, sulfolane, tetrahydrofuran, or acetonitrile, and preferably includes dimethylsulfoxide.

The reaction temperature in this step is not limited as long as the reaction proceeds, but may be, for example, from 20°C to 80°C, preferably from 23°C to 70°C, more preferably from 26°C to 60°C, and particularly preferably from 30°C to 50°C.

### <Step X-11>

Step X-11 is a step of removing the X₂ group and conducting ring-opening of the phthalimide group in the compound represented by Formula C-12 to produce a compound represented by Formula C-13; 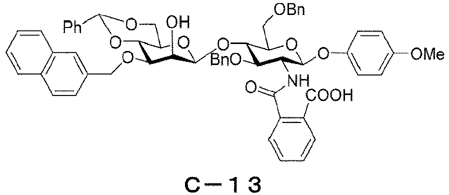 This step may be performed by using or adapting a known method of hydrolysis, and may be preferably performed, for example, by the procedure shown in Example 18.

The compound represented by Formula C-13 produced in this step and dissolved in the solvent may be used in the next step, or may be isolated and purified by recrystallization. The compound represented by Formula C-13 have a great advantage in that it can be isolated and purified by crystallization and impurities having similar structures that are difficult to remove by column chromatography purification can be almost completely removed by such crystallization. In this case, the compound represented by Formula C-13 having a purity of 99% or higher by HPLC can be obtained.

The method for the isolation and purification by recrystallization in this step includes a method for completely removing a solvent in which the compound is dissolved, by drying under reduced pressure; or a method for adding dropwise isopropanol as a poor solvent to tetrahydrofuran as a good solvent in the presence of a trace amount of water.

The recrystallization in this step may also be performed using seed crystals of the compound represented by Formula C-13. In the case of using the seed crystals, the crystallization may be conducted by, for example, a method in which a portion of isopropanol as a poor solvent is added dropwise to tetrahydrofuran as a good solvent in the presence of a trace amount of water while adding the seed crystals, and after confirming precipitation of crystals, the remaining portion of isopropanol is added dropwise.

The above step of producing the compound represented by Formula C-13 from the compound represented by Formula C-11 may be performed in a one-pot approach, for example, as shown in Example 18.

### <Step X-12>

Step X-12 is a step of conducting the ring-closing of the ring-opened phthalimide group in the compound represented by Formula C-13 by dehydration condensation, to produce a compound represented by Formula C-14; 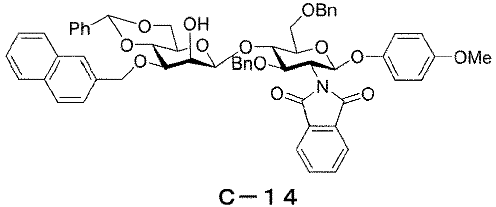 This step may be performed by using or adapting a known method, and may be preferably performed, for example, by the procedure shown in Example 19.

### <Step X-13>

Step X-13 is a step of protecting, with a benzyl group, the hydroxyl group attached to the carbon at position 2 of D-mannopyranoside in the compound represented by Formula C-14 to produce a compound represented by Formula C-15: 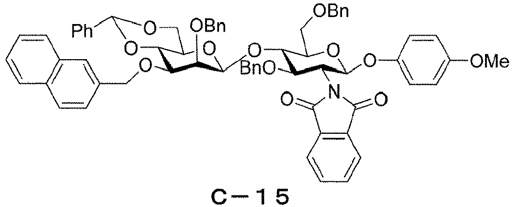 This step may be performed by using or adapting a known method, and may be preferably performed, for example, by the procedure shown in Example 20.

In an embodiment of the present invention, Step X-13 comprises a step of protecting, with a benzyl group, the hydroxyl group attached to the carbon at position 2 of D-mannopyranoside in the compound represented by Formula C-14 in the presence of lithium tert-butoxide or lithium tert-amoxide to produce the compound represented by Formula C-15. The ring-opening of phthalimide can be suppressed by performing Step X-15 in the presence of lithium tert-butoxide or lithium tert-amoxide. In addition, it can be performed safer and is easier to be scaled up as compared to a common condition using sodium hydride.

The solvent in this step is not limited as long as the reaction proceed, and includes, for example, dimethylacetamide, dimethylformamide, N-methylpyrrolidone, or N,N-dimethylimidazolidinone, and preferably includes dimethylacetamide.

The reaction temperature in this step is not limited as long as the reaction proceeds, but may be, for example, from -20°C to 100°C, preferably from -15°C to 70°C, and particularly preferably from -10°C to 50°C.

### <Step X-14>

Step X-14 is a step of selectively reducing the protecting group of benzylidene in the compound represented by Formula C-15 (for more details, see Angew. Chem. Int. Ed. 2005, 44, 1665-1668) to produce a compound represented by Formula A-4: 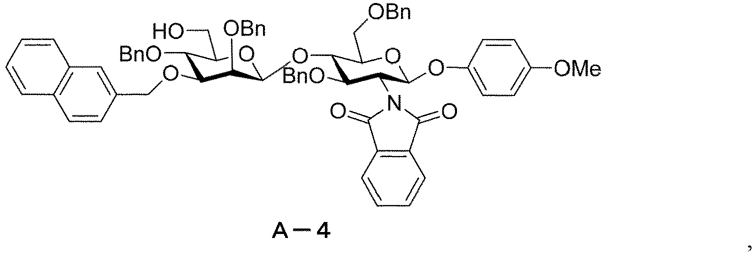 wherein only the hydroxyl group attached to the carbon at position 6 of D-mannopyranoside is deprotected. This step may be performed by using or adapting a known method, and may be preferably performed, for example, by the procedure shown in Example 21.

The compound represented by Formula A-4 that is produced in this step and dissolved in a solvent may be used in the next step, or may be isolated and purified by, for example, column chromatography purification.

In an embodiment of the present invention, this step comprises the following Steps X-15 and X-16 for stereoinversion from glucose -> mannose using a redox reaction, instead of Steps X-9 to X-12 for the stereoinversion using an S_{N}2 reaction.

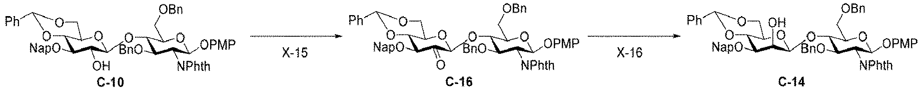

### <Step X-15>

Step X-15 is a step of oxidizing position 2 of D-glucopyranoside in the compound represented by Formula C-10 to produce a compound represented by Formula C-16: 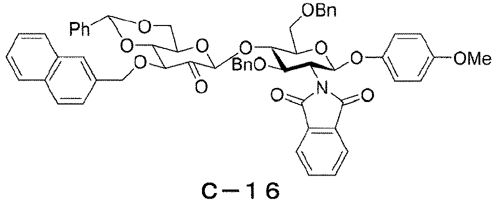 This step may be performed by using or adapting a known procedure.

### <Step X-16>

Step X-16 is a step of reducing the ketone group attached to the carbon at position 2 of 2-keto-D-glucopyranoside in the compound represented by Formula C-16 to produce a compound represented by Formula C-14: 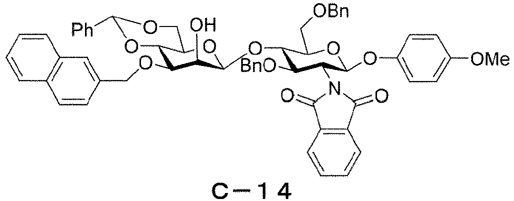 This step may be performed by using or adapting a known procedure.

The solvent in this step is not limited as long as the reaction proceeds, and includes, for example, diethyl ether, cyclopentyl methyl ether, tert-butyl methyl ether, diisopropyl ether, dipropyl ether, dibutyl ether, or 1,4-dioxane, and preferably tetrahydrofuran.

The reaction temperature in this step is not limited as long as the reaction proceeds, but may be, for example, from -80°C to 20°C. As described below, the optimum reaction temperature varies depending on the reducing agent used.

In an embodiment of the present invention, the oxo group attached to the carbon at position 2 of 2-keto-D-glucopyranoside in the compound represented by Formula C-16 may be reduced in the presence of a reducing agent selected from the group consisting of L-selectride; LS-selectride; lithium diisobutyl-tert-butoxyaluminum hydride (LDBBA); a compound represented by Formula W: 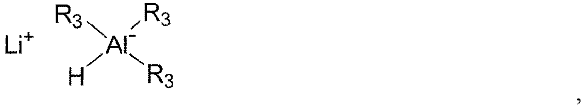 wherein R₃ is a di-tert-butylmethylphenoxide or hydride as shown in the following formula: 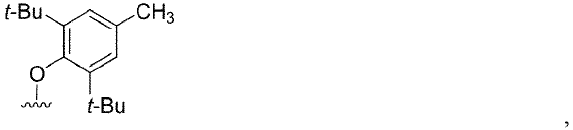 provided that at least two R₃ are each di-tert-butylmethylphenoxide; and a combination thereof. In the reduction step, for example, when NaBH₄ is used, the stereoselectivity was low (about 7:3), and it was difficult to efficiently achieve the desired stereoinversion from Gln -> Man (Org. Biomol. Chem., 2018, 16, 4720-4727). In contrast, when the reducing agent listed above is used, the selectivity of stereoinversion from Gln -> Man is greatly improved (93.6:6.4 to 98.1:1.9) as compared to the case where NaBH₄ is used.

The compound represented by Formula W, wherein three R₃ moieties are di-tert-butylmethylphenoxide, can be obtained, for example, by adding dibutylhydroxytoluene (885.41 mg, 4.02 mmol) to a tetrahydrofuran suspension (2 mL) containing lithium aluminum hydride (50.0 mg, 1.32 mmol) at 0°C, followed by stirring at 25°C. The compound represented by Formula W, wherein two R₃ moieties are di-tert-butylmethylphenoxide, can be obtained in a similar manner by using 2 molar equivalents of dibutylhydroxytoluene relative to 1 molar equivalent of lithium aluminum hydride.

As described above, the reaction temperature in this step is not limited as long as the reaction proceeds. When L-selectride, LS-selectride, or LDBBA is used as the reducing agent, the reaction temperature may be preferably from -80°C to -20°C, more preferably from -80°C to -30°C, still more preferably from -80°C to -40°C, and particularly preferably from -80°C to -50°C. When the compound represented by Formula A is used as the reducing agent, the reaction temperature may be preferably from -20°C to 20°C, more preferably from -15°C to 15°C, and particularly preferably from -10°C to 10°C. Therefore, the particularly suitable reducing agent used for this step is the compound represented by Formula W, since the reaction proceeds at the easily-handled temperature.

### <Purification of the compound represented by Formula A-5>

In Step I-1-1, the compound represented by Formula A-5 can be obtained in a purified form by the following method for purification. The method for purification comprises purifying the above compound represented by Formula A-5 by
after stopping the reaction of the compound represented by Formula A-4 with the compound represented by Formula A-3, adding a hydrophobic carrier and water to a water-soluble organic solvent containing the above resulting compound represented by Formula A-5 and contaminants to adsorb the above compound represented by Formula A-5 onto the hydrophobic carrier,
then filtering the resultant and washing the hydrophobic carrier with a mixture of the above water-soluble organic solvent and water to remove the contaminants,
and then eluting the above compound represented by Formula A-5 from the hydrophobic carrier using an organic solvent. It has become possible to efficiently produce a large quantity of a high-quality oligosaccharide using the above method for purification, since remnants of the reagents and impurities derived from a glycosyl donor and an acceptor, which are remained after glycosylation in the liquid phase synthesis of an oligosaccharide chain, can be removed by washing them with a small amount of hydrophobic carrier, and an inhibition of the reaction and side reactions caused by these impurities can be suppressed. Further, in the present invention, the number of steps of desorpting tag(s) can be reduced, and a reduction in functionality of the tag(s) during oligomerization can be prevented by utilizing the hydrophobicity of the substrate itself, as compared to conventionally developed methods, so that the oligosaccharide can be produced more efficiently. In particular, in this step, the compound represented by Formula A-5 can be easily separated and purified from degradation products derived from the compound represented by Formula A-3 by using the above method for purification.

The purification of the above compound represented by Formula A-5 is not limited to the purification in this Step I-1-1. Accordingly, in an embodiment of the present invention, provided is a method for purify the above compound represented by Formula A-5, comprising
adding a hydrophobic carrier and water to a water-soluble organic solvent containing the above compound represented by Formula A-5 and contaminants to adsorb the above compound represented by Formula A-5 onto the hydrophobic carrier;
then filtering the resultant and washing the hydrophobic carrier with a mixture of the above water-soluble organic solvent and water to remove the contaminants;
and then eluting the above compound represented by Formula A-5 from the hydrophobic carrier using an organic solvent. Further, the above method for purification can be applied to purification of organic compounds other than the sugar compounds. When the organic compound to be purified is a sugar compound, a protected oligosaccharide having a glycan structure consisting of 3-15 sugar residues, in which one or all hydroxyl groups in the sugar are protected, may be preferably purified, and in that case, the protecting group of the glycan includes, but are not limited to, alkyl ethers, benzyl ethers, silyl ethers, esters, or carbonate esters.

The above term "contaminants" refers to compounds and/or reagents other than the protected oligosaccharide (in this step, the compound represented by Formula A-5 above), and mainly means reagents used in a reaction of synthesizing a protected oligosaccharide and remnants of the reagents, sugars other than the protected oligosaccharide, such as mono- or disaccharide compounds used in the elongation reaction of the protected oligosaccharide, or byproducts generated by the deprotection reaction of the protected oligosaccharide.

The above term "hydrophobic carrier" refers to a hydrophobic adsorption material that adsorbs specific compounds including sugar compounds, and includes, for example, a resin used for packing reversed-phase partition chromatography. The "resin packed for reversed-phase partition chromatography" is selected from the group consisting of, but is not limited to, a poly(styrene/divinylbenzene) polymer gel resin, a polystyrene-divinylbenzene resin, a polyhydroxymethacrylate resin, a styrene-vinylbenzene copolymer resin, a polyvinyl alcohol resin, a polystyrene resin, a polymethacrylate resin, a chemically bonded silica gel resin, and a combination thereof.

The above "chemically bonded silica gel resin" is selected from the group consisting of (1) a resin obtained by reacting silica gel with a silane coupling agent, (2) a resin obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) a resin obtained by chemically bonding a docosyl or triacontyl group to silica gel, and (4) a combination of the resins (1) to (3). Octadecyl group-bonded silica gel resin (ODS resin) are preferably used, but the above resin is not limited to any of them.

The above "water-soluble organic solvent" is not limited to, but may be a water-soluble alcohol-based solvent (suitably C1-C4), a water-soluble nitrile-based solvent (e.g., acetonitrile, etc.), a water-soluble ether-based solvent (e.g., tetrahydrofuran, etc.), a water-soluble ketone-based solvent (e.g., acetone), a water-soluble amide-based solvent (e.g., dimethylformamide), or a water-soluble sulfoxide-based solvent (e.g., dimethyl sulfoxide). Acetonitrile may be preferably used as the solvent.

As the "organic solvent" used in the step of eluting the product of interest from the above hydrophobic carrier, a nitrile-based solvent (e.g., acetonitrile), ether-based solvent (e.g., tetrahydrofuran), ester-based solvent (e.g., ethyl acetate), ketone-based solvent (e.g., acetone), halogen-based solvent (e.g., dichloromethane), or aromatic solvent (e.g., toluene), or a mixed solvent containing at least one of the above solvent systems may be used, and acetonitrile, ethyl acetate, tetrahydrofuran, or toluene may be preferably used, but the organic solvent is not particularly limited to any of them.

The above step of the purification can be performed at the temperature from 0°C to 50°C, but the temperature is not particularly limited to such a temperature.

Steps 1-1-2 to 1-1-3 as follows, after Step 1-1-1, may be used to produce the above compound represented by Formula A-7 from the above compound represented by Formula A-5, but production steps are not limited to those steps.

### <Step I-1-2>

Step I-1-2 is a step of removing the 4-methoxyphenyl group from the compound represented by Formula A-5 to give a compound represented by Formula A-6:

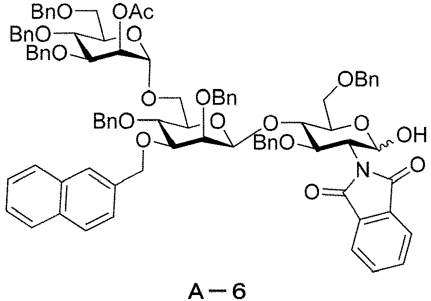

In an embodiment of the present invention, this step is a step of producing the above compound represented by Formula A-6 by reacting the above compound represented by Formula A-5 with λ³-iodane in fluorous alcohol and water to deprotect a 4-methoxyphenyl group. This step may be preferably performed by, for example, the procedure shown in Example 23.

The term "λ3-iodane" described above means a trivalent/hypervalent iodine compound. In an embodiment, a compound represented by Formula R⁴-I(OR⁵)₂ (wherein R⁴ is an unsubstituted or substituted phenyl group and R⁵ is selected from the group consisting of H, acetoxy, trifluoroacetoxy, tosyloxy, methane sulfonyloxy, and a combination thereof). As defined in the above formula, R⁴ may be a "substituted phenyl group", and its substituent includes a linear or branched, saturated or unsaturated hydrocarbon group, an oxygen-containing group (e.g., alkoxy, ester), a nitrogen-containing group (e.g., cyano, azide), or a halogen atom (e.g., fluorine atom, chlorine atom, bromine atom, iodine atom), more preferably hydrocarbon group, an oxygen-containing substituent, or a halogen atom. If the above substituent contains carbon(s), the substituent having, for example, C1-C5 or C1-C3 may be preferably used. Specific examples of the λ3-iodane include, but are not limited to, [bis(trifluoroacetoxy)iodo]benzene (PIFA), [hydroxy(tosyloxy)iodo]benzene (HTIB), (diacetoxyiodo)benzene (PIDA), [bis(trifluoroacetoxy)iodo]pentafluorobenzene, and [hydroxy(methanesulfonyloxy)iodo]benzene.

As used in the above step, the term "fluorous alcohol" means a fluorine-containing alcohol compound in which all carbon atoms except those bonded to the alcohol have fluorous. The fluorous alcohol preferably has a greater number of fluorine atoms as long as fluorine substitution is allowed. The fluorous alcohol includes, but is not limited to, a fluorous aliphatic alcohol. The hydrocarbon moiety in the fluorous aliphatic alcohol may be saturated or unsaturated, linear or branched, or cyclic. The fluorous aliphatic alcohol is, for example, a fluorous C₂-C₈ aliphatic alcohol, preferably a fluorous C₂-C₅ aliphatic alcohol, and more preferably a fluorous C₂-C₃ aliphatic alcohol. Specific examples of the fluorous alcohol include, but are not limited to a compound selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and a combination thereof.

This step is carried out in the co-presence of the above-mentioned fluorous alcohol and "water." The amount of water can be appropriately determined from the viewpoint of achieving a high yield of the product, etc. For example, the amount may be about 1.0 equivalent or more, about 1.5 equivalents or more, about 2.0 equivalents or more, or about 2.5 equivalents or more in a mole ratio based on the compound represented by Formula A-5. The amount may be about 10 or less, about 8 or less, about 5 or less, or about 3 or less in a volume ratio based on the compound represented by Formula A-5.

In this step, an "additive" may be further added to the fluorous alcohol and water. The additive is preferably selected from the group consisting of sodium dihydrogen phosphate, potassium dihydrogen phosphate, disodium hydrogen phosphate, trifluoroacetic acid, and a combination thereof. The amount of the additive can be appropriately determined, and may be, for example, about 0.5 to 8 equivalents, about 1 to 6 equivalents, or about 1.5 to 5 equivalents based on the compound represented by Formula A-5.

### <Step I-1-3>

Step I-1-3 is a step of producing the above compound represented by Formula A-7 from the above compound represented by Formula A-6. This step may be performed by using or adapting a known method, and may be preferably performed, for example, by the procedure shown in Example 24.

In an embodiment of the present invention, this step is a step of reacting the above compound represented by Formula A-6 with 2,2,2-trifluoro-N-phenylacetimidoyl chloride (TFPC) in the presence of DBU to give the above compound represented by Formula A-7. The amount of TFPC can be reduced using DBU as the base, as compared to the case where potassium carbonate, etc., is used, and the product of interest can be obtained in high yield. Since TFPC is an expensive reagent, an increase in the yield of this step is very beneficial for commercial production.

The solvent in this step is not limited as long as the reaction proceeds, and includes, for example, dichloromethane, toluene, ethyl acetate, acetonitrile, or tetrahydrofuran, and preferably dichloromethane.

The reaction temperature in this step is not limited as long as the reaction proceeds, but may be preferably from -20°C to 40°C, more preferably from -10°C to 35°C, and particularly preferably from 0°C to 30°C.

This step is preferably performed in the presence of a dehydrating agent. The dehydrating agent in this step is not limited as long as the reaction proceeds, and include,d for example, Molecular Sieves, and preferably Molecular Sieves 4A powder with a powder particle size of 10 µm or less.

In this step, the resulting compound represented by Formula A-7 dissolved in a solvent may be used in the next step when the base used in the reaction has been removed, or may be isolated and purified by column chromatography, etc. The isolation and purification using the column include isolation and purification using silica gel as a stationary phase and a dichloromethane or a toluene-ethyl acetate mixed solvent system as a mobile phase.

### <Step I-2>

Step I-2 is a step of producing a compound represented by Formula A-10: 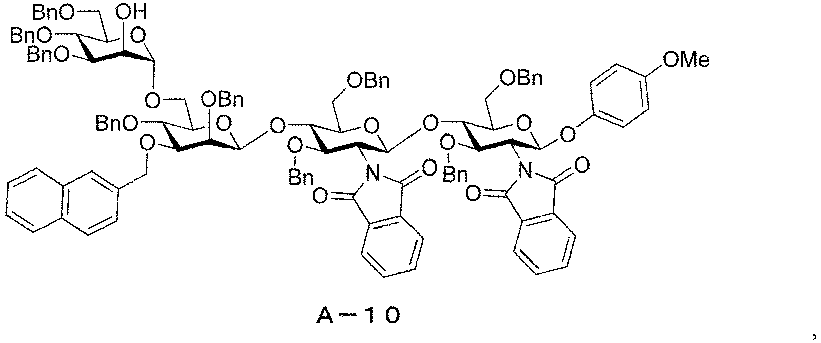 wherein the step comprising a step of:
connecting the above compound represented by Formula A-7 to a compound represented by Formula A-8: 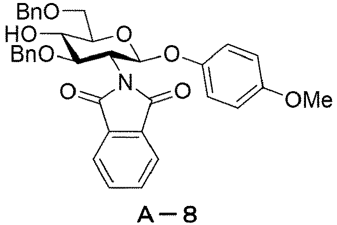 via β-1,4-glycosidic linkage to give a compound represented by Formula A-9:

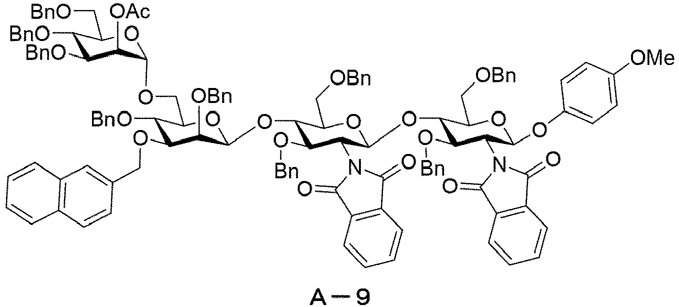

Step I-2 comprises the following Steps I-2-1 to I-2-2.

### <Step I-2-1>

Step I-2-1 is a step of connecting the compound represented by Formula A-7 to the compound represented by Formula A-8 via β-1,4-glycosidic linkage to give a compound represented by Formula A-9. The compound represented by Formula A-8 may be produced by a known method, or a commercially available product thereof may be used. The commercially available compound represented by Formula A-8 includes 4-methoxyphenyl 3,6-di-O-benzyl-2-deoxy-2-phthalimido-β-D-glucopyranoside, available by Tokyo CHEMICAL INDUSTRY CO., LTD. This step may be performed by using or adapting a known method, and may be preferably performed, for example, by the procedure shown in Example 25.

### <Purification of the compound represented by Formula A-9>

In Step I-2-1, the compound represented by Formula A-9 can be obtained in a purified form by the following method for purification. The method for purification comprises purifying the above compound represented by Formula A-9 by
after stopping the reaction of the compound represented by Formula A-7 with the compound represented by Formula A-8, adding a hydrophobic carrier and water to a water-soluble organic solvent containing the above resulting compound represented by Formula A-9 and contaminants to adsorb the above compound represented by Formula A-9 onto the hydrophobic carrier;
then filtering the resultant and washing the hydrophobic carrier with a mixture of the above water-soluble organic solvent and water to remove the contaminants;
and then eluting the above compound represented by Formula A-9 from the hydrophobic carrier using an organic solvent. As described in the method of purifying the above compound represented by Formula A-5, this method for purification makes it possible to produce a large amount of high-quality oligosaccharide efficiently in the liquid-phase synthesis of oligosaccharide chains by using a small amount of hydrophobic carrier. In particular, the monosaccharide compound represented by Formula A-8 and the tetrasaccharide compound represented by Formula A-9 have very close polarity in normal-phase silica gel column chromatography. This makes it difficult to separate them, due to the same Rf value, for example, under the typical column solvent system (hexane-ethyl acetate) condition. However, the monosaccharide can be used to easily separated from the tetrasaccharide having very close polarity by the method for purification in the present invention

The purification of the above compound represented by Formula A-9 is not limited to the purification in this Step I-2-1. Accordingly, in an embodiment of the present invention, also provided is a method comprising
adding a hydrophobic carrier and water to a water-soluble organic solvent containing the above compound represented by Formula A-9 and contaminants to adsorb the above compound represented by Formula A-9 onto the hydrophobic carrier,
then filtering the resultant and washing the hydrophobic carrier with a mixture of the above water-soluble organic solvent and water to remove the contaminants,
and then eluting the above compound represented by Formula A-9 from the hydrophobic carrier using an organic solvent to purify the above compound represented by Formula A-9.

The above term "contaminants" refers to compounds and/or reagents other than the protected oligosaccharide (in this step, the compound represented by Formula A-9), and mainly means reagents used in a reaction of synthesizing a protected oligosaccharide and remnants of the reagents, sugars other than the protected oligosaccharide, such as mono- or disaccharide compounds used in the elongation reaction of the protected oligosaccharide, or byproducts generated by the deprotection reaction of the protected oligosaccharide. The "hydrophobic carrier" (e.g., a resin used for packing reversed-phase partition chromatography), "water-soluble organic solvent," "organic solvent," and the temperature for the purification used in this step are substantially the same as those described in the method of purifying the above compound represented by Formula A-5.

### <Step I-2-2>

Step I-2-2 is a step of producing the compound represented by Formula A-10 from the compound represented by Formula A-9.

In an embodiment of the present invention, this Step I-2-2 is a step of reacting the compound represented by Formula A-9 with a strong base in the presence of an alkyl ester of perfluorocarboxylic acid in a solvent to remove the acetyl group (deacetylation reaction) to give the compound represented by Formula A-10, and may be preferably used, for example, by the procedure shown in Example 26. The deacetylation reaction can be performed in the same way as in the deacetylation reaction described in Step X-7, except that the substrate is different. By using an approach of reaction with a strong base in the presence of an alkyl ester of perfluorocarboxylic acid, the deacetylation reaction can be carried out while suppressing the ring-opening of the phthalimide group.

The use of the above deacetylation reaction is not limited to that in Step I-2-2. Accordingly, in an embodiment of the present invention, provided is a method for producing the above compound represented by Formula A-10, wherein the method comprises a step of reacting the above compound represented by Formula A-9 with a strong base in the presence of an alkyl ester of perfluorocarboxylic acid.

The alkyl ester of perfluorocarboxylic acid, the strong base, the solvent, and the reaction temperature used in this step are as described in Step X-7 above.

### <Step I-3>

Step I-3 is a step of producing the above oligosaccharide represented by Formula A-13, wherein the step comprising a step of:
connecting the above compound represented by Formula A-10 to a compound represented by Formula A-11 : 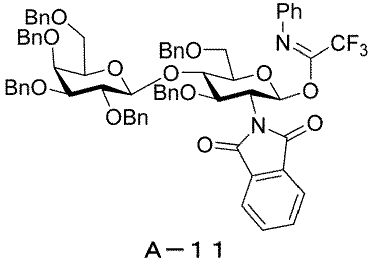 via β-1,2-glycosidic linkage to give a compound represented by Formula A-12: 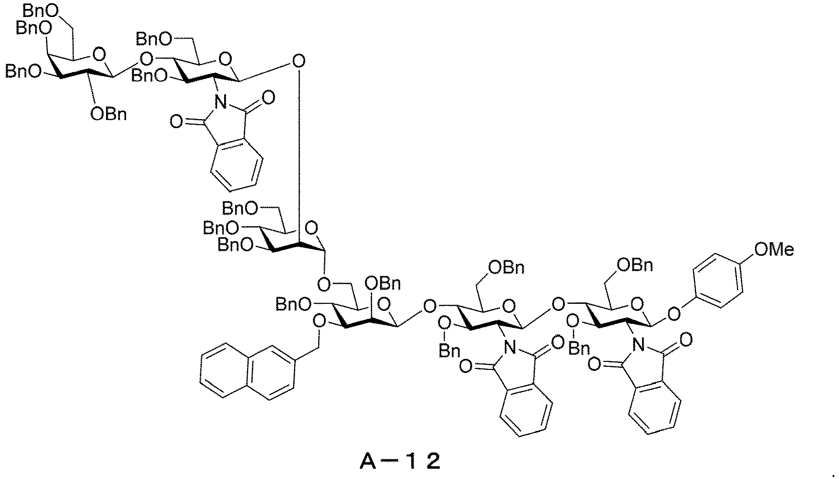 In an embodiment of the present invention, Step I-3 comprises Steps I-3-1 to I-3-2 as follows.

### <Step I-3-1>

Step I-3-1 is a step of connecting the above compound represented by Formula A-10 to the above compound represented by Formula A-11 via β-1,2-glycosidic linkage to give the above compound represented by Formula A-12. This glycosidic linkage step may be performed by using or adapting a known method, and may be preferably performed, for example, by the procedure shown in Example 27. In addition, the compound represented by Formula A-11 may be produced as follows. Further, the compound represented by Formula A-12 may be purified as described below.

### <Production of the compound represented by Formula A-11>

In one embodiment, the above compound represented by Formula A-11 is produced by steps comprising:
(Step Y-1) a step of producing a compound represented by Formula B-4: 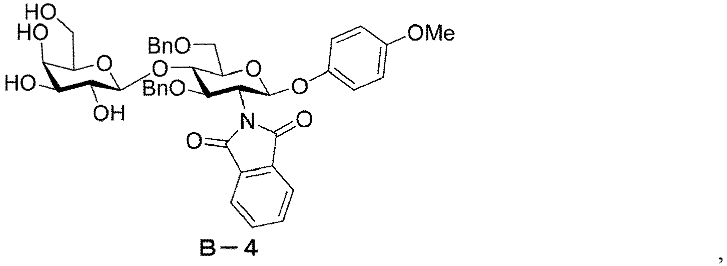 wherein the step comprising a step of:
   connecting a compound represented by Formula B-1: 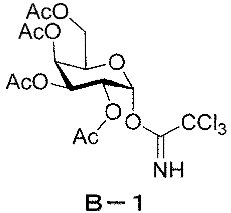 to a compound represented by Formula B-2: 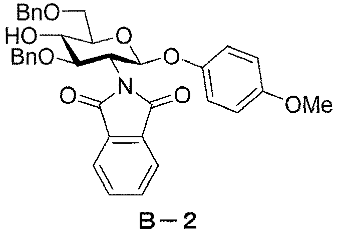 via β-1,4-glycosidic linkage to give a compound represented by Formula B-3: 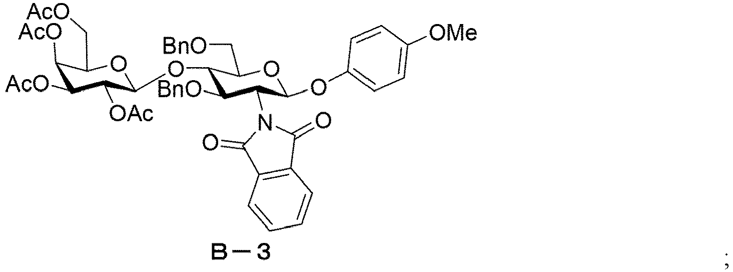 and
(Step Y-2) a step of adding lithium tert-butoxide or lithium tert-amoxide to a solvent containing the compound represented by Formula B-4 and benzyl halide or benzyl sulfonate to protect each hydroxyl group present in the compound represented by Formula B-4 with a benzyl group to give a compound represented by Formula B-5: 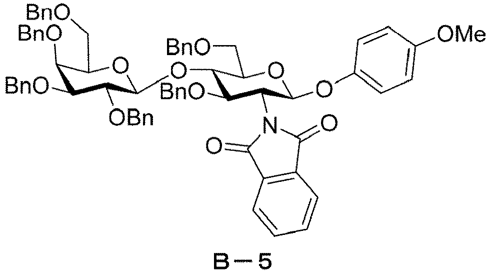 Step Y-1 above comprises steps Y-1-1 and Y-1-2, and Step Y-2 above comprises steps Y-2-1 to Y-2-3.

### <Step Y-1-1>

Step Y-1-1 is a step of connecting the above compound represented by Formula B-1 to the compound represented by Formula B-2 via β-1,4-glycosidic linkage to give the above compound represented by Formula B-3. The commercially available compound represented by Formula B-1 includes 2,3,4,6-tetra-O-acetyl-α-D-galactopyranosyl 2,2,2-trichloroacetimidate (86520-63-0) from TOKYO CHEMICAL INDUSTRY CO., LTD. In addition, the commercially available compound represented by Formula B-2 includes 4-methoxyphenyl 3,6-di-O-benzyl-2-deoxy-2-phthalimido-β-D-glucopyranoside, available by TOKYO CHEMICAL INDUSTRY CO., LTD. This step may be performed by using or adapting a known method, and may be preferably performed, for example, by the procedure shown in Example 3. For example, the compound represented by Formula B-3 can be produced by sequentially adding a solution containing the above compound represented by Formula B-1, Molecular Sieves 4A powder, and trimethylsilyl trifluoromethanesulfonate (TMSOTf) to a solution containing the above compound represented by Formula B-2.

### <Step Y-1-2>

Step Y-1-2 is a step of removing each acetyl group from the compound represented by Formula B-3 to give a compound represented by Formula B-4: 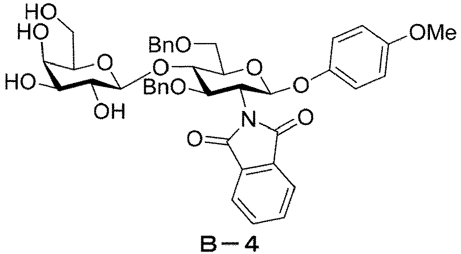 The above removal of the acetyl group (AcO) may be performed by using a known method, and may be preferably performed, for example, by the procedure shown in Example 4. For example, the compound represented by Formula B-4 can be produced by reacting the compound represented by Formula B-3 with a strong base in the presence of trifluoroacetate to remove the acetyl groups in a solvent such as toluene.

### <Step Y-2-1>

Step Y-2-1 is a step of protecting each hydroxyl group present in the compound represented by Formula B-4 with a benzyl group to give the above compound represented by Formula B-5.

In one embodiment, this Step Y-2-1 is a step of producing the above compound represented by Formula B-5 by adding lithium tert-butoxide or lithium tert-amoxide to a solvent containing the compound represented by Formula B-4 and benzyl halide (benzyl bromide, benzyl chloride, benzyl fluoride, or benzyl iodide) or benzyl sulfonate to protect each hydroxyl group present in the above compound represented by Formula B-4 with a benzyl group. In sugar derivatives like a compound N-2 represented by B-4 protected by a phthalimide group, it is sometimes necessary to benzylate a plurality of hydroxyl groups simultaneously, and in that case, the reaction should proceed while suppressing ring-opening of the phthalimide group. However, under strongly basic conditions, the ring-opening reaction of the phthalimide group readily proceeds due to a trace amount of hydroxide ions. Accordingly, under the NaH/DMAc conditions used in conventional benzylation reactions, the yield varies greatly depending on the amount of sodium hydroxide in NaH. Further, there is a disadvantage in that NaH/DMAc is not easy to be used for mass synthesis due to hazardous reagent combination and risk of explosion. The present inventors have discovered a method by which a plurality of hydroxyl groups can be benzylated simultaneously under milder conditions while suppressing ring-opening of the phthalimide group by the benzylation reaction as described above.

The use of the above benzylation reaction is not limited to that in Step Y-2-1. Thus, in one embodiment of the present invention, also provided is a method for producing the above compound represented by Formula B-5, wherein the method comprises a step of adding lithium tert-butoxide or lithium tert-amoxide to a solution containing the compound represented by Formula B-4 and benzyl halide (benzyl bromide, benzyl chloride, benzyl fluoride, or benzyl iodide) or benzyl sulfonate to protect each hydroxyl group present in the above compound represented by Formula B-4 with a benzyl group.

The solvent used in this step is not particularly limited as long as the reaction proceeds, but an amide-based solvent (dimethylformamide, dimethylacetamide), an ether-based solvent (e.g., tetrahydrofuran, dimethoxy ethane), an aromatic solvent (e.g., toluene), an hydrocarbon-based solvent (e.g., hexane), or a urea-based solvent, or a mixed solvent containing at least one of the above solvent systems may be used, and an amide-based solvent (e.g., dimethylformamide, dimethylacetamide) may be more preferably used.

In addition, the reaction in this step is preferably carried out at 0°C to 60°C, and more preferably at 30°C to 50°C.

### <Purification of the compound represented by Formula B-5>

The above compound represented by Formula B-5 may be purified by the following steps. The steps comprises conducting ring-opening of the phthalimide group in the compound represented by Formula B-5, followed by forming a salt with cinchonidine to give a crystalline compound represented by Formula B-6: 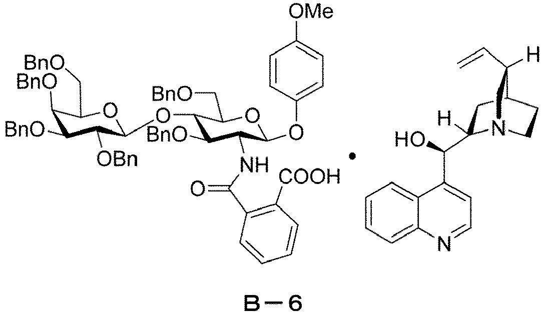 as a cinchonidine salt; separating the crystalline compound represented by Formula B-6 from non-crystalline materials; and then adding a solvent to remove the cinchonidine in the compound represented by Formula B-6 to give a compound represented by Formula B-7: 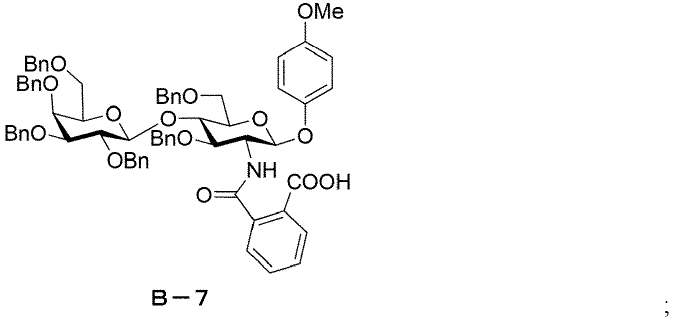 and then conducting ring-closing of the ring-opened phthalimide group in the compound represented by Formula B-7, to give the compound represented by Formula B-5. The compound represented by Formula B-6 (a cinchonidine salt of the compound represented by Formula B-7) is a crystalline compound, whereas the above compounds represented by formulas B-3, B-4, and B-5 are non-crystalline compounds. Thus, the compound represented by Formula B-5 can be crystallized by first conducting ring-opening of the phthalimide in the compound represented by Formula B-5, followed by forming a salt between the resulting carboxylic acid moiety in the phthalimide group and cinchonidine. Subsequently, the crystalline substance is separated from non-crystalline materials; then cinchonidine in the above compound represented by Formula B-6 is removed by, for example, adding an acidic solution and a solvent; and then the ring-closing of the phthalimide is conducted again, to obtain the compound represented by Formula B-5 with high-purity. The ring-opening and ring-closing of the phthalimide can be conducted using known methods. The ring-opening of the phthalimide can be conducted, for example, by adding sodium hydroxide in methanol-tetrahydrofuran. The ring-closing of the phthalimide can be conducted, for example, by adding carbonyldiimidazole (CDI) in a tetrahydrofuran solvent. This step may be preferably performed by, for example, the procedure shown in Examples 6 and 7.

### <Step Y-2-2>

Step Y-2-2 is a step of removing the 4-methoxyphenyl group from the compound represented by Formula B-5 to give a compound represented by Formula B-8:

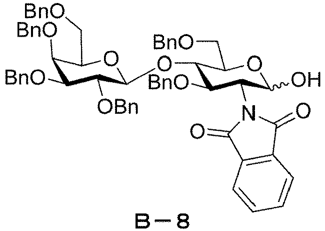

In one embodiment, Step Y-2-2 is a step of reacting the compound represented by Formula B-5 with λ³-iodane in fluorous alcohol and water to remove the 4-methoxyphenyl group to give the above compound represented by Formula B-8, and may be preferably performed, for example, by the procedure shown in Example 8. This step can be performed according to Step I-1-2 above, and the fluorous alcohol and λ3-iodane used in the step can be the same as those used in Step I-1-2 above.

### <Step Y-2-3>

Step Y-2-3 is a step of producing the above compound represented by Formula A-1 1 from the above compound represented by Formula B-8.

In an embodiment of the present invention, Step Y-2-3 is a step of reacting the above compound represented by Formula B-8 with 2,2,2-trifluoro-N-phenylacetimidoyl chloride (TFPC) in the presence of N-methylimidazole to give the above compound represented by Formula A-11, and may be preferably performed, for example, by the procedure shown in Example 9. As described for substantially the same reaction in Step I-1-3, the amount of TFPC can be reduced by using the above N-methylimidazole as the base, as compared to the case where, for example, potassium carbonate is used, and the product of interest can be obtained in high yield. The solvent and reaction temperature to be used, the feature that the reaction is preferably carried out in the presence of a dehydrating agent, and the feature that the product may be isolated and purified by column chromatography, etc., are the same as those in Step I-1-3 above.

### <Purification of the compound represented by A-12>

In Step I-3-1 above, the compound represented by Formula A-12 can be obtained in a purified form by the following method for purification. The method for purification comprises purifying the above compound represented by Formula A-12 by
after stopping the reaction of the compound represented by Formula A-10 with the compound represented by Formula A-11, adding a hydrophobic carrier and water to a water-soluble organic solvent containing the above resulting compound represented by Formula A-12 and contaminants to adsorb the above compound represented by Formula A-12 onto the hydrophobic carrier;
then filtering the resultant and washing the hydrophobic carrier with a mixture of the above water-soluble organic solvent and water to remove the contaminants;
and then eluting the above compound represented by Formula A-12 from the hydrophobic carrier using an organic solvent. As described in the method of purifying the above compound represented by Formula A-5, the above method for purification using a small amount of hydrophobic carrier makes it possible to produce a large amount of high-quality oligosaccharide efficiently in the liquid-phase synthesis of oligosaccharide chains.

The purification of the above compound represented by Formula A-12 is not limited to the purification in Step I-3-1. Accordingly, in an embodiment of the present invention, also provided is a method comprising the following:
adding a hydrophobic carrier and water to a water-soluble organic solvent containing the above compound represented by Formula A-12 and contaminants to adsorb the above compound represented by Formula A-12 onto the hydrophobic carrier;
then filtering the resultant and washing the hydrophobic carrier with a mixture of the above soluble organic solvent and water to remove the contaminants;
and then eluting the above compound represented by Formula A-12 from the hydrophobic carrier using an organic solvent to purify the above compound represented by Formula A-12.

The above term "contaminants" refers to compounds and/or reagents other than the protected oligosaccharide (in this step, the compound represented by Formula A-12), and mainly means reagents used in a reaction of synthesizing a protected oligosaccharide and remnants of the reagents, sugars other than the protected oligosaccharide, such as mono- or disaccharide compounds used in the elongation reaction of the protected oligosaccharide, or byproducts generated by the deprotection reaction of the protected oligosaccharide.

The "hydrophobic carrier" (e.g., a resin used for packing reversed-phase partition chromatography), "water-soluble organic solvent," "organic solvent," and the temperature for the purification used in the above step are substantially the same as those described in the method of purifying the above compound represented by Formula A-5.

### <Step I-3-2>

Step I-3-2 is a step of producing the oligosaccharide represented by Formula A-13 from the compound represented by Formula A-12.

In an embodiment of the present invention, Step I-3-2 is a step of reacting the compound represented by Formula A-12 with DDQ (2,3-dichloro-5,6-dicyano-p-benzoquinone) in a mixed solvent of fluorous alcohol and water to remove the 2-naphthylmethyl group in the above compound represented by Formula A-12 (de-2-naphthylmethylation reaction) to give the above oligosaccharide represented by Formula A-13, and may be performed, for example, by the procedure shown in Example 28-1.

For the above-mentioned de-2-naphthylmethylation reaction, the present inventors have found that the reaction (action) of 2,3-dichloro-5,6-dicyano-p-benzoquinone with a substrate having a 2-naphthylmethyl group attached via an oxygen atom in fluorous alcohol and water allows the reaction to be carried out under a mild condition in favorable stirring properties; and the de-2-naphthylmethylated product can be obtained in high yield. The advantages of the de-2-naphthylmethylation reaction are explained in more detail below. In the above-mentioned de-2-naphthylmethylation reaction in the present invention, the de-2-naphthylmethylated product can be obtained from a substrate, such as a sugar to which a 2-naphthylmethyl group is attached via an oxygen atom, in high yield under a mild condition. The reaction can be carried out reproducibly without any deterioration in stirring properties or adhesion to the vessel wall due to the byproduct 2,3-dichloro-5,6-dicyano-p-benzohydroquinone, and is suitable for mass synthesis of such a product. Due to the abnormally decreased freezing point of HFIP-H₂O, no coagulation of the solvent was observed even when the reaction temperature was lowered to -30°C, and thus a wide range of the temperature is applicable depending on the reactivity of the reaction substrate (melting point of HFIP: -3.3°C, H₂O: 0°C). It was found that when the denaphthylmethylation reaction of the compound represented by Formula A-12 having many benzyl groups is carried out, DDQ is used as an oxidant and HFIP-H₂O is used as a solvent, so that the reaction proceeds with superior selectivity as compared to conventional conditions. In most of the reported cases for the above conversion reaction, the reaction condition of dichloromethane-water-based bilayer system are used for the conversion reaction, and in that case, debenzylation of a plurality of benzyl groups proceeds at a constant rate with moderate yield. In addition, there are reported examples of improved conditions using β-pinene as an additive (see, for example, J. Org. Chem., 2017,82,3926), but the yield remains moderate for the compounds with a plurality of Bn groups (see, for example, Angew. Chem. Int. Ed. 2021,60,19287). Further, the selectivity especially for a substrate with 10 or more benzyl groups, such as the compound represented by Formula A-12, has not been well understood. There is a problem that DDQ and DDQ-derived byproducts deteriorate stirring properties in a dichloromethane-water system, which is not a suitable reaction condition for mass synthesis. In contrast, the DDQ/HFIP-H₂O system in the present method achieved a high selectivity of 85% or more for the compound represented by Formula A-12 having 15 benzyl groups. This method does not show any deterioration of stirring properties due to DDQ as described above. Further, due to an abnormally decreased freezing point of HFIP-H₂O, no coagulation of the solvent was observed even when the reaction temperature was lowered to -30°C, and therefore a wide range of the temperature is applicable depending on the reactivity of the reaction substrate (melting point of HFIP: -3.3°C, H₂O: 0°C).

The above de-2-naphthylmethylation reaction is not limited to the reaction in Step I-3-1. Thus, in an aspect of the present invention, also provided is a method for producing the above oligosaccharide represented by Formula A-13, wherein the method comprises a step of reacting the compound represented by Formula A-12 with DDQ (2,3-dichloro-5,6-dicyano-p-benzoquinone) in a mixed solvent of fluorous alcohol and water to remove the 2-naphthylmethyl group in the compound represented by Formula A-12.

The above "fluorous alcohol" is not limited as long as the reaction proceeds, and is preferably selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and a combination thereof.

The above de-2-naphthylmethylation reaction is not limited as long as the reaction proceeds, but is preferably carried out at -35°C to 70°C, and more preferably at -30°C to - 10°C.

The above production method provides an oligosaccharide represented by Formula A-13: 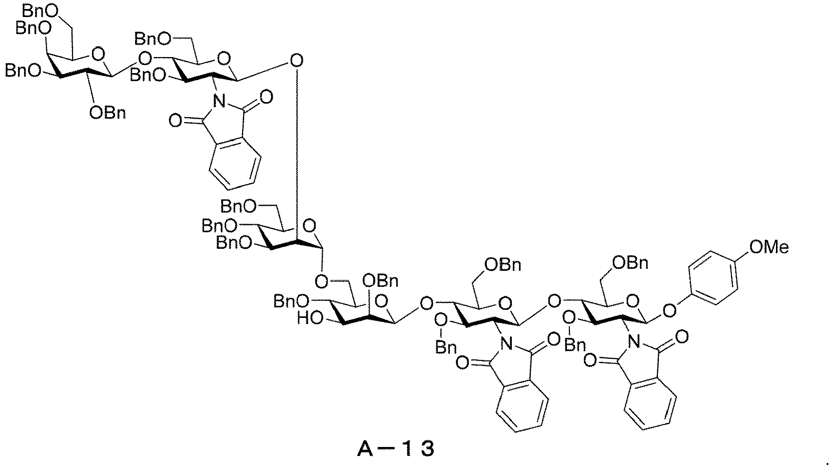 Examples of the oligosaccharide represented by Formula A-13 include those having similar protective groups such as chlorobenzyl groups instead of benzyl groups in the oligosaccharide represented by Formula A-13, as long as they have substantially the same functions or actions as those of the oligosaccharide, or a modified product thereof.

### <Purification of the compound represented by Formula A-13>

The above compound represented by Formula A-13 may be purified by the following step. In this step, the ring-opening of the phthalimide group in the compound represented by Formula A-13 is conducted, followed by forming a salt with (R)-(+)-1-(1-naphthyl)ethylamine, to give a crystalline compound represented by Formula A-14: 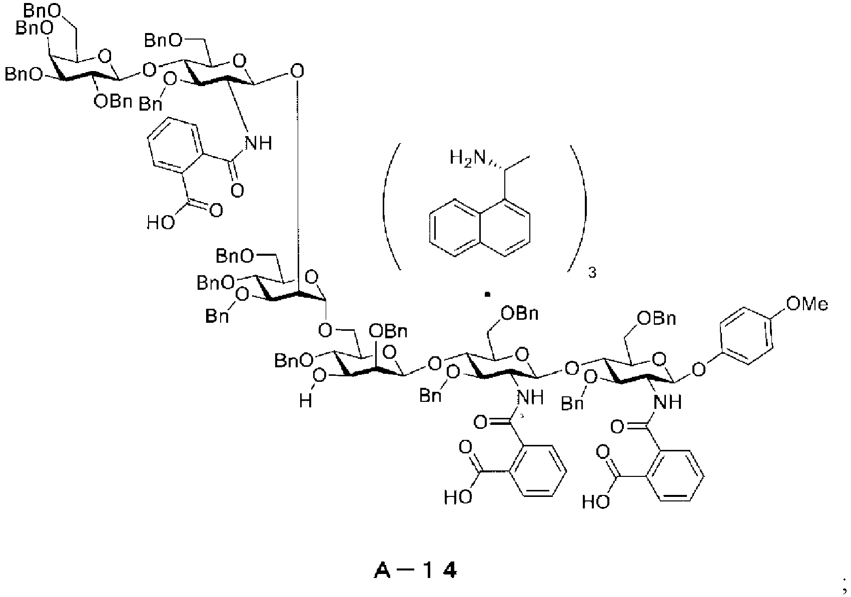 and then crystalline compound represented by Formula A-14 is separated from non-crystalline materials, followed by adding an acid aqueous solution and solvent, to remove the (R)-(+)-1-(1-naphthyl)ethylamine in the compound represented by Formula A-14, so as to produce a compound represented by Formula A-15: 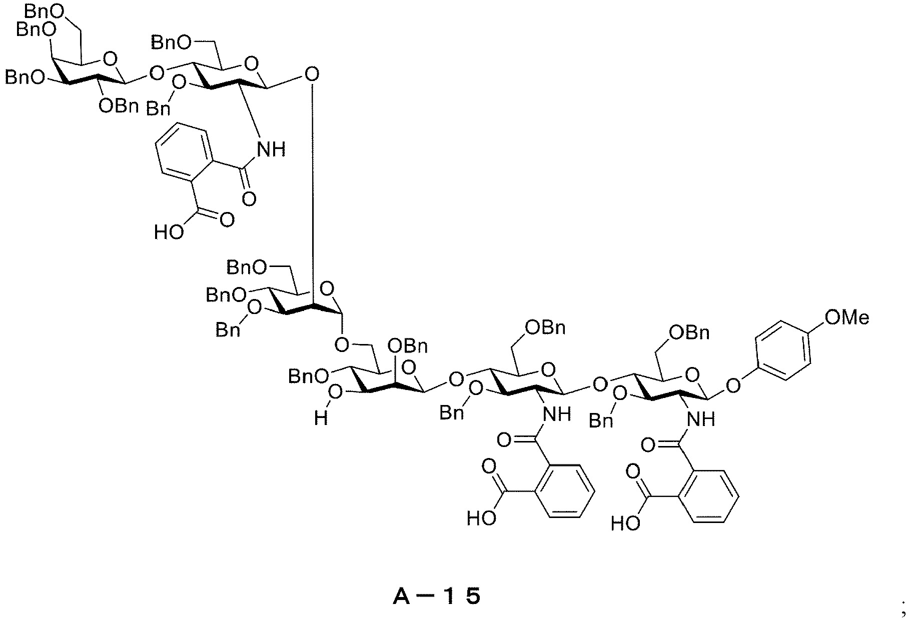 then ring-closing of the ring-opened phthalimide group in the compound represented by Formula A-15 is conducted to give the compound represented by Formula A-13. The compound represented by Formula A-14 (a (R)-(+)-1-(1-naphthyl)ethylamine salt of the compound represented by Formula A-15) is a crystalline compound, whereas the above compound represented by Formula A-13 is a non-crystalline compound. Thus, the resulting crystalline substance can be separated from non-crystalline materials by first conducting ring-opening of the phthalimide in the compound represented by Formula A-13, followed by forming a salt between the carboxylic acid moiety in the resulting phthalimide group and (R)-(+)-1-(1-naphthyl)ethylamine, and the (R)-(+)-1-(1-naphthyl)ethylamine in the compound represented by Formula A-14 is then removed by adding an acid aqueous solution and solvent to produce the compound represented by Formula A-15, followed by conducting ring-closing of the phthalimide again to give the compound represented by Formula A-13 with high-purity. The ring-opening and ring-closing of phthalimide can be conducted using known methods. The ring-opening of the phthalimide can be conducted, for example, by adding sodium hydroxide in methanol-tetrahydrofuran. The ring-closing of the phthalimide can be conducted, for example, by adding carbonyldiimidazole (CDI) in a tetrahydrofuran solvent. This step may be preferably performed by, for example, the procedure shown in Example 28-2.

### <2. Method for producing the oligosaccharide represented by Formula D-13>

In an embodiment of the present invention, provided is a novel oligosaccharide represented by Formula D-13 and a novel method for production thereof. In the present invention, the oligosaccharide represented by Formula D-13 means the following oligosaccharide.

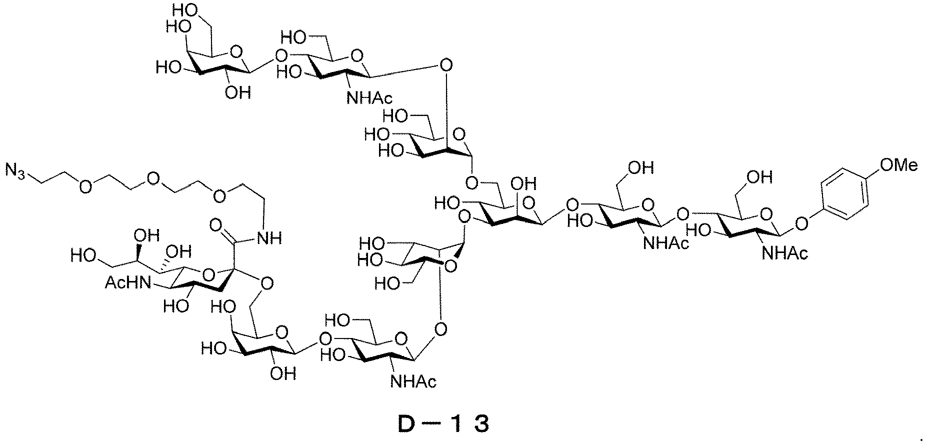

The new synthetic scheme for the above oligosaccharide represented by Formula D-13 comprises the following Steps II-1 to II-4.

### <Step II-1>

Step I-1 is a step of producing a compound represented by Formula D-2: 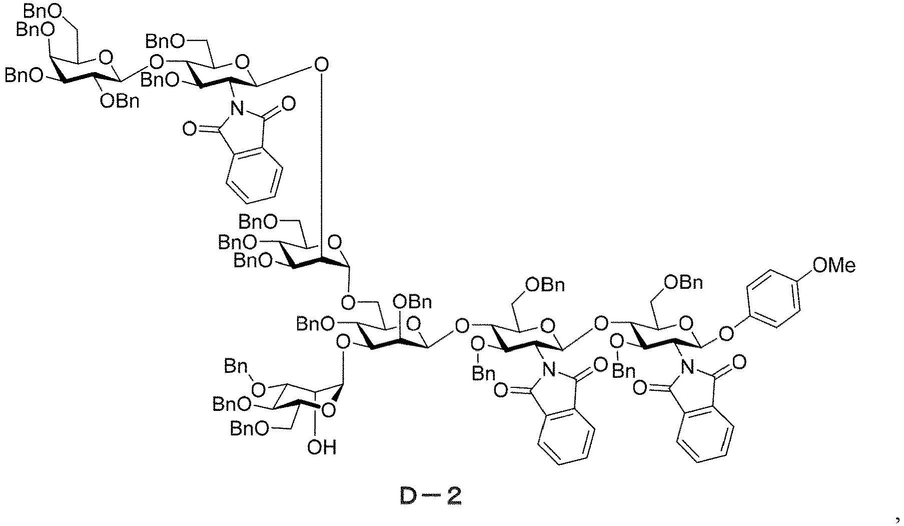 wherein the step comprising a step of:
connecting an oligosaccharide represented by Formula A-13: 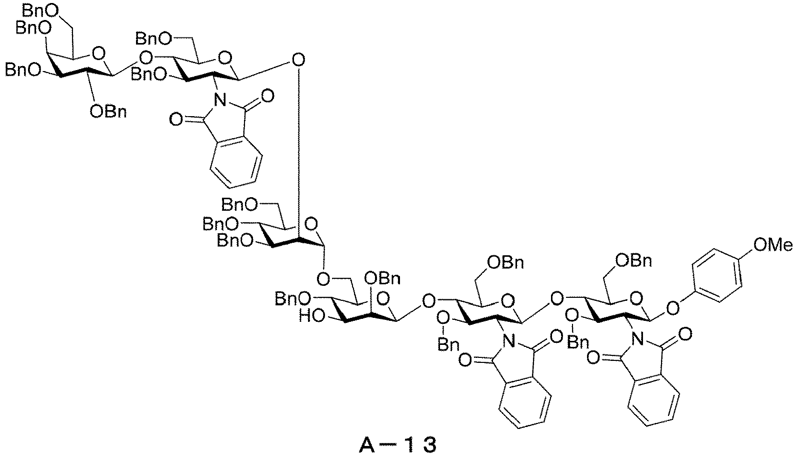 to a compound represented by Formula A-3: 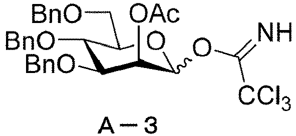 via α-1,3-glycosidic linkage to give a compound represented by Formula D-1: 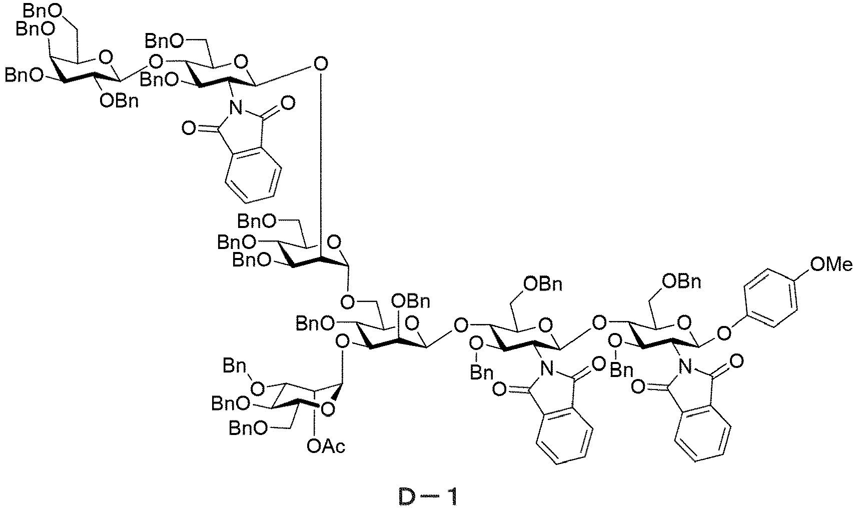 In an embodiment of the present invention, Step II-1 comprises the following Steps II-1-1 to II-1-2.

### <Step II-1-1>

Step II-1-1 is a step of connecting the above compound represented by Formula A-13 to the above compound represented by Formula A-3 via α-1,3-glycosidic linkage to produce the compound represented by Formula D-1. This step may be performed by using or adapting a known method, and may be preferably performed, for example, by the procedure shown in Example 52. For example, the compound represented by Formula A-13 may be bound to the above compound represented by Formula A-3 via α-1,3-glycosidic linkage, by sequentially adding Molecular Sieves 4A powder and trimethylsilyl trifluoromethanesulfonate (TMSOTf) in an organic solvent (e.g., toluene), to give the above compound represented by Formula D-1.

### <Purification of the compound represented by Formula D-1>

In Step II-1-1, the compound represented by Formula D-1 can be obtained in a purified form by the following method for purification. The method for purification comprises purifying the above compound represented by Formula A-9 by
after stopping the reaction of the above compound represented by Formula A-13 with the compound represented by Formula A-3, adding a hydrophobic carrier and water to a water-soluble organic solvent containing the above resulting compound represented by Formula D-1 and contaminants to adsorb the above compound represented by Formula D-1 onto the hydrophobic carrier;
then filtering the resultant and washing the hydrophobic carrier with a mixture of the above water-soluble organic solvent and water to remove the contaminants;
and then eluting the above compound represented by Formula D-1 from the hydrophobic carrier using an organic solvent to purify the above compound represented by Formula D-1. As described in the method of purifying the above compound represented by Formula A-5 in Step I-1-1, this method for purification makes it possible to produce a large amount of high-quality oligosaccharide efficiently in the liquid-phase synthesis of oligosaccharide chains by using a small amount of hydrophobic carrier.

Purification of the above compound represented by Formula D-1 is not limited to the purification in this step. Accordingly, in an embodiment of the present invention, also provided is a method comprising the following:
adding a hydrophobic carrier and water to a water-soluble organic solvent containing the above compound represented by Formula D-1 and contaminants to adsorb the above compound represented by Formula D-1 onto the hydrophobic carrier;
then filtering the resultant and washing the hydrophobic carrier with a mixture of the above water-soluble organic solvent and water to remove the contaminants;
and then eluting the above compound represented by Formula D-1 from the hydrophobic carrier using an organic solvent to purify the above compound represented by Formula D-1.

The above term "contaminants" refers to compounds and/or reagents other than the protected oligosaccharide (in this step, the compound represented by Formula D-1), and mainly means reagents used in a reaction of synthesizing a protected oligosaccharide and remnants of the reagents, sugars other than the protected oligosaccharide, such as mono- or disaccharide compounds used in the elongation reaction of the protected oligosaccharide, or byproducts generated by the deprotection reaction of the protected oligosaccharide. The "hydrophobic carrier" (e.g., a resin used for packing reversed-phase partition chromatography), "water-soluble organic solvent," "organic solvent," and the temperature for the purification used in this step are substantially the same as those described in the method of purifying the compound represented by Formula A-5 in Step I-1-1 above.

### <Step II-1-2>

Step II-1-2 is a step of removing the acetyl group from the compound represented by Formula D-1 to give the above compound represented by Formula D-2. This step may be performed by using or adapting a known method, and may be preferably performed, for example, by the procedure shown in Example 53.

In an embodiment of the present invention, Step II-1-2 is a step of reacting the above compound represented by Formula D-1 with a strong base in the presence of an alkyl ester of perfluorocarboxylic acid to remove the acetyl group to give the compound represented by Formula D-2. The deacetylation reaction can be carried out in the same way as in the deacetylation reaction described in the above Step X-7, except that the substrate is different. The deacetylation reaction can be carried out while suppressing the ring-opening of the phthalimide group by the above method using the approach in which the compound is reacted with a strong base in the presence of an alkyl ester of perfluorocarboxylic acid.

The above deacetylation reaction is not limited to use in Step II-1-2. Thus, in an embodiment of the present invention, also provided is a method for producing the above compound represented by Formula D-2, wherein the method comprises a step of reacting the above compound represented by Formula D-1 with a strong base in the presence of an alkyl ester of perfluorocarboxylic acid.

### <Step II-2>

Step II-2 is a step comprising a step of producing a compound represented by Formula D-5: 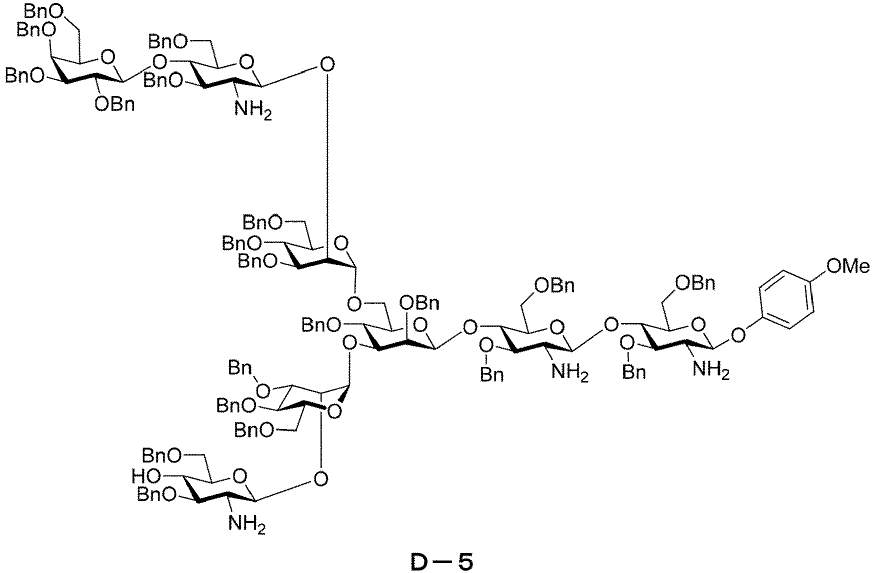 wherein the step comprises a step of:
connecting the above compound represented by Formula D-2 to the compound represented by Formula D-3: 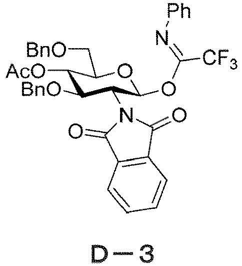 via β-1,2-glycosidic linkage to give a compound represented by Formula D-4: 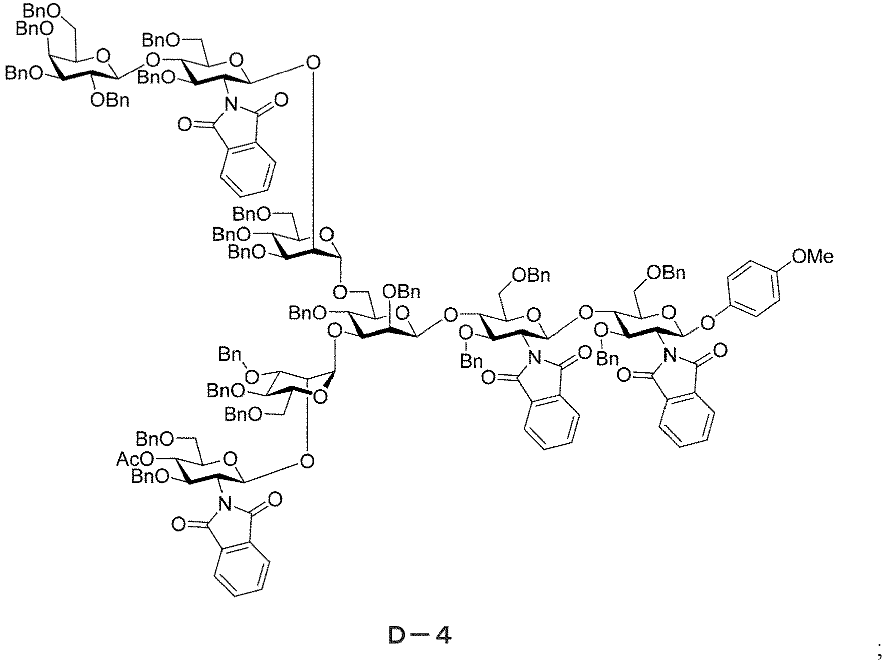 and then protecting the amino group in the compound represented by Formula D-5 with a protecting group selected from an aryloxycarbonyl (COOAr) group, an acetyl (Ac) group, a 2,2,2-trichloroethoxycarbonyl (Troc) group, or a phthalimide (Pht) group to give a compound represented by Formula D-6: 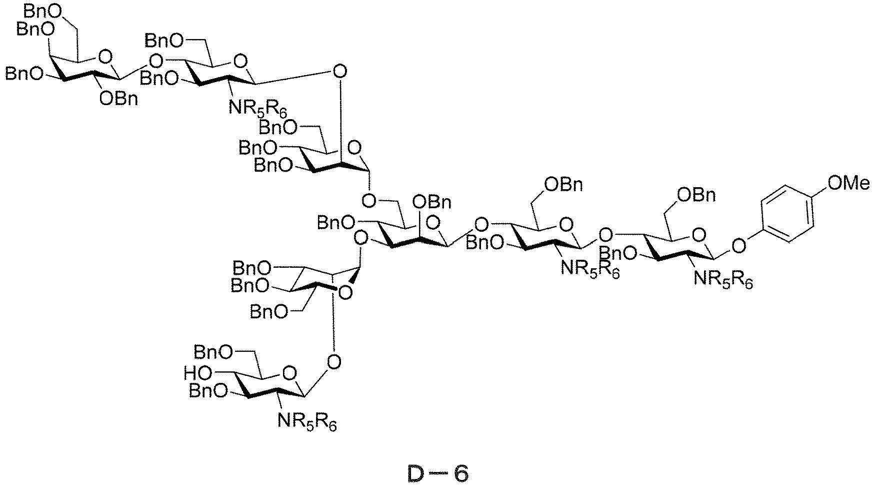 (wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with the nitrogen atom attached thereto form a phthalimide group), or
a step of removing the acetyl group from the above compound represented by Formula D-4 to give the above compound represented by Formula D-6 (wherein R₅ and R₆ together with the nitrogen atom attached thereto form a phthalimide group). In an embodiment of the present invention, Step II-2 comprises the following Steps II-2-1 to II-2-3.

### <Step II-2-1>

Step II-2-1 is a step of connecting the above compound represented by Formula D-2 to the above compound represented by Formula D-3 via β-1,2-glycosidic linkage to give the above compound represented by Formula D-4. This step of connecting via glycosidic linkage may be performed by using or adapting a known method, and may be preferably performed, for example, by the procedure shown in Example 54. For example, the compound represented by Formula D-2 may be bound to the above compound represented by Formula D-3 via β-1,2-glycosidic linkage, by sequentially adding Molecular Sieves 4A powder and trimethylsilyl trifluoromethanesulfonate (TMSOTf) in an organic solvent (e.g., toluene) to give the above compound represented by Formula D-4.

### <Production of the compound represented by Formula D-3>

The above compound represented by Formula D-3 may be produced as described in the following substeps Z-1 to Z-3.

### <Substep Z-1>

First, the hydroxyl group on the compound represented by Formula A-8: 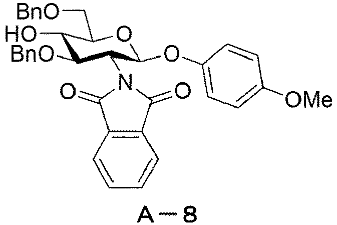 used in Step Y-1 above is protected with an acetyl group to give a compound represented by Formula F-1: 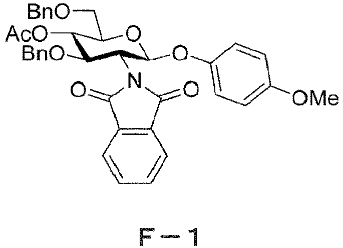 This step may be performed by using or adapting a known method, and may be preferably performed, for example, by the procedure shown in Example 34. This step can be performed by adding triethylamine, dimethylaminopyridine, and acetic anhydride to an ethyl acetate solution containing the compound represented by Formula A-8, but is not limited to such a procedure.

### <Substep Z-2>

Next, the 4-methoxyphenyl group from the compound represented by Formula F-1 is remove d to give a compound represented by Formula F-2:

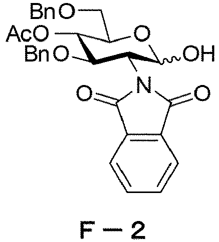

In one embodiment of the present invention, Substep Z-2 is a step of reacting the compound represented by Formula F-1 with λ3-iodane in fluorous alcohol and water to remove the 4-methoxyphenyl group to produce the compound represented by Formula F-2, and may be preferably performed, for example, by the procedure shown in Example 35. This step can be performed according to Step I-1-2 above. The fluorous alcohol and λ3-iodane used in the step can be the same as those used in Step 1-1-2 above.

### <Substep Z-3>

Then, in this step, the compound represented by Formula F-2 is reacted with 2,2,2-trifluoro-N-phenylacetimidoyl chloride (TFPC) to produce a compound represented by Formula D-3: 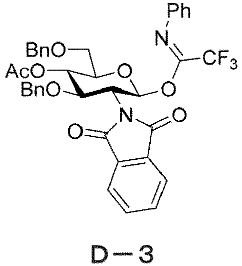 This step may be performed by using or adapting a known procedure.

In an embodiment of the present invention, Substep Z-3 is a step of reacting with 2,2,2-trifluoro-N-phenylacetimidoyl chloride (TFPC) in the presence of N-methylimidazole to give the above compound represented by Formula D-3, and may be preferably performed, for example, by the procedure shown in Example 36. As described for substantially the same reaction in Step I-1-3, the amount of TFPC can be reduced by using the above N-methylimidazole as the base as compared to the case of using potassium carbonate, etc., and the product of interest can be obtained in high yield. The solvent and reaction temperature to be used, the feature that the reaction is preferably carried out in the presence of a dehydrating agent, and the feature that the product may be isolated and purified by purification on column, etc., are the same as those in Step I-1-3 above.

### <Step II-2-2>

Step II-2-2 is a step of removing each phthalimide group as a protecting group of amino group on the above compound represented by Formula D-4 to give the compound represented by Formula D-5. This step may be preferably performed by, for example, the procedure shown in Example 55-1. For example, this step can be performed by adding n-butanol and ethylenediamine to a solution containing the compound represented by Formula D-4, but is not limited to such a procedure.

### <Purification of the compound represented by Formula D-5 (1)>

In Step II-2-2, the compound represented by Formula D-5 can be obtained in a purified form by the following method for purification. The method for purification comprises purifying the above compound represented by Formula D-5 by
after stopping the reaction of the above compound represented by Formula D-3 with the compound represented by Formula D-4, adding a hydrophobic carrier and water to a water-soluble organic solvent containing the above resulting compound represented by Formula D-5 and contaminants to adsorb the above compound represented by Formula D-5 onto the hydrophobic carrier;
then filtering the resultant and washing the hydrophobic carrier with a mixture of the above water-soluble organic solvent and water to remove the contaminants;
and then eluting the above compound represented by Formula D-5 from the hydrophobic carrier using an organic solvent. As described in the method of purifying the above compound represented by Formula A-5 in Step I-1-1, this method for purification makes it possible to produce a large amount of high-quality oligosaccharide efficiently in the liquid-phase synthesis of oligosaccharide chains by using a small amount of hydrophobic carrier.

Purification of the above compound represented by Formula D-5 is not limited to the purification in this step. Accordingly, in an embodiment of the present invention, also provided is a method comprising the following:
adding a hydrophobic carrier and water to a water-soluble organic solvent containing the above compound represented by Formula D-5 and contaminants to adsorb the above compound represented by Formula D-5 onto the hydrophobic carrier;
then filtering the resultant and washing the hydrophobic carrier with a mixture of the above water-soluble organic solvent and water to remove the contaminants;
and then eluting the above compound represented by Formula D-5 from the hydrophobic carrier using an organic solvent to purify the above compound represented by Formula D-5.

The above term "contaminants" refers to compounds and/or reagents other than the protected oligosaccharide (in this step, the compound represented by Formula D-5), and mainly means reagents used in a reaction of synthesizing a protected oligosaccharide and remnants of the reagents, sugars other than the protected oligosaccharide, such as mono- or disaccharide compounds used in the elongation reaction of the protected oligosaccharide, or byproducts generated by the deprotection reaction of the protected oligosaccharide. The "hydrophobic carrier" (e.g., a resin used for packing reversed-phase partition chromatography), "water-soluble organic solvent," "organic solvent," and the temperature for the purification used in this step are substantially the same as those described in the method of purifying the above compound represented by Formula A-5 in Step I-1-1.

### <Purification of the compound represented by Formula D-5 (2)>

The compound represented by Formula D-5 above may be purified by the following step. The purification may be performed separately from or in addition to the above purification (1) of the compound represented by Formula D-5. In this step, the compound represented by Formula D-5 is first reacted with fumaric acid to give a crystalline fumarate compound represented by Formula D-5-FMA: 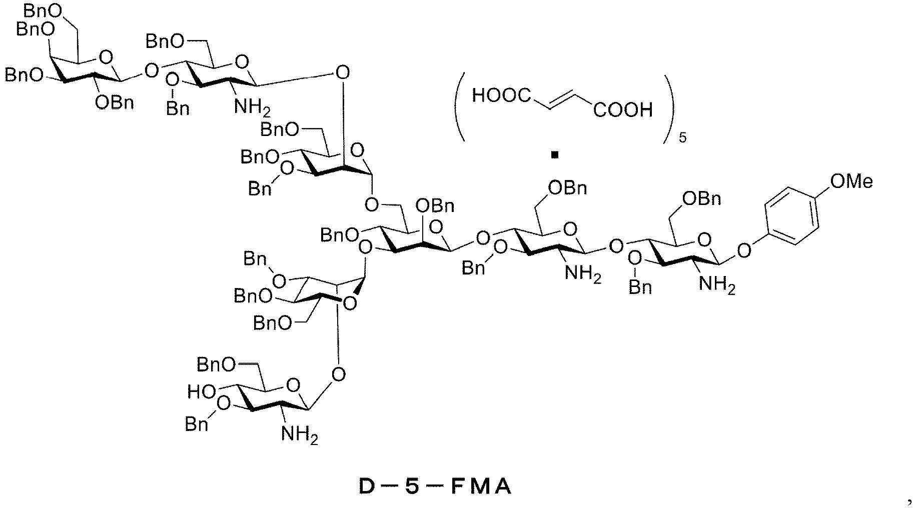 and then the crystalline compound represented by Formula D-5-FMA can be separated from non-crystalline materials. The compound represented by Formula D-5-FMA dissolved in a solvent may be used in the next Step II-2-3, or may be converted to the compound represented by Formula D-5. The conversion to the compound shown in Formula D-5 may be carried out by adding a basic aqueous solution and a solvent, etc., to remove the fumaric acid from the compound represented by Formula D-5-FMA into an aqueous layer, followed by concentrating the resulting organic layer, and as a result, the high-purity compound represented by Formula D-5 can be obtained. Impurities having similar structure such as stereoisomers, which are difficult to be removed even by purification on column, can be easily removed by the above operations. This step may be preferably performed by, for example, the procedure shown in Example 55-2.

### <Step II-2-3>

Step II-2-3 is a step of protecting each amino group in the above compound represented by Formula D-5 with a protecting group selected from an aryloxycarbonyl (COOAr) group, an acetyl (Ac) group, a 2,2,2-trichloroethoxycarbonyl (Troc) group, or a phthalimide (Pht) group to give the above compound represented by Formula D-6 (wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with the nitrogen atom attached thereto form a phthalimide group). The objective of introducing the above protecting group of the amino group is as follows: in order to produce the compound of interest (the compound represented by Formula D-13), it is more efficient to use an acetyl group as the protecting group of the amino group, since it is a linear route; however, in the glycosylation reaction of the compound represented by Formula D-6 and the compound represented by Formula D-7 in next Step II-3, if an amino group (-NHAc group) protected with an acetyl group is present in the reaction substrate, the interaction with Lewis acid causes a remarkable decrease in the reactivity of the glycosylation reaction of interest, and thus an excess amount of glycosyl donor is often required to complete the reaction. Accordingly, during the above glycosylation reaction, it is temporarily protected with a protecting group selected from an aryloxycarbonyl (COOAr) group, a 2,2,2-trichloroethoxycarbonyl (Troc) group, or a phthalimide (Pht) group on the nitrogen of the glucosamine, and the deprotection is carried out after the glycosylation reaction to form a -NHAc group, so that the above disadvantages can be avoided. It is most preferable to use the aryloxycarbonyl (COOAr) group as the protective group. The "aryl (Ar) group" in the aryloxycarbonyl means a group formed by removing one hydrogen atom on an aromatic ring in an aromatic hydrocarbon, and includes, for example, but are not limited to, a phenyl, 2-naphthyl, 1-naphthyl, 2-pyridyl, 3-pyridyl, nitrophenyl, chlorophenyl, fluorophenyl, bromophenyl, iodophenyl, methoxyphenyl, or C1-C4 alkylphenyl group, and preferably a phenyl group. It has been found that the glycosylation reaction proceeds better with the aryloxycarbonyl (COOAr) group than with other protecting groups, and in the subsequent deprotection reaction, the deprotection can be carried out under a suitable condition, e.g., at room temperature and within 1 hour under a typical hydrolysis condition.

The above step may be preferably performed by, for example, the procedure shown in Examples 56 to 59. For example, this step can be performed by adding an aqueous solution containing tetrahydrofuran and sodium bicarbonate, potassium bicarbonate, disodium hydrogen phosphate, or dipotassium hydrogen phosphate dissolved in water to a tetrahydrofuran solution containing the compound represented by Formula D-5, but is not limited to such a procedure.

Instead of Steps II-2-2 and II-2-3 above, the acetyl (Ac) group on the compound represented by Formula D-4 may be selectively removed to produce the above compound represented by Formula D-6 (wherein R₅ and R₆ together with the nitrogen atom attached thereto form a phthalimide group). The selective removal of this acetyl group can be carried out under the condition of methyl trifluoroacetate, but is not limited to such a procedure. This step causes the same result as the case where the phthalimide (Pht) group is selected as the protecting group of the amino group in the above compound represented by Formula D-5 in Steps II-2-2 and II-2-3.

### <Step II-3>

Step II-3 is a step of producing a compound represented by Formula D-11: 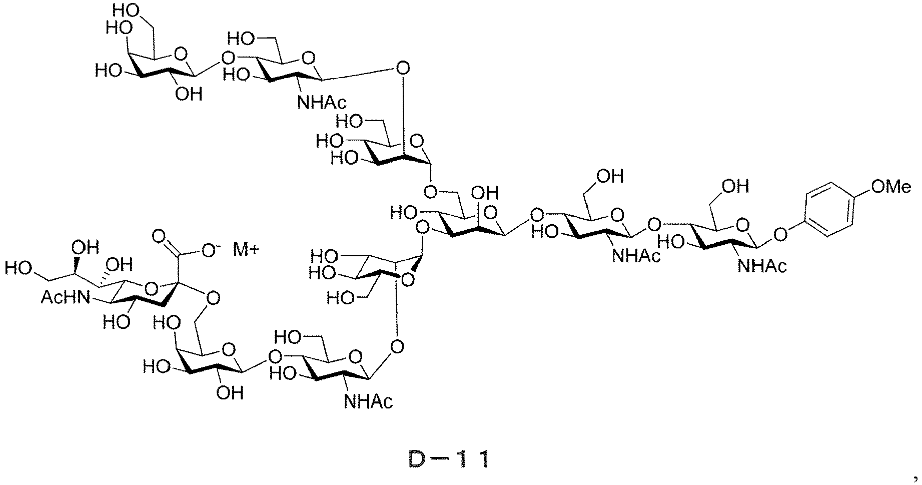 wherein the step comprising a step of
connecting the compound represented by Formula D-6 to a compound represented by Formula D-7: 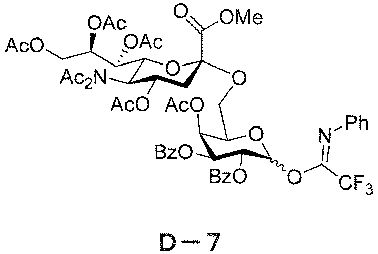 via β-1,4-glycosidic linkage to give a compound represented by Formula D-8: 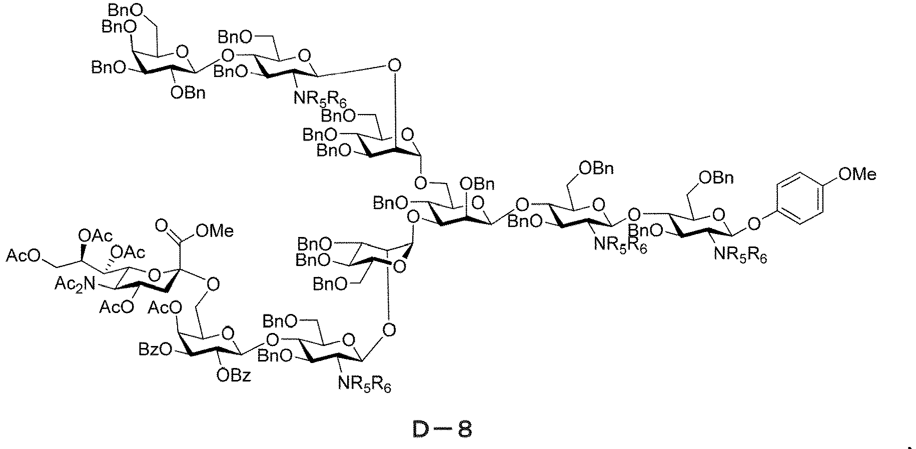
wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with the nitrogen atom attached thereto form a phthalimide group,
and then removing each amino group-protecting group in the compound represented by Formula D-8 to give a compound represented by Formula D-9: 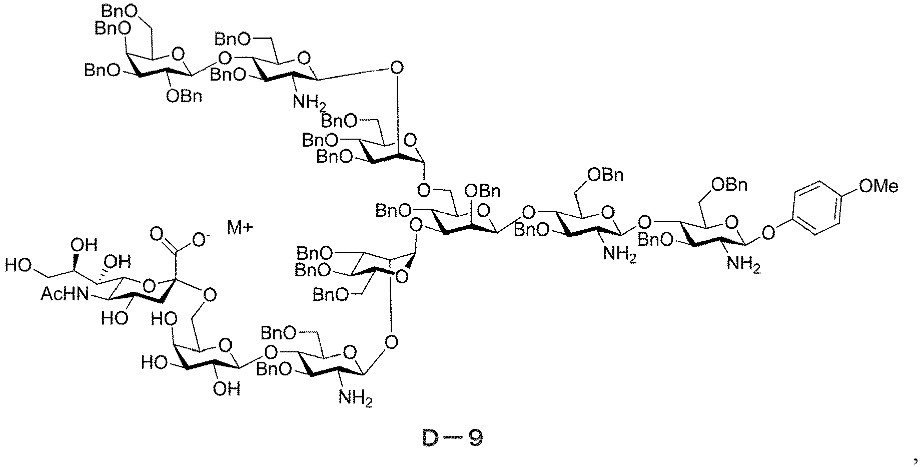
wherein M⁺ is a sodium ion, a lithium ion, a potassium ion, or a protonated triethylamine cation. In an embodiment of the present invention, Step II-3 comprises Steps II-3-1 to II-3-4 as follows.

### <Step II-3-1>

This step is a step of connecting the above compound represented by Formula D-6 to the compound represented by Formula D-7 via β-1,4-glycosidic linkage to give the compound represented by Formula D-8. The above step of connecting via glycosidic linkage may be performed by using or adapting a known method, and may be preferably performed, for example, by the procedure shown in Examples 60 to 63.

### <To produce the compound represented by Formula D-7>

In an embodiment of the present invention, the above compound represented by Formula D-7 may be produced as described in Substeps V-1 to V-11 as follows. This step comprises essential Substeps V-7 as described below, in which two molecules of monosaccharide are bound via α-2,6-glycosidic linkage to synthesize a disaccharide block. The other substeps can be performed by using or adapting usual procedures for producing a monosaccharide or oligosaccharide.

In an embodiment of the present invention, Step V comprises the following substeps:

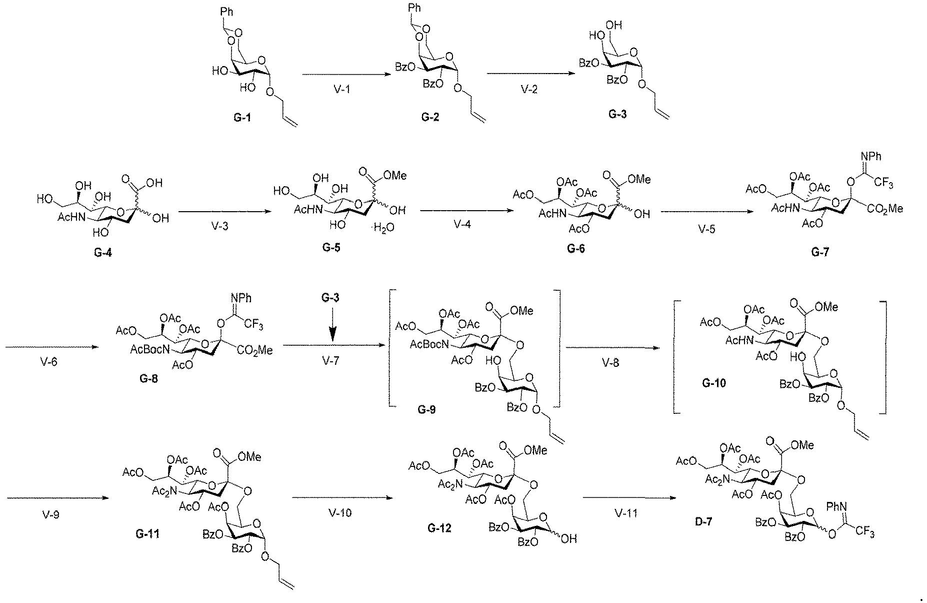

### <Substep V-1>

Substep V-1 is a step of protecting, with a benzoyl group, each hydroxyl group on the compound represented by Formula G-1: 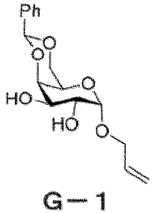 to produce a compound represented by Formula G-2: 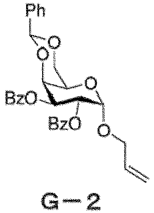 The compound represented by Formula G-1, which is the starting material for this step and is the compound specified as CAS No. 100759-10-2, may be produced by a known method, and may be produced by, for example, by the procedures shown in Examples 37 and 38. This step may be performed by using or adapting a known method, and may be preferably performed, for example, by the procedure shown in Example 39.

### <Substep V-2>

Substep V-2 is a step of removing the benzylidene protecting group from the compound represented by Formula G-2 to produce a compound represented by Formula G-3: 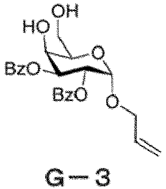 This step may be performed by using or adapting a known method, and may be preferably performed, for example, by the procedure shown in Example 40.

In an embodiment of the present invention, this step comprises a step of contacting a solvent in which the compound represented by Formula G-3 is dissolved with silica gel to conduct solid-phase extraction of the compound represented by Formula G-3. Since an unreacted compound represented by Formula G-2 and an removed benzaldehyde are not adsorbed on silica gel, the compound represented by Formula G-3 can be efficiently purified by this step.

The solvent used to dissolve the compound represented by Formula G-3 includes, but are not limited to, toluene, heptane, dichloromethane, chloroform, or a combination thereof, preferably toluene, dichloromethane, chloroform, or a combination thereof, and particularly preferably toluene.

As silica gel in this step, for example, silica gel in an amount 2 to 5 times relative to the amount of the raw material may be used; silica gel in an amount 2 to 4 times relative to the amount of the raw material may be preferably used; and silica gel in an amount about 3 times relative to the amount of the raw material may be more preferably used.

In this step, the solvent used to elute the compound represented by Formula G-3 adsorbed on silica gel is not limited as long as the solvent does not dissolve the silica gel and can elute the compound of interest. , and include, for example, cyclopentyl methyl ether, ethyl acetate, or tert-butyl methyl ether.

### <Substep V-3>

Substep V-3 is a step of conducting esterification of the carboxylic acid of the compound represented by Formula G-4: 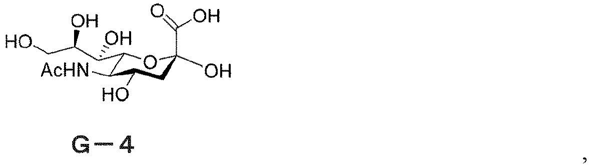 followed by adding water, to produce a compound represented by Formula G-5: 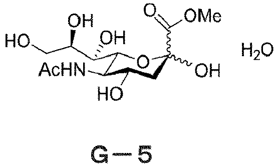 The compound represented by Formula G-4, which is the starting material for this step, may be produced by a known method, or a commercially available product thereof may be used. The commercially available compound represented by Formula G-4 includes N-acetylneuraminic acid, available by TOKYO CHEMICAL INDUSTRY CO., LTD. This step may be performed by using or adapting a known method, and may be preferably performed, for example, by the procedure shown in Example 41.

### <Substep V-4>

Substep V-4 is a step of protecting, with an acetyl group, each hydroxyl group other than the hydroxyl group attached to the carbon at position 1 in the compound represented by Formula G-5 to produce a compound represented by Formula G-6: 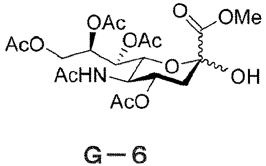 This step may be performed by using or adapting a known method, and may be preferably performed, for example, by the procedure shown in Example 42.

### <Substep V-5>

Substep V-5 is a step of reacting the compound represented by Formula G-6 with 2,2,2-trifluoro-N-phenylacetimidoyl chloride (TFPC) to produce a compound represented by Formula G-7: 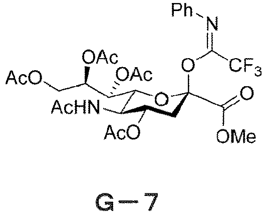 This step may be performed by using or adapting a known method, and may be preferably performed, for example, by the procedure shown in Example 43.

In an embodiment of the present invention, this step is a step of reacting the compound represented by Formula G-6 with TFPC in the presence of N-methylimidazole to produce the compound represented by Formula G-7. If N-methylimidazole is used as a base in this step, the amount of TFPC can be reduced, as compared to the case where K₂CO₃ is used, and even in that case, the product of interest can be obtained in high yield. Since TFPC is an expensive reagent, an increase in the yield in this step is very beneficial for commercial production.

The solvent in this step is not limited as long as the reaction proceeds, and includes, for example, dichloromethane, toluene, ethyl acetate, acetonitrile, or tetrahydrofuran, and preferably dichloromethane.

The reaction temperature in this step is not limited as long as the reaction proceeds, but may be preferably from 20°C to 40°C, more preferably from 10°C to 35°C, and particularly preferably from 0°C to 30°C.

This step is preferably performed in the presence of a dehydrating agent. The dehydrating agent in this step is not limited as long as the reaction proceeds, and includes, for example, Molecular Sieves, and preferably Molecular Sieves 4A powder with a powder particle size of 10 µm or less.

### <Substep V-6>

Substep V-6 is a step of protecting, with a tert-butoxycarbonyl group, the nitrogen atom of the acetamide group in the compound represented by Formula G-7 to produce a compound represented by Formula G-8: 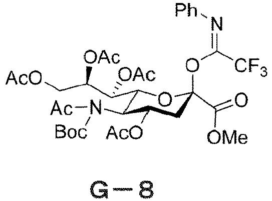 This step may be performed by using or adapting a known method, and may be preferably performed, for example, by the procedure shown in Example 44.

The compound represented by Formula G-8 that is produced in this step and dissolved in a solvent may be used in the next step or may be isolated and purified by recrystallization. The compound represented by Formula G-8 has a great advantage in that this compound can be isolated and purified by crystallization. The compound represented by Formula G-8 with HPLC purity of 99% or higher can be obtained without any impurities, and thus a glycosylation reaction in the next step can be carried out stably. The isolation and purification by recrystallization may be carried out by a method comprising adding heptane to a cyclopentyl methyl ether-containing solution to achieve crystallization.

### <Substep V-7>

Substep V-7 is a step of connecting the compound represented by Formula G-8 to the compound represented by Formula G-3 via α-2,6-glycosidic linkage to produce a compound represented by Formula G-9: 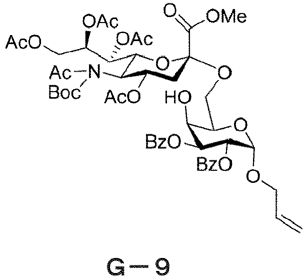 It is difficult to selectively connectan N-acetylneuraminic acid derivative to a galactose derivative via α-2,6-glycosidic linkage. For example, a method for synthesizing a disaccharide by reacting a compound represented by Formula G-7 with a compound represented by Formula G-3 has been reported (J. Org. Chem., 2016, 81, 10600-10616), but it was not easy to reproduce the reaction, and the desired yield and selectivity could not be achieved. Further, there are big problems in this reaction, i.e., the selectivity decreases as the scale increases; an acceptable range of the reaction temperature is narrow; and a large effect of heat caused in the reaction is caused. In addition, the compound represented by Formula G-7, which is one of the raw material compounds in this reaction, is very expensive, and there are also big problems of low reproducibility, yield, and selectivity in this reaction, especially for commercial production where scale-up is required. In contrast, if the compound represented by Formula G-8 with a tert-butoxycarbonyl group is used instead of the compound represented by Formula G-7 as a raw material compound, the high selectivity for connecting via α-2,6-glycosidic linkage (α : β = 93 : 7) can be well-reproduced with improved yield; further, the acceptable range of the temperature is wider, and high reproducibility, yield, and selectivity can be achieved even when it is scaled up, resulting in extremely beneficial effects on commercial production.

This step can suitably be performed in the presence of a Lewis acid. The Lewis acid in this step is not limited as long as the reaction proceeds, and includes, for example, trimethylsilyl trifluoromethanesulfonate, triisopropylsilyl trifluoromethanesulfonate, or tert-butyl dimethylsilyl trifluoromethanesulfonate, and preferably trimethylsilyl trifluoromethanesulfonate.

The solvent in this step is not limited as long as the reaction proceeds, and includes, for example, diisopropyl ether, tert-butyl methyl ether, diethyl ether, dibutyl ether, dipropyl ether, 1,4-dioxane, dichloromethane, 1,2-dichloroethane, toluene, chlorobenzene, trifluoromethylbenzene, propionitrile, or acetonitrile, and preferably cyclopentyl methyl ether.

The reaction temperature in this step is not limited as long as the reaction proceeds, but may be, for example, from -78°C to 0°C, preferably from -78°C to -20°C, more preferably from -78°C to -30°C, and particularly preferably from -78°C to -40°C.

In this step, it is preferable to use 1 to 3 equivalents of the compound represented by Formula G-3 relative to 1 equivalent of the compound represented by Formula G-8, and it is more preferable to use 1.4 to 2 equivalents of the compound represented by Formula G-3 relative to 1 equivalent of the compound represented by Formula G-8.

This step is not limited as long as the reaction proceeds, and may be performed by, for example, adding a mixed solution containing the compound represented by Formula G-8 and the compound represented by Formula G-3 (preferably, a solution containing cyclopentyl methyl ether mixed) dropwise over a long time to a Lewis acid-containing solution (preferably, cyclopentyl methyl ether solution), or by adding a solution containing the compound represented by Formula G-8 (preferably, a solution containing cyclopentyl methyl ether) dropwise over a long time to a solution containing a Lewis acid and the compound represented by Formula G-3 (preferably, a solution containing cyclopentyl methyl ether), preferably by adding a solution containing the compound represented by Formula G-8 (preferably, a solution containing cyclopentyl methyl ether) dropwise over a long time to a solution containing a Lewis acid and the compound represented by Formula G-3 (preferably, a solution containing cyclopentyl methyl ether). The time for the dropwise addition is not limited as long as the reaction proceeds, but may be, for example, from 30 minutes to 5 hours, preferably from 1 to 4 hours, more preferably from 2 to 3.5 hours, and particularly preferably about 3 hours.

In an embodiment of the present invention, this step comprises a step of contact a solvent dissolving the compound represented by Formula G-9 with silica gel to conduct solid-phase extraction of the compound represented by Formula G-9. N-phenyl trifluoroacetamide that is a byproduct of the glycosylation reaction as well as other trace impurities in the toluene solvent that are not adsorbed on silica gel are not adsorbed on silica gel, and thus the compound represented by Formula G-9 can be efficiently purified by this step.

The solvent used to dissolve the compound represented by Formula G-9 includes, but are not limited to, toluene, heptane, dichloromethane, chloroform, or a combination thereof, preferably toluene, dichloromethane, chloroform, or a combination thereof, and particularly preferably toluene.

As silica gel in this step, for example, silica gel in an amount 2 to 5 times relative to the amount of the raw material may be used; silica gel in an amount 2 to 4 times relative to the amount of the raw material may be preferably used; and silica gel in an amount about 3.5 times relative to the amount of the raw material may be more preferably used.

In this step, the solvent used to elute the compound represented by Formula G-9 adsorbed on silica gel is not limited as long as the silica gel is not dissolved in the solvent and the compound of interest can be eluted by the solvent, and includes, for example, ethyl acetate, cyclopentyl methyl ether, or tert-butyl methyl ether, and preferably ethyl acetate.

This step may be performed by, for example, the procedure shown in Example 45.

### <Substep V-8>

Substep V-8 is a step of removing the tert-butoxycarbonyl group from the compound represented by Formula G-9 to produce a compound represented by Formula G-10: 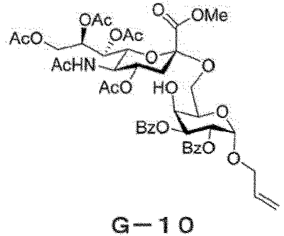 This step may be performed by using or adapting a known method, and may be preferably performed, for example, by the procedure shown in Example 46.

### <Substep V-9>

Substep V-9 is a step of further protecting, with acetyl groups, the hydroxyl group and the nitrogen atom of the acetamide group in the compound represented by Formula G-10 to produce a compound represented by Formula G-11: 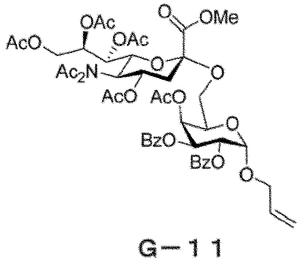 This step may be performed by using or adapting a known method, and may be preferably performed, for example, by the procedure shown in Example 47.

In an embodiment of the present invention, this step comprises a step of contact a solvent dissolving the compound represented by Formula G-11 with silica gel to conduct solid-phase extraction of the compound represented by Formula G-11. A byproduct, which is produced by acetylating the compound represented by Formula G-3 that is used in excess during the upstream glycosylation reaction, such as a diacetyl form of the compound represented by Formula G-3, is not adsorbed on silica gel, and thus the compound represented by Formula G-11 can be efficiently purified by this step.

The solvent used to dissolve the compound represented by Formula G-11, and includes, but are not limited to, toluene, heptane, dichloromethane, chloroform, or a combination thereof, preferably toluene, dichloromethane, chloroform, or a combination thereof, and particularly preferably toluene.

As the silica gel in this step, for example, silica gel in an amount 2 to 5 times relative to the amount of the raw material may be used; silica gel in an amount 2 to 4 times relative to the amount of the raw material may be preferably used; and silica gel in an amount about 3.5 times relative to the amount of the raw material may be more preferably used.

In this step, the solvent used to elute the compound represented by Formula G-1 1 adsorbed on silica gel is not limited as long as the silica gel is not dissolve in the solvent and the compound of interest can be can eluted by the solvent, and includes, for example, ethyl acetate, cyclopentyl methyl ether, or tert-butyl methyl ether, and preferably ethyl acetate.

### <Substep V-10>

Substep V-10 is a step of removing the allyl group attached to the carbon at position 1 of D-galactopyranoside in the compound represented by Formula G-11 to produce a compound represented by Formula G-12: 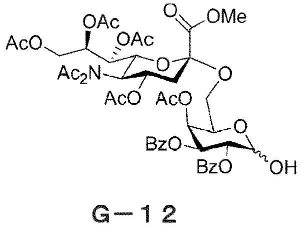 This step may be performed by using or adapting a known method, and may be preferably performed, for example, by the procedure shown in Example 48.

The compound represented by Formula G-12 that is produced in this step and dissolved in a solvent may be used in the next step, or may be isolated and purified by recrystallization. The compound represented by Formula G-12 has a great advantage in that the compound can be isolated and purified by crystallization. The compound represented by Formula G-12 with HPLC purity of 99% or higher can be obtained without any impurities. Thus, a reaction in the next step can be carried out stably. The isolation and purification by recrystallization may be performed by a method for crystallization wherein 2-propanol is added to a solution of ethyl acetate in which the compound represented by Formula G-12 is dissolved, and may be preferably performed, for example, by the procedure shown in Example 48.

### <Substep V-11>

Substep V-11 is a step of reacting the compound represented by Formula G-12 with 2,2,2-trifluoro-N-phenylacetimidoyl chloride (TFPC) to produce a compound represented by Formula D-7: 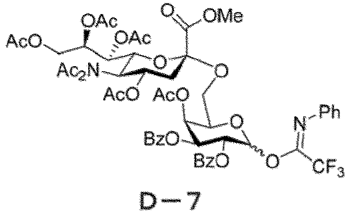 This step may be performed by using or adapting a known method, and may be preferably performed, for example, by the procedure shown in Example 49.

### <Purification of the compound represented by Formula D-8>

In Step II-3-1, the compound represented by Formula D-8 can be obtained in a purified form by the following method for purification. The method for purification comprises purifying the above compound represented by Formula D-8 by
after stopping the reaction of the compound represented by Formula D-6 with the compound represented by Formula D-7, adding a hydrophobic carrier and water to a water-soluble organic solvent containing the above resulting compound represented by Formula D-8 and contaminants to adsorb the above compound represented by Formula D-8 onto the hydrophobic carrier;
then filtering the resultant and washing the hydrophobic carrier with a mixture of the above water-soluble organic solvent and water to remove the contaminants;
and then eluting the above compound represented by Formula D-8 from the hydrophobic carrier using an organic solvent. As described in the method of purifying the above compound represented by Formula A-5 in Step I-1-1, this method for purification makes it possible to produce a large amount of high-quality oligosaccharide efficiently in the liquid-phase synthesis of oligosaccharide chains by using a small amount of hydrophobic carrier.

Purification of the above compound represented by Formula D-8 is not limited to the purification in this step. Accordingly, in an embodiment of the present invention, also provided is a method comprising
adding a hydrophobic carrier and water to a water-soluble organic solvent containing the above compound represented by Formula D-8 and contaminants to adsorb the above compound represented by Formula D-8 onto the hydrophobic carrier;
then filtering the resultant and washing the hydrophobic carrier with a mixture of the above water-soluble organic solvent and water to remove the contaminants;
and then eluting the above compound represented by Formula D-8 from the hydrophobic carrier using an organic solvent to purify the above compound represented by Formula D-8.

The above term "contaminants" refers to compounds and/or reagents other than the protected oligosaccharide (in this step, the compound represented by Formula D-8), and mainly means reagents used in a reaction of synthesizing a protected oligosaccharide and remnants of the reagents, sugars other than the protected oligosaccharide, such as mono- or disaccharide compounds used in the elongation reaction of the protected oligosaccharide, or byproducts generated by the deprotection reaction of the protected oligosaccharide. The "hydrophobic carrier" (e.g., a resin used for packing reversed-phase partition chromatography), "water-soluble organic solvent," "organic solvent," and the temperature for the purification used in this step are substantially the same as those described in the method of purifying the above compound represented by Formula A-5 in Step I-1-1.

### <Step II-3-2>

This step is a step of removing each amino group-protecting group and each alcohol acyl-based protecting group on the compound represented by Formula D-8 to give the compound represented by Formula D-9. The above removal (deprotection) of each amino group-protecting group may be carried out by using or adapting a known method, and may be preferably performed, for example, by the procedure shown in Example 64. For example, this step can be performed by sequentially adding 1,2-dimethoxyethane and potassium hydroxide, sodium hydroxide, or lithium hydroxide aqueous solution, but is not limited to such a procedure.

Steps II-3-3 to II-3-4 as follows are exemplary embodiments for producing the compound represented by Formula D-11 from the compound represented by Formula D-9, but steps for the production are not limited to them.

### <Step II-3-3>

This step is a step of protecting, with an acetyl group, each amino group on the compound represented by Formula D-9 to give a compound represented by Formula D-10: 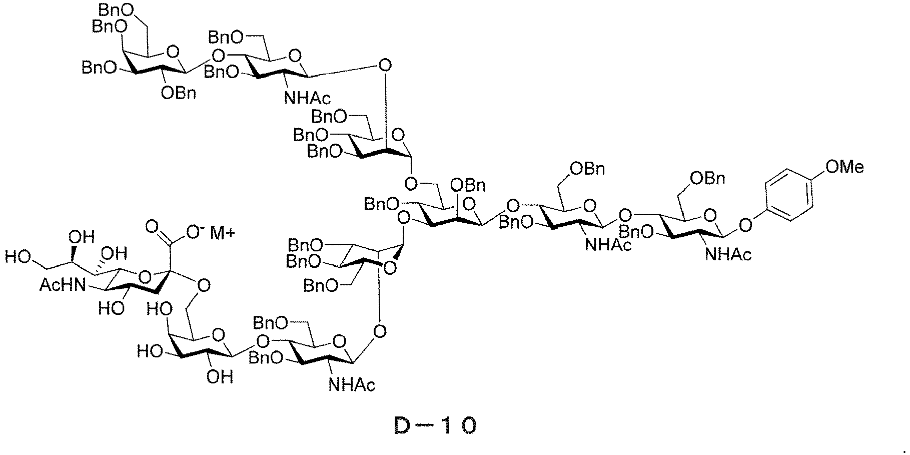 The above protection of each amino group with an acetyl group may be performed by using or adapting a known method, and may be preferably performed, for example, by the procedure shown in Example 65.

### <Purification of the compound represented by Formula D-10>

In Step II-3-3, the compound represented by Formula D-10 can be obtained in a purified form by the following method for purification. The method for purification comprises purifying the above compound represented by Formula D-10 by
adding a hydrophobic carrier and water to a water-soluble organic solvent containing the above resulting compound represented by Formula D-10 and contaminants to adsorb the above compound represented by Formula D-10 onto the hydrophobic carrier ;
then filtering the resultant and washing the hydrophobic carrier with a mixture of the above water-soluble organic solvent and water to remove the contaminants;
and then eluting the above compound represented by Formula D-10 from the hydrophobic carrier using an organic solvent. As described in the method of purifying the above compound represented by Formula A-5 in Step I-1-1, this method for purification makes it possible to produce a large amount of high-quality oligosaccharide efficiently in the liquid-phase synthesis of oligosaccharide chains by using a small amount of hydrophobic carrier.

Purification of the above compound represented by Formula D-10 is not limited to the purification in this step. Accordingly, in an embodiment of the present invention, also provided is a method comprising
adding a hydrophobic carrier and water to a water-soluble organic solvent containing the above compound represented by Formula D-10 and contaminants to adsorb the above compound represented by Formula D-10 onto the hydrophobic carrier;
then filtering the resultant and washing the hydrophobic carrier with a mixture of the above water-soluble organic solvent and water to remove the contaminants;
and then eluting the above compound represented by Formula D-10 from the hydrophobic carrier using an organic solvent to purify the above compound represented by Formula D-10.

The above term "contaminants" refers to compounds and/or reagents other than the protected oligosaccharide (in this step, the compound represented by Formula D-10), and mainly means reagents used in a reaction of synthesizing a protected oligosaccharide and remnants of the reagents, sugars other than the protected oligosaccharide, such as mono- or disaccharide compounds used in the elongation reaction of the protected oligosaccharide, or byproducts generated by the deprotection reaction of the protected oligosaccharide. The "hydrophobic carrier" (e.g., a resin used for packing reversed-phase partition chromatography), "water-soluble organic solvent," "organic solvent," and the temperature for the purification used in this step are substantially the same as those described in the method of purifying the above compound represented by Formula A-5 in Step I-1-1.

### <Step II-3-4>

This step is a step of removing a benzyl group from each benzyloxy group on the compound represented by Formula D-10 to give the above compound represented by Formula D-11. The above benzyl group may be removed by using or adapting a known method, and may be preferably performed, for example, by the procedure shown in Example 66. For example, this step can be performed by adding N-methylpyrrolidone and Pd/C to the compound represented by Formula D-10, followed by reducing pressure for displacement with nitrogen and applying hydrogen pressure for decompression, but is not limited to such a procedure.

### <Step II-4>

Step II-4 is a step comprising a step of reacting the above compound represented by Formula D-11 with an azido PEG linker compound (11-azido-3,6,9-trioxaundecan-1-amine) represented by Formula D-12: 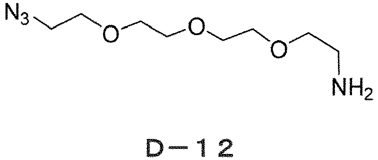 to give the above oligosaccharide represented by Formula D-13. Binding the above compound represented by Formula D-11 to the compound represented by Formula D-12 may be achieved by using or adapting a known method, and may be preferably performed, for example, by the procedure shown in Example 72. For example, this step can be achieved by sequentially adding the compound represented by Formula D-12, N-ethyl diisopropyl amine, and hexafluorophosphate (benzotriazol-1-yloxy)tripyrrolidinophosphonium, bromotripyrrolidinophosphonium-hexafluorophosphate, or 4-(4,6-dimethoxy-1,3,5-triazin-2-yl)-4-methylmorpholinium chloride to a solution containing the compound represented by Formula D-11, followed by stirring the mixture, but is not limited to such a procedure.

### <Purification of the compound represented by Formula D-12>

In an embodiment of the present invention, the above compound represented by Formula D-12 may be obtained by the method for purification comprising a step of adding, to a solution containing the crude compound represented by Formula D-12, a compound represented by Formula E-1: 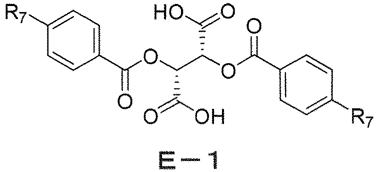 (wherein R₇ is a hydrogen atom, a methyl group, or a methoxy group) to give a crystallin compound represented by Formula E-2: 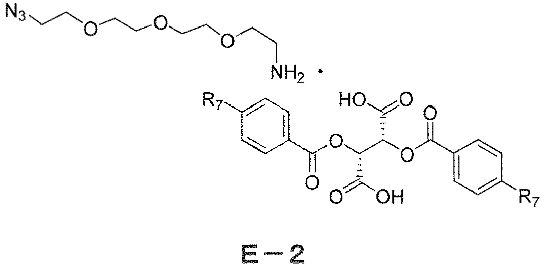 (wherein R₇ is a hydrogen atom, a methyl group, or a methoxy group); and a step of isolating the crystalline compound and then extracting the compound represented by Formula D-12 from the crystalline compound isolated. This method for purification makes it possible to obtain the compound represented by Formula D-12 with high-purity. The compound represented by Formula D-12 preferably has a purity (also referred to herein as "HPLC purity") of 95% or higher, more preferably 96% or higher or 97% or higher, and still more preferably 98% or higher or 99% or higher as measured by HPLC. As such, the objective of purifying the compound represented by D-12 is to purify dimers and other impurities that are contaminated in a commercially available reagent of the compound. In addition, in conventional technology, a strict purification by distillation or complicated column chromatography purification are required to purify them, and further in the case where an azide structure is included, inconveniently, operations for the distillation that require heating cannot be carried out due to a risk of its explosion. The present inventors have investigated a method for purification to obtain the compound represented by Formula D-12 with high-purity, and as a result, have found that when the three different tartaric acid derivatives (wherein R₇ is a hydrogen atom, a methyl group, or a methoxy group) represented by Formula E-1 above are used, the compound represented by Formula D-12 can form a salt with each tartaric acid derivative at a ratio of 1:1 and thus can be isolated as crystals. The above resulting compound represented by Formula E-2 is a new crystalline compound, and the compound represented by Formula D-12 with higher HPLC purity (preferably, with HPLC purity of 95% or higher) than that before purification can be obtained by carrying out liquid-separation using a solution containing ethyl acetate/hydrochloric acid after the isolation, followed by freeing and extraction.

The above purification method is exemplified as follows. First, the compound represented by Formula E-1 is added to a solution containing the compound represented by Formula D-12 in a solvent such as acetonitrile and water, then stirred, and after confirming that it is dissolved, a solvent such as acetonitrile is added. The resulting slurry is concentrated under reduced pressure and stirred to filter and the precipitated crystals are then filtered. The filtered crystals are washed with acetonitrile and dried under reduced pressure to obtain crystals of the compound represented by Formula E-2 (i.e. the step for producing crystals). Then, to a solution containing the resulting crystalline compound in ethyl acetate and water is added concentrated hydrochloric acid, then stirred, and liquid-separation is then conducted. The resulting aqueous layer is washed with ethyl acetate, etc., and adjusted to be basic with an aqueous sodium hydroxide solution, etc. Subsequently, sodium chloride, etc., is added and dissolved. Subsequently, a solvent such as dichloromethane is added, then stirred, and liquid-separation is carried out. The resulting organic layer is concentrated under reduced pressure. After a solvent such as acetonitrile is added and the mixture is concentrated under reduced pressure, the resulting solution is filtered and washed with a solvent such as acetonitrile. The obtained solution is concentrated under reduced pressure (extraction process) to obtain the compound represented by Formula D-12 with high HPLC purity. More preferably, for example, the method shown in Examples 67 to 71 may be performed.

Purification of the above compound represented by Formula D-12 is not limited to the purification in this step. Accordingly, in an embodiment of the present invention, also provided is a method of purifying the compound represented by Formula D-12, wherein the method comprises: a step of adding, to a solution containing the crude compound represented by Formula D-12 above, the above compound represented by Formula E-1 (wherein R₇ is a hydrogen atom, a methyl group, or a methoxy group) to give the crystallin compound represented by Formula E-2 above (wherein R₇ is a hydrogen atom, a methyl group, or a methoxy group); and a step of isolating the crystalline compound and then extracting the compound represented by Formula D-12 from the crystalline compound isolated.

### <Novel compounds>

The intermediates for the above oligosaccharide represented by Formula A-13 are useful in the production of the oligosaccharide, but can also be used for any of applications, and the use of the intermediates is not limited to that for production of the above oligosaccharide. Thus, the present invention provides the above oligosaccharide represented by Formula A-13 and intermediates thereof.

In an embodiment of the present invention, provided is the oligosaccharide represented by Formula A-13:

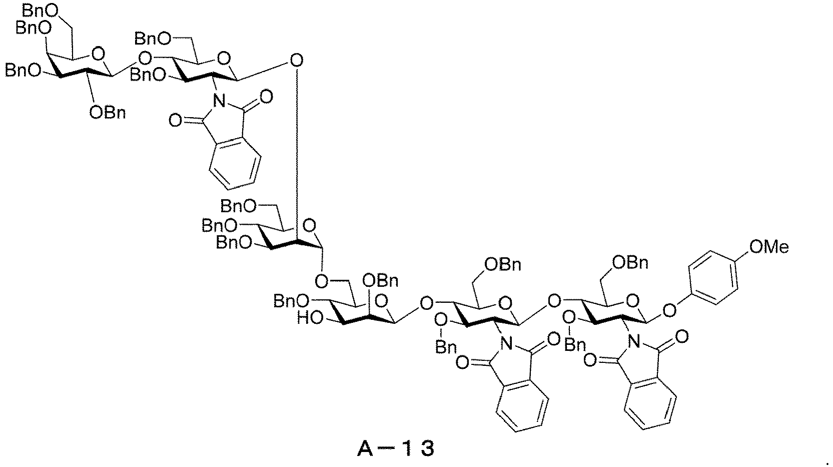

In an embodiment of the present invention, provided is the compound represented by Formula A-5:

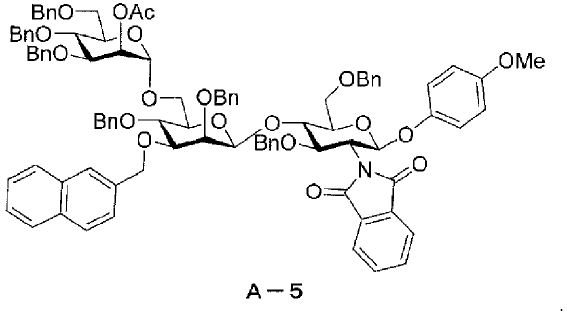

In an embodiment of the present invention, provided is the compound represented by Formula A-6:

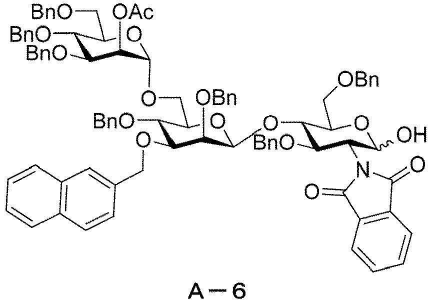

In an embodiment of the present invention, provided is the compound represented by Formula A-7:

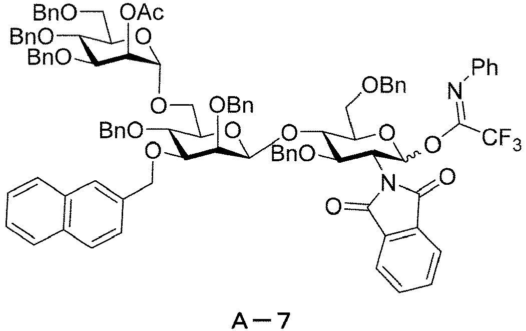

In an embodiment of the present invention, provided is the compound represented by Formula A-9:

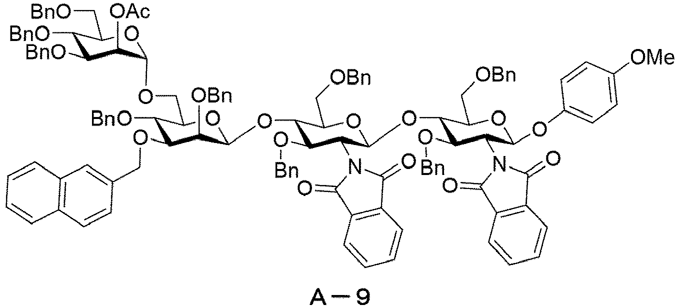

In an embodiment of the present invention, provided is the compound represented by Formula A-10:

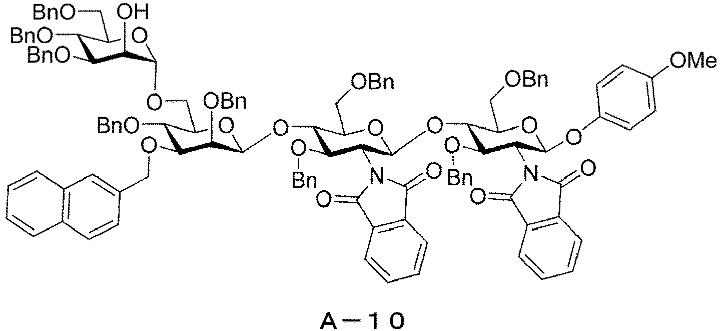

In an embodiment of the present invention, provided is the compound represented by Formula A-11:

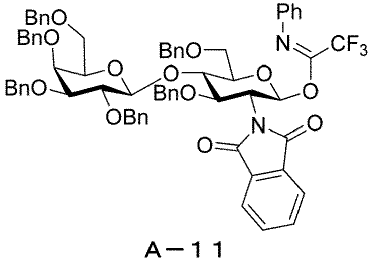

In an embodiment of the present invention, provided is the compound represented by Formula A-12:

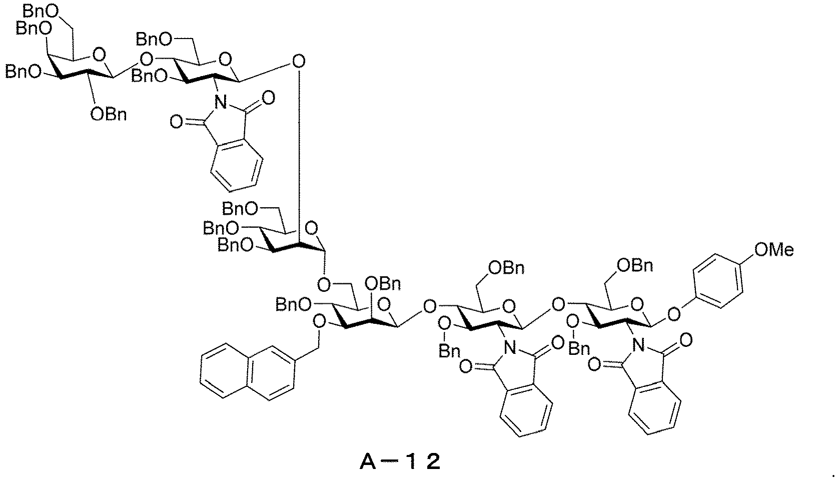

In an embodiment of the present invention, provided is the compound represented by Formula A-14:

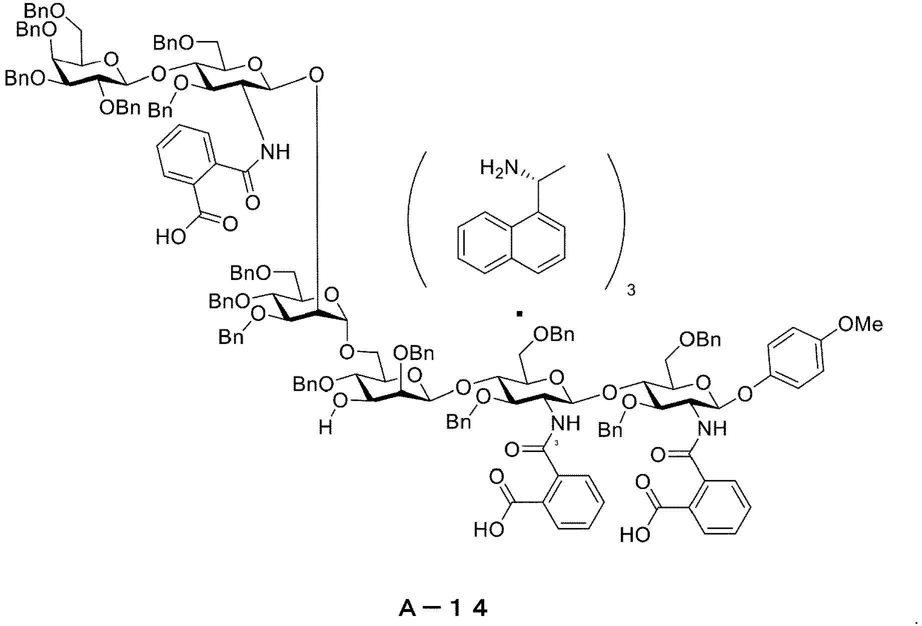

In an embodiment of the present invention, provided is the compound represented by Formula A-15:

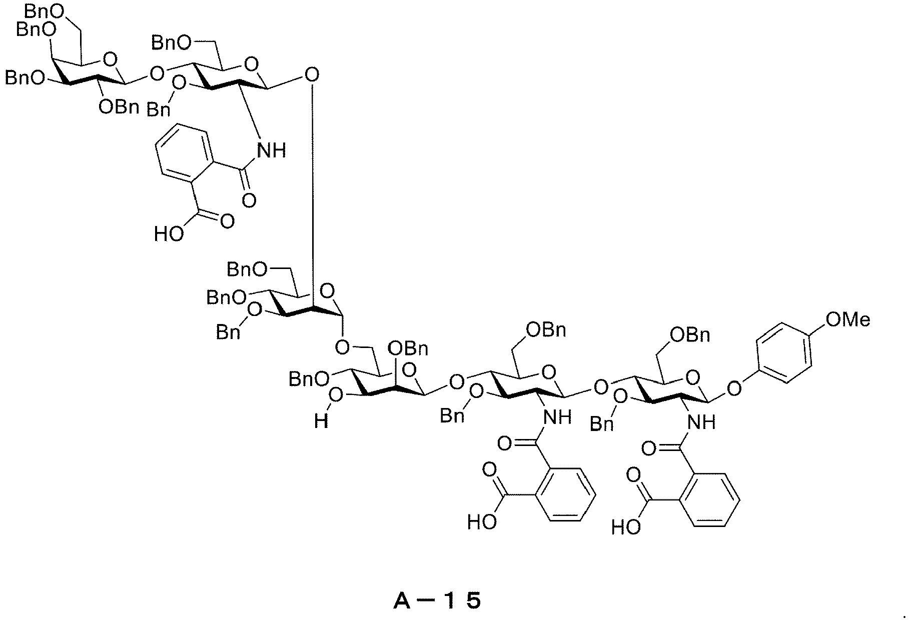

Further, the intermediates for the above compound represented by Formula A-11 are useful in the production of the compound, while the use of them is not limited to that for the production of the above compound, and they are applicable to any use. Thus, the present invention also provides the intermediates for the above compound represented by Formula A-11.

In an embodiment of the present invention, provided is the compound represented by Formula B-4:

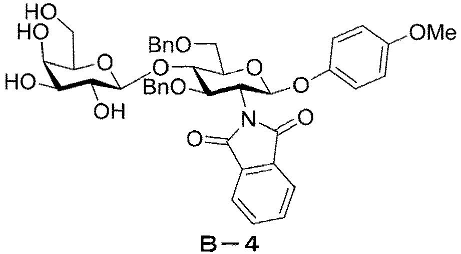

In an embodiment of the present invention, provided is the compound represented by Formula B-5:

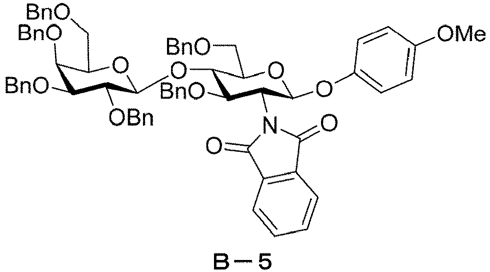

In an embodiment of the present invention, provided is the compound represented by Formula B-6:

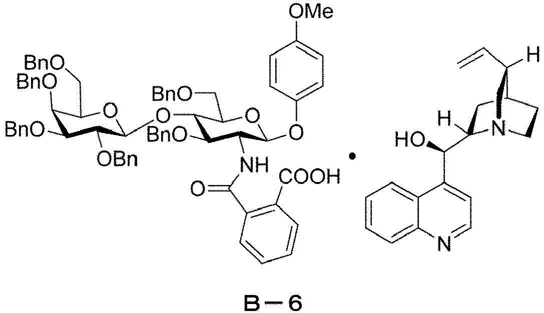

In an embodiment of the present invention, provided is the compound represented by Formula B-7:

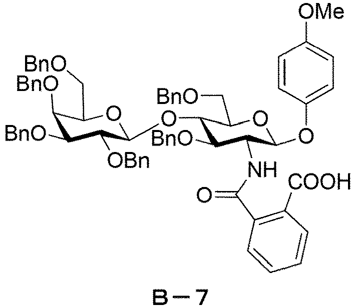

In an embodiment of the present invention, provided is the compound represented by Formula B-8:

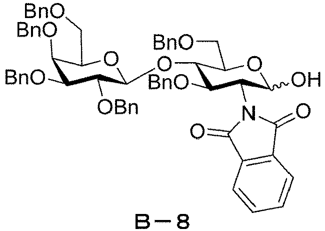

Further, the intermediates for the above oligosaccharide of Formula D-13 are useful in the production of the oligosaccharide, while the use of them is not limited to that for the production of the above compound, and they are applicable to any use. As described below, the present invention provides the above oligosaccharide represented by Formula D-13 and their intermediates (including the above compound represented by Formula A-13 and intermediates thereof).

In an embodiment of the present invention, provided is an oligosaccharide represented by Formula D-13:

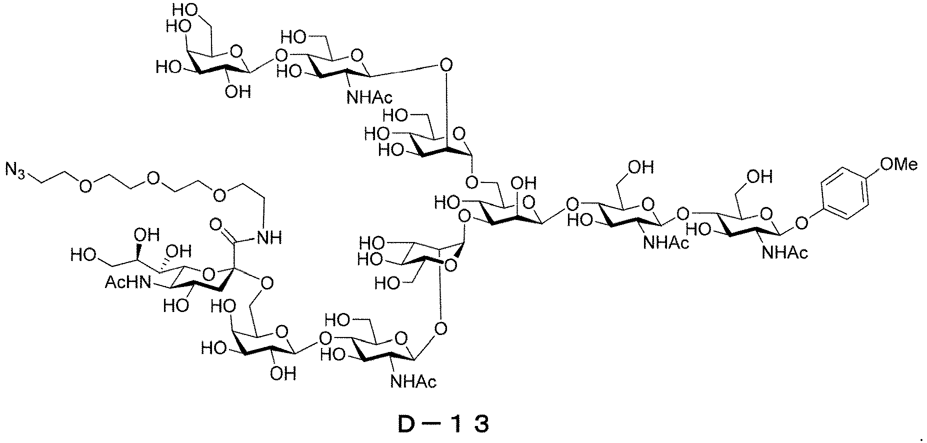

In an embodiment of the present invention, provided is the compound represented by Formula D-1:

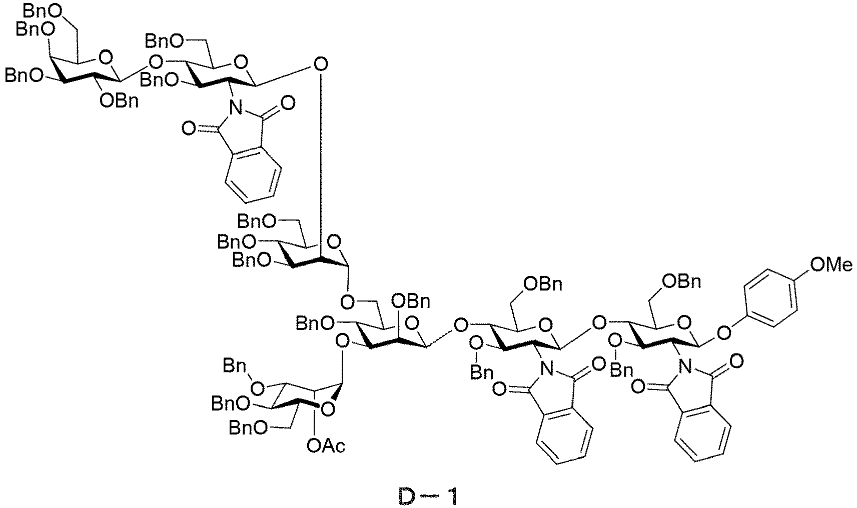

Provided is a compound represented by Formula D-2:

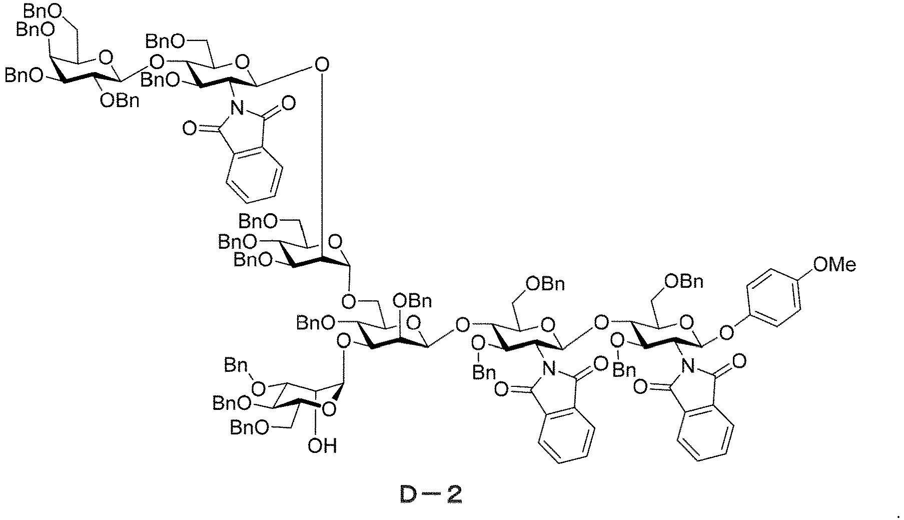

In an embodiment of the present invention, provided is the compound represented by Formula D-4:

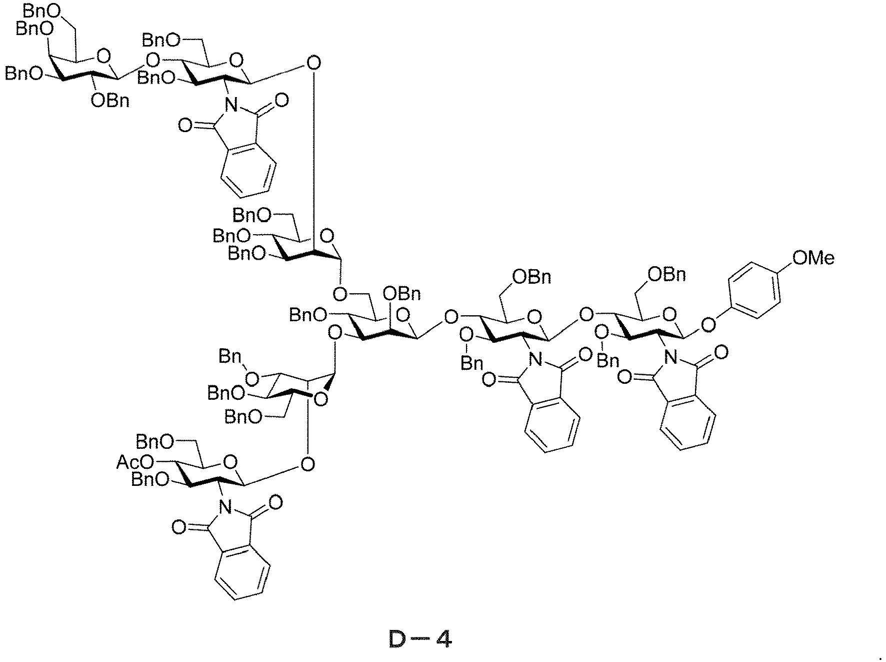

In an embodiment of the present invention, provided is the compound represented by Formula D-5:

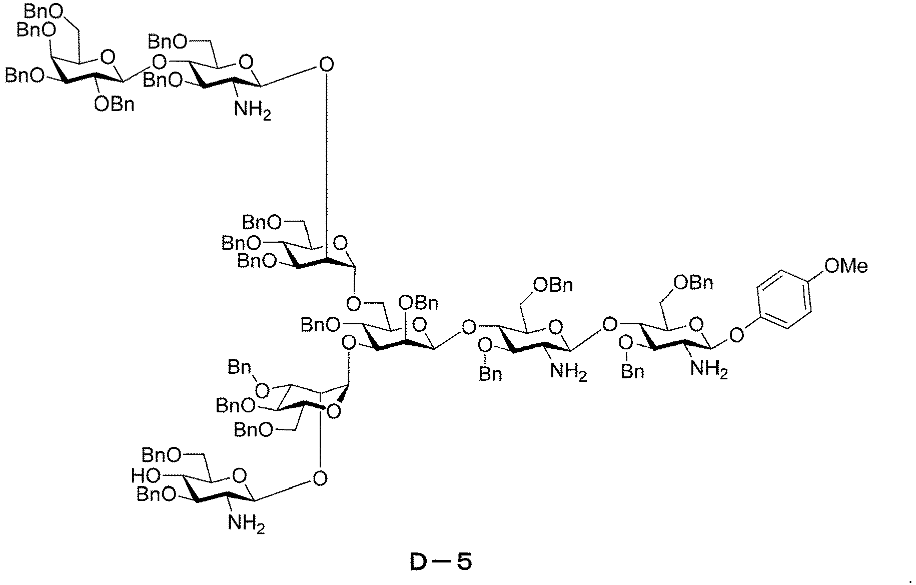

In an embodiment of the present invention, provided is the compound represented by Formula D-5-FMA:

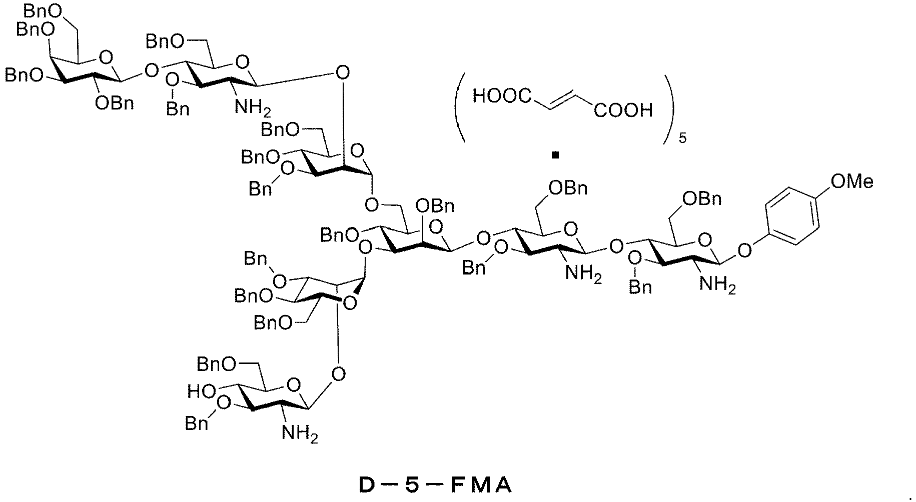

In an embodiment of the present invention, provided is the compound represented by Formula D-6: 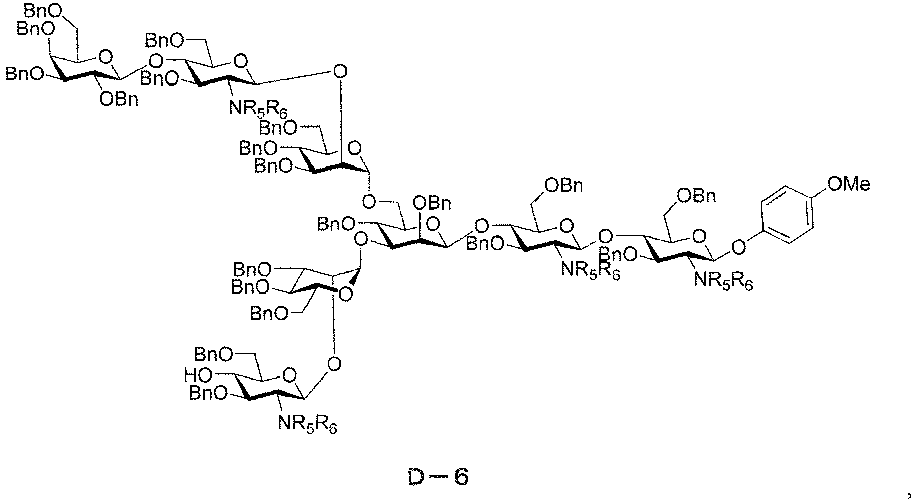 wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆, together with a nitrogen atom attached thereto form a phthalimide group.

In an embodiment of the present invention, provided is the compound represented by Formula D-8: 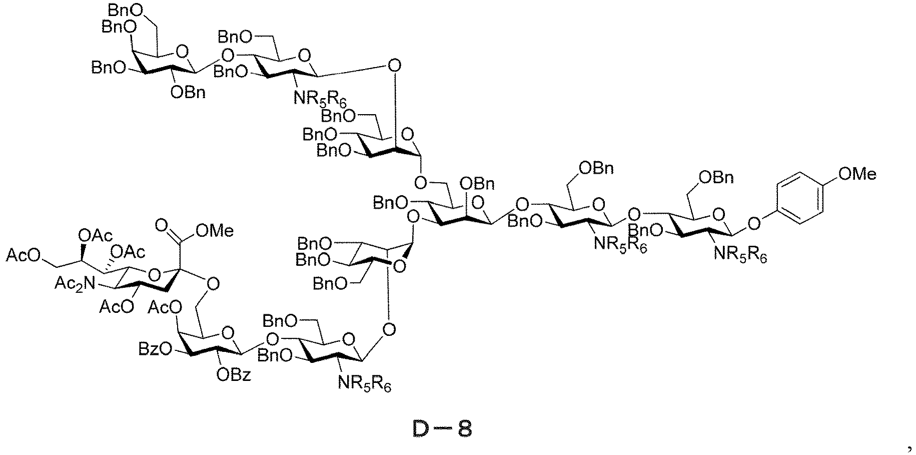 wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆, together with a nitrogen atom attached thereto form a phthalimide group.

In an embodiment of the present invention, provided is the compound represented by Formula D-9: 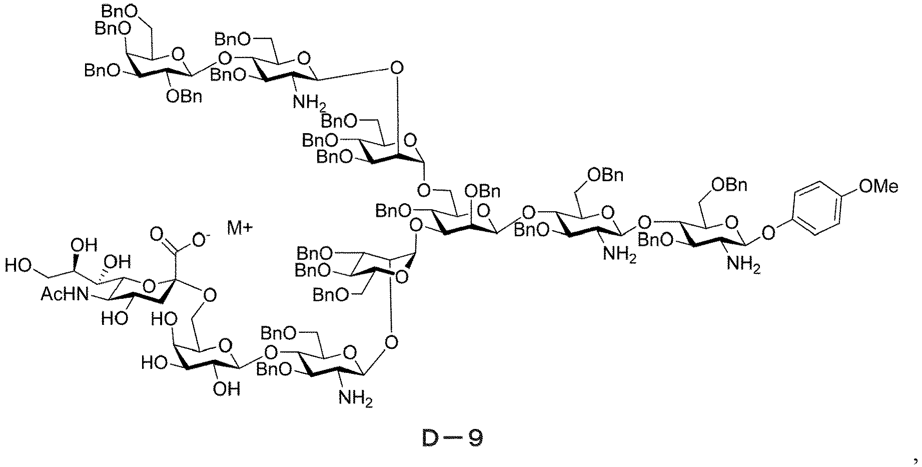 wherein M⁺ is a sodium ion, a lithium ion, a potassium ion, or a protonated triethylamine cation.

In an embodiment of the present invention, provided is the compound represented by Formula D-10: 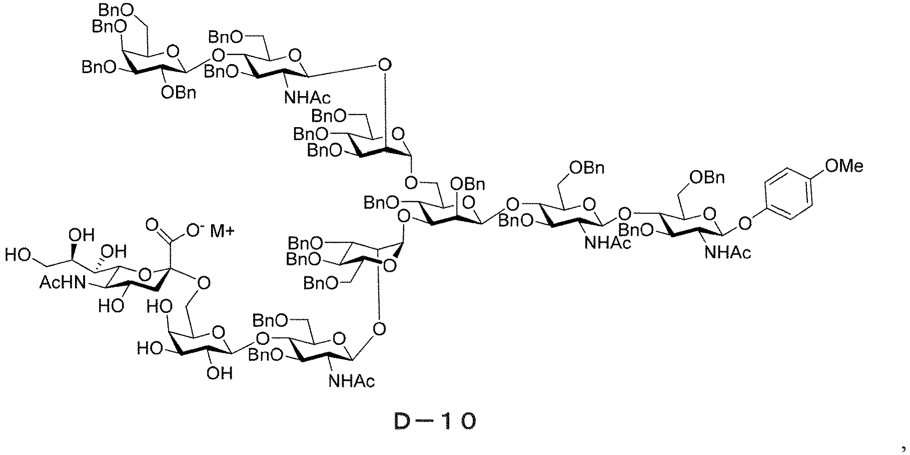 wherein M⁺ is a sodium ion, a lithium ion, a potassium ion, or a protonated triethylamine cation.

In an embodiment of the present invention, provided is the compound represented by Formula D-11: 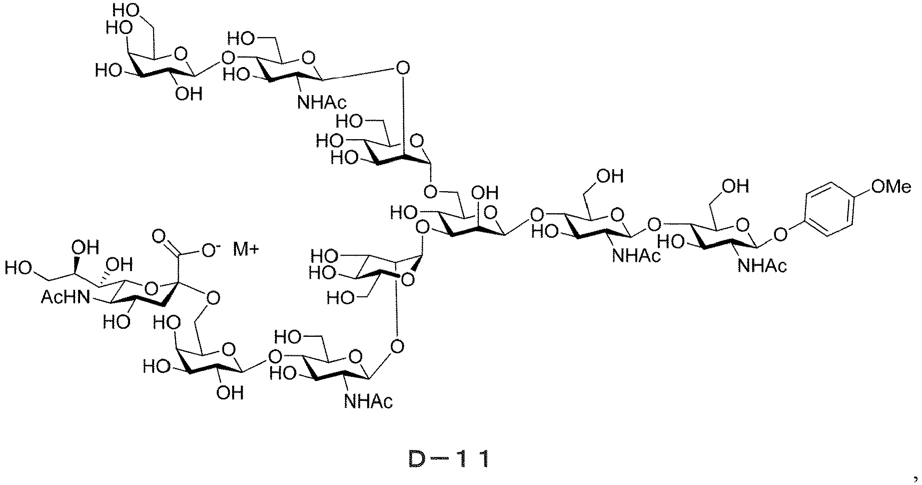 wherein M⁺ is a sodium ion, a lithium ion, a potassium ion, or a protonated triethylamine cation.

In an embodiment of the present invention, provided is a crystalline compound represented by Formula E-2: 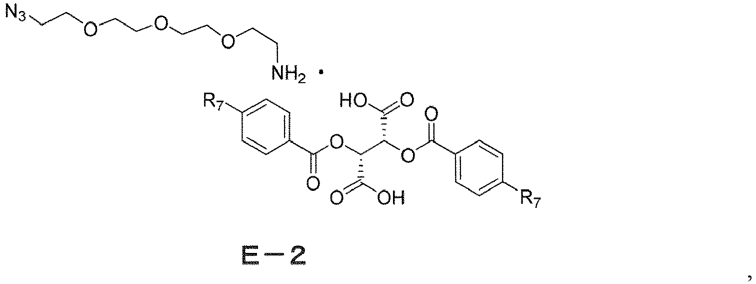 wherein R₇ is a hydrogen atom, a methyl group, or a methoxy group.

In an embodiment of the present invention, provided is the compound represented by Formula D-12: 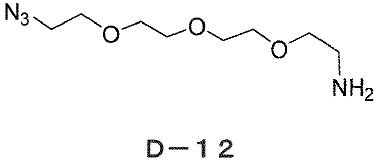 having a purity of 95% or higher as measured by HPLC. In an embodiment of the present invention, provided is the above compound represented by Formula D-12 having a purity of 95% or higher as measured by HPLC.

### <Glycoprotein, etc., and Method for production thereof>

In one embodiment of the present invention, provided is a novel glycoprotein, etc., and a novel method for production thereof, wherein a biantennary glycan having an α2,6-sialic acid structure at a non-reducing end (i.e., an oligosaccharide represented by Formula D-13) is used as a donor molecule in synthesizing, for example, a glycoprotein (especially, a glycan-remodeled antibody or its FC region-containing molecule, or an antibody-drug conjugate). As detailed below, the oligosaccharide represented by Formula D-13 obtained by the method for production of the present invention may be used, but is not limited to, for the production of a glycoprotein (in particular, a glycan-remodeled antibody or a molecule containing an Fc region thereof, or an antibody-drug conjugate) (e.g., WO2019/065964, WO2020/050406) or may be used for other applications.

It has recently been reported that a non-uniform glycan of an antibody can be remodeled by enzymatic reactions to introduce functionalized glycans uniformly (ACS Chem. Biol. 2012, 7, 110-122, ACS Med. Chem. Lett. 2016, 7, 1005-1008). Using this glycan remodeling technology, attempts have been made to synthesize a uniform antibody-drug conjugate (ADC) by introducing a drug(s) in a site-specific manner (Bioconjugate Chem. 2015, 26, 2233-2242; Angew. Chem. Int. Ed. 2016, 55, 2361-2367, US2016361436).

In the glycan remodeling, non-uniform glycan(s) attached to a protein (e.g., an antibody) is removed using hydrolase while maintaining only N-acetylglucosamine (GlcNAc) at each terminus to produce a uniform protein moiety, to which GlcNAc is attached (hereinafter, referred to as an "acceptor molecule"). Subsequently, any glycan is separately prepared (hereinafter, referred to as a "donor molecule"), and the acceptor molecule and the donor molecule are linked together by using glycosyltransferase, thereby, a uniformglycoprotein with any glycan structure can be synthesized.

In an embodiment of the present invention, the oligosaccharide represented by Formula D-13 produced by using the novel method for production of the present invention, with its terminal structure being activated, can be used as a donor molecule in the synthesis of the above uniform glycoprotein (in particular, a glycan-remodeled antibody or a molecule containing an Fc region thereof).

### EXAMPLES

In the following Examples, the room temperature indicated is from 15°C to 35°C. Silica gel chromatography was performed using Biotage Sfar HC D (20 µm, available by Biotage), reverse-phase column chromatography was performed using Universal Column ODS Premium 30-µm L-size (available by Yamazen Corporation) and Inject column ODS L-size (available by Yamazen Corporation), and preparative HPLC was performed using Agilent Preparative HPLC System (available by Agilent Technology). The preparative column used was XBridge Prep OBD (5 µm, C18, 130 Å, 250 × 30 mm; available by Waters).

The following instruments were used to measure various spectral data. ¹H-NMR and ¹³C-NMR spectra were measured using JEOL ECZ500R and ECX400P. Mass spectra were measured using Shimadzu LCMS-2010 and LCMS-2020 (available by Shimadzu Corporation), XEVO Q-Tof MS (Waters), and Q-Exactive (Thermo Fisher).

### <Synthesis of compound represented by Formula A-3>

Compound represented by Formula A-3 was synthesized according to the following synthesis scheme 1.

### [Synthesis Scheme 1]

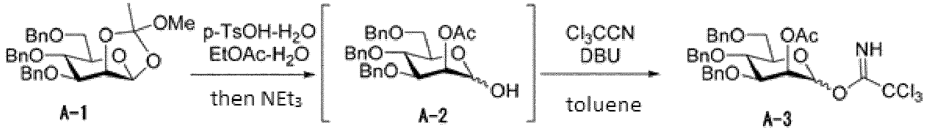

### Example 1

### 2-O-Acetyl-3,4,6-tri-O-benzyl-D-mannopyranose(a compound represented by Formula A-2)

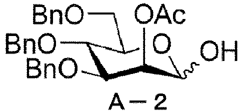

First, 3,4,6-tri-O-benzyl-1,2-O-(1-methoxyethyliden)-β-D-mannopyranose (the compound represented by Formula A-1) (40.0 g, 78.9 mmol) was added to a 1-L four-neck flask, and ethyl acetate (400 mL) was then added. Under a nitrogen atmosphere, water (2 mL) and p-TsOH·H₂O (45 mg, 0.237 mmol) were added at room temperature, and the mixture was stirred at the same temperature for 6 hours. After the completion of the reaction was checked by HPLC, triethylamine (7.99 g, 78.9 mmol) was added. The mixture was stirred overnight at the same temperature. After the completion of the acetyl group transfer was checked by HPLC, the reaction solution was mixed with 5% sodium bicarbonate in water (400 mL) for liquid-separation . Then, 20% brine (200 mL) was added to the organic layer for liquid-separation. The resulting organic layer was concentrated under reduced pressure to 80 mL, and toluene (400 mL) was added. The mixture was concentrated under reduced pressure to a liquid volume of 80 mL. Toluene (400 mL) was added again, and the mixture was concentrated again under reduced pressure to a liquid volume of 80 mL. Dehydrated toluene (120 mL) was added to prepare, as a colorless solution, a toluene solution containing 2-O-acetyl-3,4,6-tri-O-benzyl-D-mannopyranose (the compound represented by Formula A-2).

### Example 2

### 2-O-Acetyl-3,4,6-tri-O-benzyl-1-O-(2,2,2-trichloroethanimidoyl)-D-mannopyranose(the compound represented by Formula A-3)

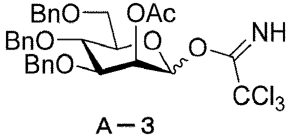

A toluene solution containing 2-O-acetyl-3,4,6-tri-O-benzyl-D-mannopyranose (the compound represented by Formula A-2) (78.9 mmol) was added to a 1-L flask, and trichloroacetonitrile (12 mL, 118 mmol) and DBU (119 µL, 0.789 mmol) were added. The mixture was stirred under nitrogen at 0°C for 2 hours. After the completion of the reaction was checked by HPLC, acetic acid (45 µL, 0.789 mmol) was added to the reaction solution at 0°C to prepare, as a brown solution, a toluene solution containing 2-O-acetyl-3,4,6-tri-O-benzyl-1-O-(2,2,2-trichloroethanimidoyl)-D-mannopyranose (78.9 mmol). This solution was used as it was in the next step.

### <To synthesize the compound represented by Formula A-11>

A compound represented by Formula A-11 was synthesized according to the following synthesis scheme 2.

### [Synthesis Scheme 2]

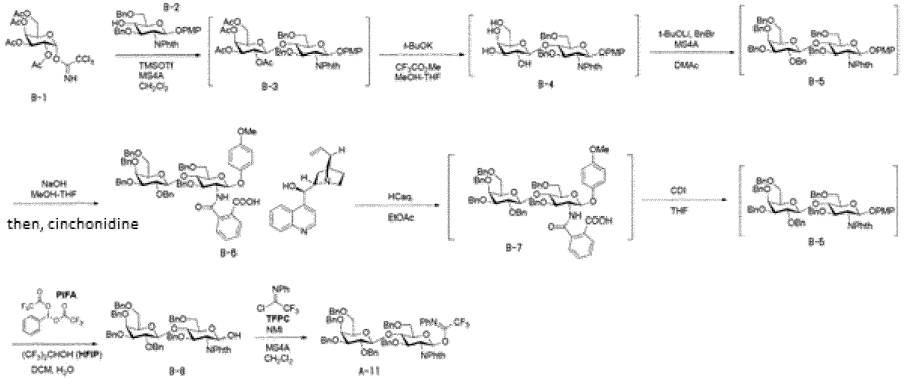

### Example 3

### 4-Methoxyphenyl 3,6-di-O-benzyl-2-deoxy-2-(1,3-dioxo-1,3-dihydro-2H-isoindol-2-yl)-4-O-(2,3,4,6-tetra-O-acetyl-β-D-galactopyranosyl)-β-D-glucopyranoside(the compound represented by Formula B-3)

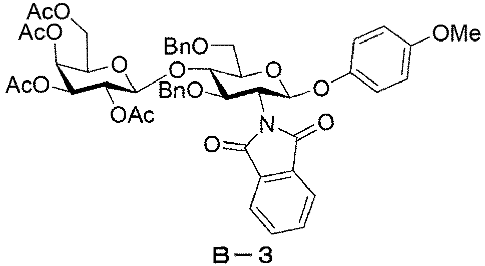

To a dichloromethane solution (17.5 L) containing 4-methoxyphenyl 3,6-di-O-benzyl-2-deoxy-2-phthalimido-β-D-glucopyranoside (the compound represented by Formula B-2) (2.508 kg, 4.211 mol) were added 2,3,4,6-tetra-O-acetyl-1-O(2,2,2-trichloroethanimidoyl)-α-D-galactopyranose (the compound represented by Formula B-1) (2.275 kg, 4.618 mol) and Molecular Sieves 4A powder (10 µm or less, 375 g). The mixture was stirred at 20°C to 30°C for 20 minutes, and then cooled to -20°C to -10°C. Subsequently, trimethylsilyl trifluoromethanesulfonate (140 g, 0.630 mol) was added dropwise over 3 minutes. The mixture was stirred at -5°C to -10°C for 3 hours, and triethylamine (106 g, 1.05 mol) was then added thereto. The temperature was raised to 0°C to 5°C, the Molecular Sieves 4A was filtered, and then washed with toluene (5 L). After the resulting solution was concentrated under reduced pressure to 12.5 L, toluene (12.5 L) was added, and the solution was liquid-separated and washed four times with a methanol (6.5 L)-water (18.5 L) mixture. The resulting organic layer was concentrated under reduced pressure to 9 L, and toluene (25 L) was added. The mixture was then concentrated under reduced pressure to 7.5 L to give a toluene solution (7.5 L) containing 4-methoxyphenyl 3,6-di-O-benzyl-2-deoxy-2-(1,3-dioxo-1,3-dihydro-2H-isoindol-2-yl)-4-O-(2,3,4,6-tetra-O-acetyl-β-D-galactopyranosyl)-β-D-glucopyranoside (the compound represented by Formula B-3).

### Example 4

### 4-Methoxyphenyl 3,6-di-O-benzyl-2-deoxy-2-(1,3-dioxo-1,3-dihydro-2H-isoindol-2-yl)-4-O-β-D-galactopyranosyl-β-D-glucopyranoside(the compound represented by Formula B-4)

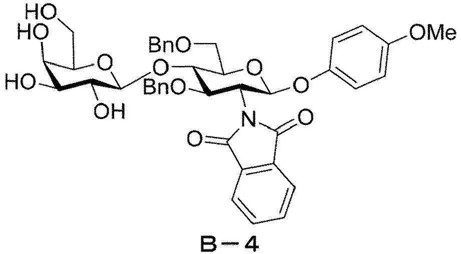

To a toluene solution (7.5 L) containing 4-methoxyphenyl 3,6-di-O-benzyl-2-deoxy-2-(1,3-dioxo-1,3-dihydro-2H-isoindol-2-yl)-4-O-(2,3,4,6-tetra-O-acetyl-β-D-galactopyranosyl)-β-D-glucopyranoside (the compound represented by Formula B-3) were added tetrahydrofuran (10 L), methanol (5 L), and methyl trifluoroacetate (538 g, 4.20 mol), respectively. The mixture was then mixed with a potassium t-butoxide-tetrahydrofuran solution (1 M, 2.1 L, 2.1 mol), and stirred at 40°C to 45°C for 2 hours. After cooling to 20°C to 25°C, acetic acid (151 g) and ethyl acetate (25 L) were added, respectively. The mixture was liquid-separated and washed three times with a sodium bicarbonate (750 g)-sodium chloride (750 g)-water (20 L) solution, and once with a sodium chloride (2.5 kg)-water (10 L) solution. The resulting organic layer was concentrated under reduced pressure to 7.5 L, and mixed with N,N-dimethylacetamide (25 L) and cyclopentylmethyl ether (37.5 L), respectively. The mixture was then concentrated under reduced pressure to 27.5 L. After that, cyclopentyl methyl ether (12.5 L) was added and the mixture was concentrated under reduced pressure to 27.5 L to give a N,N-dimethylacetamide solution (27.5 L) containing 4-methoxyphenyl 3,6-di-O-benzyl-2-deoxy-2-(1,3-dioxo-1,3-dihydro-2H-isoindol-2-yl)-4-O-β-D-galactopyranosyl-β-D-glucopyranoside (the compound represented by Formula B-4).

### Example 5

### 4-Methoxyphenyl 3,6-di-O-benzyl-2-deoxy-2-(1,3-dioxo-1,3-dihydro-2H-isoindol-2-yl)-4-O-(2,3,4,6-tetra-O-benzyl-β-D-galactopyranosyl)-β-D-glucopyranoside(the compound represented by Formula B-5)

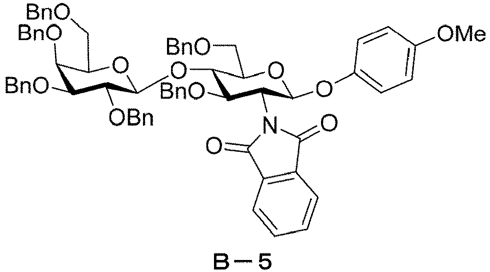

To a solution containing t-butyl alcohol (1.56 kg, 21.0 mol) in hexane (3.28 kg), n-butyl lithium-hexane solution (15.2%, 8.88 kg, 21.0 mol) was added dropwise at -15°C to 0°C over 4 hours. Methyl trifluoroacetate (26.9 g, 0.170 mol) was added to give a lithium t-butoxide-hexane solution. To an N,N-dimethylacetamide solution (27.5 L) containing 4-methoxyphenyl 3,6-di-O-benzyl-2-deoxy-2-(1,3-dioxo-1,3-dihydro-2H-isoindol-2-yl)-4-O-β-D-galactopyranosyl-β-D-glucopyranoside (the compound represented by Formula B-4) were added benzyl bromide (5.03 kg, 29.4 mol) and Molecular Sieves 4A powder (10 µm or less, 750 g). A lithium t-butoxide-hexane solution (12.1 kg) was added dropwise over 3 hours at 35°C to 45°C. After cooling to 20°C to 25°C, acetic acid (378 g, 6.29 mol) was added. The Molecular Sieves 4A was filtered and then washed with N,N-dimethylacetamide (7.5 L). Heptane (12.5 L) was added and the mixture was liquid-separated and washed. Then, t-butyl methyl ether (25 L) was added to the resulting N,N-dimethylacetamide layer, and the mixture was liquid-separated and washed three times with water (20 L). The resulting organic layer was concentrated under reduced pressure to 7.5 L to give a t-butyl methyl ether solution (7.5 L) containing 4-methoxyphenyl 3,6-di-O-benzyl-2-deoxy-2-(1,3-dioxo-1,3-dihydro-2H-isoindol-2-yl)-4-O-(2,3,4,6-tetra-O-benzyl-β-D-galactopyranosyl)-β-D-glucopyranoside (the compound represented by Formula B-5).

### Example 6

### 4-Methoxyphenyl 3,6-di-O-benzyl-2-(2-carboxybenzamido)-2-deoxy-4-O-(2,3,4,6-tetra-O-benzyl-β-D-galactopyranosyl)-β-D-glucopyranoside cinchonidine salt(the compound represented by Formula B-6)

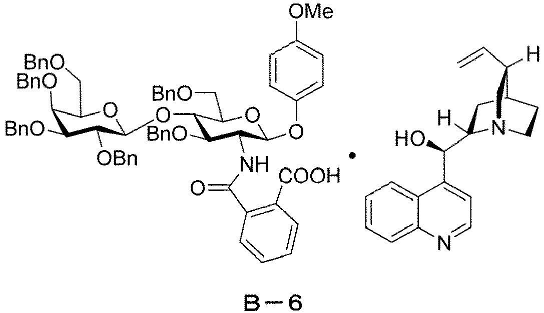

To a t-butyl methyl ether solution (7.5 L) containing 4-methoxyphenyl 3,6-di-O-benzyl-2-deoxy-2-(1,3-dioxo-1,3-dihydro-2H-isoindol-2-yl)-4-O-(2,3,4,6-tetra-O-benzyl-β-D-galactopyranosyl)-β-D-glucopyranoside (the compound represented by Formula B-5) were added tetrahydrofuran (12.5 L), methanol (5 L), and water (1 L), respectively. After cooling to 0°C to 10°C, 4 N aqueous sodium hydroxide solution (2.6 L, 10.4 mol) was added dropwise over 5 minutes. The mixture was stirred at 0°C to 10°C for 6 hours, and triethylamine (1.70 k g, 16.8 mol) was then added thereto. The mixture was then stirred at 20°C to 30°C for 16 hours. After cooling to 0°C to 10°C, 6 M HCl (3.0 L, 18.0 mol) was added dropwise over 20 min, and the temperature was raised to 20°C to 25°C. Ethyl acetate (20 L) was added, and the mixture was liquid-separated and washed with water (17.5 L), and then liquid-separated and washed with a sodium chloride (2.5 kg)-water (10 L) solution. After the resulting organic layer was concentrated under reduced pressure to 7.5 L, ethyl acetate (17.5 L) was added. The mixture was then concentrated under reduced pressure to 7.5 L. Ethyl acetate (30 L) and cinchonidine (1.36 kg, 4.62 mol), respectively, were added to the resulting solution. The mixture was stirred at 20°C to 25°C for 18 hours, and then cooled to 0°C to 5°C over 1 hour. Heptane (20 L) was then added dropwise over 1 hour. After stirring at the same temperature for 1.5 hours, the crystals formed were filtered, and the resulting crystals were washed with a mixed solvent of ethyl acetate (7.5 L)-heptane (5.6 L) cooled to 0°C to 5°C. The resulting crystals were dried under reduced pressure at 40°C to give 4-methoxyphenyl 3,6-di-O-benzyl-2-(2-carboxybenzamido)-2-deoxy-4-O-(2,3,4,6-tetra-O-benzyl-β-D-galactopyranosyl)-β-D-glucopyranoside cinchonidine salt (the compound represented by Formula B-6) (5.10 kg, the total yield through 4 steps: 85.0%).

¹H-NMR (500 MHz, CDCl₃) δ 8.78 (d, 4.6 Hz, 1H), 8.75 (d, J = 8.0 Hz, 1H), 8.07 (d, J = 8.5 Hz, 1H), 8.06 (d, J = 8.0 Hz, 2H), 7.65-7.68 (m, 3H), 7.61 (dd, 3.9, 7.7 Hz, 1H), 7.55 (d, J = 4.6 Hz, 1H), 7.43 (dd, 3.7, 7.4 Hz, 2H), 7.19-7.33 (m, 28H), 6.98-7.04 (m, 5H), 6.68-6.70 (m, 2H), 6.32 (brd, 1H), 5.39-5.46 (m, 1H), 5.35 (d, J = 6.9 Hz, 1H), 4.97 (d, J = 11 Hz, 1H), 4.83-4.91 (m, 3H), 4.66-4.73 (m, 5H), 4.55 (d, J = 12 Hz, 1H), 4.46 (s, 1H), 4.43 (d, J = 4.0 Hz, 1H), 4.35 (dd, J = 5.7, 12 Hz, 2H), 4.26 (d, J = 12 Hz, 1H), 4.22 (dd, J = 4.2, 8.4 Hz, 1H), 4.03-4.09 (m, 3H), 3.92 (d, J = 2.6 Hz, 1H), 3.74 (d, J = 3.7 Hz, 2H), 3.67-3.70 (m, 4H), 3.57-3.60 (m, 1H), 3.54 (t, J = 7.9 Hz, 1H), 3.36-3.44 (m, 3H), 3.31 (t, J = 9.2 Hz, 1H), 3.12 (dd, 10, 14 Hz, 1H), 3.02 (d, J = 13 Hz, 1H), 2.93 (m, 1H), 2.36 (s, 1H), 1.86-1.89 (m, 2H), 1.81 (dd, J = 9.0, 13 Hz, 1H), 1.53 (dd, J = 4.7, 9.5 Hz, 1H), 1.09 (dd, J = 5.7, 11 Hz, 1H).

¹³C-NMR (125 MHz, CDCl₃) δ 176.2, 169.5, 155.3, 151.6, 150.1, 148.0, 146.4, 138.88, 138.7, 138.53, 138.48, 138.39, 138.35, 138.26, 137.8, 133.4, 130.15, 130.07, 129.11, 128.73, 128.70, 128.51, 128.4, 128.34, 128.22, 128.2, 128.16, 127.95, 127.92, 127.83, 127.75, 127.57, 127.55, 127.43, 127.41, 127.19, 127.12, 124.9, 122.8, 119.5, 118.7, 116.2, 114.3, 103.2, 100.3, 82.4, 79.8, 78.7, 76.2, 75.2, 75.1, 74.8, 73.7, 73.4, 73.03, 72.99, 72.7, 68.7, 68.1, 59.8, 55.5, 55.3, 54.1, 43.4, 37.6, 27.0, 25.0, 19.1

HRMS (ESI⁺) [M+HNEt₃]⁺ calcd for C₇₅H₈₅N₂O₁₄: 1237.5995; found 1237.5977. [α]_{D}²⁰ = -21.889 (c 1.003, CDCl₃).

### Example 7

### 4-Methoxyphenyl 3,6-di-O-benzyl-2-deoxy-2-(1,3-dioxo-1,3-dihydro-2H-isoindol-2-yl)-4-O-(2,3,4,6-tetra-O-benzyl-β-D-galactopyranosyl)-β-D-glucopyranoside(the compound represented by Formula B-5)

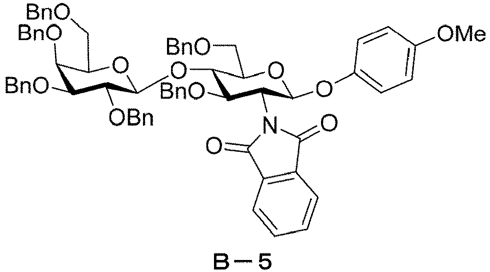

To an ethyl acetate suspension (33.8 L) containing a 4-methoxyphenyl 3,6-di-O-benzyl-2-(2-carboxybenzamido)-2-deoxy-4-O-(2,3,4,6-tetra-O-benzyl-β-D-galactopyranosyl)-β-D-glucopyranoside cinchonidine salt (the compound represented by Formula B-6) (4.50 kg, 3.15 mol) was added 0.5 M hydrochloric acid (33.8 L, 16.9 mol) at 15°C to 25°C. The mixture was stirred and dissolved. After the aqueous layer is removed, the resulting organic layer was liquid-separated and washed with a sodium chloride (4.5 kg)-water (18 L) solution, and was concentrated under reduced pressure to 6.8 L. After addition of ethyl acetate (33.8 L), the mixture was concentrated under reduced pressure to 6.8 L to give, as an ethyl acetate solution, a compound represented by Formula B-7:

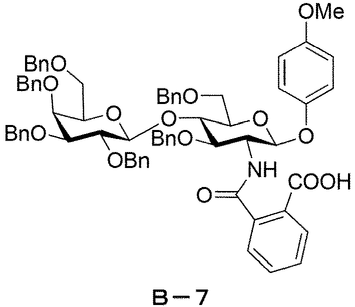

Next, tetrahydrofuran (18 L) was added to the resulting solution. After cooling to 0°C to 5°C, 1,1'-carbonyldiimidazole (765 g, 4.12 mol) was added and the mixture was stirred for 17 hours. Ethyl acetate (22.5 L), water (22.5 L), and 6 M hydrochloric acid (1.57 L, 9.42 mol), respectively, were added, and the aqueous layer was removed. The resulting organic layer was liquid-separated and washed sequentially with water (22.5 L) and a sodium chloride (4.5 kg)-water (18 L) solution. The resulting organic layer was concentrated under reduced pressure to 4.5 L. Toluene (22.5 L) was then added and concentrated under reduced pressure to 4.5 L to give a toluene solution (4.5 L) containing 4-methoxyphenyl 3,6-di-O-benzyl-2-deoxy-2-(1,3-dioxo-1,3-dihydro-2H-isoindol-2-yl)-4-O-(2,3,4,6-tetra-O-benzyl-β-D-galactopyranosyl)-β-D-glucopyranoside (the compound represented by Formula B-5).

### Example 8

### 3,6-di-O-benzyl-2-deoxy-2-(1,3-dioxo-1,3-dihydro-2H-isoindol-2-yl)-4-O-(2,3,4,6-tetra-O-benzyl-β-D-galactopyranosyl)-β-D-glucopyranose(the compound represented by Formula B-8)

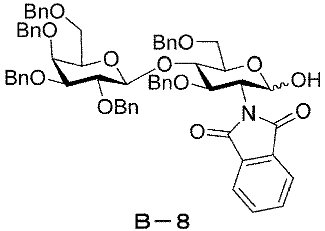

To a toluene solution (4.5 L) containing 4-methoxyphenyl 3,6-di-O-benzyl-2-deoxy-2-(1,3-dioxo-1,3-dihydro-2H-isoindol-2-yl)-4-O-(2,3,4,6-tetra-O-benzyl-β-D-galactopyranosyl)-β-D-glucopyranoside (the compound represented by Formula B-5) were added dichloromethane (6.75 L), 1,1,1,3,3,3-hexafluoro-2-propanol (6.75 L), and water (675 mL), respectively, at 15°C to 25°C. A suspension containing bis(trifluoroacetoxy)iodobenzene (2.16 kg, 5.02 mol) in dichloromethane (6.75 L) was added in 10 portions, and the mixture was stirred at 15°C to 25°C for 20 hours. The mixture was cooled to 0°C to 5°C, toluene (31.5 L) was added, and the resulting mixture was then liquid-separated and washed twice with a sodium bicarbonate (900 g)-sodium sulfite (900 g)-water (22.5 L) solution at 0°C to 20°C. The resulting organic layer was liquid-separated and washed with a sodium chloride (4.5 kg)-water (18 L) solution. The resulting organic layer was then concentrated under reduced pressure to 9 L, and toluene (22.5 L) was added. The mixture was then concentrated under reduced pressure to 9 L. Toluene (36 L) was added to the resulting solution, and the mixture was divided into two portions. Silica gel (4.5 kg) was added to each divided solution and stirred at 20°C to 25°C for 3 hours. The silica gel was then filtered and washed with toluene (45 L). The resulting silica gel was washed with an ethyl acetate (7.5 L)-toluene (22.5 L)-mixed solution. In this way, desorption from the silica gel was performed. The resulting organic layer was mixed and then concentrated under reduced pressure to 9 L to give a toluene solution (9 L) containing 3,6-di-O-benzyl-2-deoxy-2-(1,3-dioxo-1,3-dihydro-2H-isoindol-2-yl)-4-O-(2,3,4,6-tetra-O-benzyl-β-D-galactopyranosyl)-β-D-glucopyranose (the compound represented by Formula B-8).

¹H-NMR (500 MHz, CDCl₃) δ 7.60-7.87 (m, 4H), 7.18-7.36 (m, 25H), 6.96 (d, J = 6.9 Hz, 2H), 6.89 (m, 1H), 6.83 (dd, J = 7.4, 10 Hz, 2H), 5.33 (dd, J = 1.9, 3.7 Hz, 0.2H), 5.30 (dd, J = 4.3, 8.6 Hz, 0.8H), 4.92 (dd, J = 9.9, 12 Hz, 2H), 4.78-4.86 (m, 2H), 4.71 (d, J = 2.3 Hz, 2H), 4.44-4.61 (m, 3H), 4.36-4.14 (m, 3H), 4.34 (d, J = 4.6 Hz, 1H), 4.26 (d, J = 12 Hz, 1H), 4.03-4.12 (m, 2H), 3.89 (d, J = 2.8 Hz, 1H), 3.85 (dd, J = 4.0, 11 Hz, 1H), 3.77 (dd, J = 7.7, 9.7 Hz, 1H), 3.67 (dd, J = 1.6, 11 Hz, 1H), 3.56-3.60 (m, 1H), 3.35-3.49 (m, 4H), 2.88 (d, J = 8.6 Hz, 1H).

¹³C-NMR (125 MHz, CDCl₃) δ 168.2, 139.06, 139.04, 138.98, 138.91, 138.7, 138.6, 138.5, 138.10, 138.06, 133.8, 131.7, 128.40, 128.38, 128.32, 128.27, 128.24, 128.10, 127.99, 127.96, 127.90, 127.84, 127.78, 127.76, 127.72, 127.68, 127.65, 127.63, 127.55, 127.51, 127.49, 127.44, 127.42, 127.3, 126.8, 123.6, 123.3, 102.9, 102.8, 93.1, 92.8, 82.34, 82.32, 80.02, 79.99, 77.8, 77.7, 76.6, 75.43, 75.39, 75.31, 74.53, 74.49, 74.33, 73.98, 73.72, 73.63, 73.41, 73.24, 73.20, 73.07, 72.63, 72.59, 70.6, 68.3, 68.0, 67.8, 57.7, 55.8,

HRMS (ESI⁺) [M+Na]⁺ calcd for C₆₂H₆₁NNaO₁₂: 1034.4092; found 1034.4071. [α]_{D}²⁰ = +33.243 (c 1.002, CDCl₃).

### Example 9

### 3,6-Di-O-benzyl-2-deoxy-2-(1,3-dioxo-1,3-dihydro-2H-isoindol-2-yl)-4-O-(2,3,4,6-tetra-O-benzyl-β-D-galactopyranosyl)-1-O-(2,2,2-trifluoro-N-phenylethanimidoyl)-β-D-glucopyranose(the compound represented by Formula A-11)

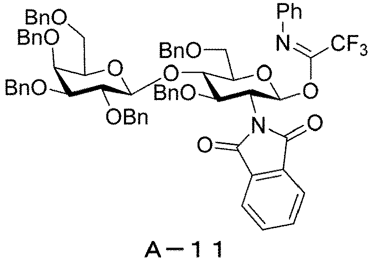

To a toluene solution (total amount: 1002 g) containing 3,6-di-O-benzyl-2-deoxy-2-(1,3-dioxo-1,3-dihydro-2H-isoindol-2-yl)-4-O-(2,3,4,6-tetra-O-benzyl-β-D-galactopyranosyl)-β-D-glucopyranose (the compound represented by Formula B-8) (solution obtained from Examples 7 and 8 and derived from 500 g, 0.349 mol of the compound represented by Formula B-6) were added dichloromethane (2.0 L), Molecular Sieves 4A powder (10 µm or less, 250 g), and N-methylimidazole (34.4 g, 0.419 mol) at 20°C to 30°C. The mixture was then mixed with 2,2,2-trifluoro-N-phenylacetimidoyl chloride (79.8 g, 0.384 mmol), and stirred at 20°C to 30°C for 17 hours. The Molecular Sieves 4A was filtered, and then washed with toluene (500 mL). The resulting solution was cooled to 0°C to 10°C. The solution was made to pass through a column packed with silica gel (1.5 kg) wetted with dichloromethane cooled to 0°C to 5°C. The filtrate was washed with dichloromethane (15 L) cooled to 0°C to 5°C, and was fractionated every 2.5 L to 3 L to obtain fractions. Next, the fractions were washed with 3% ethyl acetate-containing dichloromethane (10 L) mixture, and were fractionated every 2.5 L to 3 L to obtain fractions. The first to seventh fractions obtained were combined and concentrated under reduced pressure to 1 L to give a toluene-dichloromethane mixed solution containing 3,6-di-O-benzyl-2-deoxy-2-(1,3-dioxo-1,3-dihydro-2H-isoindol-2-yl)-4-O-(2,3,4,6-tetra-O-benzyl-β-D-galactopyranosyl)-1-O-(2,2,2-trifluoro-N-phenylethanimidoyl)-β-D-glucopyranose (the compound represented by Formula A-11). This solution was used as it was in Example 27.

¹H-NMR (500 MHz, CDC1₃) δ 7.62-7.82 (m, 4H), 7.01-7.53 (m, 31H), 6.98-7.01 (m, 1H), 6.95 (d, J = 7.2 Hz, 2H), 6.80-6.88 (m, 3H), 6.64 (brd, 2H), 4.93 (d, J = 12 Hz, 1H), 4.89 (d, J = 12 Hz, 1H), 4.83 (d, J = 11 Hz, 1H), 4.77 (d, J = 11 Hz, 1H), 4.67-4.72 (m, 2H), 4.56 (d, J = 12 Hz, 1H), 4.53 (d, J = 12 Hz, 1H), 4.36-4.48 (m, 5H), 4.28 (d, J = 12 Hz, 1H), 4.14 (t, J = 8.7 Hz, 1H), 3.89 (d, J = 2.6 Hz, 1H), 3.85 (d, J = 7.7 Hz, 1H), 3.76 (t, J = 8.4 Hz, 1H), 3.38-3.50 (m, 4H).

¹³C-NMR (125 MHz, CDCl₃) δ 167.6, 143.4, 143.1, 139.0, 138.68, 138.66, 138.5, 137.08, 137.06, 135.2, 133.92, 131.5, 129.3, 129.1, 129.0, 128.5, 128.41, 128.37, 128.31, 128.27, 128.22, 128.90, 127.86, 127.69, 127.56, 127.45, 127.44, 127.2, 126.9, 126.2, 124.3, 123.4, 120.6, 120.5, 119.3, 116.3 (q, J = 148.5 Hz), 102.9, 93.5, 82.3, 79.9, 76.6, 76.0, 75.4, 74.5, 73.61, 73.41, 73.08, 73.05, 72.6, 68.3, 67.2, 54.8.

HRMS (ESI⁺) [M+Na]⁺ calcd for C₇₀H₆₅F₃N₂NaO₁₂: 1205.4387; found 1205.43835. [α]_{D}²⁰ = +66.645 (c 1.099, CDCl₃).

### <To synthesize the compound represented by Formula A-13>

The compound represented by Formula A-13 was synthesized according to the following synthesis scheme 3.

### [Synthesis Scheme 3]

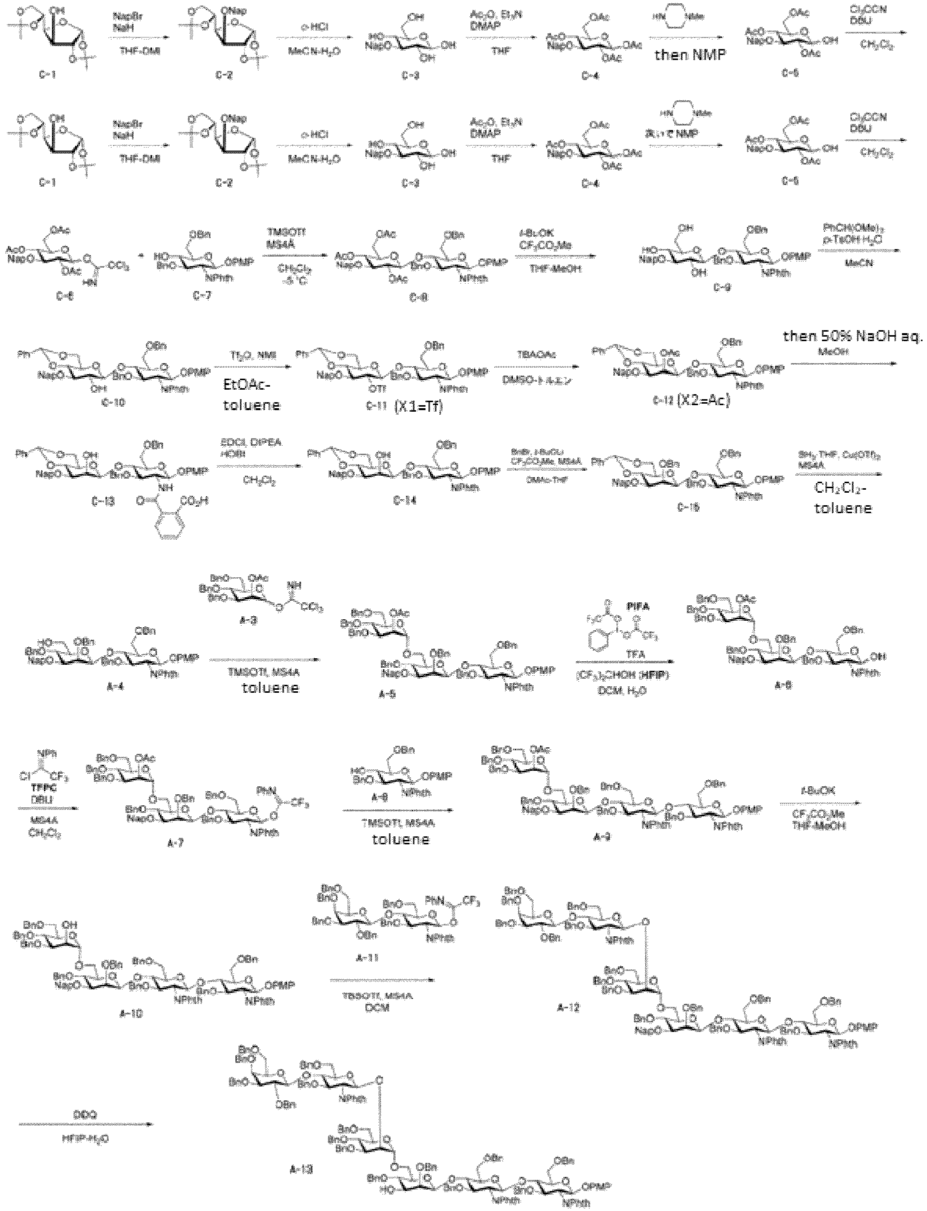

### Example 10

### 1,2:5,6-Bis-O-(1-methylethyliden)-3-O-(2-naphthylmethyl)-α-D-glucofuranose(the compound represented by Formula C-2)

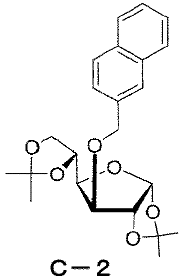

A tetrahydrofuran (900 mL) solution containing sodium hydride (55.32 g, 1.38 mol, content: 50-72%) was cooled to 0°C. Next, a tetrahydrofuran (1.05 L) solution containing 1,2:5,6-bis-O-(1-methylethyliden)-α-D-glucofuranose (the compound represented by Formula C-1) (300.00 g, 1.15 mol) was added dropwise over 1 hour. The temperature was then raised to 25°C and 1,3-dimethyl-2-imidazolidinone (150 mL) and 2-bromomethylnaphthalene (280.31 g, 1.27 mol) were added. After stirring at 25°C for 6 hours, the completion of the reaction was checked by HPLC. Ethylenediamine (anhydrous) (13.85 g, 230.52 mmol) was added, and the mixture was stirred for another 1 hour. The solution was cooled to 0°C and 10% aqueous citric acid solution (1.2 L) was added over 1 hour. The reaction liquid was diluted with heptane (3 L) and separated into an organic layer and an aqueous layer. The organic layer was washed with water (900 mL) and concentrated under reduced pressure until the liquid volume reached 900 mL. Acetonitrile (3 L) was further added, and the mixture was concentrated again until the liquid volume reached 900 mL to prepare, as an acetonitrile solution, crude 1,2:5,6-bis-O-(1-methylethyliden)-3 -O-(2-naphthylmethyl)-α-D-glucofuranose (the compound represented by Formula C-2). This compound was used as it was in the next step.

### Example 11

### 3-O-(2-Naphthylmethyl)-D-glucopyranose(the compound represented by Formula C-3)

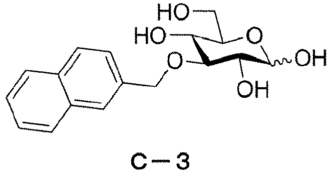

To a solution (900 mL) containing the crude compound represented by Formula C-2 obtained in Example 10 were added acetonitrile (1.5 L), water (600 mL), and concentrated hydrochloric acid (17.51 g, 172.89 mmol), and the mixture was stirred at 55°C for 18.5 hours. After the completion of the reaction was checked by HPLC, the reaction liquid was cooled to 0°C and the pH in the system was adjusted to 6.25 with a 4 N aqueous sodium hydroxide solution (43.22 mL). The reaction liquid was diluted with heptane (900 mL) and separated into an acetonitrile layer and a heptane layer. Ethyl acetate (2.4 L) and water (600 mL) were added to the acetonitrile layer, and the mixture was liquid-separated to give an organic layer A and an aqueous layer. A mixed solution of ethyl acetate (1.5 L) and tetrahydrofuran (1.5 L) was added to the aqueous layer again, and the mixture was liquid-separated to give an organic layer B and an aqueous layer. The organic layers A and B were mixed, washed with saturated brine (600 mL), and concentrated under reduced pressure until the liquid volume reached 1.5 L (crystals were found to precipitate during the concentration step). Ethyl acetate (4.5 L) was further added, and the mixture was concentrated again until the liquid volume reached 3 L. To this suspension were added ethyl acetate (1.5 L) and cyclopentyl methyl ether (1.5 L), and the mixture was stirred at 55°C for 1 hour. Heptane (3 L) was added dropwise over 1.5 hours. After stirring for 1 hour, the mixture was cooled to 0°C. After that, the precipitated crystals were filtered and washed with an ethyl acetate (1.2 L)/heptane (600 mL) mixed solution cooled to 0°C. The resulting crystals were dried at 40°C under reduced pressure to afford 3-O-(2-naphthylmethyl)-D-glucopyranose (the compound represented by Formula C-3) (356.95 g, yield 96.7%).

¹H-NMR (500 MHz, DMSO-d₆) δ 7.85-7.90 (m, 4H), 7.59 (dd, J = 8.0, 1.5 Hz, 1H), 7.46-7.51 (m, 2H), 6.69 (d, J = 6.0 Hz, 1H), 5.12 (dd, J = 5.0, 3.0 Hz, 2H), 4.94-5.00 (m, 2H), 4.53 (t, J = 6.0 Hz, 1H), 4.35 (dd, J = 8.0, 6.5 Hz, 1H), 3.70 (ddd, J = 11.5, 5.0, 2.0 Hz, 1H), 3.45-3.50 (m, 1H), 3.25-3.31 (m, 2H), 3.11-3.16 (m, 2H).

¹³C-NMR (125 MHz, DMSO-d₆) δ 137.3, 132.8, 132.3, 127.6, 127.5, 127.4, 126.1, 126.0, 125.6, 125.5, 96.9, 85.4, 76.7, 74.8, 73.7, 69.9, 61.1.

HRMS (ESI⁻) [M-H]⁻ calcd for C₁₇H₂₀O₆: 320.1260; found 319.1175.

### Example 12

### 2,4,6-Tri-O-acetyl-3-O-(2-naphthylmethyl)-D-glucopyranose(the compound represented by Formula C-5)

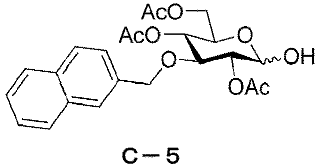

To a tetrahydrofuran (675 mL) solution containing 3-O-(2-naphthylmethyl)-D-glucopyranose (the compound represented by Formula C-3) (150.00 g, 468.25 mmol) were added triethylamine (236.92 g, 2.34 mol) and 4-dimethylaminopyridine (0.29 g, 2.34 mmol). After the mixture was cooled to 0°C, acetic anhydride (195.99 g, 1.92 mol) was added dropwise over 30 minutes. The temperature was then raised to 25°C, and after 3 hours of stirring, the completion of the reaction was checked by HPLC. The reaction liquid was cooled to 10°C and 1-methylpiperazine (60.97 g, 608.73 mmol) was added. After stirring at 35°C for 18 hours, the completion of the reaction was checked by HPLC. The mixture was then cooled to 0°C. The pH was adjusted to 6.36 with 6 N aqueous hydrochloric acid (480 mL), and the solution was diluted with heptane (375 mL) and separated into an organic layer and an aqueous layer. The organic layer was washed with a saturated sodium bicarbonate aqueous solution (450 mL) and then water (450 mL) and concentrated under reduced pressure until the liquid volume reached 450 mL. Ethyl acetate (2.25 L) was added, the mixture was concentrated again until the liquid volume reached 450 mL, and the same operation was repeated one more time. Dichloromethane (2.25 L) was added to this solution, the mixture was concentrated until the liquid volume reached 450 mL, and the same operation was repeated one more time to prepare, as a dichloromethane solution, crude 2,4,6-tri-O-acetyl-3-O-(2-naphthylmethyl)-D-glucopyranose (the compound represented by Formula C-5). This compound was used as it was in the next step.

The step from the compound represented by Formula C-3 to the compound represented by Formula C-5 was carried out in a one-pot process.

### Example 13

### 2,4,6-Tri-O-acetyl-3-O-[(naphthalen-2-yl)methyl]-1-O-(2,2,2-trichloroethanimidoyl)-D-glycero-hexopyranose(the compound represented by Formula C-6)

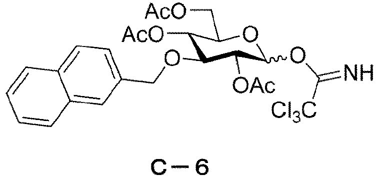

To a solution (450 ml) containing the crude 2,4,6-tri-O-acetyl-3-O-(2-naphthylmethyl)-D-glucopyranose (the compound represented by Formula C-5) obtained in Example 12 were added dichloromethane (450 mL) and trichloroacetonitrile (338.03 g, 2.34 mol), and the mixture was cooled to 0°C. Next, 1,8-diazabicyclo[5.4.0]-7-undecene (5.70 g, 37.46 mmol) was added dropwise. After stirring at 0°C for 14.5 hours, the completion of the reaction was checked by HPLC. Acetic acid (2.25 g, 37.46 mmol) was then added. Silica gel 60N (particle size: 40 to 50 µm; 150 g; available by KANTO CHEMICAL CO., INC.) was added to the solution, and the mixture was stirred for 1.5 hours and then filtered. The silica gel was washed with dichloromethane (1.5 L) and the filtrate was concentrated under reduced pressure until the liquid volume reached 450 mL. Dichloromethane (1.5 L) was further added, and the solution was concentrated until the liquid volume reached 450 mL to prepare, as a dichloromethane solution, 2,4,6-tri-O-acetyl-3-O-[(naphthalen-2-yl)methyl]-1-O-(2,2,2-trichloroethanimidoyl)-D-glycero-hexopyranose (the compound represented by Formula C-6). This compound was used as it was in the next step.

### Example 14

### 4-Methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside(the compound represented by Formula C-8)

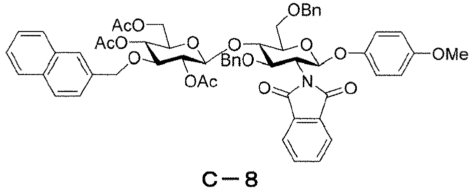

To a solution (450 mL) containing the 2,4,6-tri-O-acetyl-3-O-[(naphthalen-2-yl)methyl]-1-O-(2,2,2-trichloroethanimidoyl)-D-glycero-hexopyranose (the compound represented by formula -6) obtained in Example 13 were added 4-methoxyphenyl 3,6-di-O-benzyl-2-deoxy-2-(1,3-dioxo-1,3-dihydro-2H-isoindol-2-yl)-β-D-glucopyranoside (the compound represented by C-7) (276.66 g, 468.25 mmol), dichloromethane (4.2 L), and Molecular Sieves 4A powder (10µm or less, 83.00 g), and the mixture was cooled to -5°C. Trimethylsilyl trifluoromethanesulfonate (10.41 g, 46.83 mmol) was added dropwise to this suspension over 20 minutes, and the mixture was then stirred for 3 hours. After the completion of the reaction was checked by HPLC, triethylamine (23.69 g, 234.13 mmol) was added. After the suspension was filtered, the filtrate was washed with ethyl acetate (2.8 L) and concentrated under reduced pressure until the liquid volume reached 1.4 L. Ethyl acetate (4.2 L) was further added, the mixture was concentrated until the liquid volume reached 1.4 L, and the same operation was repeated one more time. To this solution was added ethyl acetate (2.8 L), and the mixture was washed with a saturated sodium bicarbonate aqueous solution (830 mL) and water (830 mL). The resulting organic layer was then concentrated under reduced pressure to a liquid volume of 830 mL. Next, 2-propanol (4.2 L) was added, and the mixture was concentrated to a liquid volume of 1.4 L. The suspension was then warmed to 65°C. Ethyl acetate (830 mL) was added. After the mixture was stirred at 65°C for 2 hours, 2-propanol (5.53 L) was added dropwise over 2 hours. After this suspension was cooled to 0°C, the crystals were filtered and washed with 2-propanol (1.4 L) cooled to 0°C. The resulting crystals were dried under reduced pressure at 40°C to give crude 4-methoxyphenyl 3,6-di-O-benzyl-2-deoxy-2-(1,3-dioxo-1,3-dihydro-2H-isoindol-2-yl)-4-O- f 2,4,6-tri-O-acetyl-3-O-[(naphthalen-2-yl)methyl]-β-D-glucopyranosyl}-β-D-glucopyranoside (the compound represented by Formula C-8) (355.34 g, yield 90.5%, in terms of compound represented by Formula C-3).

The resulting crude compound represented by Formula C-8 (350.00 g) was mixed with methyl isobutyl ketone (2.1 L). The compound was dissolved at 50°C, and ethyl cyclohexane (1.4 L) was added dropwise over 1 hour. 4-Methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (the compound represented by Formula C-8) (70.00 mg) were added, and the mixture was stirred for 1 hour. After the crystals were found to precipitate, ethylcyclohexane (4.9 L) was added dropwise over 2 hours. The suspension was cooled to room temperature and stirred for 14.5 hours. The precipitated crystals were then filtered, and washed with a mixed solution of methyl isobutyl ketone (350 mL) and ethyl cyclohexane (1.4 L). The resulting crystals were dried under reduced pressure at 40°C to give 4-methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (the compound represented by Formula C-8) (331.74 g, yield 94.8%).

¹H-NMR (500 MHz, CDC1₃) δ 7.81-7.84 (m, 4H), 7.69 (br, 1H), 7.65 (br, 3H), 7.46-7.51 (m, 2H), 7.28-7.36 (m, 6H), 7.00 (dd, J = 7.0, 1.5 Hz, 2H), 6.77-6.84 (m, 5H), 6.66-6.69 (m, 2H), 5.59, (d, J = 9.0 Hz, 1H), 5.09-5.15 (m, 2H), 4.82 (d, J = 12.5 Hz, 1H), 4.77 (d, J = 12.0 Hz, 1H), 4.71-4.77 (m, 2H), 4.60 (d, J = 8.0 Hz, 1H), 4.50 (d, J = 12.5 Hz, 1H), 4.45 (d, J = 13.0 Hz, 1H), 4.36 (dd, J = 11.0, 8.5 Hz, 1H), 4.28 (dd, J = 11.0, 8.5 Hz, 1H), 4.20 (dd, J = 12.5, 5.0 Hz, 1H), 4.10 (dd, J = 10.0, 8.5 Hz, 1H), 3.99 (dd, J = 12.0, 2.0 Hz, 1H), 3.80 (br, 2H), 3.69 (s, 3H), 3.58-3.62 (m, 2H), 3.44 (ddd, J = 10.0, 4.5, 2.5 Hz, 1H), 1.98 (s, 3H), 1.938 (s, 3H), 1.937 (s, 3H).
¹³C-NMR (125 MHz, CDCl₃) δ 171.0, 169.5, 169.1, 155.6, 151.0, 138.7, 138.2, 135.5, 133.9, 133.4, 133.2, 128.7, 128.4, 128.23, 128.20, 128.06, 128.05, 127.9, 127.2, 126.5, 126.2, 125.7, 123.5, 118.9, 114.5, 100.7, 97.8, 80.6, 78.5, 76.8, 75.2, 74.8, 74.1, 73.8, 73.1, 72.1, 69.9, 67.8, 62.2, 55.78, 55.75, 21.1, 20.94, 20.85.

HRMS (ESI⁺) [M+H]⁺ calcd for C₅₈H₅₈NO₁₆: 1024.3750; found 1024.3706.

The spectrum was found to correspond to the following literature: Org. Biomol. Chem., 2018, 16, 4720-4727.

### Example 15

### 4-Methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside(the compound represented by Formula C-9)

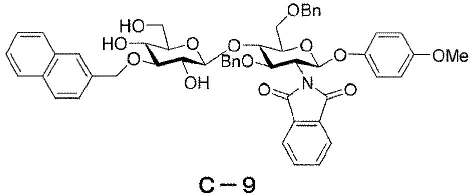

To a tetrahydrofuran (150 mL) solution containing 4-methoxyphenyl 3,6-di-O-benzyl-2-deoxy-2-(1,3-dioxo-1,3-dihydro-2H-isoindol-2-yl)-4-O-{2,4,6-tri-O-acetyl-3-O-[(naphthalen-2-yl)methyl]-β-D-glucopyranosyl}-β-D-glucopyranoside (the compound represented by Formula C-8) (30.00 g, 29.29 mmol) were added methanol (90 mL) and methyl trifluoroacetate (3.75 g, 29.29 mmol). The mixture was stirred at 25°C for 10 min, and potassium tert-butoxide (1 mol/L tetrahydrofuran solution) (14.7 mL, 14.65 mmol) was then added. Next, the temperature was raised to 55°C. After stirring for 2 hours, the completion of the reaction was checked by HPLC. The reaction liquid was cooled to 25°C and acetic acid (1.76 g, 29.29 mmol) and ethyl acetate (300 mL) were added in this order. This solution was washed twice with 1% sodium chloride aqueous solution (300 mL) and concentrated under reduced pressure to a liquid volume of 90 mL. Ethyl acetate (450 mL) was added, and the mixture was concentrated again until the liquid volume reached 90 mL. Acetonitrile (450 mL) was further added, and the mixture was concentrated until the liquid volume reached 90 mL to prepare, as an acetonitrile solution, 4-methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside (the compound represented by Formula C-9). This compound was used as it was in the next step.

### Example 16

### 4-Methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside(the compound represented by Formula C-10)

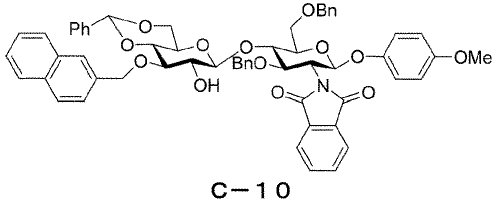

To a solution (90 mL) containing the 4-methoxyphenyl 3,6-di-O-benzyl-2-deoxy-2-(1,3-dioxo-1,3-dihydro-2H-isoindol-2-yl)-4-O-{3-O-[(naphthalen-2-yl)methyl]-β-D-glucopyranosyl}-β-D-glucopyranoside (the compound represented by Formula C-9) were added acetonitrile (210 mL), benzaldehyde dimethyl acetal (5.13 g, 33.69 mmol), and p-toluenesulfonic acid monohydrate (0.17 g, 0.88 mmol), and the mixture was stirred at 25°C for 30 minutes. Toluene (600 mL) was added to this solution, and the solution was concentrated until the liquid volume reached 300 mL. At this time point, the completion of the reaction was checked by HPLC. Further, 1-methylimidazole (12.03 g, 146.47 mmol) was added, and the mixture was concentrated until the liquid volume reached 90 mL to prepare, as a 1-methylimidazole-containing toluene solution, 4-methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside (the compound represented by Formula C-10). This compound was used as it was in the next step.

### Example 17

### 4-Methoxyphenyl 3,6-di-O-benzyl-4-O-{4,6-O-benzyliden-3-O-[(naphthalen-2-yl)methyl]-2-O-(trifluoromethanesulfonyl)-β-D-glucopyranosyl}-2-deoxy-2-(1,3-dioxo-1,3-dihydro-2H-isoindol-2-yl)-β-D-glucopyranoside(the compound represented by Formula C-11, wherein X₁ is a Tf group)

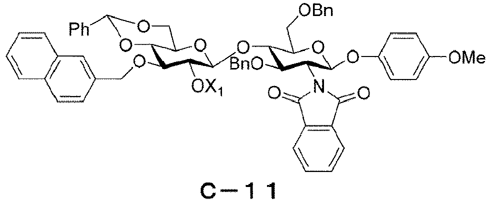

To a (90 mL, 1-methylimidazole-containing) solution containing the 4-methoxyphenyl 3,6-di-O-benzyl-4-O-{4,6-O-benzyliden-3-O-[(naphthalen-2-yl)methyl]-β-D-glucopyranosyl}-2-deoxy-2-(1,3-dioxo-1,3-dihydro-2H-isoindol-2-yl)-β-D-glucopyranoside (the compound represented by Formula C-10) obtained in Example 16 was added ethyl acetate (210 mL), and the mixture was cooled to 0°C. Trifluoromethanesulfonic anhydride (16.53 g, 58.59 mmol) was added dropwise to this solution over 1 hour, followed by stirring for 30 minutes. After the completion of the reaction was checked by HPLC, water (300 mL) was added and organic and aqueous layers were separated. The organic layer was washed twice with water (300 mL) and once with a saturated sodium chloride aqueous solution (150 mL) and then concentrated under reduced pressure to a liquid volume of 90 mL. Ethyl acetate (300 mL) was added, and the mixture was concentrated again until the liquid volume reached 90 mL to prepare, as an ethyl acetate solution, 4-methoxyphenyl 3,6-di-O-benzyl-4-O-{4,6-O-benzyliden-3-O-[(naphthalen-2-yl)methyl]-2-O-(trifluoromethanesulfonyl)-β-D-glucopyranosyl}-2-deoxy-2-(1,3-dioxo-1,3-dihydro-2H-isoindol-2-yl)-β-D-glucopyranoside(the compound represented by Formula C-11, wherein X₁ is a Tf group). This compound was used as it was in the next step.

### Example 18

### 4-Methoxyphenyl 3,6-di-O-benzyl-4-O-{4,6-O-benzyliden-3-O-[(naphthalen-2-yl)methyl]-P-D-mannopyranosyl}-2-(2-carboxybenzamido)-2-deoxy-P-D-glucopyranoside(the compound represented by Formula C-13)

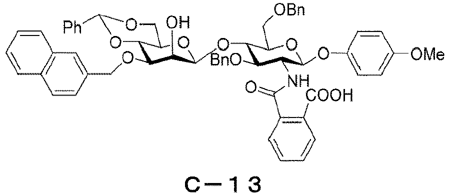

To a solution (90 mL) containing 4-methoxyphenyl 3,6-di-O-benzyl-4-O-{4,6-O-benzyliden-3-O-[(naphthalen-2-yl)methyl]-2-O-(trifluoromethanesulfonyl)-β-D-glucopyranosyl}-2-deoxy-2-(1,3-dioxo-1,3-dihydro-2H-isoindol-2-yl)-β-D-glucopyranoside (the compound represented by Formula C-11 (wherein X₁ is a Tf group)) obtained in Example 17 were added dimethyl sulfoxide (150 mL) and tetrabutylammonium acetate (17.67 g, 58.59 mmol). Next, the temperature was raised to 30°C. After 17 hours of stirring, the completion of the reaction was checked by HPLC. Toluene (150 mL) was added to this reaction liquid, and the mixture was concentrated under reduced pressure until the liquid volume reached 165 mL. Methanol (45 mL) and 50% aqueous sodium hydroxide solution (3.52 g, 87.88 mmol) were added, and the mixture was stirred at 25°C for 1.5 hours. After the completion of the reaction was checked by HPLC, ethyl acetate (450 mL) and water (300 mL) were added for liquid separation. Water (300 mL) was added to the resulting organic layer. The mixture was cooled to 0°C, and adjusted to pH 2.73 with 6 N hydrochloric acid under strong stirring. Tetrahydrofuran (300 mL) was added to the separated organic layer, and the mixture was concentrated under reduced pressure until the liquid volume reached 150 mL. Tetrahydrofuran (300 mL) was added, the mixture was concentrated again until the liquid volume reached 90 mL, and the internal temperature was then adjusted to 45°C. Tetrahydrofuran (60 mL) was added, and the mixture was then cooled to 25°C. Next, 2-propanol (150 mL) and water (15 mL) were added. 4-Methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (the compound represented by Formula C-13) (30 mg) were then added. The mixture was stirred at 25°C for 14 hours, and after the crystals were found to precipitate, 2-propanol (210 mL) was added dropwise over 1 hour. The mixture was then cooled to 0°C. After stirring for 2 hours, the precipitated crystals were filtered and washed with 2-propanol (150 mL) cooled to 0°C. The resulting crystals were dried under reduced pressure at 40°C to give 4-methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (the compound represented by Formula C-13) (27.74 g, yield 94.3%, in terms of compound represented by Formula C-8).
*The step from the compound represented by Formula C-12 to the compound represented by Formula C-13 was carried out in a one-pot process.
*For the seed crystals, a part of the reaction solution was divided into small portions and concentrated to precipitate solids for use.
*This reaction proceeds well with tetrabutylammonium acetate available by TOKYO CHEMICAL INDUSTRY CO., LTD. (product code: T2694, purity: >90.0%) or Sigma-Aldrich (product code: 86849, purity: >90%). In other manufacturers, tetrabutylammonium acetate may contain excess acetic acid, in which case the reaction tends to be significantly slow. As an alternative method, a similar conversion reaction is possible using cesium acetate (see details below).
*The conversion of the compound represented by Formula C-11 to the compound represented by Formula C-12 was also feasible under the conditions using cesium acetate (3 equivalents) and dimethyl sulfoxide at 50°C for 24 hours. Subsequently, substantially the same reaction (the compound represented by Formula C-12 -> compound represented by Formula C-13) was followed by post-treatment to prepare the compound represented by Formula C-13.

¹H-NMR (500 MHz, CDC1₃) δ 8.02 (dd, J = 6.0, 2.0 Hz, 1H), 7.69-7.83 (m, 4H), 7.38-7.49 (m, 12H), 7.32-7.34 (m, 2H), 7.16-7.29 (m, 8H), 6.97 (ddd, J = 9.0, 4.0, 2.5 Hz, 2H), 6.76 (ddd, J = 9.5, 3.5, 2.5 Hz, 2H), 5.51 (s, 1H), 5.40 (d, J = 6.0 Hz, 1H), 4.83-4.91 (m, 3H), 4.76 (d, J = 11.5 Hz, 1H), 4.55 (d, J = 0.5 Hz, 1H), 4.49 (d, J = 12.0 Hz, 1H), 4.36 (d, J = 12.0 Hz, 1H), 4.26-4.30 (m, 1H), 4.16 (t, J = 6.5 Hz, 1H), 4.05-4.09 (m, 2H), 3.99 (dd, J = 3.0, 0.5 Hz, 1H), 3.93 (t, J = 9.5 Hz, 1H), 3.80-3.86 (m, 2H), 3.71 (s, 3H), 3.65-3.69 (m, 1H), 3.56 (t, J = 10.0 Hz, 1H), 3.51 (dd, J = 10.0, 3.5 Hz, 1H), 3.13 (td, J = 9.5, 5.0 Hz, 1H).

¹³C-NMR (125 MHz, CDCl₃) δ 170.9, 168.4, 155.3, 151.4, 138.7, 138.0, 137.6, 136.2, 135.4, 133.4, 133.3., 132.2, 132.1, 130.7, 130.3, 129.2, 128.6, 128.49, 128.45, 128.11, 128.07, 128.0, 127.89, 127.87, 127.8, 126.8, 126.4, 126.3, 126.2, 125.8, 118.6, 114.7, 101.7, 100.4, 99.1, 78.3, 76.7, 76.4, 75.1, 73.7, 73.3, 72.5, 69.9, 69.4, 68.5, 67.0, 55.8, 54.4. HRMS(ESI⁺)[M+H]⁺ calcd for C₅₉H₅₈NO₁₄: 1004.3852; found 1004.3873.

### Example 19

### 4-Methoxyphenyl 3,6-di-O-benzyl-4-O-{4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (the compound represented by Formula C-14).

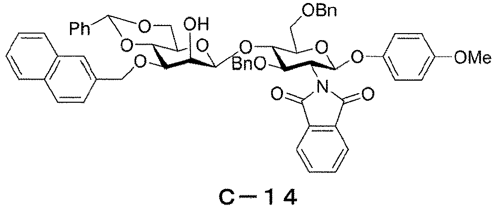

To a dichloromethane (30 mL) solution containing 4-methoxyphenyl 3,6-di-O-benzyl-4-O-{4,6-O-benzyliden-3-O-[(naphthalen-2-yl)methyl]-β-D-mannopyranosyl}-2-(2-carboxybenzamido)-2-deoxy-β-D-glucopyranoside (the compound represented by Formula C-13) (6.00 g, 5.98 mmol) were added 1-hydroxybenzotriazole monohydrate (0.18 g, 1.20 mmol), N,N-diisopropylethylamine (0.85 g, 6.57 mmol), and 1-(3-dimethylaminopropyl)-3-ethylcarbodiimide hydrochloride (1.26 g, 6.57 mmol). The temperature was raised to 40°C, and the mixture was stirred for 31 hours. After the completion of the reaction was checked by HPLC, the reaction solution was cooled to 0°C. Ethyl acetate (90 mL) and water (60 mL) were then added, and under strong stirring, 6 N hydrochloric acid was added until pH 7 was reached. An organic layer and an aqueous layer were separated. The resulting organic layer was washed with water (60 mL) and then saturated brine (30 mL). The solution was concentrated under reduced pressure until the liquid volume reached 12 mL. Tetrahydrofuran (60 mL) was added, and the mixture was concentrated again until the liquid volume reached 9 mL to prepare a tetrahydrofuran solution containing crude 4-methoxyphenyl 3,6-di-O-benzyl-4-O-{4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (the compound represented by Formula C-14). This compound was used as it was in the next step.

### Example 20

### 4-Methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside(the compound represented by Formula C-15)

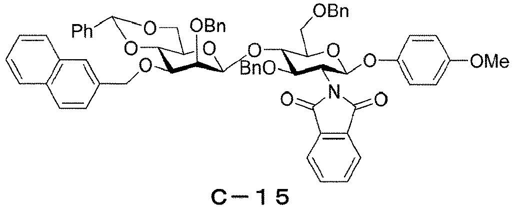

To a solution containing the crude 4-methoxyphenyl 3,6-di-O-benzyl-4-O-{4,6-O-benzyliden-3-O-[(naphthalen-2-yl)methyl]-β-D-mannnopyranosyl}-2-deoxy-2-(1,3-dioxo-1,3-dihydro-2H-isoindol-2-yl)-β-D-glucopyranoside (the compound represented by Formula C-14) were added N,N-dimethylacetamide (60 mL), benzyl bromide (1.53 g, 8.96 mmol), methyl trifluoroacetate (0.15 g, 1.20 mmol), and Molecular Sieves 4A (1.8 g). The mixture was cooled to 0°C. A lithium tert-butoxide-containing tetrahydrofuran solution (note that this solution was used and prepared such that a mixture of 2-methyl-2-propanol (0.66 g, 8.96 mmol) and tetrahydrofuran (2.4 mL) was cooled to 0°C, and a hexane solution containing normal butyl lithium (1.55 mol/L) (5.78 mL, 8.96 mmol) was added, and the mixture was stirred for 30 minutes) was added, and the mixture was stirred for 3 hours. After the completion of the reaction was checked by HPLC, ethylenediamine (anhydrous) (0.18 g, 2.99 mmol) was added. The mixture was stirred for another 1 hour. Acetic acid (0.72 g. 11.95 mmol) was added to this solution, and the mixture was filtered. After the Molecular Sieves 4A was washed with ethyl acetate (90 mL), water (60 mL) was added for liquid separation. The organic layer was washed twice with water (60 mL) and then concentrated under reduced pressure until the liquid volume reached 12 mL. Toluene (30 mL) was added, and the mixture was concentrated again until the liquid volume reached 12 mL. Toluene (18 mL) and silica gel 60N (spherical; particle size: 40 to 50 µm; available by KANTO CHEMICAL CO., INC.) (9 g) were then added, and the mixture was stirred at 25°C for 30 minutes. The suspension was filtered, and the silica gel was washed with a mixed solution of toluene (191 mL) and ethyl acetate (19 mL). The filtrate was concentrated under reduced pressure until the liquid volume reached 9 mL to prepare a toluene solution containing crude 4-methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (the compound represented by Formula C-15).

### Example 21

### 4-Methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside(the compound represented by Formula A-4)

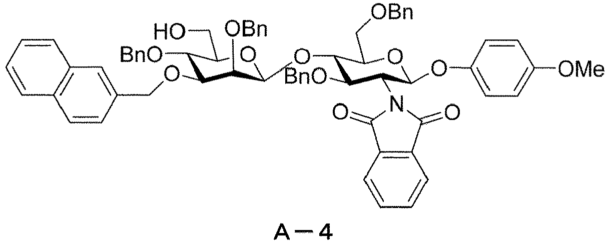

To a solution containing the crude 4-methoxyphenyl 3,6-di-O-benzyl-4-O-{2-O-benzyl-4,6-O-benzyliden-3-O-[(naphthalen-2-yl)methyl]-β-D-mannopyranosyl}-2-deoxy-2-(1,3-dioxo-1,3-dihydro-2H-isoindol-2-yl)-β-D-glucopyranoside (the compound represented by Formula C-15) obtained (5.50 g (5.48 mmol) equivalent in terms of the compound represented by Formula C-14 was used) were added dichloromethane (16.5 mL) and Molecular Sieves 4A (550 mg), and the mixture was cooled to 0°C. A borane-tetrahydrofuran complex (0.91 mol/L tetrahydrofuran solution) (18.06 mL, 16.43 mmol) and copper(II) trifluoroacetate (0.59 g, 1.64 mmol) were added, and the mixture was then stirred for 3 hours. After the completion of the reaction was checked by HPLC, methanol (5.5 mL) was added and the mixture was stirred for another 30 minutes. The solution is filtered. After the Molecular Sieves 4A was washed with ethyl acetate (110 mL), 0.5 N hydrochloric acid (55 mL) was added, and the mixture was then stirred for 30 minutes. An organic layer and an aqueous layer were separated. The organic layer was washed with 0.5 N hydrochloric acid (55 mL) and saturated brine (27.5 mL), and the resulting solution was then concentrated to dryness under reduced pressure. The resulting material was purified by silica gel column chromatography (silica gel: 300 g; hexane: ethyl acetate = 55:45 -> 30:70) to give 4-methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside (the compound represented by Formula A-4) (4.94 g, yield 83.6%, HPLC area: 98.54%).

¹H-NMR (500 MHz, CDC1₃) δ 7.67-7.84 (m, 8H), 7.44-7.49 (m, 4H), 7.40 (dd, J = 8.0, 1.5 Hz, 1H), 7.21-7.33 (m, 13H), 6.87-6.93 (m, 5H), 6.82 (ddd, J = 9.5, 4.0, 2.5 Hz, 2H), 6.70 (ddd, J = 9.0, 4.0, 2.0 Hz, 2H), 5.64 (d, J = 8.5 Hz, 1H), 4.94 (d, J = 12.5 Hz, 1H), 4.91 (d, J = 10.0 Hz, 1H), 4.90 (s, 2H), 4.66 (s, 2H), 4.60 (d, J = 11.0 Hz, 1H), 4.59 (d, J = 12.0 Hz, 1H), 4.55 (s, 1H), 4.40-4.46 (m, 3H), 4.33 (dd, J = 11.0, 9.0 Hz, 1H), 4.06 (dd, J = 9.5, 8.5 Hz, 1H), 3.80-3.85 (m, 2H), 3.70-3.76 (m, 2H), 3.71 (s, 3H), 3.61-3.68 (m, 2H), 3.45-3.48 (m, 1H), 3.44 (dd, J = 9.5, 3.0 Hz, 1H), 3.23 (ddd, J = 9.5, 5.5, 2.5 Hz, 1H), 1.97 (br-t, 1H).

¹³C-NMR (125 MHz, CDCl₃) δ 155.6, 151.1, 138.9, 138.64, 138.56, 138.0, 135.9, 134.0, 133.5, 133.2, 131.8, 128.7, 128.6, 128.4, 128.3, 128.20, 128.19, 128.15, 128.1, 120.04, 128.01, 127.9, 127.7, 127.6, 127.3, 126.4, 126.3, 126.1, 125.8, 123.6, 119.0, 114.6, 101.2, 98.0, 82.6, 79.0, 77.2, 75.9, 75.4, 75.3, 75.2, 75.1, 74.8, 74.7, 73.8, 72.1, 68.7, 62.6, 55.82, 55.78, 34.4, 30.5.

HRMS(ESI⁻)[M+HCOz]⁻ calcd for C₆₇H₆₄NO₁₅: 1122.4281; found 1122.4285.

### Example 22

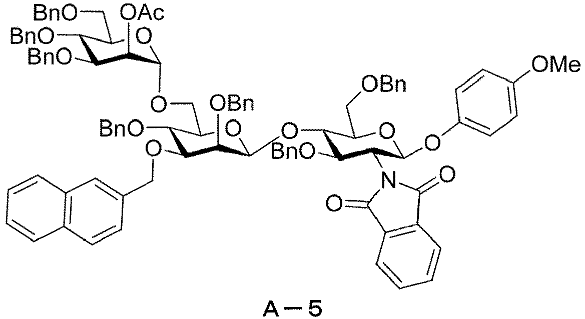

A toluene solution (78.4 mmol equivalents) containing 4-methoxyphenyl 3,6-di-O-benzyl-2-deoxy-4-O-{2,4-di-O-benzyl-3-O-[(naphthalen-2-yl)methyl]-β-D-mannopyranosyl}-2-(1,3-dioxo-1,3-dihydro-2H-isoindol-2-yl)-β-D-glucopyranoside (the compound represented by Formula A-4) (65.0 g, 60.3 mmol) and 2-O-acetyl-3,4,6-tri-O-benzyl-1-O-(2,2,2-trichloroethanimidoyl)-D-mannopyranose (the compound represented by Formula A-3) was added to a 1-L four-neck flask, and dissolved in 650 mL of toluene. Molecular Sieves 4A powder (10 µm or less, 13.0 g) was then added. Trimethylsilyl trifluoromethanesulfonate (2.7 mL, 15.1 mmol) was added dropwise over 15 minutes at -15°C under nitrogen, and the mixture was stirred at the same temperature for 30 minutes. After the completion of the reaction was checked by HPLC, triethylamine (4.2 mL, 30.2 mmol) was added. The temperature was raised to room temperature. The reaction solution was filtered through Celite and then washed with acetonitrile (195 mL). The filtrate was concentrated under reduced pressure, acetonitrile (650 mL) was added to the concentrated residue, and silica gel 120RP-18 (particle size: 40 to 50 µm; 97.5 g; available by KANTO CHEMICAL CO., INC.) for reversed phase was added. Water (130 mL) was added dropwise over 30 minutes to adsorb the target substance onto the solid phase, and the mixture was then filtered. After the solid phase was washed with acetonitrile/water (3/1,326 mL) (the filtrate was discarded), the target substance was desorbed with an acetonitrile (585 mL)-ethyl acetate (65 mL) solution. The filtrate was concentrated under reduced pressure to give, as a white amorphous material, 4-methoxyphenyl 2-O-acetyl-3,4,6-tri-O-benzyl-α-D-mannopyranosyl-(1→6)-2,4-di-O-benzyl-3-O-[(naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-5) ) (70.5 g, isolation yield: 94%).

¹H-NMR (CDCl₃) σ 7.85-7.80 (m, 1H), 7.78 (d, J = 8.6 Hz, 1H), 7.74-7.58 (m, 5H), 7.50-7.43 (m, 5H), 7.39 (dd, J = 8.6, 1.7 Hz, 1H), 7.32-7.02 (m, 30H), 6.92-6.89 (m, 2H), 6.78-6.76 (m, 2H), 6.71-6.62 (m, 5H), 5.59 (d, J = 8.6 Hz, 1H), 5.36 (dd, J = 2.9, 2.3 Hz, 1H), 5.00-4.90 (m, 4H), 4.87 (d, J= 12.6 Hz, 1H), 4.79 (d, J = 11.5 Hz, 1H), 4.66-4.50 (m, 7H), 4.43 (dd, J = 11.5, 2.3 Hz, 1H), 4.40-4.34 (m, 3H), 4.28 (dd, J = 10.3, 8.6 Hz, 1H), 4.22 (d, J = 11.5 Hz, 1H), 4.08 (dd, J = 9.7, 9.2 Hz, 1H), 3.94 (dd, J = 9.2, 9.2 Hz, 1H), 3.89-3.83 (m, 3H), 3.83-3.58 (m, 10H), 3.55-3.50 (m, 1H), 3.42 (dd, J = 9.2, 2.9 Hz, 1H), 3.37-3.31 (m, 1H), 1.90 (s, 3H).

¹³C-NMR (CDCl₃) σ 167.0, 155.3, 150.8, 138.8, 138.7, 138.51, 138.49, 138.4, 138.0, 137.9, 135.6, 133.6, 133.2, 132.9, 131.6, 128.44, 128.41, 128.32, 128.26, 128.22, 128.19, 128.12, 127.86, 127.81, 127.75, 127.71, 127.69, 127.63, 127.56, 127.44, 127.37, 127.27, 127.0, 126.3, 126.2, 125.9, 125.7, 123.2, 118.7, 114.3, 101.9, 98.3, 97.6, 82.7, 79.6, 77.8, 76.6, 75.04, 74.98, 74.91, 74.89, 74.75, 74.4, 74.3, 74.1, 74.0, 73.4, 73.3, 71.70, 71.65, 71.3, 68.8, 68.6, 68.3, 67.1, 55.6, 55.5, 20.9.

HRMS (ESI⁺) [M+HNEt₃]⁺ calcd for C₁₀₁H₁₀₉N₂O₁₉⁺: 1654.7653; found 1654.7618. [α]_{D}²⁰ = +31.589 (c 1.002, CDCl₃).

### Example 23

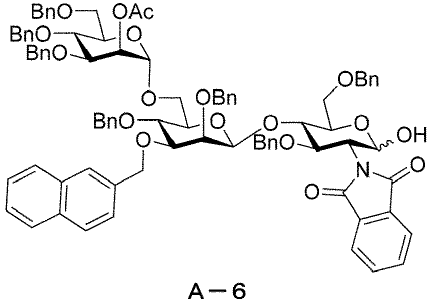

4-Methoxyphenyl 2-O-acetyl-3,4,6-tri-O-benzyl-α-D-mannopyranosyl-(1→6)-2,4-di-O-benzyl-3-O-[(naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-5) (44.0 g, 28.3 mmol) was added to a 1-L four-neck flask, and was mixed with dichloromethane (228 mL), 1,1,1,3,3,3-hexafluoro-2-propanol (163 mL), and water (14 mL). A dichloromethane solution (80 mL) containing [bis(trifluoroacetoxy)iodo]benzene (25.9 g, 56.6 mmol) and trifluoroacetic acid (6.9 mL, 84.9 mmol) were added at 2°C under nitrogen, and the mixture was stirred at the same temperature for 8 hours. After the completion of the reaction was checked by HPLC, a sodium bicarbonate (18.7 g)-water (228 mL) solution was added. The mixture was then stirred for 5 minutes, and mixed with a sodium sulfite (11.7 g)-water (228 mL) solution. After stirring for 5 minutes, the mixture was allowed to stand and the dichloromethane layer was separated. The resulting organic layer was concentrated under reduced pressure to 97.5 mL, and ethyl acetate (325 mL) and a sodium chloride (23.4 g)-water (211 mL) solution were each added. After stirring for 5 minutes, the mixture was allowed to stand and the organic layer was separated. The resulting organic layer was concentrated under reduced pressure to 97.5 mL, and toluene (890 mL) was then added. The mixture was concentrated again under reduced pressure to 97.5 mL. Toluene (164 mL) and dichloromethane (65 mL) were added, silica gel 60N (particle size: 40 to 50 µm; 130 g; available by KANTO CHEMICAL CO., INC.) was added, and the target substance was adsorbed on the silica gel and then filtered. A dichloromethane (130 mL)-toluene (520 mL) solution was used for washing (the filtrate was discarded), and the target substance was desorbed from the solid phase by using an ethyl acetate (220 mL)-dichloromethane (455 mL) solution. The filtrate was concentrated under reduced pressure, mixed with toluene (228 mL), and then concentrated under reduced pressure to 97.5 mL to give, as a toluene solution, 2-O-acetyl-3,4,6-tri-O-benzyl-α-D-mannopyranosyl-(1→6)-2,4-di-O-benzyl-3-O-[(naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-D-glucopyranose (the compound represented by Formula A-6). This solution was used as it was in the next step.

¹H-NMR (CDCl₃) σ 7.85-7.80 (m, 1H), 7.78 (d, J = 8.6 Hz, 1H), 7.72 (s, 1H), 7.70-7.64 (m, 1H), 7.63 (brs, 2H), 7.48-7.43 (m, 4H), 7.39 (dd, J = 8.6, 1.7 Hz, 1H), 7.33-7.10 (m, 30H), 6.92-6.87 (m, 2H), 6.77-6.66 (m, 3H), 5.38 (dd, J = 3.4, 1.7 Hz, 1H), 5.23 (dd, J = 8.6, 8.6 Hz, 1H), 4.97-4.86 (m, 4H), 4.84 (d, J = 12.6 Hz, 1H), 4.79 (d, J = 10.9 Hz, 1H), 4.63 (d, J = 11.5 Hz, 1H), 4.60-4.48 (m, 7H), 4.46 (d, J = 11.5 Hz, 1H), 4.37 (dd, J = 12.0, 3.4 Hz, 1H), 4.32 (dd, J = 10.9, 8.6 Hz, 1H), 4.23 (d, J = 10.9 Hz, 1H), 4.06 (dd, J = 9.2, 9.2 Hz, 1H), 4.01 (dd, J = 10.9, 9.2 Hz, 1H), 3.92 (dd, J = 9.2, 3.4 Hz, 1H), 3.89-3.74 (m, 6H), 3.72-3.65 (m, 2H), 3.64-3.57 (m, 2H), 3.48 (dd, J = 10.9, 1.1 Hz, 1H), 3.42-3.34 (m, 2H), 2.68 (dd, J = 9.2, 1.1 Hz, 1H), 1.95 (s, 3H).

¹³C-NMR (CDCl₃) σ 170.2, 167.9, 138.85, 138.79, 138.6, 138.5, 138.4, 138.0, 137.8, 135.6, 133.6, 133.2, 132.9, 131.6, 128.55, 128.50, 128.33, 128.24, 128.20, 128.16, 127.9, 127.80, 127.75, 127.69, 127.66, 127.62, 127.54, 127.45, 127.36, 127.33, 126.97, 126.22, 126.17, 125.9, 125.6, 123.2, 101.1, 97.9, 92.9, 82.6, 78.6, 78.0, 76.1, 74.9, 74.8, 74.4, 74.15, 74.13, 74.0, 73.5, 73.2, 71.7, 71.6, 71.3, 68.8, 68.768.4, 67.0, 57.6, 20.9.

HRMS (ESI⁺) [M+HNEt₃]⁺ calcd for C₉₄H₁₀₃N₂O₁₈⁺: 1548.7234; found 1548.7237. [α]_{D}²⁰ = +32.528 (c 1.006, CDCl₃).

### Example 24

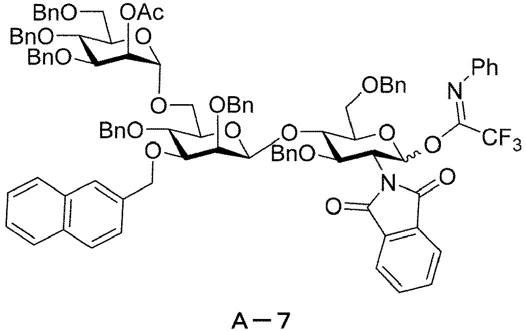

A toluene solution containing 2-O-acetyl-3,4,6-tri-O-benzyl-α-D-mannopyranosyl-(1→6)-2,4-di-O-benzyl-3-O-[(naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-D-glucopyranose (the compound represented by Formula A-6) obtained in Example 23 was added to a 1-L four-neck flask. Dichloromethane (260 mL) and Molecular Sieves 4A powder (10 µm or less, 21.8 g) were added, and the flask was cooled to 0°C. Next, 1,8-diazabicyclo[5.4.0]und-7-ene (5.08 mL, 34.0 mmol) and 2,2,2-trifluoro-N-phenylacetimidoyl chloride (5.25 mL, 31.1 mmol) were added, and the mixture was stirred for 5 hours. The reaction solution was filtered through a neutral silica gel pad (silica gel 60N; particle size: 40 to 50 µm; 130 g; available by KANTO CHEMICAL CO., INC.) packed with dichloromethane. The silica gel pad was washed with 10% ethyl acetate/dichloromethane (1760 mL, 220 mL each collected), and the main residue was concentrated under reduced pressure. Toluene (228 mL) was then added, and the mixture was concentrated again under reduced pressure to 97.5 mL to give a toluene solution containing 2-O-acetyl-3,4,6-tri-O-benzyl-α-D-mannopyranosyl-(1→6)-2,4-di-O-benzyl-3-O-[(naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-1-O-(2,2,2-trifluoro-N-phenylethanimidoyl)-D-glucopyranose (the compound represented by Formula A-7). This solution was used as it was in the next step.

¹H-NMR (CDCl₃) σ 7.85-7.80 (m, 1H), 7.78 (d, J = 8.6 Hz, 1H), 7.72 (s, 1H), 7.70-7.64 (m, 3H), 7.50-7.43 (m, 4H), 7.39 (dd, J = 8.6, 1.7 Hz, 1H), 7.33-7.08 (m, 30H), 7.06-7.01 (m, 1H), 6.88 (d, J = 6.9 Hz, 2H), 6.70-6.60 (m, 4H), 5.35 (dd, J = 2.9, 1.7 Hz, 1H), 4.97-4.88 (m, 4H), 4.84 (d, J = 13.2 Hz, 1H), 4.79 (d, J = 10.9 Hz, 1H), 4.65-4.49 (m, 7H), 4.43 (dd, J = 11.5, 4.0 Hz, 1H), 4.36 (dd, J = 12.0, 7.4 Hz, 1H), 4.22 (d, J = 11.5 Hz, 1H), 4.11-4.04 (m, 1H), 3.93 (dd, J = 9.7, 9.7 Hz, 1H), 3.89-3.83 (m, 2H), 3.82-3.69 (m, 4H), 3.64 (dd, J = 10.9, 4.0 Hz, 1H), 3.52 (dd, J = 10.9, 1.1 Hz, 1H), 3.39 (dd, J = 9.7, 2.9 Hz, 1H), 3.33-3.28 (m, 1H), 1.89 (s, 3H).

¹³C-NMR (CDCl₃) σ 170.0, 167.4, 143.0, 138.8, 138.7, 138.5, 138.4, 138.0, 137.6, 135.6, 133.7, 133.2, 133.0, 131.5, 128.56, 128.53, 128.4, 128.32.128.30, 128.26, 128.22, 128.19, 128.14, 128.12, 127.90, 127.86, 127.81, 127.74, 127.72, 127.69, 127.54, 127.46, 127.38, 127.28, 127.0, 126.3, 126.2, 126.0, 125.6, 124.3, 123.3, 119.3, 101.8, 98.2, 82.6, 79.0, 77.8, 76.2, 75.5, 75.0, 74.91, 74.89, 74.7, 74.5, 74.3, 74.1, 74.0, 73.4, 73.3, 71.7, 71.7, 71.3, 68.8, 68.3, 68.0, 67.0, 54.7, 20.8.

HRMS (ESI⁺) [M+HNH₃]⁺ calcd for C₉₆H₉₁F₃N₃O₁₈⁺: 1635.6591; found 1635.6549. [α]_{D}²⁰ = +62.169 (c 1.002, CDCl₃).

### Example 25

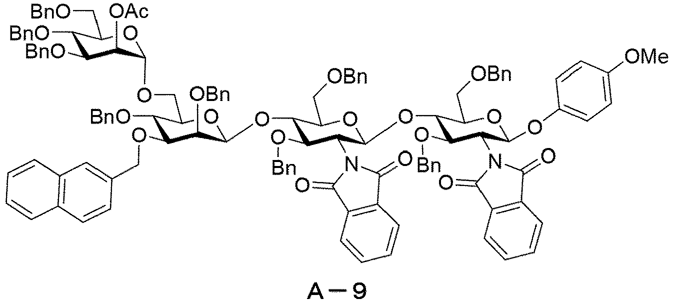

A toluene solution containing 2-O-acetyl-3,4,6-tri-O-benzyl-α-D-mannopyranosyl-(1→6)-2,4-di-O-benzyl-3-O-[(naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-1-O-(2,2,2-trifluoro-N-phenylethanimidoyl)-D-glucopyranose (the compound represented by Formula A-7) obtained in Example 24, toluene (488 mL), and 4-methoxyphenyl 3,6-di-O-benzyl-2-deoxy-2-(1,3-dioxo-1,3-dihydro-2H-isoindol-2-yl)-β-D-glucopyranoside (the compound represented by Formula A-8) (21.55 g, 36.2 mmol) were added to a 1-L four-neck flask, and Molecular Sieves 4A powder (14.6 g) was added. Trimethylsilyl trifluoromethanesulfonate (545 µL, 2.83 mmol) was added dropwise over 5 minutes at -15°C under nitrogen, and the mixture was stirred at the same temperature for 1 hour. After the completion of the reaction was checked by HPLC, triethylamine (1.67 mL, 11.32 mmol) was added. The temperature was raised to room temperature. The reaction solution was filtered and washed with acetonitrile (160 mL). The filtrate was concentrated under reduced pressure to 97.5 mL, and acetonitrile (650 mL) was added and concentrated again under reduced pressure to 97.5 mL. Acetonitrile (488 mL) and silica gel 120RP-18 (particle size 40 to 50 µm; 130 g; available by KANTO CHEMICAL CO., INC.) for reversed phase were added. Water (146 mL) was added dropwise over 30 minutes to adsorb the target substance onto the solid phase, and the mixture was then filtered. After the solid phase was washed with acetonitrile (536 mL)-water (146 mL) (the filtrate was discarded), the target substance was desorbed with acetonitrile (975 mL)-ethyl acetate (244 mL). The filtrate was concentrated under reduced pressure and azeotroped twice with toluene (325 mL) to reduce the final liquid volume to 97.5 mL. This resulted in a toluene solution containing 4-methoxyphenyl 2-O-acetyl-3,4,6-tri-O-benzyl-α-D-mannopyranosyl-(1→6)-2,4-di-O-benzyl-3-O-[(naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-9). A portion of this solution (90 mL, 26.1 mmol in terms of the compound represented by Formula A-4) was collected and used in the next step.

¹H-NMR (CDCl₃) σ 7.86-7.81 (m, 1H), 7.81-7.75 (m, 2H), 7.74-7.60 (m, 7H), 7.56-7.44 (m, 5H), 7.40 (dd, J = 8.6, 1.1 Hz, 1H), 7.28-7.05 (m, 35H), 6.95-6.91 (m, 2H), 6.91-6.88 (m, 2H), 6.78-6.62 (m, 8H), 6.61-6.55 (m, 2H), 5.42 (d, J = 8.6 Hz, 1H), 5.33 (dd, J = 2.9, 1.7 Hz, 1H), 5.23 (d, J = 8.0 Hz, 1H), 4.98 (d, J = 12.6 Hz, 1H), 4.94-4.85 (m, 4H), 4.82 (d, J = 13.2 Hz, 1H), 4.76 (d, J = 10.9 Hz, 1H), 4.65-4.29 (m, 16H), 4.25-4.12 (m, 5H), 4.04 (dd, J = 10.3, 8.0 Hz, 1H), 3.95 (dd, J = 9.2, 9.2 Hz, 1H), 3.89-3.80 (m, 3H), 3.77 (d, J = 9.2 Hz, 1H), 3.73 (d, J = 10.9 Hz, 1H), 3.70-3.65 (m, 1H), 3.64 (s, 3H), 3.63-3.57 (m, 2H), 3.53-3.46 (m, 2H), 3.44-3.35 (m, 4H), 3.30-3.23 (m, 2H), 1.83 (s, 3H).

¹³C-NMR (CDCl₃) σ 169.8, 168.1, 167.4, 155.2, 150.8, 138.9, 138.71, 138.68, 138.55, 138.48, 138.36, 137.9, 135.6, 133.8, 133.6, 133.2, 133.0, 131.8, 131.6, 131.5, 128.5, 128.34, 128.32, 128.29, 128.26, 128.20, 128.17, 128.12, 128.10, 127.89, 127.85, 127.80, 127.77, 127.69, 127.65, 127.52, 127.47, 127.44, 127.36, 127.29, 127.28, 126.90, 126.87, 126.3, 126.2, 126.0, 125.7, 123.5, 123.2, 123.1, 118.5, 114.2, 102.1, 98.2, 97.4, 97.1, 82.7, 80.0, 77.8, 76.6, 75.9, 74.9, 74.8, 74.63, 74.61, 74.56, 74.49, 74.3, 74.06, 74.03, 73.3, 73.2, 72.6, 71.7, 71.6, 71.2, 68.7, 68.3, 68.11, 68.06, 68.8, 56.5, 55.6, 55.5, 20.8.

HRMS (ESI⁺) [M+HNEt₃]⁺ calcd for C₁₂₉H₁₃₄N₃O₂₅⁺: 2125.9334; found 2125.9267. [α]_{D}²⁰ = +30.098 (c 1.008, CDCl₃).

### Example 26

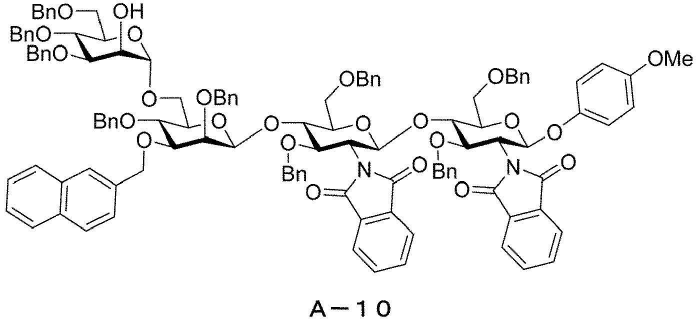

A toluene solution containing 4-methoxyphenyl 2-O-acetyl-3,4,6-tri-O-benzyl-α-D-mannopyranosyl-(1→6)-2,4-di-O-benzyl-3-O-[(naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-9) (90 mL, 26.1 mmol: compound represented by Formula A-4) obtained in Example 25 was added to a 1-L four-neck flask, and mixed with tetrahydrofuran (165 mL), methanol (75 mL), and methyl trifluoroacetate (2.76 mL, 27.8 mmol). After stirring at room temperature under nitrogen for 5 minutes, a tetrahydrofuran solution containing 1 M potassium t-butoxide (13.9 mL, 13.9 mmol) was added. The mixture was stirred at 40°C for 1 hour. After the completion of the reaction was checked by HPLC, the mixture was cooled to room temperature. Acetic acid (1.11 mL, 19.4 mmol) was added, and ethyl acetate (330 mL) and water (270 mL) were then added. Triethylamine was added until pH 7 was reached. Sodium chloride (2.7 g) was then added, and the mixture was stirred for 5 minutes. After the mixture was allowed to stand, the organic layer obtained by liquid-separation was washed twice with water (270 mL). The organic layer was concentrated under reduced pressure to 90 mL. Toluene (300 mL) was added, and the mixture was concentrated again under reduced pressure to 90 mL. Toluene (300 mL) was added, and the mixture was concentrated again under reduced pressure to 90 mL to give a toluene solution containing 4-methoxyphenyl 3,4,6-tri-O-benzyl-α-D-mannopyranosyl-(1→6)-2,4-di-O-benzyl-3-O-[(naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-10). This solution was used as it was in the next step.

¹H-NMR (CDCl₃) σ 7.85-7.81 (m, 1H), 7.79 (d, J = 8.6 Hz, 1H), 7.78-7.72 (m, 2H), 7.72-7.60 (m, 6H), 7.59-7.54 (m, 1H), 7.50-7.44 (m, 4H), 7.41 (dd, J = 8.6, 1.1 Hz, 1H), 7.31-7.13 (m, 32H), 7.10-7.05 (m, 2H), 6.97-6.93 (m, 2H), 6.90-6.86 (m, 2H), 6.78-6.67 (m, 8H), 6.61-6.56 (m, 2H), 5.43 (d, J = 8.6 Hz, 1H), 5.25 (d, J = 8.0 Hz, 1H), 4.99-4.86 (m, 4H), 4.83 (d, J = 12.6 Hz, 1H), 4.71 (d, J = 10.9 Hz, 1H), 4.64 (d, J = 12.0 Hz, 1H), 4.61 (d, J = 12.0 Hz, 1H), 4.56 (d, J = 6.9 Hz, 1H), 4.53-4.39 (m, 7H), 4.39-4.31 (m, 5H), 4.30 (d, J = 11.5 Hz, 1H), 4.25-4.14 (m, 4H), 4.05 (dd, J = 9.7, 8.6 Hz, 1H), 3.94-3.88 (m, 2H), 3.86-3.80 (m, 2H), 3.76-3.68 (m, 3H), 3.68-3.59 (m, 5H), 3.57-3.35 (m, 7H), 3.31-3.25 (m, 2H), 2.15 (d, J = 4.0 Hz, 1H).

¹³C-NMR (CDCl₃) σ 168.2, 167.5, 155.2, 150.8, 138.91, 138.88, 138.6, 138.52, 138.47, 138.38, 128.32, 128.0, 137.9, 135.7, 133.9, 133.6, 133.3, 133.0, 131.8, 131.6, 131.4, 128.5, 128.29, 128.23, 128.19, 128.13, 128.0, 127.92, 127.87, 127.82, 127.79, 127.76, 127.70, 127.56, 127.53, 127.46, 127.36, 127.31, 127.28, 126.9, 126.3, 126.2, 126.0, 125.7, 123.5, 123.2, 123.1, 118.6, 114.2, 101.9, 99.7, 97.4, 97.1, 82.7, 79.7, 79.6, 76.7, 75.9, 75.3, 74.9, 74.8, 74.73, 74.68, 74.63, 74.5, 74.4, 74.2, 74.1, 73.2, 73.2, 72.6, 71.8, 71.3, 71.2, 68.9, 68.13, 68.07, 67.8, 66.6, 56.5, 55.6, 55.5.

HRMS (ESI⁺) [M+HNEt₃]⁺ calcd for C₁₂₇H₁₃₂N₃O₂₄⁺: 2083.9229; found 2083.9150. [α]_{D}²⁰ = +27.776 (c 1.007, CDCl₃).

### Example 27

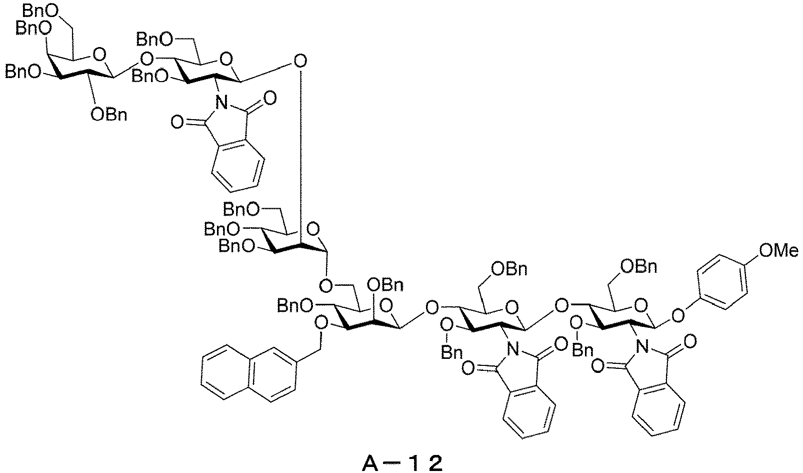

To a toluene solution containing 4-methoxyphenyl 3,4,6-tri-O-benzyl-α-D-mannopyranosyl-(1→6)-2,4-di-O-benzyl-3-O-[naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-10) obtained in Example 26 was added dichloromethane (450 mL) and a toluene solution containing 3,6-di-O-benzyl-2-deoxy-2-(1,3-dioxo-1,3-dihydro-2H-isoindol-2-yl)-4-O-(2,3,4,6-tetra-O-benzyl-β-D-galactopyranosyl)-1-O-(2,2,2-trifluoro-N-phenylethanimidoyl)-β-D-glucopyranose (the compound represented by Formula A-11) (118.2 g, 36.2 mmol) in a 1-L four-neck flask. Molecular Sieves 4A powder (10 µm or less, 15.0 g) was added. Then, t-butyldimethylsilyl trifluoromethanesulfonate (2.4 mL, 10.4 mmol) was added dropwise over 5 minutes at -78°C under nitrogen, and the mixture was stirred at the same temperature for 10 hours. Triethylamine (4.6 mL, 33.2 mmol) was added, and the temperature was raised to room temperature. The reaction solution was filtered through Celite and washed with acetonitrile (150 mL). The filtrate was concentrated under reduced pressure to 150 mL, and acetonitrile (600 mL) was added. The mixture was then concentrated again under reduced pressure to 150 mL. Acetonitrile (600 mL) and silica gel 120RP-18 (particle size: 40 to 50 µm; 135 g; available by KANTO CHEMICAL CO., INC.) for reversed phase were added. Water (120 mL) was added dropwise over 30 minutes to adsorb the target substance onto the solid phase, and the mixture was then filtered. After the solid phase was washed with acetonitrile (900 mL)-water (135 mL) (the washing liquid was discarded), the target substance was desorbed from the solid phase by using an acetonitrile (840 mL)-ethyl acetate (210 mL) solution. The desorption solution was concentrated under reduced pressure. Toluene (300 mL) was then added, and the mixture was concentrated under reduced pressure to 90 mL to give a toluene solution containing 4-methoxyphenyl 2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)-2,4-di-O-benzyl-3-O-[(naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-12). This solution was used as it was in the next step.

¹H-NMR (CDCl₃) σ 7.84-7.73 (m, 4H), 7.70-7.57 (7H), 7.55-7.03 (m, 68H), 6.99-6.89 (m, 11H), 6.88-6.83 (m, 1H), 6.82-6.67 (m, 7H), 6.67-6.62 (m, 2H), 6.62-6.56 (m, 3H), 5.42 (d, J = 8.6 Hz, 1H), 5.23 (d, J = 8.6 Hz, 1H), 5.02 (d, J = 8.6 Hz, 1H), 4.95-4.89 (m, 3H), 4.86-4.79 (m, 5H), 4.77-4.66 (m, 4H), 4.64-4.08 (m, 30H), 4.06-3.98 (m, 3H), 3.91-3.68 (m, 9H), 3.65 (s, 3H), 3.58 (d, J = Hz, 1H), 3.54-3.32 (m, 13H), 3.32-3.27 (m, 1H), 3.22-3.16 (m, 3H), 2.84 (dd, J = 10.9, 5.7 Hz, 1H).

¹³C-NMR (CDCl₃) σ 168.4, 168.2, 167.4, 155.2, 150.8, 139.1, 139.02, 139.99, 138.93, 138.8, 138.7, 138.52, 138.48, 138.38, 138.32, 138.06, 138.01, 135.6, 133.7, 133.61.133.56, 133.3, 133.2, 132.9, 131.8, 131.6, 131.4, 128.54, 128.50, 128.39, 128.34, 128.17, 128.13, 128.10, 128.00, 127.96, 127.89, 127.82, 127.78, 127.67, 127.64, 127.62, 127.59, 127.51, 127.48, 127.46, 127.41, 127.39, 127.29, 127.20, 127.05, 127.01, 126.9, 126.7, 126.3, 126.2, 125.9, 125.7, 123.5, 123.2, 123.1, 123.0, 118.6, 114.2, 102.9, 102.7, 97.8, 97.4, 97.0, 96.9, 83.1, 82.5, 80.4, 79.9, 77.7, 77.4, 76.9, 76.6, 75.7, 75.3, 75.175.0, 74.8, 74.7, 74.6, 74.52, 74.46, 74.0, 73.95, 73.85, 73.7, 73.4, 73.2, 73.0, 72.9, 72.58, 72.55, 72.52, 72.48, 72.0, 71.9, 69.8, 69.7, 68.2, 3, 68.20, 68.06, 68.03, 67.0, 56.6, 55.6, 55.5

HRMS (ESI⁺) [M+HNEt₃]⁺ calcd for C₁₉₁H₁₇₅N₄O₃₅⁺: 3078.3350; found 3078.3200. [α]_{D}²⁰ = +9.276 (c 1.002, CDCl₃).

### Example 28-1

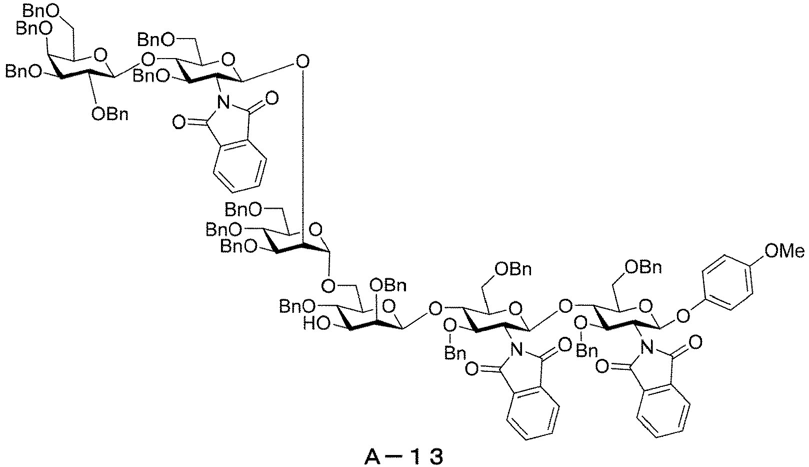

A toluene solution containing 4-methoxyphenyl 2,3,4,6-tetra-O-benzyl-β-D-galactopyranosy1-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)-2,4-di-O-benzyl-3-O-[(naphthalen-2-yl)methyl]-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-12) (64.2 mmol equivalents) obtained in Example 27 was added to a 3-L four-neck flask, and was mixed with 1,1,1,3,3,3-hexafluoro-2-propanol (1337 mL) and water (133.7 mL). After the reaction solution was cooled to about -30°C, 2,3-dichloro-5,6-dicyano-p-benzoquinone (17.48 g, 77.0 mmol) was added. The mixture was then stirred for 38 hours. After sufficient progress of the reaction was checked, a sodium sulfite (4.04 g, 32.1 mmol)-water (95.6 mL) solution was added to stop the reaction. The temperature was raised to room temperature over 1 hour. Dichloromethane (1910 mL) and a sodium bicarbonate (38.2 g)-sodium sulfite (38.2 g)-water (1910 mL) solution were each added, and the mixture was stirred for 5 minutes. The mixture was allowed to stand and then liquid-separated. The resulting organic layer was concentrated under reduced pressure to 573 mL. The residue was mixed with toluene (955 mL), and the mixture was concentrated under reduced pressure to 573 mL. Ethyl acetate (955 mL) and a sodium chloride (95.5 g)-water (860 mL) solution were added, and the mixture was stirred for 5 minutes. The mixture was allowed to stand and liquid-separated. The resulting organic layer was concentrated under reduced pressure to 382 mL. Acetonitrile (1910 mL) was then added, and the mixture was concentrated again under reduced pressure to 382 mL. The residue was mixed with acetonitrile (1528 mL) and silica gel 120RP-18 (particle size 40 to 50 µm; 573 g; available by KANTO CHEMICAL CO., INC.) for reversed phase. Water (1242 mL) was added dropwise over 30 minutes to adsorb the target substance onto the solid phase, and the mixture was then filtered. After the solid phase was washed with acetonitrile (1337 mL)-water (573 mL) (the washing solution was discarded), the solid phase was washed with methanol (955 mL). The target substance was then desorbed from the solid phase by using an acetonitrile (6876 mL)-tetrahydrofuran (764 mL) solution. The desorption solution is concentrated under reduced pressure and dried to give 167 g of crude 4-methoxyphenyl 2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4, 6-tri-O-benzyl-α-D-mannopyranosyl-(1→6)-2,4-di-O-benzyl-D-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-13).

The crude material obtained was purified by preparative HPLC to give, as a purified product, 96 g of 4-methoxyphenyl 2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-a-D-mannopyranosyl-(1→6)-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3 -dioxo-1,3-dihydro-2H-isoindol-2-yl)-β-D-glucopyranoside (the compound represented by Formula A-13) (total yield of 36.1% from the compound represented by Formula C-13).

¹H-NMR (CDCl₃) σ 7.75 (d, J = 8.0 Hz, 1H), 7.69-7.40 (10H), 7.55-7.10 (m, 54H), 7.06-6.97 (5H), 6.97-6.89 (m, 8H), 6.88-6.84 (m, 1H), 6.82-6.73 (m, 5H), 6.72-6.67 (m, 2H), 6.62-6.56 (m, 5H), 5.42 (d, J = 8.6 Hz, 1H), 5.22 (d, J = 8.0 Hz, 1H), 5.03 (d, J = 12.0 Hz, 1H), 4.99 (d, J = 8.6 Hz, 1H), 4.92 (d, J = 10.9 Hz, 1H), 4.87-4.79 (m, 5H), 4.75 (d, J = 11.5 Hz, 1H), 4.70 (d, J = 12.0 Hz, 1H), 4.67 (d, J = 12.0 Hz, 1H), 4.63 (d, J = 6.3 Hz, 1H), 4.61 (d, J = 6.9 Hz, 1H), 4.60-3.97 (m, 30H), 3.88 (d, J = 2.9 Hz, 1H), 3.84 (d, J = 12.0 Hz, 1H), 3.82-3.67 (m, 5H), 3.66-3.60 (m, 5H), 3.54-3.33 (m, 14H), 3.27-3.23 (m, 1H), 3.20 (d, J = 10.9 Hz, 1H), 3.15-3.09 (m, 2H), 2.83-2.78 (m, 1H), 2.27-2.21 (m, 1H).

¹³C-NMR (CDCl₃) σ 168.4, 168.2, 167.4, 167.3, 155.2, 150.8, 139.1, 139.0, 138.9, 138.8, 138.7, 138.51, 138.49, 138.44, 138.38, 138.37, 138.35, 138.26, 138.0, 137.7, 133.8, 133.6, 122.49, 133.41, 133.36, 131.8, 131.4, 128.6, 128.52, 128.49, 128.39, 128.34, 128.30, 128.2, 128.14, 128.10, 128.06, 128.0, 127.85, 127.82, 127.7, 127.69, 127.65, 127.56, 127.53, 127.48, 127.38, 127.33, 127.26, 127.1, 127.0, 126.9, 126.7, 123.5, 123.2, 123.09, 123.05, 122.98, 118.5, 114.2, 102.9, 102.6, 97.8, 97.4, 97.0, 96.9, 82.5, 80.5, 79.9, 78.5, 77.8, 77.4, 76.9, 76.7, 75.87, 75.80, 75.2, 75.0, 74.79, 74.77, 74.62, 74.59, 74.48, 74.41, 74.2, 74.0, 73.9, 73.6, 73.4, 73.0, 72.9, 72.7, 72.62, 72.56, 72.0, 69.8, 68.24, 68.20, 68.1, 67.8, 67.2, 56.6, 55.6, 55.5, 53.4.

HRMS (ESI⁺) [M+HNEt₃]⁺ calcd for C₁₇₂H₁₈₃N₄O₃₅⁺: 2938.2725; found 2938.2516. [α]_{D}²⁰ = +14.385 (c 1.005, CDCl₃).

### Example 28-2

The compound represented by Formula A-13 was purified by using a technique according to the following scheme. As shown below, the method for purification made it possible to obtain the high-purity compound represented by Formula A-13 without HPLC preparative purification.

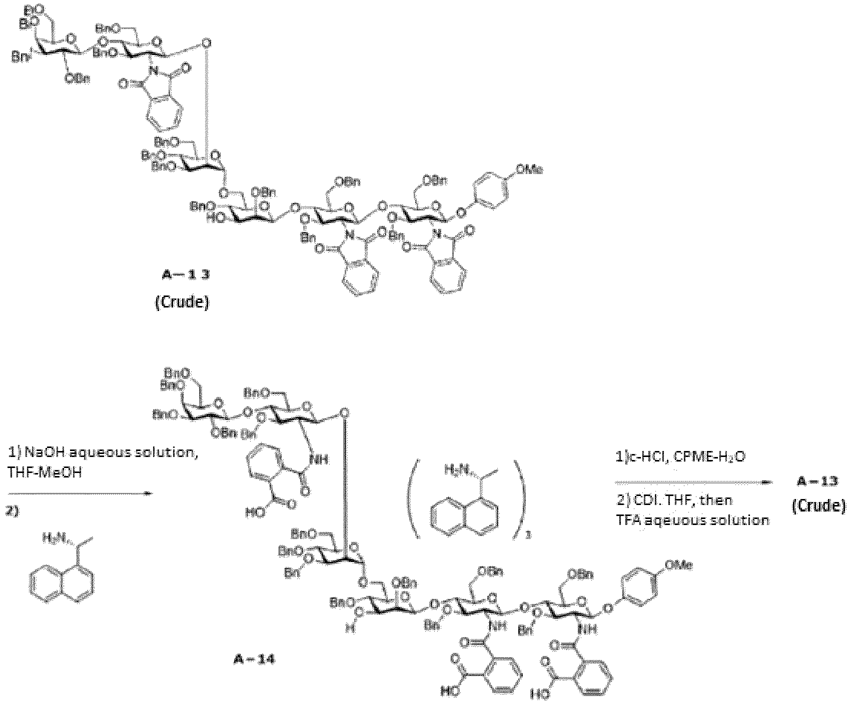

To synthesize methoxyphenyl 2,3,4,6-tetra-O-benzyl-β-D-galactopyranosyl-(1→4)-3,6-di-O-benzyl-2-deoxy-2-(2-carboxybenzamido)-β-D-glucopyranosyl-(1→2)-3,4,6-tri-O-benzyl-α-D-mannopyranosyl-(1→6)-2,4-di-O-benzyl-β-D-mannopyranosyl-(1→4)-3,6-di-O-benzyl-2-deoxy-2-(2-carboxybenzamido)-β-D-glucopyranosyl-(1→4)-3,6-di-O-benzyl-2-deoxy-2-(2-carboxybenzamido)-β-D-glucopyranoside tri(R)-(+)-1-(1-naphthyl)ethylamine salt (the compound represented by Formula A-14)

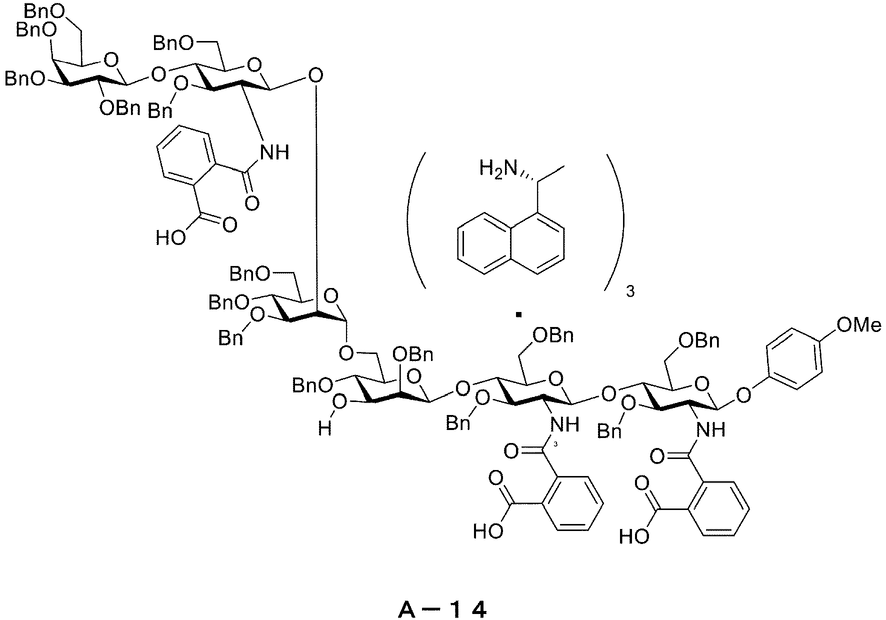

First, 4-methoxyphenyl 2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-13) (25.00 g, 8.82 mmol, purity: 72.2 PA%) was dissolved in dichloromethane (250 mL). The solution was made to pass through silica gel (62.5 g, Chromatorex SMB100-20/45 available by FUJI SILYSIA CHEMICAL LTD.). Elution was performed with a diisopropyl ether/dichloromethane mixed solvent (7/93, 1000 mL) (during flow-through and elution operations, the sample was fractionated every 125 mL and the solution was collected). Fractions were selected by HPLC while the purity was measured, and the selected fractions were mixed and then concentrated to 38 mL. Thereafter, tetrahydrofuran (125 mL) was added to the concentrated solution, and the mixture was concentrated to 38 mL. Tetrahydrofuran (100 mL), methanol (38 mL), and aqueous sodium hydroxide solution (4 M, 16.5 mL, 7.5 equivalents) were then added dropwise. The temperature was raised to 45°C and the mixture was stirred for 1 hour. After the completion of the reaction was checked, the mixture was cooled to 0°C. Hydrochloric acid (6 M, 11 mL, 7.5 equivalents) was added dropwise below 10°C for neutralization. Ethyl acetate (250 mL) and 3% brine (250 mL) were added, and the pH of the aqueous layer was adjusted to 2.0 or less with hydrochloric acid (6 M) under stirring. The solution was liquid-separated to remove the aqueous layer, and the resulting organic layer was washed with 3% brine (250 mL). The organic layer was concentrated to 38 mL, and ethyl acetate (250 mL) was added. The mixture was concentrated to 38 mL. The concentrated solution was mixed with ethyl acetate (138 mL), (R)-(+)-1-(1-naphthyl)ethylamine (5.28 g, 3.5 equivalents), and seed crystals (0.03 g). After stirring at 25°C for at least 12 hours, the mixture was cooled to 0°C. Heptane (88 mL) was added dropwise over 1 hour. The mixture was stirred for 2 hours, and the precipitated crystals were then filtered to obtain wet crystals (purity: 92.9 PA%). The resulting wet crystals were mixed with ethyl acetate (125 mL), stirred at 35°C for 30 minutes, cooled to 25°C, and then stirred for 12 hours. The slurry solution was cooled to 0°C over 1 hour, and heptane (75 mL) was added dropwise over 1 hour. After stirring at 0°C for 2 hours, the precipitated crystals were filtered and dried under reduced pressure to give, as white crystals, 4-methoxyphenyl 2,3,4,6-tetra-O-benzyl-β-D-galactopyranosyl-(1→4)-3,6-di-O-benzyl-2-deoxy-2-(2-carboxybenzamido)-β-D-glucopyranosyl-(1→2)-3,4, 6-tri-O-benzyl-α-D-mannopyranosyl-(1→6)-2,4-di-O-benzyl-β-D-mannopyranosyl-(1→4)-3,6-di-O-benzyl-2-deoxy-2-(2-carboxybenzamido)-β-D-glucopyranosyl-(1→4)-3,6-di-O-benzyl-2-deoxy-2-(2-carboxybenzamido)-β-D-glucopyranoside tri(R)-(+)-1-(1-naphthyl)ethylamine salt (the compound represented by Formula A-14) (18.93 g, yield: 63.1%, purity: 97.2 PA%). Powder X-ray crystallographic analysis of the obtained crystals is shown below.

### <Measuring instrument>

### Powder X-ray crystal analyzer: Rigaku Corporation

### <Measurement conditions>

Wavelength : Cuka / 1.541862 Å
Goniometer: MiniFlex 300/600
Scanning speed: CONTINUOUS
Scanning speed/counting time: 10.00
Step width: 0.02 deg
Scanning axis: 20/0
Scanning range: 3.00 to 40.00 deg
Filter: K-beta(x1)
Rotation: Yes

### <Powder X-ray crystal analysis measurement chart for the compound represented by Formula A-14>

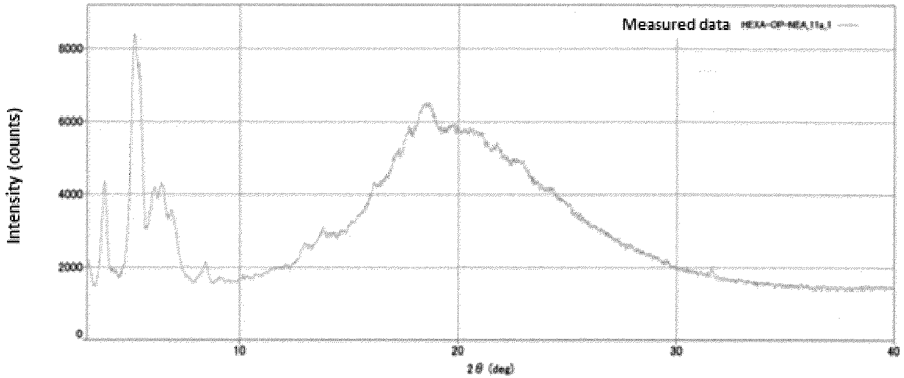

Conversion of compound represented by Formula A-14 to compound represented by Formula A-13

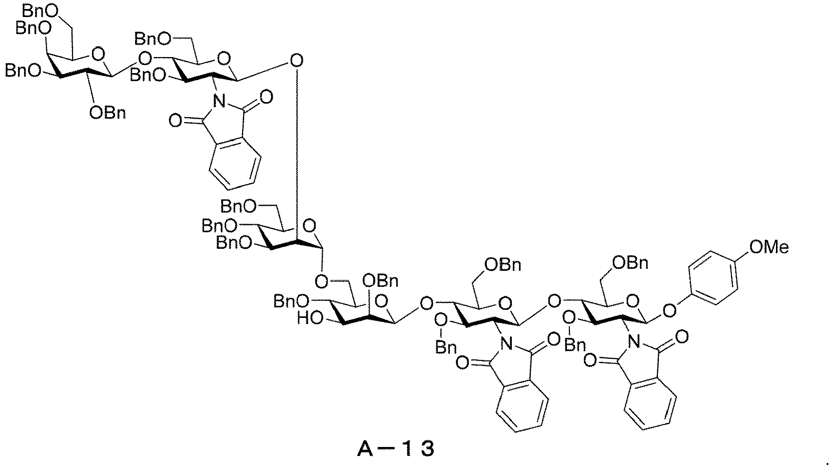

First, 4-methoxyphenyl 2,3,4,6-tetra-O-benzyl-β-D-galactopyranosyl-(1→4)-3,6-di-O-benzyl-2-deoxy-2-(2-carboxybenzamido)-β-D-glucopyranosyl-(1→2)-3,4,6-tri-O-benzyl-α-D-mannopyranosyl-(1→6)-2,4-di-O-benzyl-β-D-mannopyranosyl-(1→4)-3,6-di-O-benzyl-2-deoxy-2-(2-carboxybenzamido)-β-D-glucopyranosyl-(1→4)-3,6-di-O-benzyl-2-deoxy-2-(2-carboxybenzamido)-β-D-glucopyranoside tri(R)-(+)-1-(1-naphthyl)ethylamine salt (the compound represented by Formula A-14) (18.00 g, purity: 97.2 PA%) was mixed with cyclopentyl methyl ether (90 mL). The mixture was washed three times with 1 M aqueous hydrochloric acid solution (180 mL) and then with 5% brine (90 mL). The resulting organic layer was concentrated to 18 mL, mixed with cyclopentyl methyl ether (90 mL), and concentrated under reduced pressure to 18 mL. To the concentrated solution were added tetrahydrofuran (90 mL) and then carbonyldiimidazole (6.86 g, 8 equivalents). The mixture was stirred at 35°C for 1 hour. After the completion of the reaction was checked (a product was found in which in addition to the ring-closing reaction of the phthalimide, imidazole carbonylation of the hydroxyl group proceeded), water (9 mL) and trifluoroacetic acid (12.1 g, 20 equivalents) were added. The mixture was heated to 60°C and stirred for 20 hours. After the progress of deimidazole carbonylation was checked, the mixture was then cooled to room temperature. Ethyl acetate (90 mL) and water (90 mL) were added for liquid separation. The resulting organic layer was then washed with 5% sodium bicarbonate in water (90 mL) and water (90 mL) in this order. The resulting organic layer was concentrated under reduced pressure to 18 mL. Toluene (90 mL) was added. The mixture was concentrated to 18 mL, and then mixed with dichloromethane (162 mL). This dichloromethane solution was made to pass through silica gel (36 g, Chromatorex SMB100-20/45 available by FUJI SILYSIA CHEMICAL LTD.). Elution was performed with a diisopropyl ether/dichloromethane mixed solvent (7/93, 720 mL) (during flow-through and elution operations, the sample was fractionated every 90 mL and the solution was collected). Fractions were selected by HPLC while the purity was measured, and the selected fractions were mixed and concentrated to 18 mL. Cyclopentyl methyl ether (90 mL) was added to the concentrated solution and the mixture was concentrated to 36 mL. This solution was then added dropwise to isopropanol (630 mL) cooled to 0°C over 30 minutes under stirring. The mixture was stirred at 0°C for 2 hours. The slurry solution was then filtered and washed with isopropanol (180 mL) at 0°C. The resulting powder was dried under reduced pressure to give, as white powder, 4-methoxyphenyl 2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-13) (14.1 g, yield: 93%, purity: 97.0 PA%).

### [Analytical conditions]

Column: Xbridge Phenyl 3.5 µm, 4.6ϕ × 150 mm (Waters)
Wavelength: 220 nm
Oven: 40°C

### Eluent:

(A) 10 mM AcONH₄ solution, (B) acetonitrile
Gradient: 0 min (B) concentration 85%
30 min (B) concentration 100%
30.01 min (B) concentration 85%
35 min (B) concentration 85%

### Flow rate:

1 mL/min

### Injection:

5 µL

HRMS (ESI⁺) [M+NH₄]⁺calcd forC₁₇₂H₁₆₈N₃O₃₅: 2852.1718; found: 2852.1694

1H-NMR (500 MHz, CDCl₃) δ σ7.75 (d, J = 8.0 Hz, 1H), 7.69-7.40 (10H), 7.55-7.10 (m, 54H), 7.06-6.97 (5H), 6.97-6.89 (m, 8H), 6.88-6.84 (m, 1H), 6.82-6.73 (m, 5H), 6.72-6.67 (m, 2H), 6.62-6.56 (m, 5H), 5.42 (d, J = 8.6 Hz, 1H), 5.22 (d, J = 8.0 Hz, 1H), 5.03 (d, J = 12.0 Hz, 1H), 4.99 (d, J = 8.6 Hz, 1H), 4.92 (d, J = 10.9 Hz, 1H), 4.87-4.79 (m, 5H), 4.75 (d, J = 11.5 Hz, 1H), 4.70 (d, J = 12.0 Hz, 1H), 4.67 (d, J = 12.0 Hz, 1H), 4.63 (d, J = 6.3 Hz, 1H), 4.61 (d, J = 6.9 Hz, 1H), 4.60-3.97 (m, 30H), 3.88 (d, J = 2.9 Hz, 1H), 3.84 (d, J = 12.0 Hz, 1H), 3.82-3.67 (m, 5H), 3.66-3.60 (m, 5H), 3.54-3.33 (m, 14H), 3.27-3.23 (m, 1H), 3.20 (d, J = 10.9 Hz, 1H), 3.15-3.09 (m, 2H), 2.83-2.78 (m, 1H), 2.27-2.21 (m, 1H)

13C-NMR (125 MHz, CDCl₃) δ 168.4, 168.2, 167.4, 167.3, 155.2, 150.8, 139.1, 139.0, 138.9, 138.8, 138.7, 138.51, 138.49, 138.44, 138.38, 138.37, 138.35, 138.26, 138.0, 137.7, 133.8, 133.6, 122.49, 133.41, 133.36, 131.8, 131.4, 128.6, 128.52, 128.49, 128.39, 128.34, 128.30, 128.2, 128.14, 128.10, 128.06, 128.0, 127.85, 127.82, 127.7, 127.69, 127.65, 127.56, 127.53, 127.48, 127.38, 127.33, 127.26, 127.1, 127.0, 126.9, 126.7, 123.5, 123.2, 123.09, 123.05, 122.98, 118.5, 114.2, 102.9, 102.6, 97.8, 97.4, 97.0, 96.9, 82.5, 80.5, 79.9, 78.5, 77.8, 77.4, 76.9, 76.7, 75.87, 75.80, 75.2, 75.0, 74.79, 74.77, 74.62, 74.59, 74.48, 74.41, 74.2, 74.0, 73.9, 73.6, 73.4, 73.0, 72.9, 72.7, 72.62, 72.56, 72.0, 69.8, 68.24, 68.20, 68.1, 67.8, 67.2, 56.6, 55.6, 55.5, 53.4

### Example 29

Comparative experiments were carried out on the deacylation reaction to produce the compound represented by Formula C-9 from the compound represented by Formula C-8 in Example 15 above while using the reaction conditions shown in the table below. Entries 1 to 3 are Comparative Examples, and Entries 4 and 5 are Examples of the present invention.

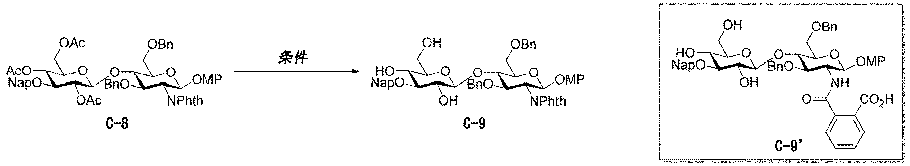

**[Table 1]**

| Entry | Conditions | HPLC PAR (C-9/C-9') (%) |
|---|---|---|
| 1 | NaOMe, DCM, r.t., 1 h | N.D. / 98.8% |
| 2 | 2M HCl/MeOH, THF, r.t.∼5D°C, 1 | N.D. / 47.7% (decomposition was observed) |
| | 4.5 h | |
| 3 | *t*-BuOK, THF-MeOH, 60°C, 5 h | 84.5% / 7.8% |
| 4 | *t*-BuOK, CF₃CO₂Me, THF-MeOH, 6 | 98.9% / N.D. |
| | 0°C, 2 h | |
| 5 | LHMDS, CF₃CO₂Me, THF-MeOH, 60°C, 2 h | 98.4% / N.D. |

| | | |
|---|---|---|
| - In the case of NaOMe, the ring-opening reaction proceeded completely due to the effect of moisture in the reagent (Entry 1). - Under acidic conditions, the ring-opening of the phthalimide group could be inhibited, but the reaction rate was slow and decomposition proceeded (Entry 2). - When t-BuOK was used, the target substance was produced in about 85% yield, but about 8% of the ring-opened product was by-produced due to the influence of moisture in the reagent and solvent (Entry 3). - When CF₃CO₂Me was added, the reaction was complete with almost complete suppression of the ring-opened product. t-BuOK and LHMDS as bases gave comparable results (Entries 4 and 5). | | |

### Example 30

The de-2-naphthylmethylation reaction of the compound represented by Formula A-12 containing 15 benzyl groups and one 2-naphthylmethyl group was carried out under conventional conditions (CH₂Cl₂-H₂O) (Entry 1) or the present method (HFIP-H₂O) (Entries 2 and 3) as shown in the table below, and analyzed by HPLC to calculate the area peak ratio of the target substance (the compound represented by Formula A-13) vs. the excess reactant in the debenzylated form. The table below shows the results.

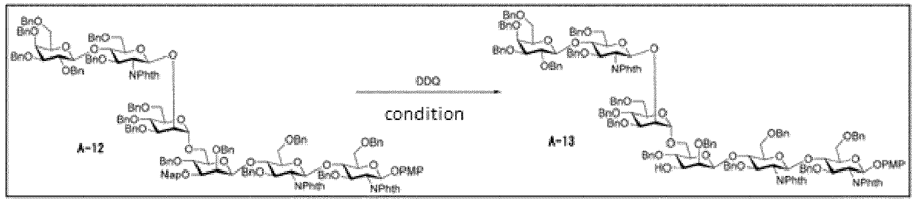

**[Table 2]**

| Entry | Conditions | HPLC PAR (Compound of formula A-131 Debenzylated byproduct) (%) |
|---|---|---|
| 1 | DDQ, CH₂Cl₂, H₂O, 0°C | 55.5. / 25.8% |
| 2 | DDQ, HFIP, H₂O, 0°C | 71.7 / 10.8% |
| 3 | DDQ, HFIP, H₂O, -30°C | 87.4% / 6.6% |

| | | |
|---|---|---|
| - Entry 1: under the conventional method conditions, the debenzylated form selectivity was moderate. - Entry 2: the selectivity was improved by using HFIP. - Entry 3: The best results were obtained by performing the reaction at an even lower temperature while using HFIP. | | |

### Example 31

The de-2-naphthylmethylation reaction of the compound represented by Formula A-4 containing four benzyl groups and one 2-naphthylmethyl group was carried out under conventional conditions (CH₂Cl₂-H₂O ) (Entry 1), the present method (HFIP-H₂O) (Entry 2), acidic conditions (Entry3), or hydrogenation conditions (Entry4) as shown in the table below. HPLC analysis was used to calculate the area peak ratio of the target substance (the compound represented by Formula A-4' below). The table below shows the results.

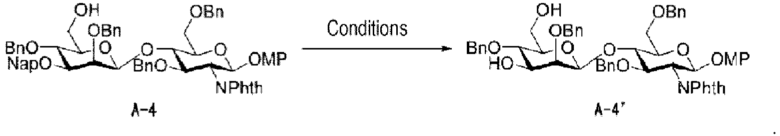

**[Table 3]**

| Entry | Conditions | HPLC PAR. (Compound of formula A-4') (%) |
|---|---|---|
| 1 | DDQ, KH₂PO₄, CH₂Cl₂, H₂O, Room temperature | 85.5 |
| 2 | DDQ, HFIP, H₂O, 0°C | 94.0 |
| 3 | 4MHCl/EtOAc, HFIP, CH₂Cl₂, Room temperature | 19.2 |
| 4 | H₂, Pd/C, EtOH, THF, 50c | 43.7 |

| | | |
|---|---|---|
| - Entry 1: the reaction proceeded in moderate to high yields even under conventional method conditions. - Entry 2: the reaction was improved by using HFIP. - Entry 3: the HCl/HFIP conditions reported in a scientific article (J. Org. Chem. 2015, 80, 8796-8806) were applied, but the reaction was complicated and resulted in low yields. - Entry 4: when the hydrogenation conditions were used, removing the benzyl group was also observed, resulting in a low yield. | | |

### Example 32

The de-2-naphthylmethylation reaction of the compound represented by Formula A-10 containing nine benzyl groups and one 2-naphthylmethyl group was carried out under conventional conditions (CH₂Cl₂-H₂O) (Entry 1) or the present method (HFIP-H₂O) (Entry 2) as shown in the table below. HPLC analysis was used to calculate the area peak ratio of the target substance (the compound represented by Formula A-10' below). The table below shows the results.

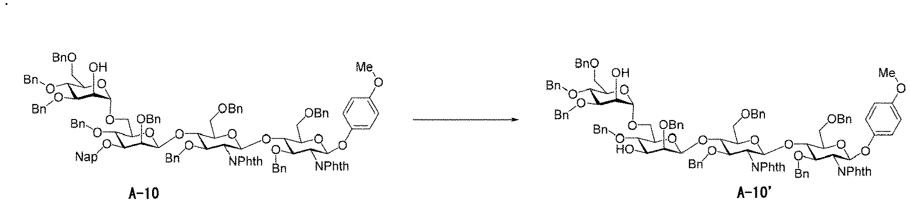

**[Table 4]**

| Entry | Conditions | HPLC PAR (TM) (%) |
|---|---|---|
| 1 | DDQ, CH₂Cl₂, H₂O, 0°C | 66.9 |
| 2 | DDQ, HFIP, H₂O, 0°C | 82.2 |

### Example 33

### <To separate and purify compound represented by Formula A-8 and compound represented by Formula A-9>

The following shows an example of separating and purifying the compound represented by Formula A-8, which is a sugar acceptor used in tetrasaccharide synthesis, and the compound represented by Formula A-9. The sugar acceptor compound represented by Formula A-8 and the tetrasaccharide compound represented by Formula A-9 have very close polarity in normal-phase silica gel column chromatography. This made it difficult to separate them, for example, under the typical column solvent system (hexane-ethyl acetate) conditions because of the same Rf value. However, the present invention can be used to easily separate the monosaccharide from the tetrasaccharide with very close polarity through silica gel.

The experimental manipulation is as follows. First, triethylamine was added to the post-reaction solution to stop the reaction, followed by filtration through Molecular Sieves, concentration, and addition of acetonitrile. After octadecyl-modified silica gel was added to the solution, water was added to adsorb the tetrasaccharide compound represented by Formula A-9. HPLC analysis of the filtrate at this time revealed that the tetrasaccharide compound represented by Formula A-9 was adsorbed, while the monosaccharide compound represented by Formula A-8 was present in the filtrate. Acetonitrile-water was optionally added, and the monosaccharide compound represented by Formula A-8 was washed out. Subsequently, the tetrasaccharide compound represented by Formula A-9 was extracted with acetonitrile and toluene. Tables 1 and 2 below show the purification results of the compound represented by Formula A-8.

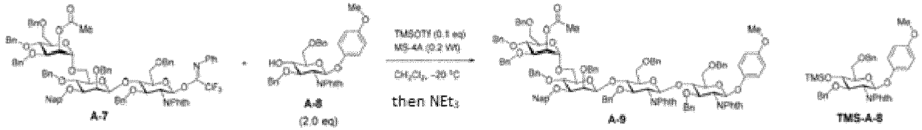

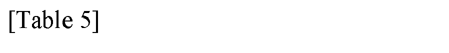

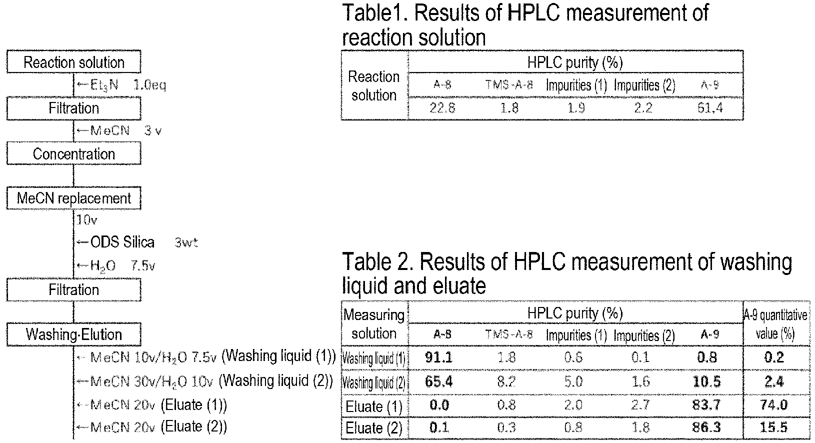

### <To synthesize the compound represented by Formula D-3>

The compound represented by Formula D-3 was synthesized according to the following synthesis scheme Z.

### [Synthesis Scheme Z]

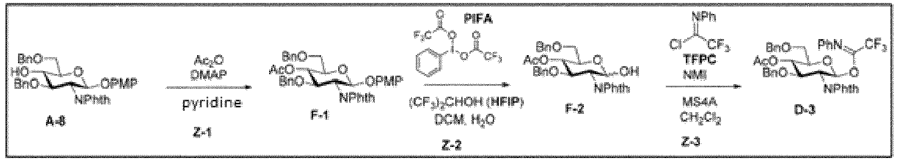

### Example 34

### 4-Methoxyphenyl 4-O-acetyl-3,6-di-O-benzyl-2-deoxy-2-(1,3-dioxo-1,3-dihydro-2H-isoindol-2-yl)-β-D-glucopyranoside(the compound represented by Formula F-1)

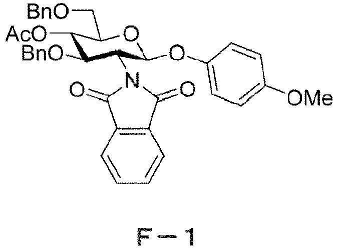

### (Substep Z-1)

An ethyl acetate solution (200 mL) containing 4-methoxyphenyl 3,6-di-O-benzyl-2-deoxy-2-(1,3-dioxo-1,3-dihydro-2H-isoindol-2-yl)-β-D-glucopyranoside (the compound represented by Formula A-8) (50.0 g, 83.94 mmol) was mixed with triethylamine (11.04 g, 109.12 mmol), dimethylaminopyridine (0.31 g, 2.52 mmol), and acetic anhydride (11.10 g, 109.12 mmol), and the mixture was stirred at 20°C for 4 hours. After the completion of the reaction was checked by HPLC, ethanol (500 mL) and water (150 mL) were added dropwise. The slurry solution was stirred for 1 hour, and the precipitated crystals were filtered. The filtered crystals were washed with a mixture of ethanol and water (150/50 mL) and dried under reduced pressure at 40°C to give 4-methoxyphenyl 4-O-acetyl-3,6-di-O-benzyl-2-deoxy-2-(1,3-dioxo-1,3-dihydro-2H-isoindol-2-yl)-β-D-glucopyranoside (the compound represented by Formula F-1) (49.0 g, yield 91%) as white crystals.

¹H-NMR (500 MHz, CDCl₃) δ 7.85-7.60 (m, 4H), 7.24-7.34 (m, 5H), 7.04-7.00 (m, 2H), 6.96-6.87 (m, 3H), 6.84 (dt, J = 9.0, 3.0 Hz, 2H), 6.67 (dt, J = 8.5, 2.5 Hz, 2H), 5.66 (t, J = 4.0 Hz, 1H), 5.22-5.18 (m, 1H), 4.64 (d, J = 12.0 Hz, 1H), 4.55-4.48 (m, 4H), 4.36 (d, J= 12.0 Hz, 1H), 3.90-3.84 (m, 1H), 3.68 (s, 3H), 3.68-3.64 (m, 2H), 1.98 (s, 3H).

¹³C-NMR (125 MHz, CDCl₃) δ 169.6, 155.3, 150.6, 137.8, 137.5, 133.9, 128.2, 128.0, 127.7, 127.7, 127.5, 127.4, 123.3, 118.4, 114.3, 97.4, 76.8, 73.9, 73.7, 73.5, 72.2, 69.4, 55.4, 55.3, 20.8.

HRMS(ESI⁺)[M+H]⁺ calcd for C₃₇H₃₆NO₉: 638.2385; found 638.2401.

### Example 35

### 4-O-Acetyl-3,6-di-O-benzyl-2-deoxy-2-(1,3-dioxo-1,3-dihydro-2H-isoindol-2-yl)-D-glucopyranoside(the compound represented by Formula F-2)

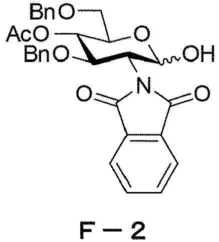

### (Substep Z-2)

To a solution containing 4-methoxyphenyl 4-O-acetyl-3,6-di-O-benzyl-2-deoxy-2-(1,3-dioxo-1,3-dihydro-2H-isoindol-2-yl)-β-D-glucopyranoside (the compound represented by Formula F-1) (49.0 g, 76.84 mmol) in dichloromethane (392 mL), hexafluoro-2-propanol (245 mL), and water (25 mL) was added [bis(trifluoroacetoxy)iodo]benzene (46.3 g, 107.58 mmol) at 25°C or below, and the mixture was stirred at the same temperature for 4 hours. After the completion of the reaction was checked by HPLC, ethyl acetate (1225 mL) was added. After the mixture was cooled on ice, water (490 mL) dissolving sodium hydrogen carbonate (24.5 g) and sodium sulfite (24.5 g) was poured, and the mixture was then liquid-separated to obtain an organic layer. The organic layer obtained was washed again with water (490 mL) dissolving sodium hydrogen carbonate (24.5 g) and sodium sulfite (24.5 g), and further washed with 20% brine (245 g). The resulting organic layer was concentrated under reduced pressure to 490 mL (crystals were found to precipitate during concentration), and heptane (735 mL) was added dropwise. The resulting slurry solution was cooled to 0°C-5°C, and stirred at the same temperature for 1 hour. The precipitated crystals were then filtered. The filtered crystals were washed with a mixture of ethyl acetate and heptane (39/118 mL) at 0°C-5°C and dried under reduced pressure at 40°C to give 4-O-acetyl-3,6-di-O-benzyl-2-deoxy-2-(1,3-dioxo-1,3-dihydro-2H-isoindol-2-yl)-D-glucopyranoside (the compound represented by Formula F-2) (37.5 g, yield 92%) as white crystals.

¹H-NMR (400 MHz, CDCl₃) δ 7.71-7.65 (m, 4H), 7.34-7.26 (m, 5H), 7.01-6.87 (m, 5H), 5.36 (dd, J = 8.0, 8.0 Hz, 1H), 5.13 (dd, J = 8.4, 10.0 Hz, 1H), 4.59 (d, J = 12.4 Hz, 1H), 4.54 (s, 2H), 4.50 (dd, J = 8.4, 10.4 Hz, 1H), 4.33 (d, J = 12.4 Hz, 1H), 4.17 (dd, J = 8.4, 10.4 Hz, 1H), 3.79 (ddd, J = 8.4, 5.2, 4.8 Hz, 1H), 3.61-3.53 (m, 2H), 3.41 (d, J = 8.0 Hz, 1H), 1.93 (s, 3H).

¹³C-NMR (100 MHz, CDCl₃) δ 169.8, 168.1, 137.7, 137.7, 134.0, 131.6, 128.4, 128.2, 128.0, 127.8. 127.7, 127.5, 123.4, 116.2, 92.9, 73.9, 73.7, 73.5, 72.2, 69.3, 57.1, 20.9. HRMS(ESI⁻)[M-H]⁻ calcd for C₃₀H₂₈NO₈: 530.1820; found 530.1841.

### Example 36

### 4-O-Acetyl-3,6-di-O-benzyl-2-deoxy-2-(1,3-dioxo-1,3-dihydro-2H-isoindol-2-yl)-O-[2,2,2-trifluoro-N-phenylethanimidoyl]-β-D-glucopyranoside(the compound represented by Formula D-3)

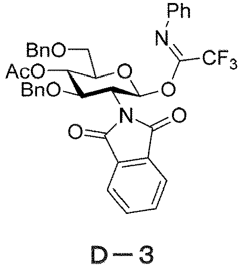

### (Substep Z-3)

First, 4-O-acetyl-3,6-di-O-benzyl-2-deoxy-2-(1,3-dioxo-1,3-dihydro-2H-isoindol-2-yl)-D-glucopyranoside (the compound represented by Formula F-2) (20.0 g, 37.63 mmol) was added to a 500-mL recovery flask, and was mixed with dichloromethane (200 mL) and Molecular Sieves 4A powder (10 µm or less, 10.0 g). N-Methylimidazole (3.40 g, 41.39 mmol) and 2,2,2-trifluoro-N-phenylacetimidoyl chloride (8.20 g, 39.51 mmol) were sequentially added at 0°C under nitrogen, and the mixture was stirred at the same temperature for 18 hours. After the completion of the reaction was checked by HPLC, the reaction solution was filtered and washed with dichloromethane (100 mL). The filtrate was filtered through a neutral silica gel pad (silica gel 60N; particle size: 40 to 50 µm; 60 g; available by KANTO CHEMICAL CO., INC.) packed with dichloromethane, and collected every 100 mL. The silica gel pad was washed with dichloromethane (400 mL, 100 mL each collected) and ethyl acetate/dichloromethane (1:4; 400 mL, 100 mL each collected). The selected fractions were concentrated until the liquid volume reached 40 mL. Toluene (200 mL) was added, and the mixture was concentrated again until the liquid volume reached 40 mL. Toluene (200 mL) was further added, and the mixture was concentrated until the liquid volume reached 40 mL to give a toluene solution containing crude 4-O-acetyl-3,6-di-O-benzyl-2-deoxy-2-(1,3-dioxo-1,3-dihydro-2H-isoindol-2-yl)-O-[2,2,2-trifluoro-N-phenylethanimidoyl]-β-D-glucopyranoside (the compound represented by Formula D-3). This was used as it was in the next step.

### <To synthesize the compound represented by Formula D-7>

The compound represented by Formula D-7 was synthesized according to the following synthesis scheme V.

### [Synthesis Scheme V]

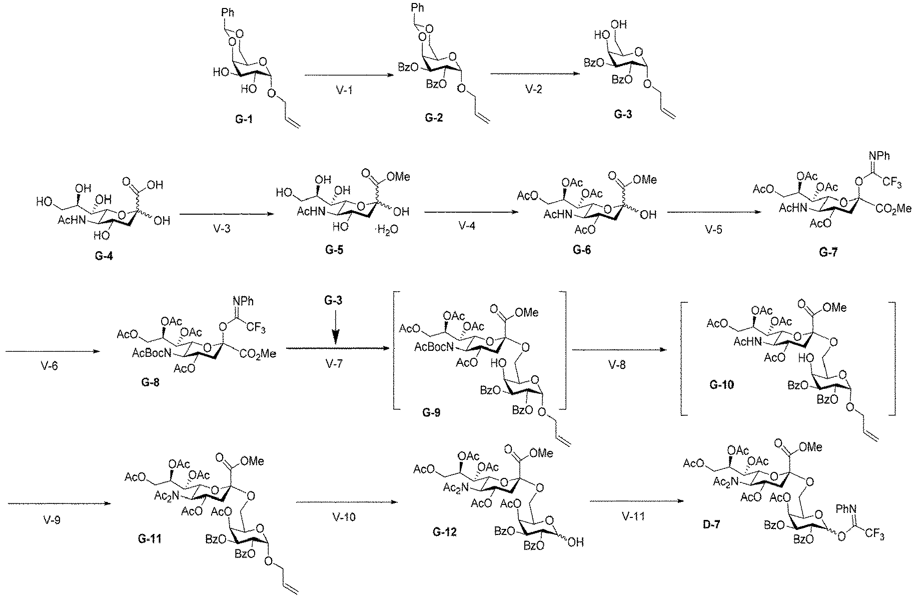

First, the compound represented by Formula G-1 was synthesized according to the following synthesis scheme W.

### [Synthesis Scheme W]

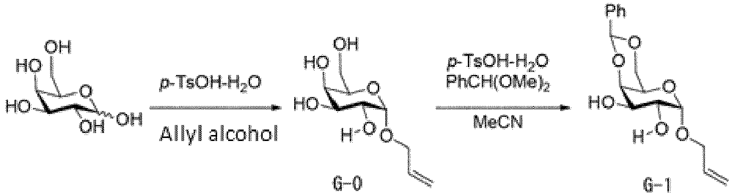

### Example 37

Allyl α-D-galactopyranoside(the compound represented by G-0)

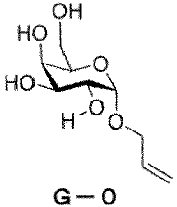

D-galactopyranose (20.00 g, 111.01 mmol) was added to a 500-mL four-neck flask, and allyl alcohol (200.0 mL) was then added. Under a nitrogen atmosphere, p-TsOH·H₂O (2.11 g, 11.10 mmol) was added at room temperature. The temperature was raised to 70°C, and the mixture was stirred for 24 hours. The reaction solution was cooled to 40°C, and mixed with triethylamine (1.69 g, 16.65 mmol), stirred for 5 min, and the reaction solution was then concentrated under reduced pressure to a liquid volume of 100 mL. Next, nBuOH (200 mL) was added dropwise to this concentrated liquid over 30 minutes, and the mixture was stirred at room temperature for 1 hour. The reaction solution was then concentrated under reduced pressure to a liquid volume of 80 mL and stirred at room temperature overnight. The suspension was filtered. The resulting crystals were washed with nBuOH (40 mL) at 0°C and dried under reduced pressure at 40°C to give allyl α-D-galactopyranoside (the compound represented by G-0) (8.41 g, yield: 34.4%) as white crystals.

¹H-NMR (500 MHz, METHANOL-D4) δ 5.97 (qd, J = 11.2, 5.7 Hz, 1H), 5.33 (dd, J = 17.2, 1.7 Hz, 1H), 5.17 (dd, J = 10.6, 1.4 Hz, 1H), 4.22 (dd, J = 13.2, 5.2 Hz, 1H), 4.04 (dd, J = 13.0, 6.0 Hz, 1H), 3.88 (d, J = 1.7 Hz, 1H), 3.82-3.66 (m, 5H).

¹³C-NMR (125 MHz, METHANOL-D4) δ 62.74, 69.36, 70.21, 71.08, 71.51, 72.49, 99.46, 117.47, 135.69.

MS (ESI) m/z: 221 (M+H)⁺, 219 (M-H)⁻.

### Example 38

### Prop-2-en-1-yl 4.6-O-benzylidene-α-D-galactopyranoside(the compound represented by Formula G-1)

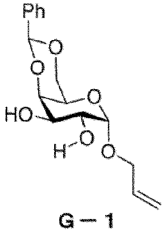

Under a nitrogen atmosphere, acetonitrile (5.0 mL), benzaldehyde dimethyl acetal (5.18 g, 34.1 mmol), and p-TsOH·H₂O (431.9 mg, 2.27 mmol) were added to a 100-mL recovery flask, followed by addition of allyl α-D-galactopyranoside (5.00 g, 22.7 mmol) little by little. The temperature was then raised to 40°C, and the solution was stirred for 30 minutes. Subsequently, triethylamine (344.6 mg, 3.41 mmol) was added, and the mixture was stirred for 5 min, followed by dropwise addition of isopropyl alcohol (75 mL) at 40°C. The reaction solution was concentrated under reduced pressure until the liquid volume reached 25 mL and stirred at 0°C overnight. Subsequently the suspension was filtered. The resulting crystals were then washed with isopropyl alcohol (5 mL) chilled to 0°C and dried under reduced pressure at 40°C to give prop-2-en-1-yl 4.6-O-benzyliden-α-D-galactopyranoside (the compound represented by Formula G-1) (5.35 g, yield: 76.3%) as white crystals.

¹H-NMR (500 MHz, METHANOL-D4) δ 7.53-7.52 (dd, J = 7.5, 2.0 Hz, 2H), 7.34 (m, 3H), 5.98 (qd, J = 11.1, 5.4 Hz, 1H), 5.59 (s, 1H), 5.35 (d, J = 18.9 Hz, 1H), 5.19 (d, J = 10.3 Hz, 1H), 4.95 (d, J = 4.0 Hz, 1H), 4.26 (d, J = 3.4 Hz, 1H), 4.22 (dd, J = 13.2, 5.2 Hz, 1H), 4.13 (s, 2H), 4.08 (q, J = 6.5 Hz, 1H), 3.92 (ddd, J = 24.1, 10.3, 3.4 Hz, 2H), 3.74 (s, 1H). ¹³C-NMR (125 MHz, METHANOL-D4) δ 64.60, 69.70, 70.03, 70.08, 70.34, 78.07, 100.22, 102.28, 117.58, 127.54, 129.02, 129.86, 135.59, 139.77.

MS (ESI) m/z: 309 (M+H)⁺, 307 (M-H)⁻.

### Example 39

### Prop-2-en-1-yl 2,3-di-O-benzoyl-4,6-O-benzyliden-α-D-galactopyranoside(the compound represented by Formula G-2)

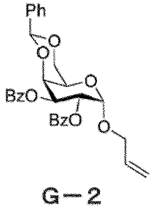

### (Substep V-1)

To a pyridine solution (150 mL) containing prop-2-en-1-yl 4,6-O-benzylidene-α-D-galactopyranoside (the compound represented by Formula G-1) (30.0 g, 97.30 mmol) was added dropwise at 40°C benzoyl chloride (47.87 g, 340.54 mmol). The mixture was stirred at the same temperature for 2 hours. After the completion of the reaction was checked by HPLC, the mixture was cooled to 20°C-30°C. After ethanol (450 mL) was poured, water (300 mL) was added dropwise over 30 minutes. The resulting slurry solution was stirred at 20°C-30°C for 1 hour. The precipitated crystals were then filtered. The filtered crystals were washed with a mixture of ethanol and water (75/75 mL) and dried under reduced pressure at 40°C to give prop-2-en-1-yl 2,3-di-O-benzoyl-4,6-O-benzyliden-α-D-galactopyranoside (the compound represented by Formula G-2) (47.9 g, yield 95%) as white crystals.

¹H-NMR (500 MHz, CDCl₃) δ 8.03-7.98 (m, 4H), 7.55-7.46 (m, 4H), 7.40-7.30 (m, 7H), 5.90-5.78 (m, 2H), 5.82 (s, 1H), 5.57 (s, 1H), 5.42 (d, J = 1.7 Hz, 1H), 5.31 (dd, J = 17.2, 1.7 Hz, 1H), 5.15 (dd, J = 1.7, 10.5 Hz, 1H), 4.66 (s, 2H), 4.33 (d, J = 12.5 Hz, 1H), 4.26 (dd, J = 12.5, 4.5 Hz, 1H), 4.12 (dd, J = 12.5, 1.0 Hz, 1H), 4.08 (dd, J = 6.5, 13.5 Hz, 1H), 3.95 (s, 1H).

¹³C-NMR (125 MHz, CDCl₃) δ 166.1, 165.8, 137.5, 133.4, 133.2, 133.1, 129.8, 129.7, 129.5, 129.4, 128.8, 128.3, 128.1, 126.1, 117.5, 100.6, 96.2, 74.2, 69.3, 69.1, 68.7, 68.6, 62.4.

HRMS (ESI⁺) [M+H]⁺ calcd for C₃₀H₂₉O₈: 517.1857; found 517.1880.

### Example 40

### Prop-2-en-1-yl 2,3-di-O-benzoyl-α-D-galactopyranoside(the compound represented by Formula G-3)

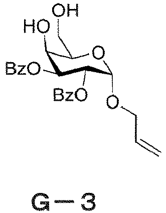

### (Substep V-2)

An acetonitrile (377 mL) solution containing prop-2-en-1-yl 2,3-di-O-benzoyl-4,6-O-benzyliden-α-D-galactopyranoside(the compound represented by Formula G-2) (47.1 g, 91.18 mmol) was heated to 45°C. Water (24 mL) and concentrated hydrochloric acid (9.2 g, 91.18 mmol) were added, and the mixture was stirred at the same temperature for 30 minutes. Water (353 mL) was added dropwise at 45°C-50°C over 3 hours, and the mixture was stirred for another 30 minutes. After the completion of the reaction was checked by HPLC, sodium acetate (11.22 g, 136.78 mmol) was added and ethyl acetate (942 mL) and water (471 mL) were poured. After cooling to 25°C or below, the mixture was liquid-separated to obtain an organic layer. The organic layer obtained was washed twice with water (471 mL) and further washed with 20% brine (236 mL). The organic layer was concentrated under reduced pressure to 141 mL, and toluene (707 mL) was added. The mixture was concentrated again under reduced pressure to 141 mL. Toluene (236 mL) was added to the concentrated solution obtained, and the mixture was concentrated under reduced pressure to 141 mL. The concentrated liquid was cooled to 0°C to 5°C and a toluene (330 mL) slurry solution containing neutral silica gel (silica gel 60N; particle size: 40 to 50 µm; 141 g; available by KANTO CHEMICAL CO., INC.) cooled to 0 to 5°C was poured. The mixture was stirred at the same temperature for 15 minutes, and the product was adsorbed on silica gel, followed by filtration. The silica gel solid phase containing the product was washed with toluene (942 mL) at 0°C-5°C (the filtrate at the time of the toluene wash was discarded). The target substance was then desorbed from silica gel by using cyclopentyl methyl ether (707 mL) to give a cyclopentyl methyl ether solution containing prop-2-en-1-yl 2,3-di-O-benzoyl-α-D-galactopyranoside (the compound represented by Formula G-3) (quantitative value 36.2 g, quantitative yield 93%). This solution was used in the next step.

### Example 41

### Methyl 5-acetamido-3,5-dideoxy-D-glycero-D-galacto-non-2-ulopyranosonate monohydrate(the compound represented by Formula G-5)

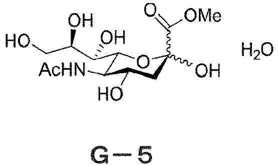

### (Substep V-3)

To a methanol (321 mL) solution containing 5-acetamido-3,5-dideoxy-D-glycero-D-galacto-non-2-uropyranosonic acid (the compound represented by Formula G-4) (40.1 g, 129.66 mmol) and methyl orthoformate (15.60 mL, 142.59 mmol) was added sulfuric acid (1.0 g, 10.20 mmol), and the mixture was heated to 40°C and stirred for 3 hours. After the completion of the reaction was checked by HPLC, the mixture was cooled to 25°C. Dimethylacetamide (40 mL) was added, and the mixture was concentrated under reduced pressure to 160 mL. The temperature of the resulting concentrated solution was adjusted to 15°C. Water (20 mL) and ethyl acetate (722 mL) were poured. The mixture was then stirred at 25°C for 1 hour. The slurry solution was cooled to 0°C-5°C, and then stirred at the same temperature for 2 hours. The precipitated crystals were filtered. The filtered crystals were washed with ethyl acetate (80 mL) at 0°C-5°C and dried under reduced pressure at 40°C to give methyl 5-acetamido-3,5-dideoxy-D-glycero-D-galacto-non-2-ulopyranosonate monohydrate (the compound represented by Formula G-5) (41.1 g, yield 93%) as white crystals.

¹H-NMR (500 MHz, CD₃OD) δ 4.07-3.98 (m, 2H), 3.85-3.77 (m, 2H), 3.78 (s, 3H), 3.72-3.68 (m, 1H), 3.62 (dd, J = 10.9, 5.7 Hz, 1H), 3.48 (dd, J = 9.2, 1.1 Hz, 1H), 2.22 (dd, J = 12.9, 4.9 Hz, 1H), 2.02 (s, 3H), 1.89 (dd, J = 12.6, 11.5 Hz, 1H).

¹³C-NMR (125 MHz, CD₃OD) δ 175.2, 175.1, 171.8, 96.6, 72.1, 72.0, 71.6, 70.1, 67.9, 64.8, 54.4, 54.3, 53.2, 40.7, 22.7, 22.7.
HRMS(ESI⁺)[M+H]⁺ calcd for C₁₂H₂₂NO₉: 324.1289; found 324.1288.

### Example 42

### Methyl 5-acetamido-4,7, 8,9-tetra-O-acetyl-3, 5-dideoxy-D-glycero-D-galacto-non-2-ulopyranosonate(the compound represented by Formula G-6)

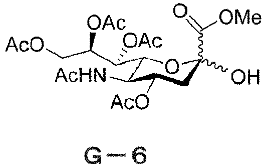

### (Substep V-4)

The temperature of an acetonitrile (403 mL) slurry solution containing methyl 5-acetamido-3,5-dideoxy-D-glycero-D-galacto-non-2-ulopyranosonate monohydrate (the compound represented by Formula G-5) (40.3 g, 118.07 mmol) was adjusted to 25°C. Acetic anhydride (60.27 g, 590.36 mmol) and paratoluenesulfonic acid monohydrate (1.12 g, 5.89 mmol) were added, and the mixture was stirred at 25°C for 24 hours. The reaction liquid was cooled to 15°C, acetic anhydride (12.05 g, 118.03 mmol) was added, and the mixture was then stirred at the same temperature for 47 hours. After the completion of the reaction was checked by HPLC, methanol (40 mL) was added. The temperature was adjusted to 25°C, and the mixture was stirred at the same temperature for 2 hours. Next, sodium acetate (0.97 g, 11.82 mmol) was added, and the mixture was stirred at the same temperature for another 1 hour. The reaction liquid was concentrated under reduced pressure to 120 mL, and cooled to 0°C-5°C. Ethyl acetate (403 mL) and water (161 mL) were then poured, and under stirring at 0°C-5°C, triethylamine was added to adjust the pH to 7.0. The liquid-separated organic layer was washed twice with 10% brine (121 mL), and concentrated under reduced pressure to 200 mL. Ethyl acetate (605 mL) was added to the concentrated solution, and the mixture was concentrated again under reduced pressure to 200 mL. Ethyl acetate (40 mL) was added to the concentrated solution. Seed crystals were inoculated, and the mixture was stirred at 25°C for 4 hours. Heptane (302 mL) was then added dropwise over 30 minutes. The resulting slurry solution was stirred at 25°C for 2 hours, and the precipitated crystals were then filtered. The filtered crystals were washed with a mixture of ethyl acetate and heptane (67/135 mL) and dried under reduced pressure at 35°C to give methyl 5-acetamido-4,7,8,9-tetra-O-acetyl-3,5-dideoxy-D-glycero-D-galacto-non-2-ulopyranosonate (the compound represented by Formula G-6) (44.1 g, yield 76%) as white crystals.

¹H-NMR (500 MHz, CDCl₃) δ 6.28 (d, J = 10.3 Hz, 1H), 5.41 (dd, J = 4.6, 2.3 Hz, 1H), 5.28-5.23 (m, 1H), 5.21-5.14 (m, 1H), 5.09 (s, 1H), 4.62 (dd, J = 12.3, 2.6 Hz, 1H), 4.28 (dd, J = 10.3, 2.3 Hz, 1H), 4.21-4.11 (m, 1H), 4.04 (dd, J = 12.3, 8.3 Hz, 1H), 3.85 (s, 3H), 2.24-2.20 (m, 2H), 2.16 (s, 3H), 2.12 (s, 3H), 2.03 (s, 3H), 2.01 (s, 3H), 1.91 (s, 3H).

¹³C-NMR (125 MHz, CDCl₃) δ 171.5, 171.1, 170.8, 170.3, 170.2, 168.9, 94.9, 72.1, 71.4, 69.1, 68.3, 62.5, 53.2, 49.1, 36.1, 23.0, 21.0, 20.8, 20.7, 20.7.

HRMS (ESI⁺) [M+H]⁺ calcd for C₂₀H₃₀NO₁₃: 492.1712; found 492.1712.

### Example 43

### Methyl 5-acetamido-4,7,8,9-tetra-O-acetyl-3,5-dideoxy-2-O-(2,2,2-trifluoro-N-phenylethanimidoyl)-D-glycero-β-D-galacto-non-2-ulopyranosonate(the compound represented by Formula G-7)

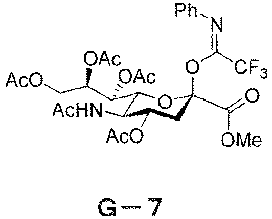

### (Substep V-5)

The temperature of a dichloromethane (352 mL) slurry solution containing methyl 5-acetamido-4,7,8,9-tetra-O-acetyl-3,5-dideoxy-D-glycero-D-galacto-non-2-ulopyranosonate (the compound represented by Formula G-6) (44.0 g, 89.53 mmol) and Molecular Sieves 4A powder (powder particle size: 10 µm or less) (22 g) was adjusted to 20°C. After stirring at the same temperature for 30 minutes, 2,2,2-trifluoro-N-phenylacetimidoyl chloride (26.02 g, 125.35 mmol) was added. Next, N-methylimidazole (11.03 g, 134.33 mmol) was added dropwise, and the mixture was stirred at 20°C for 7.5 hours. The completion of the reaction was checked by HPLC. The reaction liquid was then filtered and washed with dichloromethane (88 mL) to obtain a filtrate. The filtrate obtained was cooled to 0°C and mixed with cold water (440 mL). Under stirring at 0°C-5°C, triethylamine was added to adjust the pH to 7.5. The mixture was stirred at 0°C-5°C for 30 minutes, and then liquid-separated. The resulting organic layer was washed twice with cold water (440 mL) and then cooled 20% brine (220 mL), and concentrated under reduced pressure to 88 mL. Ethyl acetate (440 mL) was added to the concentrated solution, and the mixture was concentrated again under reduced pressure to 88 mL. The concentrated solution was mixed with t-butyl methyl ether (308 mL). Seed crystals were then inoculated, and the mixture was stirred at 20°C for 4 hours. Heptane (264 mL) was added dropwise to the resulting slurry solution over 1 hour. The mixture was stirred at the same temperature for 2 hours. The precipitated crystals were then filtered. The filtered crystals were washed with a mixture of t-butyl methyl ether and heptane (132/88 mL) and dried under reduced pressure at 35°C to give methyl 5-acetamido-4,7,8,9-tetra-O-acetyl-3,5-dideoxy-2-O-(2,2,2-trifluoro-N-phenylethanimidoyl)-D-glycero-β-D-galacto-non-2-ulopyranosonate (the compound represented by Formula G-7) (39.5 g, yield 67%) as white crystals.

¹H-NMR (500 MHz, CDCl₃) δ 7.30-7.24 (m, 2H), 7.09 (t, J = 7.4 Hz, 1H), 6.72 (d, J = 8.0 Hz, 2H), 5.76 (d, J = 9.7 Hz, 1H), 5.48-5.45 (m, 1H), 5.30 (td, J = 10.9, 4.8 Hz, 1H), 5.15-5.10 (m, 1H), 4.60 (dd, J = 12.6, 2.3 Hz, 1H), 4.30 (q, J = 10.3 Hz, 1H), 4.23 (dd, J = 10.3, 2.3 Hz, 1H), 4.11 (dd, J = 12.3 Hz, 7.7 Hz, 1H), 3.81 (s, 3H), 2.79 (dd, J = 13.5, 4.9 Hz, 1H), 2.21-2.15 (m, 1H), 2.16 (s, 3H), 2.10 (s, 3H), 2.07 (s, 3H), 1.90 (s, 3H), 1.75 (s, 3H).

¹³C-NMR (125 MHz, CDCl₃) δ 171.0, 170.7, 170.4, 170.2, 170.1, 165.3, 142.6, 141.0 (q, ²J_{C-F} = 36.0 Hz), 128.8, 124.6, 119.0, 115.1 (q, ¹J_{C-F} = 284.4 Hz), 99.7, 73.6, 71.9, 68.3, 68.0, 62.4, 53.1, 48.6, 35.6, 23.0, 20.8, 20.8, 20.7, 20.3.
HRMS(ESI⁺)[M+NH_{4]}⁺ calcd for C₂₈H₃₇F₃N₃O₁₃: 680.2273; found 680.2314.

### Example 44

### Methyl 4,7,8,9-tetra-O-acetyl-5-[acetyl(tert-butoxycarbonyl)amino]-3,5-dideoxy-2-O-(2,2,2-trifluoro-N-phenylethanimidoyl)-D-glycero-β-D-galacto-non-2-ulopyranosonate(the compound represented by Formula G-8)

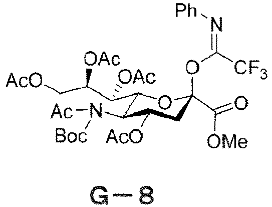

### (Substep V-6)

To a tetrahydrofuran (390 mL) solution containing methyl 5-acetamido-4,7,8,9-tetra-O-acetyl-3,5-dideoxy-2-O-(2,2,2-trifluoro-N-phenylethanimidoyl)-D-glycero-β-D-galacto-non-2-ulopyranosonate (the compound represented by Formula G-7) (39.0 g, 58.86 mmol) were added di-tert-butyl bicarbonate (27.05 g, 123.94 mmol) and dimethylaminopyridine (1.80 g, 14.73 mmol), and the mixture was heated to reflux temperature. The mixture was stirred under reflux for 30 minutes. After the completion of the reaction was checked by HPLC, the reaction solution was concentrated under reduced pressure to 117 mL. Toluene (195 mL) was added to the concentrated solution, and the mixture was concentrated again under reduced pressure to 117 mL. The concentrated solution was filtered using a silica gel-filled funnel (silica gel 60N; particle size: 40 to 50 µm; 117 g; toluene wet packed; available by KANTO CHEMICAL CO., INC.) and washed with a toluene-ethyl acetate mixture (8/2) (975 mL) to obtain a filtrate. The filtrate obtained was concentrated under reduced pressure (to a weight of 59 g), and mixed with cyclopentyl methyl ether (23 mL). The temperature of the solution was adjusted to 20°C, heptane (156 mL) was added dropwise over 15 minutes, and the mixture was then stirred at the same temperature for 1 hour. After the crystals were found to precipitate, heptane (312 mL) was added dropwise over 1 hour. The precipitated crystals were then filtered. The filtered crystals were washed with heptane (78 mL) and dried under reduced pressure at 35°C to give methyl 4,7,8,9-tetra-O-acetyl-5-[acetyl(tert-butoxycarbonyl)amino]-3,5-dideoxy-2-O-(2,2,2-trifluoro-N-phenylethanimidoyl)-D-glycero-β-D-galacto-non-2-ulopyranosonate (the compound represented by Formula G-8) (37.0 g, yield 82%) as white crystals.

¹H-NMR (500 MHz, CDCl₃)
Note: Detected as a ca. 1/4 isomer mixture.
Major isomer: δ 7.27 (t, J = 8.9 Hz, 2H), 7.09 (t, J = 7.2 Hz, 1H), 6.73 (d, J = 8.0 Hz, 2H), 5.75-5.65 (m, 1H), 5.31 (d, J = 4.6 Hz, 1H), 5.18-5.14 (m, 1H), 5.15 (d, J = 6.0 Hz, 2H), 4.54 (dd, J = 12.0, 2.0 Hz, 1H), 4.08 (dd, J = 12.6, 6.9 Hz, 1H), 3.84 (s, 3H), 2.90 (dd, J = 13.7, 5.2 Hz, 1H), 2.39 (s, 3H), 2.25 (dd, J = 13.7, 11.2 Hz, 1H), 2.09 (s, 3H), 2.07 (s, 3H), 1.99 (s, 3H), 1.77 (s, 3H), 1.62 (s, 9H). Minor isomer: δ 6.76 (d, J = 8.0 Hz, 2H), 5.85-5.80 (m, 1H), 5.29-5.25 (m, 1H), 5.22-5.19 (m, 1H), 4.44 (d, J = 11.0 Hz, 1H), 4.15-4.11 (m, 1H), 3.03 (dd, J= 14.0, 5.0 Hz, 1H), 2.41 (s, 3H), 2.12 (s, 3H), 2.00 (s, 3H), 1.88 (s, 3H), 1.74 (s, 3H), 1.54 (s, 9H). ¹³C-NMR (125 MHz, CDCl₃) Mixture: δ 173.7, 170.4, 170.2, 170.0, 169.9, 165.4, 151.7, 142.8, 128.7, 124.5, 119.1, 100.6, 85.2, 72.8, 71.3, 67.7, 65.9, 62.0, 53.1, 52.0, 36.7, 27.9, 27.7, 26.6, 20.8, 20.7, 20.6, 20.3.

HRMS (ESI⁺) [M+NH_{4]}⁺ calcd for C₃₃H₄₅F₃N₃O₁₅: 780.2797; found 780.2801.

### Example 45

### Prop-2-en-1-yl2,3-di-O-benzoyl-6-O-{4,7,8,9-tetra-O-acetyl-5-[acetyl(tert-butoxycarbonyl)amino]-3,5-dideoxy-1-methyl-D-glycero-α-D-galacto-non-2-uropyranosyl}-α-D-galactopyranoside(the compound represented by Formula G-9)

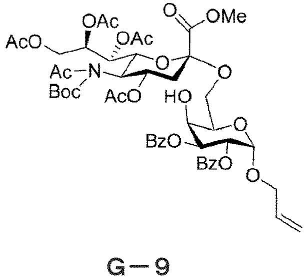

### (Substep V-7)

A cyclopentyl methyl ether solution containing prop-2-en-1-yl 2,3-di-O-benzoyl-α-D-galactopyranoside (the compound represented by Formula G-3) (quantitative value 31.46 g, 73.43 mmol) was concentrated under reduced pressure to 105 mL. This solution was added to a cyclopentyl methyl ether (175 mL) solution containing methyl 4,7,8,9-tetra-O-acetyl-5-[acetyl(tert-butoxycarbonyl)amino]-3, 5-dideoxy-2-O-(2,2,2-trifluoro-N-phenylethanimidoyl)-D-glycero-β-D-galacto-non-2-ulopyranosonate (the compound represented by Formula G-8) (35.0 g, 45.89 mmol). Next, cyclopentyl methyl ether was added to the resulting mixed solution, and the total volume was adjusted to 350 mL (cyclopentyl methyl ether mixed solution containing compound represented by Formula G-3 and compound represented by Formula G-8). Cyclopentyl methyl ether (525 mL) and Molecular Sieves 4A powder (powder particle size: 10 µm or less) (17.5 g) were added to another vessel. After the mixed solution was cooled to -60°C, trimethylsilyl trifluoromethanesulfonate (4.2 mL, 23.24 mmol) was added. To this solution, a cyclopentyl methyl ether mixed solution containing compound represented by Formula G-3 and compound represented by Formula G-8 was added dropwise over 4.5 hours at -60°C under strong stirring, and the mixture was stirred at the same temperature for 2 hours. After the completion of the reaction was checked by HPLC, triethylamine (4.5 mL, 32.12 mmol) was added. The reaction liquid was heated to 0°C. After that, Celite 545 (35.00 g) was added, and the reaction liquid was filtered and washed with cyclopentyl methyl ether (175 mL). Water (350 mL) was added to the filtrate and the liquid was separated. Next, 0.5 N hydrochloric acid water (350 mL) was added to the organic layer, and the mixture was stirred at 20°C for 2 hours. After the decomposition of byproducts was checked by HPLC, the liquid was separated to obtain an organic layer. The organic layer was washed with water (350 mL) and then 20% brine (175 mL), and concentrated under reduced pressure to 70 mL. Toluene (700 mL) was added to the concentrated solution, and the mixture was concentrated under reduced pressure to 70 mL. The concentrated solution was mixed again with toluene (700 mL) and neutral silica gel (silica gel 60N; particle size: 40 to 50 µm; 158 g; available by KANTO CHEMICAL CO., INC.) and stirred at 20°C for 30 minutes. The product was adsorbed on silica gel, and then filtered. The silica gel solid phase containing the product was washed with toluene (1575 mL) (the filtrate at the time of the toluene wash was discarded). The target substance was desorbed from silica gel by using ethyl acetate (875 mL). The resulting ethyl acetate solution was concentrated under reduced pressure to 70 mL. Toluene (175 mL) was added, and the mixture was concentrated again under reduced pressure to 70 mL to give a toluene solution containing prop-2-en-1-yl 2,3-di-O-benzoyl-6-O- f 4,7,8,9-tetra-O-acetyl-5-[acetyl(tert-butoxycarbonyl)amino]-3,5-dideoxy-1-methyl-D-glycero-α-D-galacto-non-2-uropyranosyl}-α-D-galactopyranoside (the compound represented by Formula G-9). This solution was used in the next step.

### Example 46

### Prop-2-en-1-yl 6-O-{5-acetamido-4,7,8,9-tetra-O-acetyl-3,5-dideoxy-1-methyl-D-glycero-α-D-galacto-non-2-uropyranosyl}-2,3-di-O-benzoyl-α-D-galactopyranoside(the compound represented by Formula G-10)

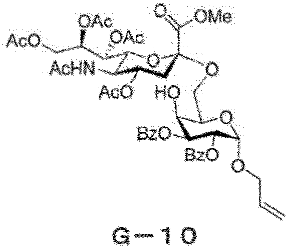

### (Substep V-8)

To the toluene solution containing prop-2-en-1-yl 2,3-di-O-benzoyl-6-O- f 4,7,8,9-tetra-O-acetyl-5-[acetyl(tert-butoxycarbonyl)amino]-3,5-dideoxy-1-methyl-D-glycero-α-D-galacto-non-2-uropyranosyl}-α-D-galactopyranoside (the compound represented by Formula G-9) obtained in Example 45 were added dichloromethane (525 mL) and copper(II) trifluoromethanesulfonate (8.30 g, 22.95 mmol). The temperature was raised to 40°C and the mixture was stirred at the same temperature for 2 hours. After the completion of the reaction was then checked by HPLC, the mixture was cooled to 25°C and concentrated under reduced pressure to 70 mL. The concentrated solution was mixed with ethyl acetate (525 mL), and washed three times with 5% brine (350 mL). Heptane (263 mL) was then added to the organic layer, and the mixture was washed four times with 20% methanol in water (525 mL). HPLC was used to check whether impurities derived from the β-removed form of compound 8, a byproduct of the glycosylation reaction, were removed into the aqueous layer. The organic layer was then concentrated under reduced pressure to 70 mL. Isopropenyl acetate (525 mL) was added to the concentrated solution, and the mixture was concentrated under reduced pressure to 350 mL to give an isopropenyl acetate solution containing prop-2-en-1-yl 6-O-{5-acetamido-4,7,8,9-tetra-O-acetyl-3,5-dideoxy-1-methyl-D-glycero-α-D-galacto-non-2-uropyranosyl}-2,3-di-O-benzoyl-α-D-galactopyranoside (the compound represented by Formula G-10). This solution was used in the next step.

### Example 47

### Prop-2-en-1-yl4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-α-D-galactopyranoside(the compound represented by Formula G-11)

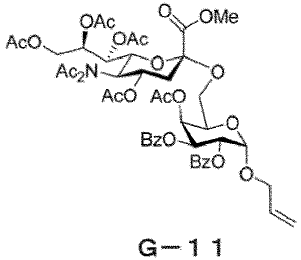

### (Substep V-9)

To the isopropenyl acetate solution containing prop-2-en-1-yl 6-O-{5-acetamide-4,7,8,9-tetra-O-acetyl-3,5-dideoxy-1-methyl-D-glycero-α-D-galacto-non-2-uropyranosyl}-2,3-di-O-benzoyl-α-D-galactopyranoside (the compound represented by Formula G-10) obtained in Example 46 was added paratoluenesulfonic acid monohydrate (0.88 g, 4.62 mmol). The temperature was raised to reflux temperature (internal temperature at or near 90°C) and the mixture was stirred at the same temperature for 3 hours. After the completion of the reaction was checked by HPLC, the mixture was cooled to 25°C. Triethylamine (0.95 mL, 6.85 mmol) was added, and the mixture was concentrated under reduced pressure to 70 mL. Toluene (350 mL) was added to the concentrated solution, and the mixture was concentrated again under reduced pressure to 70 mL. The concentrated solution was mixed with toluene (630 mL) and then neutral silica gel (silica gel 60N: particle size: 40 to 50 µm; 123 g; available by KANTO CHEMICAL CO., INC.). The mixture was stirred at the same temperature for 30 minutes. The product was adsorbed on the silica gel, and then filtered. The silica gel solid phase containing the product was washed with toluene (1925 mL) and then a toluene/ethyl acetate mixture (97/3, 1400 mL) (the filtrate at the time of the wash was discarded). The target substance was desorbed from the silica gel solid phase containing the product by using ethyl acetate (1050 mL). The resulting ethyl acetate solution was concentrated under reduced pressure to give prop-2-en-1-yl 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-α-D-galactopyranoside (the compound represented by Formula G-11) (30.1 g, yield 67% (in terms of compound represented by Formula G-8)) as a white foamy solid (containing 0.42 equivalents of toluene (about 4% by weight)).

¹H-NMR (500 MHz, CDCl₃) δ 7.99 (d, J = 8.4 Hz, 2H), 7.88 (dd, J = 8.4, 1.4 Hz, 2H), 7.53-7.47 (m, 2H), 7.37 (dt, J = 14.7, 6.9 Hz, 4H), 5.90-5.82 (m, 1H), 5.82 (dd, J = 10.9, 3.4 Hz, 1H), 5.73 (d, J = 2.9 Hz, 1H), 5.58 (dd, J = 10.9, 4.0 Hz, 1H), 5.51 (td, J = 10.6, 5.0 Hz, 1H), 5.35-5.30 (m, 3H), 5.17-5.15 (m, 2H), 4.94 (dd, J = 10.3, 1.7 Hz, 1H), 4.38-4.27 (m, 3H), 4.20-4.13 (m, 2H), 4.08 (dd, J = 13.2, 5.7 Hz, 1H), 3.94 (dd, J = 10.3, 6.3 Hz, 1H), 3.82 (s, 3H), 3.50 (dd, J = 9.7, 7.4 Hz, 1H), 2.73 (dd, J = 13.2, 5.2 Hz, 1H), 2.37 (s, 3H), 2.31 (s, 3H), 2.19 (s, 3H), 2.15 (s, 3H), 2.14 (s, 3H), 2.03 (s, 3H), 1.97 (s, 3H), 1.86 (dd, J = 13.2, 10.9 Hz, 1H).

¹³C-NMR (125 MHz, CDCl₃) δ 174.5, 173.6, 170.5, 170.1, 169.9, 169.8, 169.6, 167.3, 166.0, 165.5, 133.5, 133.3, 133.1, 129.8, 129.5, 129.4, 128.4, 128.3, 117.5, 98.6, 95.5, 77.2, 69.8, 68.9, 68.6, 68.6, 68.3, 68.3, 67.5, 67.0, 66.7, 62.4, 61.8, 57.0, 52.9, 38.7, 27.9, 25.9, 21.0, 20.9, 20.7, 20.7, 20.6.
HRMS(ESI⁺)[M+H]⁺ calcd for C₄₇H₅₆NO₂₂: 986.3288; found 986.3277.

The obtained compound was found to correspond to the spectrum in the following literature:
J. Org. Chem., 2016, 81, 10600-10616.

### Example 48

### 4-O-Acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-D-galactopyranose(the compound represented by Formula G-12)

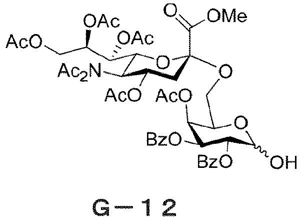

### (Substep V-10)

The pressure of a methanol solution (290 mL) containing prop-2-en-1-yl 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-α-D-galactopyranoside (the compound represented by Formula G-11) (29.00 g, 29.41 mmol), 1,3-dimethylbarbituric acid (9.19 g, 58.86 mmol), and triphenylphosphine (2.31 g, 8.81 mmol) was reduced. The solution was subjected to nitrogen substitution. This operation was repeated five times for deaeration. Palladium(II) acetate (0.66 g, 2.94 mmol) was then added, and the mixture was stirred at 40°C for 12 hours. After the completion of the reaction was checked by HPLC, toluene (580 mL) and water (1015 mL) were added. The resulting liquid was separated to obtain an organic layer. The organic layer was washed four times with 20% methanol water (580 mL) to remove 1,3-dimethylbarbituric acid into the aqueous layer. The organic layer was concentrated under reduced pressure to 58 mL, and toluene (435 mL) was added. The mixture was concentrated again under reduced pressure to 58 mL. The concentrated solution was mixed with toluene (383 mL), chloroform (197 mL), and neutral silica gel (silica gel 60N; particle size: 40 to 50 µm; 145 g; available by KANTO CHEMICAL CO., INC.). The mixture was stirred for 30 minutes. The product was adsorbed on the silica gel, and then filtered. The silica gel solid phase containing the product was washed with toluene/chloroform mixture (2/1, 4350 mL) (the filtrate at the time of the wash was discarded). The target substance was desorbed from the silica gel solid phase containing the product by using ethyl acetate (870 mL). SH silica gel (29.00 g) was added to the resulting ethyl acetate solution. The mixture was stirred for 30 minutes, filtered, and then washed with ethyl acetate (145 mL) to obtain an ethyl acetate solution containing the product of interest. The resulting solution was concentrated under reduced pressure to 58 mL, and toluene (145 mL) was added. The mixture was concentrated again under reduced pressure to 58 mL. The concentrated solution was purified by silica gel column chromatography (silica gel 60N, 40-50 µm, 290 g; mobile phase hexane/ethyl acetate 50/50 to 30/70; available by KANTO CHEMICAL CO., INC.) and the selected fractions were concentrated under reduced pressure to 29 mL. The concentrated solution was mixed with ethyl acetate (290 mL) and activated carbon (Shirasagi A, 14.5 g), stirred for 30 minutes, filtered, and then washed with ethyl acetate (87 mL) to obtain a purified ethyl acetate solution containing the product of interest. The resulting solution was concentrated under reduced pressure to give 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-D-galactopyranose (the compound represented by Formula G-12) (18.70 g, 67% yield) as a white foamy solid.

¹H-NMR (500 MHz, CDCl₃) Major isomer: δ 7.98-8.03 (m, 2H), 7.89 (t, 2H, J = 8.9 Hz), 7.52-7.48 (m, 2H), 7.39-7.34 (m, 4H), 5.92 (dd, 1H, J = 10.3, 2.9 Hz), 5.82 (d, 1H, J = 3.4 Hz), 5.68 (t, 1H, J = 2.3 Hz), 5.59-5.48 (m, 2H), 5.37 (td, 1H, J = 7.5, 2.5 Hz), 5.17 (d, 1H, J = 7.5 Hz), 5.02 (d, 1H, J = 10.5 Hz), 4.74-4.71 (m, 1H), 4.44-4.38 (m, 1H), 4.16-4.08 (m, 2H), 3.85 (s, 3H), 3.80 (m, 1H), 3.60-3.54 (m, 1H), 2.76 (dd, 1H, J = 13.0, 6.0 Hz), 2.38 (s, 3H), 2.33 (s, 3H), 2.31 (s, 3H), 2.15 (s, 3H), 2.12 (s, 3H), 2.04 (s, 3H), 1.98 (s, 3H), 1.93-1.87 (m, 1H).

¹³C-NMR (125 MHz, CDCl₃) α/β mixture: 174.5, 173.8, 173.6, 171.7, 171.0, 170.5, 170.3, 170.3, 169.9, 169.8, 169.8, 169.6, 167.6, 167.3, 166.3, 166.0, 165.6, 165.4, 133.3, 133.2, 133.2, 133.1, 129.8, 129.7, 129.5, 129.4, 129.4, 129.3, 129.0, 128.4, 128.3, 99.1, 98.8, 95.9, 91.0, 72.2, 71.6, 71.5, 69.9, 69.9, 69.3, 69.3, 68.8, 68.5, 68.1, 67.5, 67.5, 67.3, 67.0, 66.6, 62.9, 62.6, 62.4, 60.3, 57.2, 56.8, 53.0, 52.9, 38.6, 38.3, 27.9, 27.8, 26.1, 25.7, 21.0, 20.9, 20.8, 20.7, 20.7, 20.6, 20.5.
HRMS(ESI⁻)[M+HCOO]⁻ calcd for C₄₅H₅₂NO₂₄: 990.2885; found 990.2873.

### Process for purifying 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-D-galactopyranose (the compound represented by Formula G-12) by crystallization

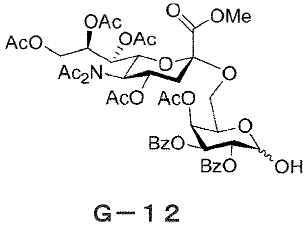

First, 4-O-Acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-D-galactopyranose (the compound represented by Formula G-12) (3.00 g, 3.17 mmol; sialyl moiety α/β ratio = 95.7/4.3) was dissolved in ethyl acetate (4 mL). Next, 2-propanol (60 mL) was added, and the mixture was stirred at 25°C and then concentrated under reduced pressure to 18 mL. The slurry solution was stirred at 0°C for 3 hours, and the precipitated crystals were then filtered. The filtered crystals were washed with cold 2-propanol (9 mL) and dried under reduced pressure at 40°C to give 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-D-galactopyranose (the compound represented by Formula G-12) (2.66 g, yield 88.7%; sialyl moiety α/β ratio => 99.9/N.D.) as white crystals.

### [Analytical conditions]

Column: CAPCELL PAK ADME ϕ4.6 × 150 mm, film thickness 3 µm
Wavelength: 220 nm
Oven: 40°C
Eluent: (A) 0.1% trifluoroacetic acid solution, (B) acetonitrile
Gradient: 0 to 150 min (B) concentration 40%
   150.1 min (B) concentration 95%
   155 min (B) concentration 95%
   155.1 min (B) concentration 40%
   160 min (B) concentration 40%
Flow rate: 1 mL/min
Injection: 5 µL

### Example 49

### 4-O-Acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose(the compound represented by Formula D-7)

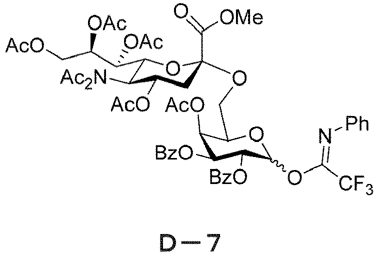

### (Substep V-11)

First, 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-D-galactopyranose (the compound represented by Formula G-12) (20.0 g, 21.1 mmol) was added to a 500-mL recovery flask. Dichloromethane (200 mL) and Molecular Sieves 4A powder (10 µm or less, 10.0 g) were added, and the mixture was cooled to 0°C. N-Methylimidazole (1.91 g, 23.3 mmol) and 2,2,2-trifluoro-N-phenylacetimidoyl chloride (4.39 g, 21.1 mmol) were added at the same temperature under nitrogen. The temperature was raised to room temperature, and the mixture was stirred for 24 hours. After the completion of the reaction was checked by HPLC, the reaction solution was filtered and washed with dichloromethane (100 mL). The filtrate was filtered through a neutral silica gel pad (silica gel 60N; particle size: 40 to 50 µm; 60 g; available by KANTO CHEMICAL CO., INC.) packed with dichloromethane, and collected every 100 mL. The silica gel pad was washed with ethyl acetate/dichloromethane (1:9; 1000 mL, 200 mL each collected), and the selected fractions were concentrated under reduced pressure to give 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (the compound represented by Formula D-7) (20.1 g, yield: 85%) as a white amorphous material.

¹H-NMR (500 MHz, CDCl₃) δ 7.99 (dd, 2H, J = 8.3, 1.4 Hz), 7.91-7.88 (m, 2H), 7.59-7.56 (m, 1H), 7.51 (tt, 1H, J = 9.7, 2.4 Hz), 7.44-7.34 (m, 4H), 7.12 (t, 2H, J = 7.7 Hz), 7.01 (t, 1H, J = 7.4 Hz), 6.82 (brs, 1H), 6.43 (brs, 2H), 5.87-5.77 (m, 3H), 5.52 (td, 1H, J = 11.0, 5.0 Hz), 5.38-5.34 (m, 1H), 5.18 (dd, 1H, J = 8.6, 1.7 Hz), 4.95 (dd, 1H, J = 10.0, 2.0 Hz), 4.53 (brs, 1H), 4.29 (dd, 1H, J = 12.6, 2.9 Hz), 4.21-4.09 (m, 3H), 4.04 (dd, 1H, J = 10.0, 6.0 Hz), 3.84 (s, 3H), 3.53 (dd, 1H, J = 10.3, 7.4 Hz), 2.76 (dd, 1H, J = 12.9, 5.4 Hz), 2.39 (s, 3H), 2.31 (s, 3H), 2.18 (s, 3H), 2.17 (s, 3H), 2.14 (s, 3H), 2.02 (s, 3H), 1.98 (s, 3H), 1.86 (dd, 1H, J = 13.0, 11.0 Hz). ¹³C-NMR (125 MHz, CDCl₃) δ 174.5, 173.5, 170.5, 170.1, 169.8, 169.7, 169.6, 167.2, 165.5, 165.4, 165.3, 142.9, 133.6, 133.3, 129.8, 129.7, 129.6, 129.5, 129.0, 128.7, 128.6, 128.5, 128.4, 124.2, 119.0, 98.7, 98.5, 70.4, 69.8, 68.3, 68.2, 67.5, 67.5, 66.9, 66.7, 62.0, 61.7, 60.3, 56.9, 53.7, 52.9, 38.7, 31.7, 29.2, 27.9, 26.0, 25.8, 21.0, 21.0, 20.9, 20.8, 20.7, 20.6, 20.5.
HRMS(ESI⁺)[M+NH_{4]}⁺ calcd for C₅₂H₅₉F₃N₃O₂₂: 1134.3537; found 1134.3564.

The spectrum was found to correspond to the following literature:
J. Org. Chem., 2016, 81, 10600-10616.

### <Synthesis of compound represented by Formula D-13>

Compound represented by Formula D-13 was synthesized according to the following synthesis scheme 4.

### [Synthesis Scheme 4]

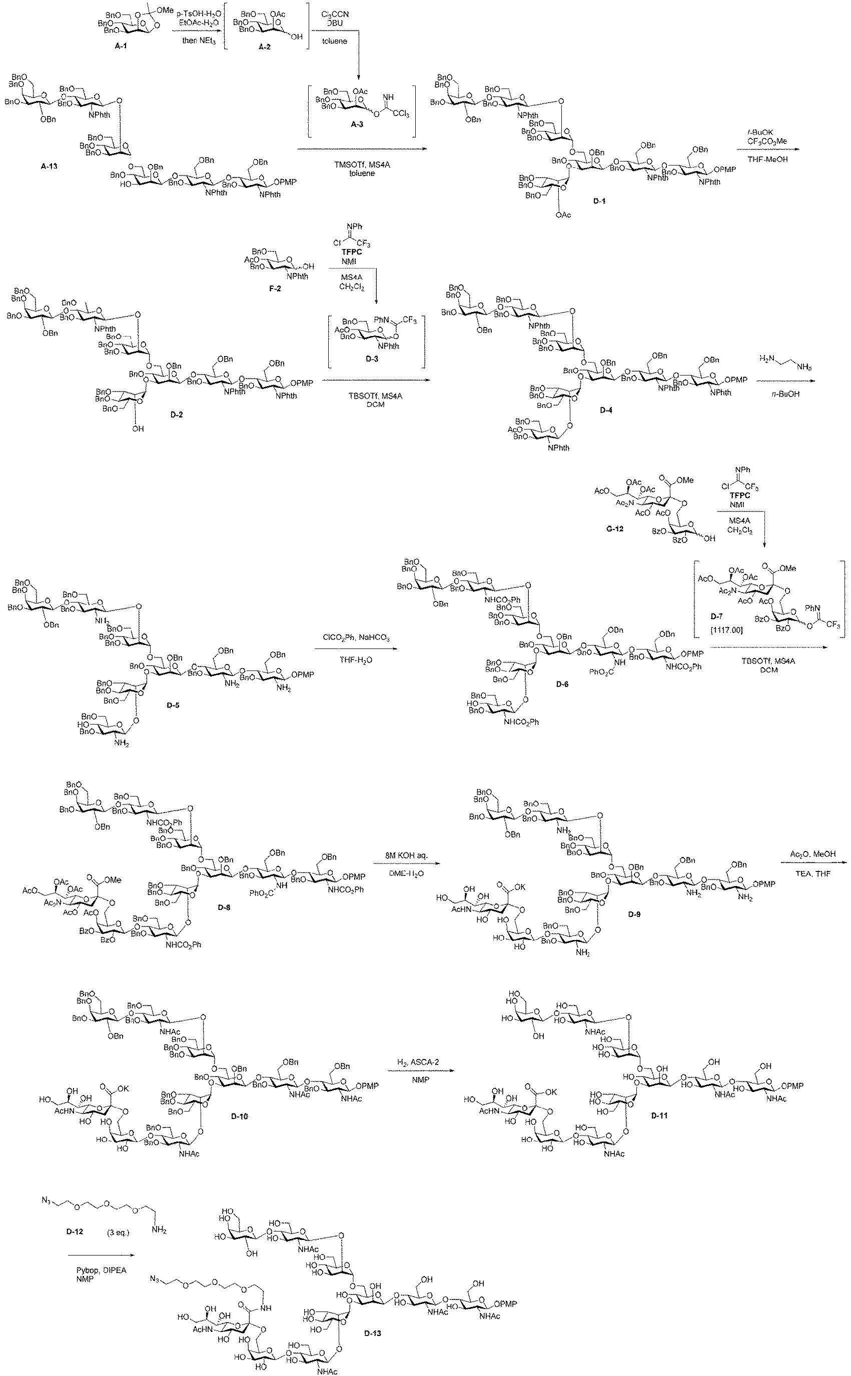

### Example 50

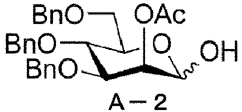

First, 3,4,6-tri-O-benzyl-1,2-O-(1-methoxyethyliden)-β-D-mannopyranose (the compound represented by Formula A-1) (4.56 g, 9.00 mmol) was added to a 200-mL recovery flask, and ethyl acetate (45.6 mL) was then added. Under a nitrogen atmosphere, water (0.23 mL) and p-TsOH·H₂O (5.1 mg, 0.027 mmol) were added at room temperature, and the mixture was stirred at the same temperature for 8 hours. After the completion of the reaction was checked by HPLC, triethylamine (1.25 mL, 9.00 mmol) was added. The mixture was stirred overnight at the same temperature. After the completion of the acetyl-group transfer was checked by HPLC, the reaction solution was mixed with 5% sodium bicarbonate in water (45 mL) for liquid-separation. The resulting organic layer was mixed with 20% brine (22.8 mL) for liquid-separation. The resulting organic layer was concentrated under reduced pressure to 9 mL, and toluene (45.6 mL) was added. The mixture was concentrated under reduced pressure to a liquid volume of 9 mL. Toluene (45.6 mL) was added again, and the mixture was concentrated again under reduced pressure to a liquid volume of 9 mL. Dehydrated toluene (13.7 mL) was added to prepare, as a colorless solution, a toluene solution containing 2-O-acetyl-3,4,6-tri-O-benzyl-D-mannopyranose (the compound represented by Formula A-2).

### Example 51

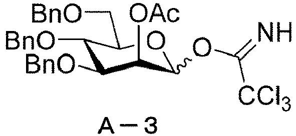

A toluene solution containing 2-O-acetyl-3,4,6-tri-O-benzyl-D-mannopyranose (the compound represented by Formula A-2) (9.00 mmol) was added to a 100-mL recovery flask. The mixture was cooled to 0°C, and trichloroacetonitrile (1.95 g, 13.5 mmol) and DBU (13.5 µL, 8.96 µmol) were then added. The mixture was stirred under nitrogen at 0°C for 4 hours. After the completion of the reaction was checked by HPLC, acetic acid (5.2 µL, 8.96 µmol) was added to the reaction solution at 0°C to prepare, as a brown solution, a toluene solution containing 2-O-acetyl-3,4,6-tri-O-benzyl-1-O-(2,2,2-trichloroethanimidoyl)-D-mannopyranose (the compound represented by Formula A-3) (9.00 mmol). This solution was used as it was in the next step.

### Example 52

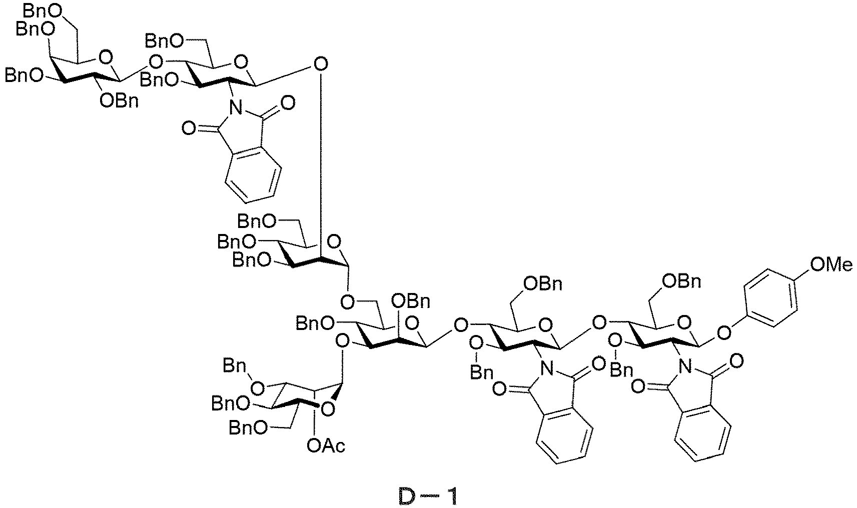

A toluene solution (1.27 mmol equivalents) containing 4-methoxyphenyl 2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula A-13) (3.0 g, 1.06 mmol) and 2-O-acetyl-3,4,6-tri-O-benzyl-1-O-(2,2,2-trichloroethanimidoyl)-D-mannopyranose (the compound represented by Formula A-3) was added to a 100-mL recovery flask, and mixed with toluene (30 mL) and Molecular Sieves 4A powder (10 µm or less, 600 mg). Trimethylsilyl trifluoromethanesulfonate (38.5 µL, 0.212 mmol) was added dropwise over 15 minutes at -15°C under nitrogen, and the mixture was stirred at the same temperature for 1 hour. After the completion of the reaction was checked by HPLC, triethylamine (0.19 mL, 1.06 mmol) was added. The mixture was stirred at room temperature for 30 minutes. The reaction solution was filtered and then washed with acetonitrile (30 mL). The resulting filtrate was concentrated under reduced pressure to a liquid volume of 6 mL. Acetonitrile (30 mL) was added again, and the mixture was concentrated again under reduced pressure to a liquid volume of 6 mL. Acetonitrile (30 mL) was added to this concentrated liquid. Silica gel 120RP-18 (particle size 40 to 50 µm; 9.0 g; available by KANTO CHEMICAL CO., INC.) for reversed phase was then added. Water (20 mL) was added dropwise over 30 minutes to adsorb the target substance onto the solid phase, and the mixture was then filtered. After the solid phase was washed with acetonitrile/water (5:4, 90 mL) (the filtrate was discarded), the target substance was desorbed with acetonitrile/tetrahydrofuran (9:1, 180 mL). The resulting filtrate was concentrated under reduced pressure to a liquid volume of 6 mL. Toluene (30 mL) was added to the solution obtained, and the mixture was concentrated under reduced pressure to a liquid volume of 6 mL. Toluene (30 mL) was added again, and the mixture was concentrated again under reduced pressure to a liquid volume of 6 mL to give a toluene solution containing crude 4-methoxyphenyl 2-O-acetyl-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula D-1). This was used as it was in the next step.

### Example 53

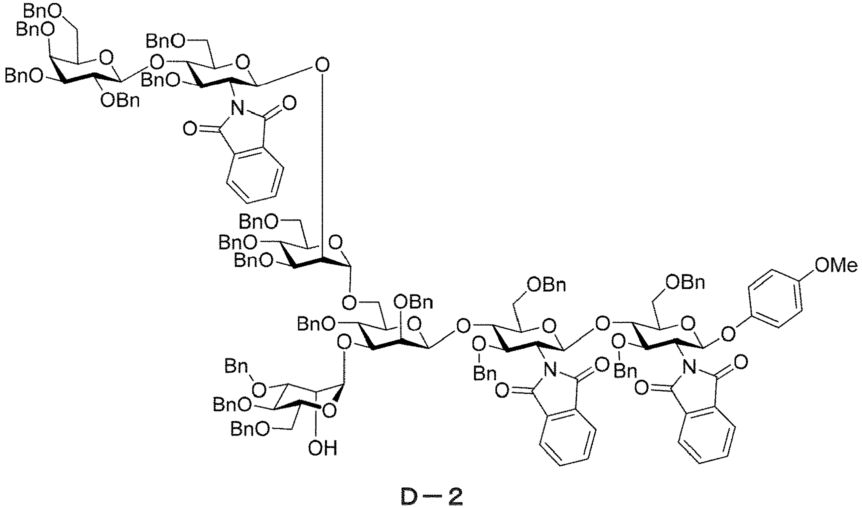

A toluene solution containing 4-methoxyphenyl 2-O-acetyl-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-(1,3-dioxo-1,3dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula D-1) (1.06 mmol equivalents) was added to a 100-mL recovery flask, and mixed with tetrahydrofuran (17.6 mL), methanol (10.5 mL), and methyl trifluoroacetate (0.11 mL, 1.06 mmol). The mixture was stirred at 25°C for 10 minutes, and potassium tert-butoxide (1 mol/L tetrahydrofuran solution) (0.53 mL, 0.53 mmol) was added. The temperature was then raised to 40°C, and after 2 hours of stirring, the completion of the reaction was checked by HPLC. The reaction solution was cooled to 25°C and acetic acid (0.06 mL, 1.03 mmol) and ethyl acetate (35 mL) were added in this order. This solution was washed twice with 3% brine (35 mL) and once with 20% brine (17.5 mL), and then concentrated under reduced pressure until the liquid volume reached 6 mL. Toluene (35 mL) was added, and the mixture was concentrated again until the liquid volume reached 6 mL. Toluene (35 mL) was further added, and the mixture was concentrated until the liquid volume reached 6 mL to give a toluene solution containing crude 4-methoxyphenyl 3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula D-2). This was used as it was in the next step.

### Example 54

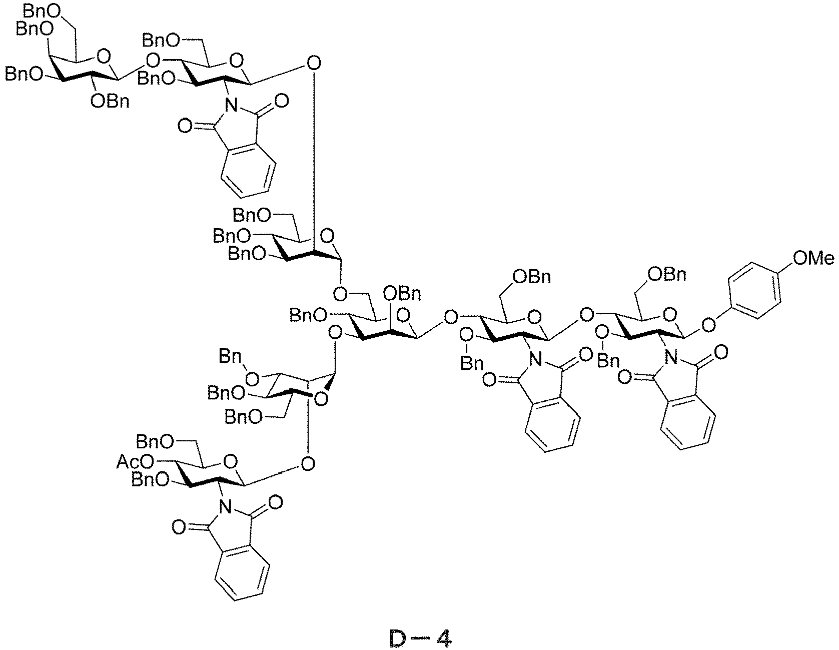

A toluene solution containing 4-methoxyphenyl 3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula D-2) (1.06 mmol equivalents) and a toluene solution containing 4-O-acetyl-3,6-di-O-benzyl-2-deoxy-2-(1,3-dioxo-1,3-dihydro-2H-isoindol-2-yl)-O-[2,2,2-trifluoro-N-phenylethanimidoyl]-β-D-glucopyranoside (the compound represented by Formula D-3) (the toluene solution obtained in Example 39 was quantified, and 1.38 mmol equivalents were added) were added to a 100-mL recovery flask, and mixed with dichloromethane (34.7 mL) and Molecular Sieves 4A powder (700 mg). Then, tert-butyldimethylsilyl trifluoromethanesulfonate (0.12 mL, 0.53 mmol) was added dropwise over 5 minutes at -78°C under nitrogen, and the mixture was stirred at the same temperature for 9 hours. After the completion of the reaction was checked by HPLC, triethylamine (0.15 mL, 1.06 mmol) was added. The mixture was stirred at room temperature for 30 minutes. The reaction solution was filtered and washed with toluene (30 mL). The resulting filtrate was concentrated under reduced pressure to a liquid volume of 6 mL to give a toluene solution containing crude 4-methoxyphenyl 4-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-a-D-mannopyranosyl-(1→3)-[2,3,4,6-tetra-O-benzyl-P-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula D-4). This was used as it was in the next step.

### Example 55-1

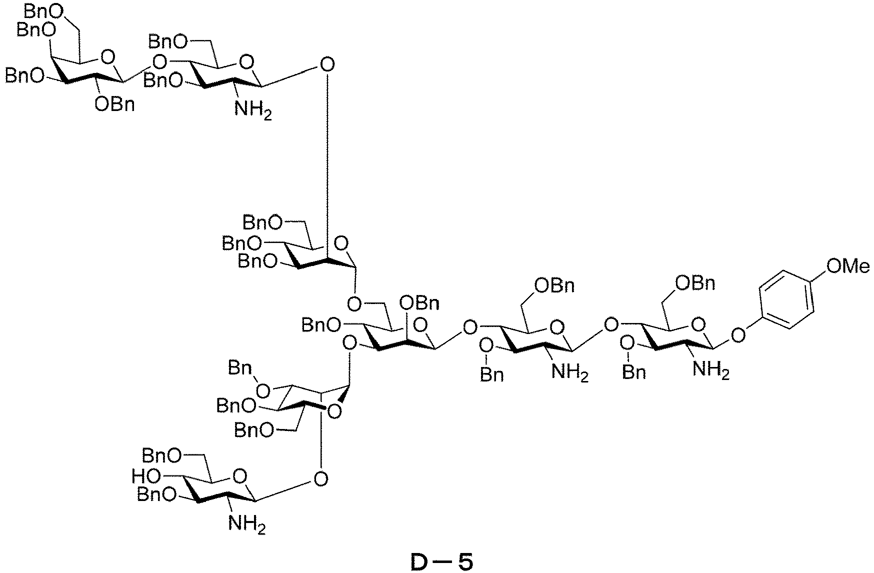

A toluene solution containing 4-methoxyphenyl 4-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula D-4) (1.06 mmol equivalents) was added to a 200-mL recovery flask, and mixed with n-butanol (12 mL) and ethylenediamine (12 mL). The mixture was stirred under nitrogen at 95°C for 12 hours. After the completion of the reaction was checked by HPLC, the reaction solution was cooled to room temperature and concentrated under reduced pressure to a liquid volume of 6 mL. This concentrated liquid was mixed with acetonitrile (40 mL) and then silica gel 120RP-18 (particle size: 40 to 50 µm; 12.0 g; available by KANTO CHEMICAL CO., INC.) for reversed phase. Water (40 mL) was added dropwise over 30 minutes to adsorb the target substance onto the solid phase, and the mixture was then filtered. After the solid phase was washed with acetonitrile/water (3:2, 120 mL) (the filtrate was discarded), the target substance was desorbed with acetonitrile/tetrahydrofuran (9:1, 180 mL). The resulting filtrate was concentrated under reduced pressure to a liquid volume of 6 mL. Tetrahydrofuran (40 mL) was further added, and the mixture was concentrated again under reduced pressure to a liquid volume of 6 mL to give a tetrahydrofuran solution containing crude 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside (the compound represented by Formula D-5). This was used as it was in the next step (Example 56).

### Example 55-2

The compound represented by Formula D-5 was purified by the following procedure comprising a step of obtaining fumarate crystals of the compound. This method for purification has achieved a significant improvement in the purity of the compound represented by the Formula D-5-FMA, and also made it possible to remove several impurities including anomeric isomers into the filtrate.

Method of purifying 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-P-D-galactopyranosyl-(1→4)-2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside pentafumarate (the compound represented by Formula D-5-FMA) by crystallization.

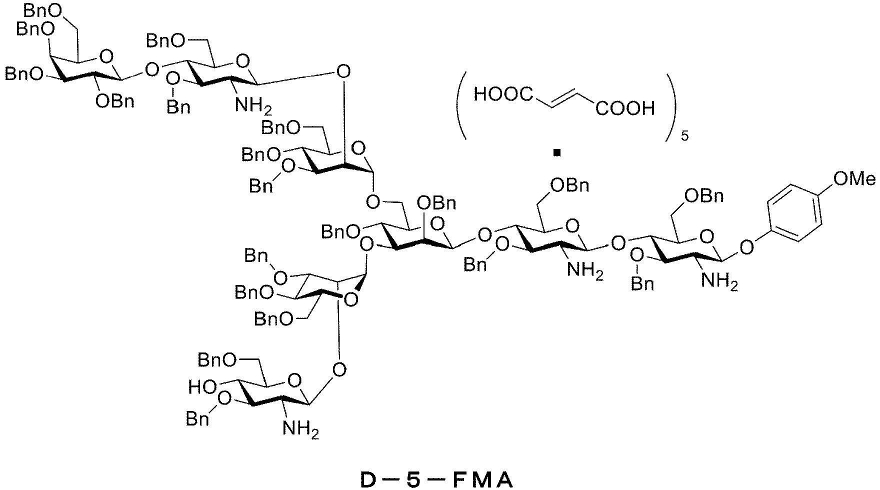

A fumaric acid (143 mg)-containing tetrahydrofuran solution (3.5 mL) was poured into a solution containing 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside (the compound represented by Formula D-5) in an acetonitrile-ethyl acetate solvent (2.5 mL, a solution derived from the hexasaccharide intermediate through 4 steps in a non-isolated manner and containing 1.14 g of the compound represented by Formula D-5 as a theoretical amount). Isopropyl acetate (10 mL) was added dropwise over 30 minutes or longer with stirring at 25°C. After inoculation with seed crystals (1 mg) and stirring for 1 hour or longer, a fumaric acid (82 mg)-containing tetrahydrofuran solution (2 mL) was added dropwise over 1 hour or longer. The mixture was then stirred at 25°C for 30 minutes. Heptane (10 mL) was added dropwise over 1 hour or longer, and the mixture was stirred for 30 minutes or longer. The slurry solution was filtered, and the resulting crystals were dried under reduced pressure at 25°C to give, as white crystals, 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside pentafumarate (the compound represented by Formula D-5-FMA) (1.15 g, yield: 86% (total yield from the hexasaccharide intermediate through 4 steps), HPLC purity: 97.0% (PA%)).

### [Analytical conditions]

Column: Xselect Fluoro-Phenyl 3.5 µm, 4.6ϕ × 150 mm (Waters)
Wavelength: 220 nm
Oven: 40°C
Eluent: (A) 10 mM NH₄HCO₃ aqueous solution, (B) acetonitrile
Gradient: 0 min (B) concentration 75%
   25 min (B) concentration 80%
   30 min (B) concentration 100%
   30.01 min (B) concentration 75%
   35 min (B) concentration 75%

### Flow rate:

1 mL/min

### Injection:

5 µL

HRMS (ESI⁺) [M+H]⁺ calcld for C₁₉₅H₂₁₃N₄O₃₈⁺: 3218.4852; found 3218.4861 1H-NMR (500 MHz, CD₃OD) δ 7.54 (d, J = 7.0 Hz, 2H), 7.48-7.15 (m, 100H), 6.94 (d, J = 9.5 Hz, 2H), 6.84 (s, 10H), 5.43 (s, 1H), 5.34 (d, J = 9, 5 Hz, 1H), 5.25-5.01 (m, 6H), 4.93-4.29 (m, 40H), 4.21-3.43 (m, 41H), 3.42-3.40 (m, 24H), 3.23-2.93 (m, 6H),
13C-NMR (125 MHz, CD₃OD) δ 155.3, 151.4, 139.3, 139.2, 139.06, 139.02, 138.85, 138.83, 138.78, 138.69, 138.5, 138.34, 133.31, 138.19, 138.16, 138.14, 138.07, 137.5, 128.5, 128.45, 128.43, 128.37, 128.34, 128.31, 128.28, 128.26, 128.20, 128.15, 128.10, 128.04, 127.95, 127.86, 127.80, 127.75, 127.72, 127.65, 127.62, 127.59, 127.52, 127.50, 127.4, 127.36, 127.33, 127.31, 127.2, 126.0, 118.6, 114.4, 103.40, 103.36, 102.7, 102.63, 102.59, 101.2, 99.8, 97.9, 84.3, 83.2, 83.1, 82.8, 82.4, 81.6, 79.9, 78.4, 78.2, 77.9, 77.5, 76.1, 76.0, 75.6, 75.5, 75.2, 75.2, 75.1, 74.91, 74.89, 74.8, 74.7, 74.58, 74.55, 74.5, 74.4, 74.3, 74.2, 74.1, 73.9, 73.84, 73.77, 73.4, 73.26, 73.23, 73.1, 73.06, 72.98, 72.93, 72.8, 72.6, 72.5, 71.6, 71.5, 71.3, 70.5, 69.4, 68.79, 68.75, 68.3, 68.2, 67.1, 57.1, 56.4, 55.6, 55.4.

### Example 56

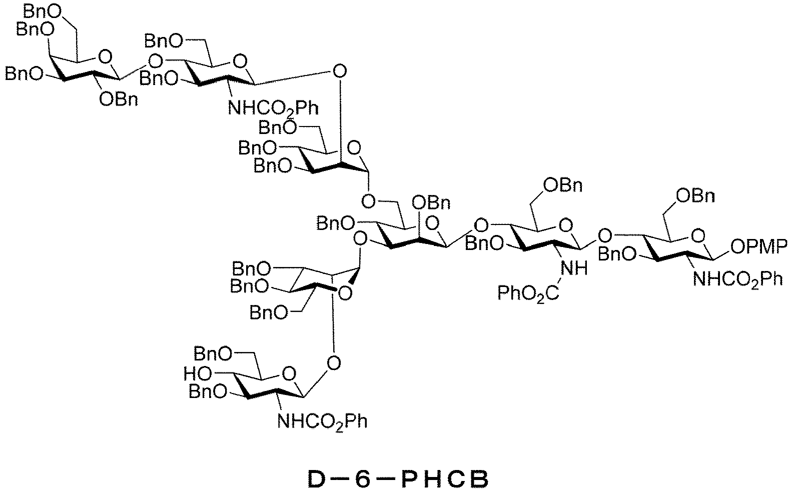

A tetrahydrofuran solution containing 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside (the compound represented by Formula D-5) (1.06 mmol equivalents) was added to a 100-mL recovery flask, and mixed with an aqueous solution having tetrahydrofuran (17.1 mL) and sodium bicarbonate (0.58 g, 6.89 mmol) dissolved in water (10.2 mL). Phenyl chloroformate (0.83 g, 5.30 mmol) was added, and the mixture was then stirred for 1 hour. After the completion of the reaction was checked by HPLC, ethyl acetate (34 mL) and water (34 mL) were added. The resulting liquid was then separated. The resulting organic layer was concentrated under reduced pressure to a liquid volume of 6 mL, and mixed with acetonitrile (34 mL) and then silica gel 120RP-18 (particle size: 40 to 50 µm; 10.2 g; available by KANTO CHEMICAL CO., INC.) for reversed phase. Water (34 mL) was added dropwise over 30 minutes to adsorb the target substance onto the solid phase, and the mixture was then filtered. After the solid phase was washed with acetonitrile/water (3:2, 90 mL) (the filtrate was discarded), the target substance was desorbed with acetonitrile/tetrahydrofuran (9:1, 180 mL). The resulting filtrate was concentrated under reduced pressure to a liquid volume of 6 mL. Toluene (34 mL) was further added, and the mixture was concentrated again under reduced pressure to a liquid volume of 6 mL. This operation was performed twice to give a toluene solution containing crude 4-methoxyphenyl 3,6-di-O-benzyl-2-deoxy-2-[(phenoxycarbonyl)amino]-β-D-glucopyranosyl-(1→2)-3,4,6-triO-benzyl-α-D-mannopyranosyl-(1→3)-[2,3,4,6-tetra-O-benzyl-β-D-galactopyranosyl-(1→4)-3,6-di-O-benzyl-2-deoxy-2-[(phenoxycarbonyl)amino]-β-D-glucopyranosyl-(1→2)-3,4,6-triO-benzyl-α-D-mannopyranosyl-(1→6)]-2,4-di-O-benzyl-β-D-mannopyranosyl-(1→4)-3,6-di-O-benzyl-2-deoxy-2-[(phenoxycarbonyl)amino]-β-D-glucopyranosyl-(1→4)-3,6-di-O-benzyl-2-deoxy-2-[(phenoxycarbonyl)amino]-β-D-glucopyranoside (the compound represented by Formula D-6-PHCB). This was used as it was in the next step.

### Example 57

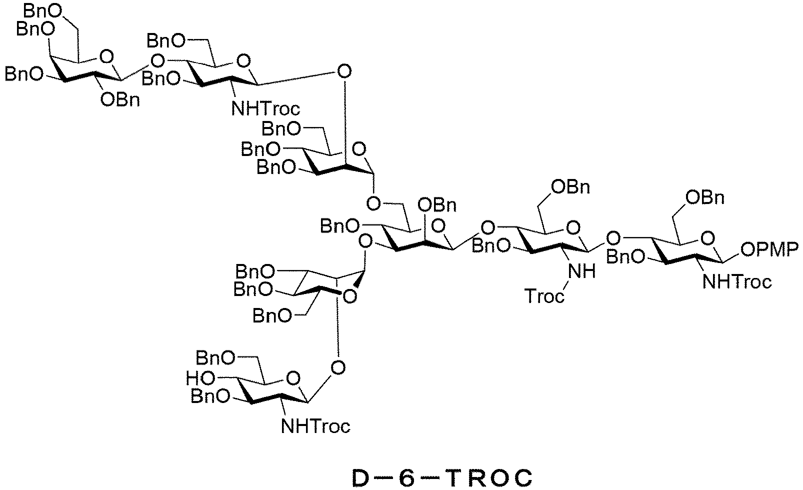

Under a nitrogen atmosphere, 4-methoxyphenyl 4-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula D-4) (2.44 g) was mixed with n-butanol (12 mL) and ethylenediamine (12 mL). The mixture was stirred under nitrogen at 90°C for 12 hours. After the completion of the reaction was checked by HPLC, the reaction solution was cooled to room temperature and concentrated under reduced pressure. The residue was mixed with acetonitrile (50 mL) and then silica gel 120RP-18 (particle size: 40 to 50 µm; 7.5 g; available by KANTO CHEMICAL CO., INC.) for reversed phase. Water (50 mL) was added dropwise. The mixture then was filtered, and the filtered material was washed with acetonitrile/water (1:1, 200 mL). Then, to elute the target substance from the filtered material, the material was washed with acetonitrile (25 mL) and toluene (200 mL), respectively. The filtrate containing the target substance was concentrated under reduced pressure. The resulting residue was mixed with an aqueous solution having tetrahydrofuran (25 mL) and sodium bicarbonate (0.36 g) dissolved in water (12.5 mL). Here, 2,2,2-triethyl chloroformate (0.43 mL) was added, and the mixture was then stirred for 1 hour. After the completion of the reaction was checked by HPLC, ethyl acetate (25 mL) and water (25 mL) were added. The resulting liquid was then separated. The resulting organic layer was concentrated under reduced pressure, and the residue was mixed with acetonitrile (25 mL) and then silica gel 120RP-18 (particle size: 40 to 50 µm; 7.5 g; available by KANTO CHEMICAL CO., INC.) for reversed phase. Water (10 mL) was added dropwise to adsorb the target substance onto the solid phase, and the mixture was then filtered. The filtered material was washed with acetonitrile/water (5:1, 60 mL). The filtered material was then washed with toluene (200 mL) to elute the target substance from the filtered material. The filtrate containing the target substance was concentrated under reduced pressure and then dried to give 2.39 g of crude 4-methoxyphenyl 3,6-di-O-benzyl-2-deoxy-2-[(2,2,2-trichloroethoxycarbonyl)amino]-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-[(2,2,2-trichloroethoxy)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,.6-di-O-benzyl-2-deoxy-2-[(2,2,2-trichloroethoxy)amino]-β-D-glucopyranosyl-(1→4)-3,6-di-O-benzyl-2-deoxy-2-[(2,2,2-trichloroethoxy)amino]-β-D-glucopyranoside (the compound represented by Formula D-6-TROC). This was used as it was in the next step.

### Example 58

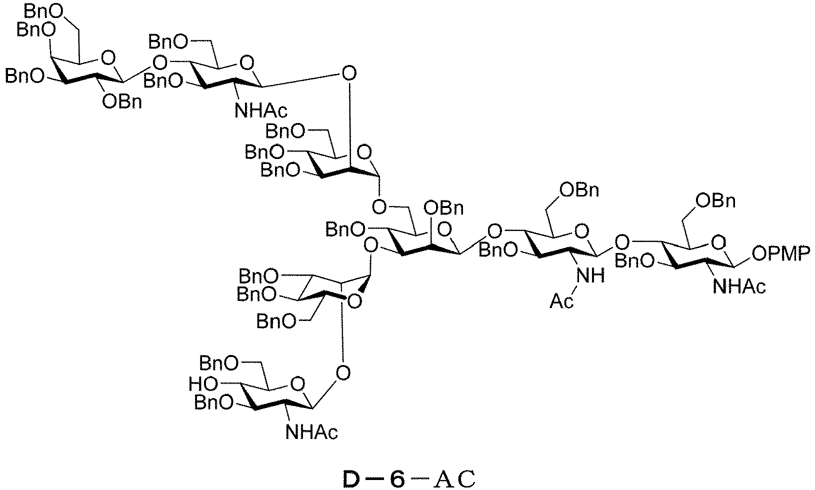

A n-butanol (1.65 mL)/ethylenediamine (3.3 mL) solution containing 4-methoxyphenyl 4-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula D-4) (330 mg, 0.088 mmol) was stirred at 90°C for 44 hours. Next, 2-methyltetrahydrofuran (5.0 mL) was added. The mixture was then cooled to room temperature and water (3 mL) was added. The aqueous layer was removed. The resulting organic layer was then washed three times with 10% methanol water (3 mL), and concentrated to dryness to prepare, as a crude form, 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside (the compound represented by Formula D-5).

To the resulting crude 4-methoxyphenyl 2-amino-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside (the compound represented by Formula D-5) were sequentially added2-methyltetrahydrofuran (6.6 mL), methanol (3.3 mL), acetic anhydride (124.1 µL, 1.31 mmol), and triethylamine (145 µL , 1.56 mmol) . After stirring for 6 hours, the reaction solution was concentrated to dryness. The resulting crude material was purified by preparative HPLC (acetonitrile/water 93% -> 100%) to give 4-methoxyphenyl 2-acetamido-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-acetamido-3,6-di-O-benzyl-2-deoxy-β-D-glucopyranosyl-(1→4)-2-acetamido-3,6-di-O-benzyl-2-deoxy-β-D-glucopyranoside (the compound represented by Formula D-6-AC) (295.60 mg, yield: 80%).

### Example 59

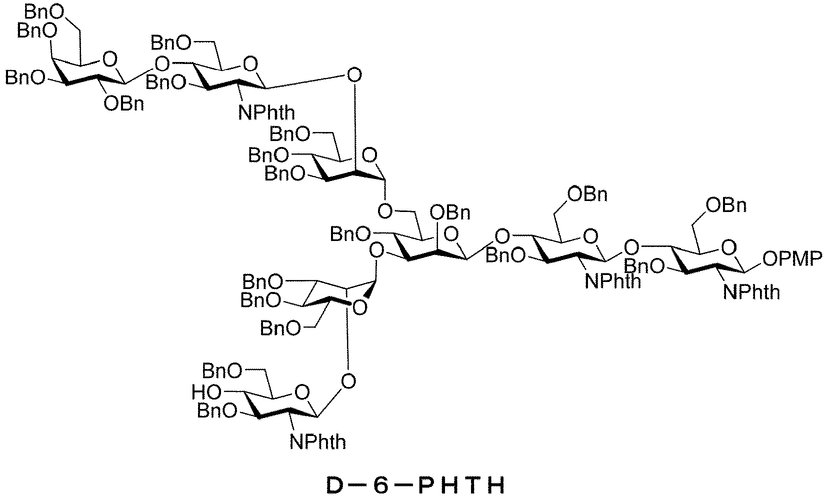

Under a nitrogen atmosphere, 4-methoxyphenyl 4-acetyl-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-(1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-(1,3-dioxo-1,3-dihydro-2H-isoindol-2-yl)-β-D-glucopyranosyl-(1→4)-3,6-di-O-benzyl-2-deoxy-2-(1,3-dioxo-1,3-dihydro-2H-isoindol-2-yl)-β-D-glucopyranoside (the compound represented by Formula D-4) (0.24 g) was mixed with tetrahydrofuran (2 mL), methanol (1 mL), methyl trifluoroacetate (0.04 mL), and 1 M t-butoxy potassium tetrahydrofuran solution (0.25 mL). After the completion of the reaction was checked by HPLC, acetic acid (0.02 mL) was added. The reaction solution was concentrated under reduced pressure. Ethyl acetate (2 mL) and water (1 mL) were added to the residue, and the mixture was liquid-separated. The organic layer was concentrated. The residue was then purified by silica gel column chromatography to give 0.14 g of 4-methoxyphenyl 3,6-di-O-benzyl-2-deoxy-2-1,3-dioxo-1,3-dihydro-2H-isoindol-2-yl)-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-[1,3-dioxo-1,3-dihydro-2H-isoindol-2-yl)-β-D-glucopyranosyl-(1→4)-3,6-di-O-benzyl-2-deoxy-2-1,3-dioxo-1,3-dihydro-2H-isoindol-2-yl)-β-D-glucopyranoside (the compound represented by Formula D-6-PHTH).

### Example 60

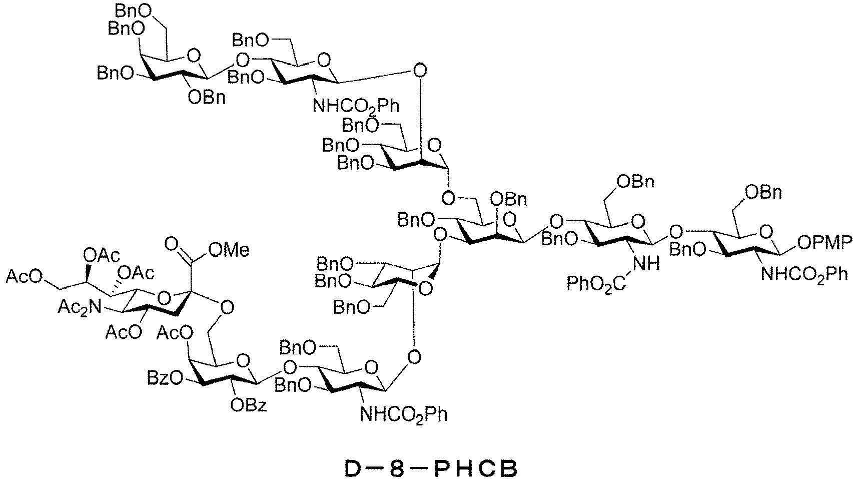

A toluene solution containing 4-methoxyphenyl 3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-[(phenoxycarbonyl)amino]-β-D-glucopyranosyl-(1→4)-3,6-di-O-benzyl-2-deoxy-2-[(phenoxycarbonyl)amino]-β-D-glucopyranoside (the compound represented by Formula D-6-PHCB) (1.06 mmol equivalents) obtained in the above Example 56 and 4-O-acetyl-2,3-di-O-benzoyl-6-O-[4,7, 8,9-tetra-O-acetyl-3, 5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (the compound represented by Formula D-7) (1.78 g, 1.59 mmol) were added to a 100-mL recovery flask, and mixed with dichloromethane (39.2 mL) and Molecular Sieves 4A powder (10 µm or less, 0.78 g). Trimethylsilyl trifluoromethanesulfonate (24.4 µL, 0.11 mmol) was added dropwise over 5 minutes at -15°C under nitrogen, and the mixture was stirred at the same temperature for 1 hour. After the completion of the reaction was checked by HPLC, triethylamine (0.15 mL, 1.06 mmol) was added. The mixture was stirred at room temperature for 30 minutes. The reaction solution was filtered and then washed with acetonitrile (40 mL). The resulting filtrate was concentrated under reduced pressure to a liquid volume of 6 mL. This solution was mixed with acetonitrile (40 mL) and then silica gel 120RP-18 (particle size: 40 to 50 µm; 12.0 g; available by KANTO CHEMICAL CO., INC.) for reversed phase. Water (40 mL) was added dropwise over 30 minutes to adsorb the target substance onto the solid phase, and the mixture was then filtered. After the solid phase was washed with acetonitrile/water (3:2, 90 mL) (the filtrate was discarded), the target substance was desorbed with acetonitrile/tetrahydrofuran (9:1, 180 mL). The resulting filtrate was concentrated to dryness to give, as a white amorphous material, crude 4-methoxyphenyl 4,7,8,9-tetra-O-acetyl-3,5-dideoxy-5-(diacetylamino)-1-O-methyl-D-glycero-α-D-galactonon-2-uropyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-[(phenoxycarbonyl)amino]-β-D-glucopyranosyl-(1→2)-3,4,6-triO-benzyl-α-D-mannopyranosyl-(1→6)]-2,4-di-O-benzyl-β-D-mannopyranosyl-(1→4)-3,6-di-O-benzyl-2-deoxy-2-[(phenoxycarbonyl)amino]-β-D-glucopyranosyl-(1→4)-3,6-di-O-benzyl-2-deoxy-2-[(phenoxycarbonyl)amino]-β-D-glucopyranoside (the compound represented by Formula D-8-PHCB) (4.53 g, 92.3% (total yield from the compound represented by Formula A-13)). This was used as it was in the next step.

### Example 61

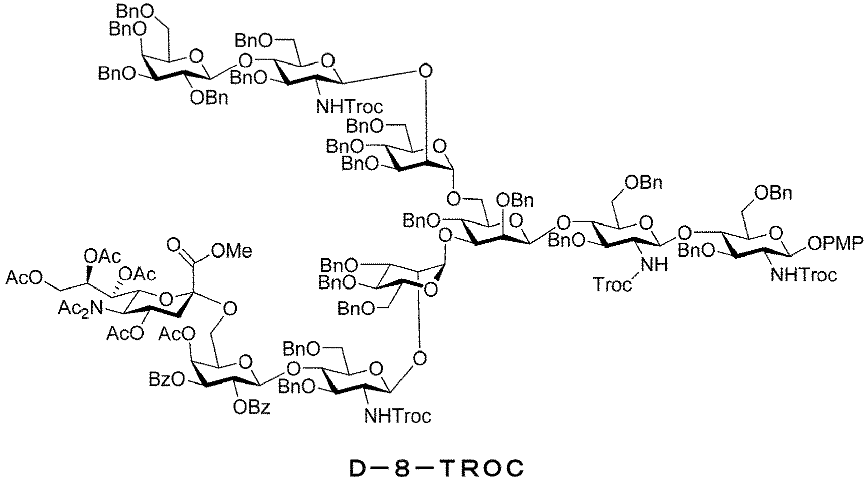

Under a nitrogen atmosphere, a dichloromethane solution (25 mL) containing 3,6-di-O-benzyl-2-deoxy-2-[(2,2,2-trichloroethoxycarbonyl)amino]-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-[(2,2,2-trichloroethoxy)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,.6-di-O-benzyl-2-deoxy-2-[(2,2,2-trichloroethoxy)amino]-β-D-glucopyranosyl-(1→4)-3,6-di-O-benzyl-2-deoxy-2-[(2,2,2-trichloroethoxy)amino]-β-D-glucopyranoside (the compound represented by Formula D-6-TROC) (2.37 g) obtained in Example 57 was mixed with 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (the compound represented by Formula D-7) (1.01 g) and Molecular Sieves 4A powder (10 µm or less, 0.50 g). Triisopropyl silyl trifluoromethanesulfonate (50 µL) was added dropwise at -15°C, and the mixture was stirred at the same temperature for 1 hour. After the completion of the reaction was checked by HPLC, triethylamine (84 µL) was added. The temperature was raised to room temperature. The reaction solution was filtered and then washed with acetonitrile (50 mL). The resulting filtrate was concentrated under reduced pressure. The residue was purified by reversed-phase silica gel chromatography (Biotage SfarBio C4 D 50 g, 95% MeCNaq.) to give, as a white amorphous material, 1.38 g of 4-methoxyphenyl 4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-[(2,2,2-trichloroethoxycarbonyl)amino]-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-[(2,2,2-trichloroethoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-[(2,2,2-trichloroethoxycarbonyl)amino]-β-D-glucopyranosyl-(1→4)-3,6-di-O-benzyl-2-deoxy-2-[(2,2,2-trichloroethoxycarbonyl)amino]-β-D-glucopyranoside (the compound represented by Formula D-8-TROC).

### Example 62

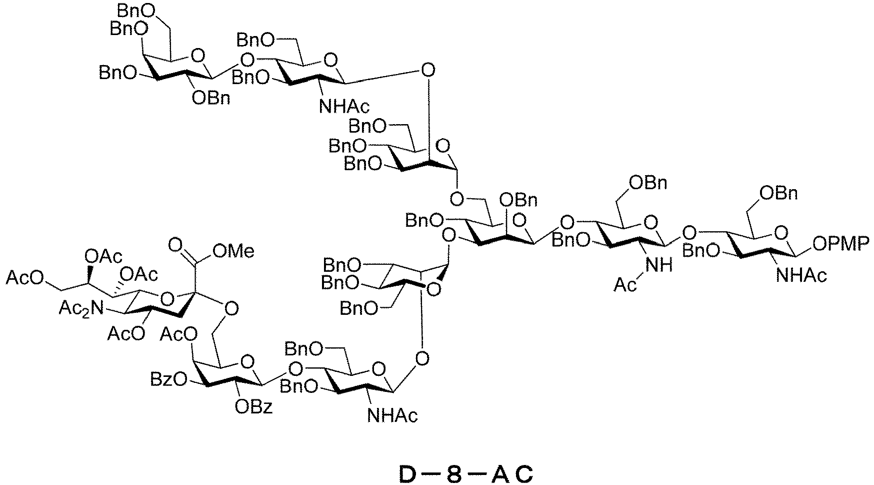

A dichloromethane solution (0.9 mL) containing 4-methoxyphenyl 2-acetamido-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-acetamido-3,6-di-O-benzyl-2-deoxy-β-D-glucopyranosyl-(1→4)-2-acetamido-3,6-di-O-benzyl-2-deoxy-β-D-glucopyranoside (the compound represented by Formula D-6-AC) (30 mg, 8.85 µmol) obtained in Example 58 above and 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (the compound represented by Formula D-7) (29.7 mg, 0.03 mmol) was mixed with Molecular Sieves 4A powder (6 mg, 0.2 wt), and the mixture was cooled to 0°C. After stirring for 10 min, triisopropylsilyl trifluoromethanesulfonate (0.95 µL, 3.54 µmol) was added. After stirring for 1 hour, tert-butyldimethylsilyl trifluoromethanesulfonate (0.81 µL, 3.54 µmol) was added. Further 1 hour later, tert-butyldimethylsilyl trifluoromethanesulfonate (0.81 µL, 3.54 µmol) was added. Two hours later, 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (the compound represented by Formula D-7) (10.0 mg ,0.01 mmol) was added. After stirring for additional 15 hours, triethylamine (10 µL) was added. The Molecular Sieves was filtered off from the resulting reaction suspension at room temperature and washed with dichloromethane (3 mL). The obtained solution was concentrated to dryness, and acetonitrile (1 mL), methanol (0.1 mL), and trifluoroacetic acid (5 µL) were added sequentially. After stirring for 1 hour, triethylamine (10 µL) was added (reaction solution 1).

A dichloromethane solution (6.3 mL) containing 4-methoxyphenyl 2-acetamido-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-acetamido-3,6-di-O-benzyl-2-deoxy-β-D-glucopyranosyl-(1→4)-2-acetamido-3,6-di-O-benzyl-2-deoxy-β-D-glucopyranoside (the compound represented by Formula D-6-AC) (206.5 mg, 0.06 mmol) and 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (the compound represented by Formula D-7) (238.3 mg, 0.21 mmol) was mixed with Molecular Sieves 4A powder (20 mg, 0.2 wt), and the mixture was cooled to 0°C. After stirring for 10 minutes, tert-butyldimethylsilyl trifluoromethanesulfonate (7.0 µL, 0.03 mmol) was added. After stirring for 24 hours , 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (the compound represented by Formula D-7) (68 mg, 0.06 mmol) and tert-butyldimethylsilyl trifluoromethanesulfonate (7.0 µL, 0.03 mmol) were added in this order. Further 7 hours later, 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (the compound represented by Formula D-7) (68 mg, 0.06 mmol) and tert-butyldimethylsilyl trifluoromethanesulfonate (7.0 µL, 0.03 mmol) were added. After 14 hours, triethylamine (20 µL) was added. The Molecular Sieves was filtered off from the resulting reaction suspension at room temperature and then washed with dichloromethane (3 mL). The obtained solution was concentrated to dryness, and acetonitrile (4 mL), methanol (0.1 mL), and trifluoroacetic acid (10 µL) were added sequentially. After stirring at 0°C for 1 hour, triethylamine (25 µL) was added (reaction solution 2).

After both solutions were mixed, the concentrated and dried crude material was purified by reversed-phase chromatography (acetonitrile/water 90% -> 100%). The mixture obtained by concentrating and drying the target substance-containing fractions was purified by silica gel column chromatography (ethyl acetate/hexane 50% -> 85%) to give 4-methoxyphenyl 4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-benzoyl-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-acetamido-3,6-di-O-benzyl-2-deoxy-β-D-glucopyranosyl-(1→4)-2-acetamido-3,6-di-O-benzyl-2-deoxy-β-D-glucopyranoside (the compound represented by Formula D-8-AC) (210.5 mg, yield: 70%).

### Example 63

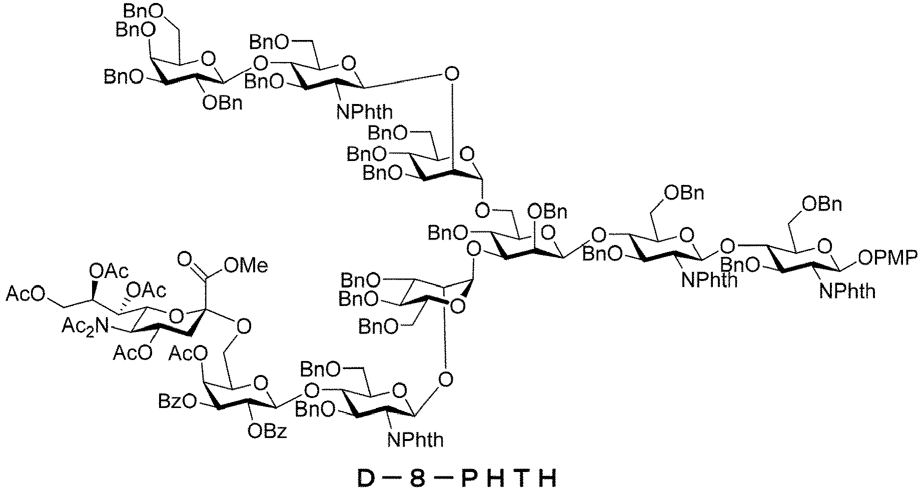

Under a nitrogen atmosphere, a dichloromethane solution (0.15 mL) containing 4-methoxyphenyl 3,6-di-O-benzyl-2-deoxy-2-1,3-dioxo-1,3-dihydro-2H-isoindol-2-yl)- β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-1,3-dioxo-1,3-dihydro-2H-isoindol-2-yl)-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-[1,3-dioxo-1,3-dihydro-2H-isoindol-2-yl)-β-D-glucopyranosyl-(1→4)-3,6-di-O-benzyl-2-deoxy-2-1,3-dioxo-1,3-dihydro-2H-isoindol-2-yl)-β-D-glucopyranoside (the compound represented by Formula D-6-PHTH) (14.8 mg) obtained in Example 59 above was mixed with 4-O-acetyl-2,3-di-O-benzoyl-6-O-[4,7,8,9-tetra-O-acetyl-3,5-dideoxy-5-(diacetylamino)-1-methyl-D-glycero-α-D-galacto-non-2-uropyranosyl]-1-O-(2,2,2-trifluoro-N-phenylethanimidoyl)-D-galactopyranose (the compound represented by Formula D-7) (9.1 mg) and Molecular Sieves 4A powder (10 µm or less, 3.6 mg). Trimethylsilyl trifluoromethanesulfonate (0.25 µL) was added dropwise at -15°C, and the mixture was stirred at the same temperature for 1 hour. After the completion of the reaction was checked by HPLC, triethylamine was added. The temperature was raised to room temperature. The reaction solution was purified by preparative TLC (toluene-ethyl acetate) to give, as a white amorphous material, 7.9 mg of 4-methoxyphenyl 4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-[(1,3-dioxo-1,3-dihydro-2H-isoindol-2-yl)]-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-[(1,3-dioxo-1,3-dihydro-2H-isoindol-2-yl)]-β-D-glucopyranosyl-(1→2)-3,4, -tri-O-benzyl-α-D-mannopyranosyl-(1→6)]-2,4-di-O-benzyl-β-D-mannopyranosyl-(1→4)-3,6-di-O-benzyl-2-deoxy-2-[(1,3-dioxo-1,3-dihydro-2H-isoindol-2-yl)]-β-D-glucopyranosyl-(1→4)-3,6-di-O-benzyl-2-deoxy-2-[(1,3-dioxo-1,3-dihydro-2H-isoindol-2-yl)]-β-D-glucopyranoside (the compound represented by Formula D-8-PHTH).

### Example 64

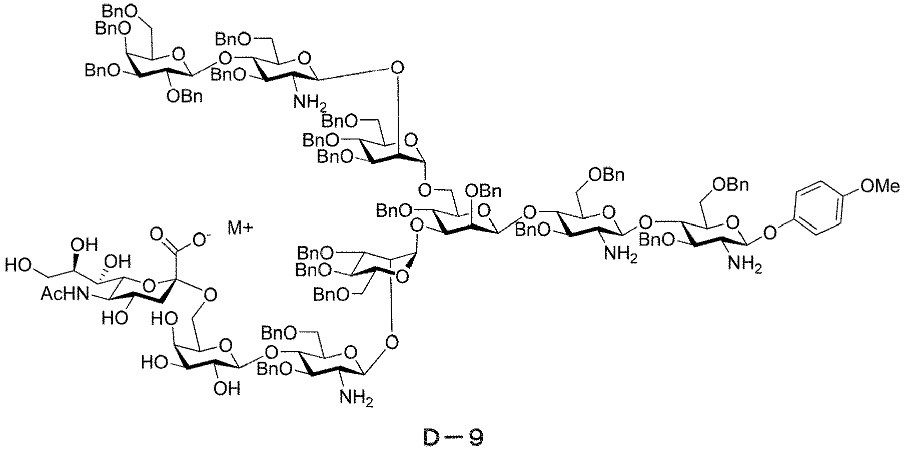

First, 4-methoxyphenyl 4,7,8,9-tetra-O-acetyl-3,5-dideoxy-5-(diacetylamino)-1-O-methyl-D-glycero-α-D-galacto-non-2-uropyranosyl-(2→6)-4-O-acetyl-2,3-di-O-benzoyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-3,6-di-O-benzyl-2-deoxy-2-[(phenoxycarbonyl)amino]-β-D-glucopyranosyl-(1→2)-3,4,6-tri-O-benzyl-α-D-mannopyranosyl-(1→6)]-2,4-di-O-benzyl-β-D-mannopyranosyl-(1→4)-3,6-di-O-benzyl-2-deoxy-2-[(phenoxycarbonyl)amino]-β-D-glucopyranosyl-(1→4)-3,6-di-O-benzyl-2-deoxy-2-[(phenoxycarbonyl)amino]-β-D-glucopyranoside (the compound represented by Formula D-8-PHCB) (3.14 g, 0.678 mmol) obtained in the Example 60 above was added to a 100-mL recovery flask, and mixed sequentially with 1,2-dimethoxyethane (28.3 mL), water (9.4 mL), and lithium hydroxide aqueous solution (4 M, 3.39 mL, 13.6 mmol). The mixture was then stirred for 1 hour. After the completion of the reaction was checked by HPLC, acetic acid (0.19 mL, 4.89 mmol) was added. Next, water (31 mL) was added, and a gelatinous solid then precipitated. The resulting solid was filtered, and the solid phase was washed with 1,2-dimethoxyethane/water (9:10, 31 mL) to give, as a wet solid, crude 4-methoxyphenyl 5-acetamido-3,5-dideoxy-D-glycero-α-D-galacto-non-2-uropyranosyl-(2→6)-β-D-galactopyranosyl-(1→4)-2-amino-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside (the compound represented by Formula D-9). This was used as it was in the next step.

From the compound represented by Formula D-8 obtained in Examples 61 to 63 above, the amino group-protecting groups were also removed under the deprotection reaction conditions shown in the table below, and the HPLC purity of the compound represented by Formula D-9 obtained is also shown in the same table. As shown in the table, the phenyloxycarbonyl (COOPh) group was used as an amino group-protecting group. Particularly, in this case, the deprotection is possible within 1 hour at room temperature under general hydrolysis conditions. The series of glycosylation, deprotection, and subsequent amino group acetylation steps can be performed in the best yield without degrading the substrate.

**[Table 6]**

| N-Protecting group | Sugar donor (NEGA-4) required amount | Deprotection reaction conditions | Product HPLC purity (%) |
|---|---|---|---|
| Ac | >5 equivalents | - | 84% |
| Troc | <1.5 equivalents | LiOH solution, THF-dioxane, room temperature, 10 hours | 79% |
| Phth | <1. 5 equivalents | 1) LiCI, pyridine, 100°C, overnight | 72% |
| | | 2) Ethylenediamine, BuOH, 90°C, overnight | |
| CO₂Ph | <1.5 equivalents | LiOH solution, DME-H₂O, room temperature, 1 hour | 93% |

### Example 65

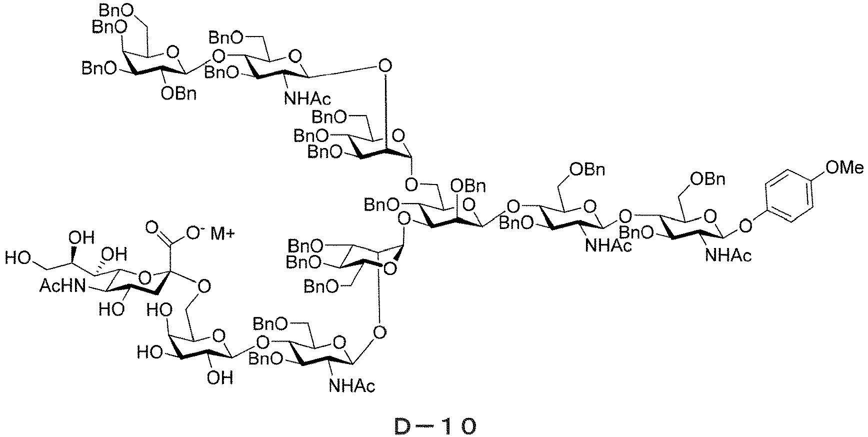

A wet solid of 4-methoxyphenyl 5-acetamido-3,5-dideoxy-D-glycero-α-D-galactonon-2-uropyranosyl-(2→6)-β-D-galactopyranosyl-(1→4)-2-amino-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-amino-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-amino-3,6-di-O-benzyl-2-deoxy-β-D-glucopyranosyl-(1→4)-2-amino-3,6-di-O-benzyl-2-deoxy-β-D-glucopyranoside (the compound represented by Formula D-9) (0.678 mmol equivalents) was added to a 100-mL recovery flask, and mixed sequentially with tetrahydrofuran (18.8 mL), methanol (9.4 mL), and triethylamine (3.31 mL, 23.7 mmol). Acetic anhydride (1.59 mL, 17.0 mmol) was then added, and the mixture was stirred at room temperature for 2 hours. After the completion of the reaction was checked by HPLC, water (31.4 mL) and ethyl acetate (62.8 mL) were added to reaction solution for liquid separation. The resulting organic layer was concentrated to dryness to give crude 4-methoxyphenyl 5-acetamido-3,5-dideoxy-D-glycero-α-D-galacto-non-2-uropyranosyl-(2→6)-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-acetamido-3,6-di-O-benzyl-2-deoxy-β-D-glucopyranosyl-(1→4)-2-acetamido-3,6-di-O-benzyl-2-deoxy-β-D-glucopyranoside (the compound represented by Formula D-10).

### Example 66

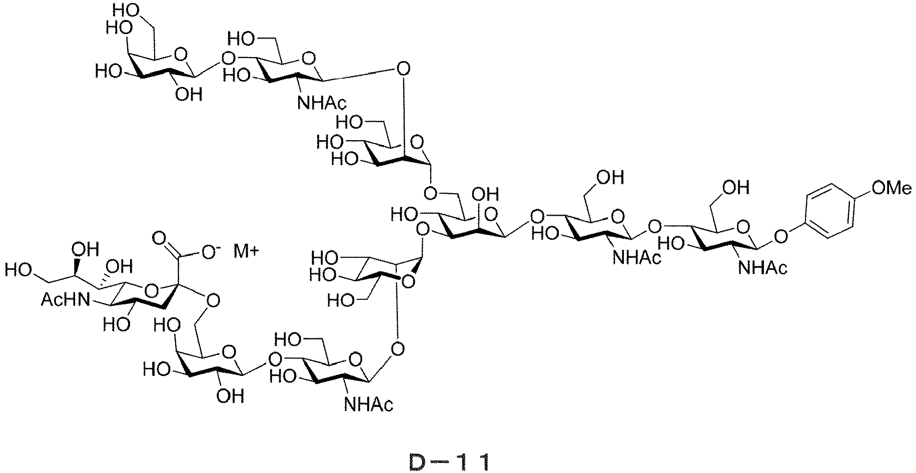

First, 4-methoxyphenyl 5-acetamido-3,5-dideoxy-D-glycero-α-D-galacto-non-2-uropyranosyl-(2→6)-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→3)-[2,3,4,6-tetra-O-benzyl-β-D-galactopyranosyl-(1→4)-2-acetamido-3,6-di-O-benzyl-2-deoxy-β-D-glucopyranosyl-(1→2)-3,4,6-tri-O-benzyl-α-D-mannopyranosyl-(1→6)]-2,4-di-O-benzyl-β-D-mannopyranosyl-(1→4)-2-acetamido-3,6-di-O-benzyl-2-deoxy-β-D-glucopyranosyl-(1→4)-2-acetamido-3,6-di-O-benzyl-2-deoxy-β-D-glucopyranoside (the compound represented by Formula D-10) (0.678 mmol equivalents) was mixed with N-methylpyrrolidone (26.1 mL) and 5% Pd/C (EA type; water content: 54.9%; 3.92 g; available by Kawaken Fine Chemicals Co., Ltd.). Pressure reduction and then nitrogen replacement were repeated three times. After that, hydrogen pressurization and then pressure release were repeated three times. The mixture was stirred for 48 hours at an outside temperature of 50°C and under a hydrogen pressure of 0.5 MPa. After the completion of the reaction was checked by HPLC, the reaction solution was filtered under reduced pressure in a glove box with nitrogen replacement. The filtered material was washed with N-methylpyrrolidone (35 mL). The filtrate was mixed with toluene (45 mL), and concentrated to 55 mL. This operation was repeated three times, namely the dehydration operation was performed to give an N-methylpyrrolidone solution containing crude 4-methoxyphenyl 5-acetamido-3,5-dideoxy-D-glycero-α-D-galacto-non-2-uropyranosyl-(2→6)-β-D-galactopyranosyl-(1→4)-2-acetamido-2-deoxy-β-D-glucopyranosyl-(1→2)-α-D-mannopyranosyl-(1→3)-[β-D-glucopyranosyl-(1→4)-2-acetamido-2-deoxy-β-D-glucopyranosyl-(1→2)-α-D-mannopyranosyl-(1→6)]-β-D-mannopyranosyl-(1→4)-2-acetamido-2-deoxy-β-D-glucopyranosyl-(1→4)-2-acetamido-2-deoxy-β-D-glucopyranoside (the compound represented by Formula D-11). This was used as it was in the next step. The reactivity is almost the same with 5% Pd/C (EA type; available by Kawaken Fine Chemicals Co., Ltd.) or ASCA-2 (available by N.E. CHEMCAT CORPORATION) instead of the above 5% Pd/C (EA type; available by Kawaken Fine Chemicals Co., Ltd.) as a catalyst.

### Example 67

The compound represented by Formula D-12 was purified according to the following synthesis scheme W. The compound shown in the lower left below is an example of a compound where "R₇" in Formula E1 is methyl.

### [Synthesis Scheme W]

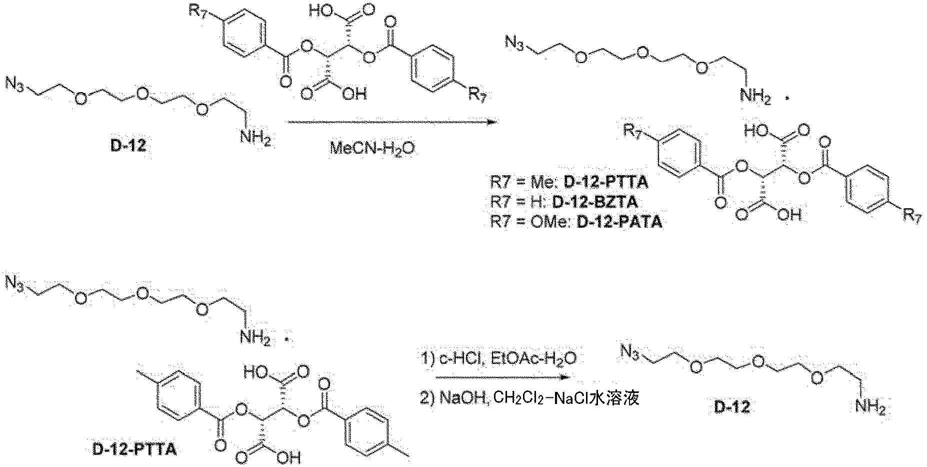

### Example 68

### 11-Azido-3,6,9-trioxaundecan-1-amine (-)-di-p-toluoyl-L-tartrate(the compound represented by Formula E-2 (R₇ = Me))

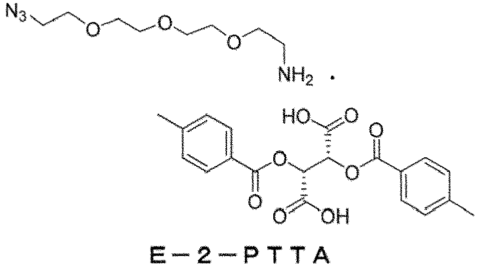

To a solution containing 11-azido-3,6,9-trioxaundecan-1-amine (the compound represented by Formula D-12) (10.0 g, 45.82, 94.3% HPLC purity) in acetonitrile (24 mL) and water (6 mL) was added (-)-di-p-toluoyl-L-tartaric acid (17.70 g, 45.82 mmol). After the mixture was stirred at 35°C and the dissolution was checked, acetonitrile (300 mL) was added dropwise at the same temperature over 1 hour. The resulting slurry solution was concentrated under reduced pressure to 200 mL, and stirred at 25°C for 30 minutes. Then, the precipitated crystals were filtered. The filtered crystals were washed with acetonitrile (30 mL) and dried under reduced pressure at 40°C to give, as white crystals, 11-azido-3,6,9-trioxaundecan-1-amine (-)-di-p-toluoyl-L-tartrate (the compound represented by Formula E-2-PTTA) (24.40 g, yield: 88.0%, HPLC purity: 99.2%) (melting point: 152°C).

¹H-NMR (500 MHz, DMSO) δ 7.82 (d, J = 9.0 Hz, 4H), 7.30 (d, J = 9.0 Hz, 4H), 5.61 (s, 2H), 3.59 (t, J = 5.0 Hz, 2H), 3.56-3.47 (m, 10H), 3.39 (t, J = 5.5 Hz, 2H), 2.91 (t, J = 6.0 Hz, 2H), 2.36 (s, 6H). ¹³C-NMR (125 MHz, DMSO) δ 168.06, 164.80, 143.63, 129.25, 129.18, 126.93, 71.68, 69.72, 69.63, 69.59, 69.57, 69.20, 66.68, 49.96, 38.40, 21.14.

Substantially the same procedure can be performed using (+)-di-p-toluoyl-D-tartaric acid instead of (-)-di-p-toluoyl-L-tartaric acid.

### Example 69

### 11-Azido-3,6,9-trioxaundecan-1-amine (-)-dibenzoyl-L-tartrate(the compound represented by Formula E-2-BZTA)

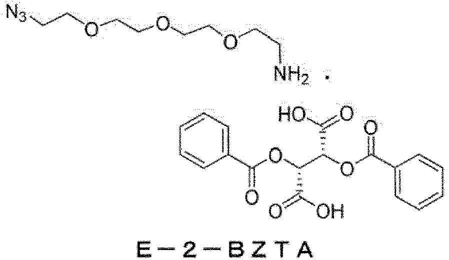

To a solution containing 11-azido-3,6,9-trioxaundecan-1-amine (the compound represented by Formula D-12) (5.0 g, 22.91 mmol) in acetonitrile (12 mL) and water (3 mL) was added (-)-dibenzoyl-L-tartaric acid (8.21 g, 22.91 mmol). After the mixture was stirred at 35°C and the dissolution was checked, acetonitrile (150 mL) was added dropwise at the same temperature over 1 hour. The resulting slurry solution was concentrated under reduced pressure to 125 mL, and acetonitrile (100 mL) was added. The mixture was concentrated again under reduced pressure to 125 mL. The slurry solution was stirred at 25°C for 30 minutes, and the precipitated crystals were then filtered. The filtered crystals were washed with acetonitrile (15 mL) and dried under reduced pressure at 40°C to give, as white crystals, 11-azido-3,6,9-trioxaundecan-1-amine (-)-dibenzoyl-L-tartrate (the compound represented by Formula E-2-BZTA) (10.6 g, yield: 80%) (melting point: 131°C).

¹H-NMR (500 MHz, DMSO) δ 7.94 (d, J = 8.5 Hz, 4H), 7.64 (t, J = 7.5 Hz, 2H), 7.51 (t, J = 7.5 Hz, 4H), 5.65 (s, 2H), 3.59 (t, J = 5.0 Hz, 2H), 3.56-3.47 (m, 10H), 3.39 (t, J = 5.5 Hz, 2H), 2.90 (t, J = 5.0 Hz, 2H).

¹³C-NMR (125 MHz, DMSO) δ 167.98, 164.86, 133.38, 129.63, 129.22, 128.66, 72.10, 69.73, 69.63, 69.59, 69.57, 69.21, 66.69, 49.97, 38.38.

Substantially the same procedure can be performed using (+)-dibenzoyl-D-tartaric acid instead of (-)-dibenzoyl-L-tartaric acid.

### Example 70

### 11-Azido-3,6,9-trioxaundecan-1-amine (-)-di-p-anisoyl-L-tartrate(the compound represented by Formula E-2 (R₇ = OMe))

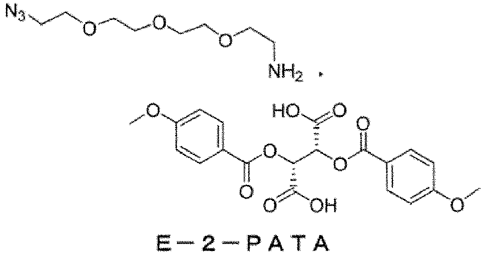

To a solution containing 11-azido-3,6,9-trioxaundecan-1-amine (the compound represented by Formula D-12) (5.0 g, 22.91 mmol) in acetonitrile (12 mL) and water (3 mL) was added (-)-di-p-anisoyl-L-tartaric acid (9.58 g, 22.91 mmol). After the mixture was stirred at 35°C and the dissolution was checked, acetonitrile (150 mL) was added dropwise at the same temperature over 1 hour. The resulting slurry solution was concentrated under reduced pressure to 100 mL, and stirred at 25°C for 30 minutes. Then, the precipitated crystals were filtered. The filtered crystals were washed with acetonitrile (15 mL) and dried under reduced pressure at 40°C to give, as white crystals, 11-azido-3,6,9-trioxaundecan-1-amine (-)-di-p-anisoyl-L-tartrate (the compound represented by Formula E-2-PATA (R₇ = OMe)) (11.4 g, yield: 78%) (melting point: 153°C).

¹H-NMR (500 MHz, DMSO) δ 7.89 (d, J = 9.0 Hz, 4H), 7.02 (d, J = 9.0 Hz, 4H), 5.59 (s, 2H), 3.82 (s, 6H), 3.59 (t, J = 5.0 Hz, 2H), 3.56-3.47 (m, 10H), 3.39 (t, J = 5.5 Hz, 2H), 2.91 (t, J = 5.5 Hz, 2H). ¹³C-NMR (125 MHz, DMSO) δ 168.25, 164.50, 163.14, 131.34, 121.88, 113.92, 71.64, 69.73, 69.63, 69.59, 69.21, 66.70, 55.48, 49.97, 38.38.

Substantially the same procedure can be performed using (+)-di-p-anisoyl-D-tartaric acid instead of (-)-di-p-anisoyl-L-tartaric acid.

### Example 71

### 11-Azido-3,6,9-trioxaundecan-1-amine (the compound represented by Formula D-12)

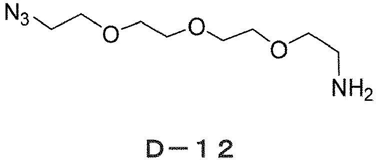

To a solution containing 11-azido-3,6,9-trioxaundecan-1-amine (-)-di-p-toluoyl-L-tartrate (the compound represented by Formula E-2-PTTA) (12.0 g, 19.85 mmol) obtained in Example 68 in ethyl acetate (120 mL) and water (18 mL) was added concentrated hydrochloric acid (2.41 g, 23.82 mmol). The mixture was stirred at 25°C, and then liquid-separated. The resulting aqueous layer was washed twice with ethyl acetate (120 mL), adjusted to pH 11 with 10 N aqueous sodium hydroxide solution (2.15 mL, 21.50 mmol). Sodium chloride (0.6 g) was added, and then dissolved. Dichloromethane (120 mL) was added, stirred, and then liquid-separated to give the organic layer. Further, the aqueous layer was mixed with dichloromethane (120 mL), stirred, and then liquid-separated to give the organic layer. These organic layers were combined. The combined organic layer was concentrated under reduced pressure to 12 mL, and ethyl acetate (120 mL) was added. The mixture was concentrated under reduced pressure to 12 mL. The resulting solution was filtered to remove inorganic salts and washed with acetonitrile (6 mL). The resulting solution was concentrated under reduced pressure to give 11-azido-3,6,9-trioxaundecan-1-amine (the compound represented by Formula D-12) with HPLC purity of 99.2% (3.7 g, yield: 85%) as a pale yellow oily compound.

¹H-NMR (500 MHz, CDCl₃) δ 3.69-3.62 (m, 10H), 3.51 (t, J = 5.5 Hz, 2H), 3.39 (t, J = 5.5 Hz, 2H), 2.87 (t, J = 5.5 Hz, 2H).

### <Conditions for analyzing purity of compound represented by Formula D-12>

The purity of the compound represented by Formula D-12 was determined under the following HPLC analysis conditions. The sample solution was measured, a blank solution was then measured, and the blank peak was removed to calculate the HPLC purity of the compound represented by Formula D-12.

### <Preparation of sample solution>

In a 10-mL measuring flask, 50 mg of the compound represented by Formula D-12 was weighed, and 2 mL of acetonitrile and 100 µL of triethylamine were added and mixed. Next, 50 µL of acetic anhydride was added, mixed, and then allowed to stand for 15 min to acetyl-derivatize the compound represented by Formula D-12. The sample solution was prepared by adding 150 µL of 4 N aqueous sodium hydroxide solution, mixing, and then filling the flask with 50% acetonitrile water.

### <Preparation of blank solution>

In a 10-mL measuring flask, 2 mL of acetonitrile and 100 µL of triethylamine were added and mixed. Next, 50 µL of acetic anhydride was added, mixed, and then allowed to stand for 15 minutes. The sample solution was prepared by adding 150 µL of 4 N aqueous sodium hydroxide solution, mixing, and then filling the flask with 50% acetonitrile water.

### <HPLC analysis conditions>

Instrument used: SHIMADZU HPLC (LC-20AD)
Column: Xbridge C18 3.5 µm, 4.6 × 150 mm (Waters)
Mobile phase A: 10 mM AcONH₄ aqueous solution
Mobile phase B: CH₃CN

**[Table 7]**

| Gradient conditions: | | |
|---|---|---|
| Time/Solvent | 10 mM AcONH₄ aqueous solution | CH₃CN |
| 0 min | 90 % | 10 % |
| 13 min | 35 % | 65 % |
| 15 min | 35 % | 65 % |

Flow rate: 1 mL/min
Detection wavelength: 210 nm
Column temperature: 40°C
Injection volume: 5 µL
Retention time: 7.3 min (acetylated compound represented by Formula D-12), 9.0 min (dimer of acetylated compound represented by Formula D-12)

### Example 72

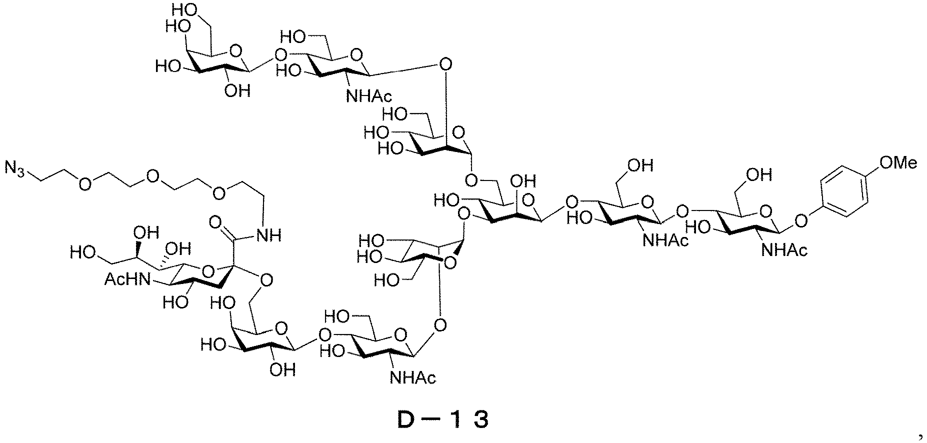

An N-methylpyrrolidone solution containing 4-methoxyphenyl 5-acetamido-3,5-dideoxy-D-glycero-α-D-galacto-non-2-uropyranosyl-(2→6)-β-D-galactopyranosyl-(1→4)-2-acetamido-2-deoxy-β-D-glucopyranosyl-(1→2)-α-D-mannopyranosyl-(1→3)-[β-D-galactopyranosyl-(1→4)-2-acetamido-2-deoxy-β-D-glucopyranosyl-(1→2)-α-D-mannopyranosyl-(1→6)]-β-D-mannopyranosyl-(1→4)-2-acetamido-2-deoxy-β-D-glucopyranosyl-(1→4)-2-acetamido-2-deoxy-β-D-glucopyranoside (the compound represented by Formula D-11) (0.678 mmol equivalents) was added to a 100-mL recovery flask, and mixed sequentially with 11-azido-3,6,9-trioxaundecan-1-amine (the compound represented by Formula D-12) (0.45 g, 2.03 mmol), N-ethyldiisopropylamine (0.36 mL, 2.03 mmol), and (benzotriazol-1-yloxy)tripyrrolidinophosphonium hexafluorophosphate (1.06 g, 2.03 mmol). The mixture was stirred at room temperature for 1 hour. After the completion of the reaction was checked by HPLC, acetonitrile (300 mL) was added dropwise to the reaction solution to precipitate a gelatinous solid. The suspension was centrifuged. The white gelatinous solid was washed twice with acetonitrile (30 mL). The resulting white gelatinous solid was mixed with water (10 mL), and filtered through a membrane filter. The filtrate was purified preparatively by HPLC (2 mL per charge). The target substance-containing fractions were concentrated under reduced pressure and lyophilized to prepare, as white solids, 4-methoxyphenyl N-(2-{2-[2-(2-azidoethoxy)ethoxy]ethoxy}ethyl)-5-acetyl-neuramamido-(2→6)-β-D-galactopyranosyl-(1→4)-2-acetamido-2-deoxy-β-D-glucopyranosyl-(1→2)-α-D-mannopyranosyl-(1→3)-[β-D-galactopyranosyl-(1→4)-2-acetamido-2-deoxy-β-D-glucopyranosyl-(1→2)-α-D-mannopyranosyl-(1→6)]-β-D-mannopyranosyl-(1→4)-2-acetamido-2-deoxy-β-D-glucopyranosyl-(1→4)-2-acetamido-2-deoxy-β-D-glucopyranoside (the compound represented by Formula D-13) (1.01 g, yield: 66% (total yield from the compound represented by D8)) (HPLC purity: 98.2% (detection wavelength 220 nm)).

¹H-NMR (500 MHz, D₂O) δ 1.73 (t, J = 12.0 Hz, 1H), 1.87-2.07 (brs, 15H), 2.59 (dd, J = 4, 12.0 Hz, 1H), 3.26-3.90 (m, 77H), 4.00 (brs, 1H), 4.08 (brs, 1H), 4.14 (brs, 1H), 4.30-4.38 (m, 2H), 4.50-4.54 (m, 3H), 4.65 (brs, 1H), 4.78 (d, J = 5.5 Hz, 1H), 4.83 (brs, 1H), 4.93 (d, J = 8.5 Hz, 1H), 5.03 (brs, 1H), 6.86 (d, J = 9.5 Hz, 2H), 6.93 (d, J = 9.5 Hz, 2H).

¹³C-NMR (125 MHz, D₂O) δ 22.71, 22.76, 22.89, 22.99, 23.06, 23.91, 38.85, 39.41, 50.80, 52.35, 55.22, 55.50, 55.62, 56.45, 60.58, 60.62, 60.84, 61.64, 62.28, 62.34, 63.29, 63.68, 66.31, 66.43, 67.63, 67.91, 67.92, 67.99, 68.45, 68.97, 69.14, 69.18, 69.80, 69.85, 70.06, 70.10, 70.16, 70.28, 70.83, 71.29, 71.62, 71.73, 72.66, 72.71, 72.73, 73.09, 73.16, 73.50, 74.12, 74.16, 74.19, 75.02, 75.05, 75.34, 75.36, 75.99, 76.98, 77.06, 79.19, 79.65, 80.10, 81.11, 81.48, 97.67, 100.02, 100.10, 100.18, 101.07, 102.03, 103.60, 104.29, 115.72, 118.92, 151.66, 155.49, 163.48, 169.72, 175.26, 175.28, 175.39, 175.46, 175.64.

HRMS (ESI) [M+H]⁺ (m/z): calcd for C₈₈H₁₄₄N₉O₅₇: 2238.8641; found 2238.8604 [M+H]⁺).

## Claims

1. A method for producing an oligosaccharide represented by Formula A-13: 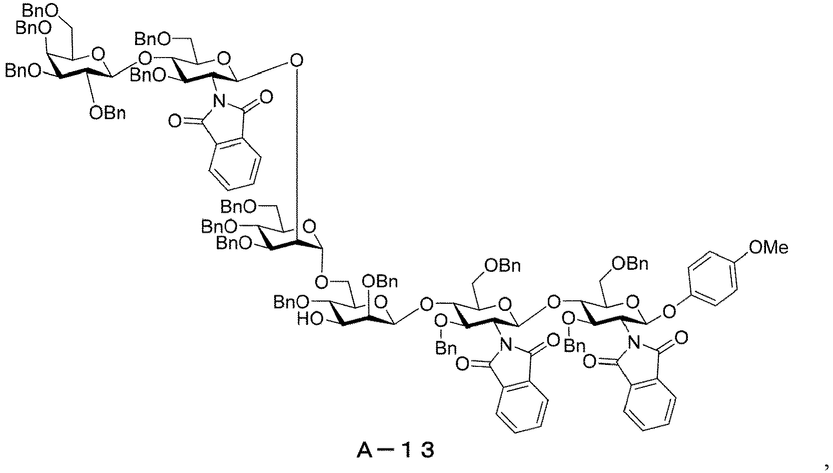 wherein the method comprises steps of:
(Step I-1) producing a compound represented by Formula A-7: 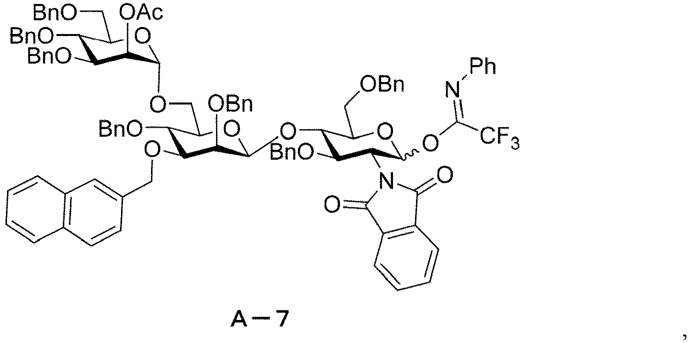 the step comprising a step of:
connecting a compound represented by Formula A-3: 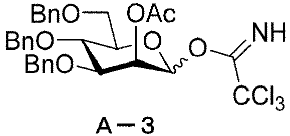 to a compound represented by Formula A-4: 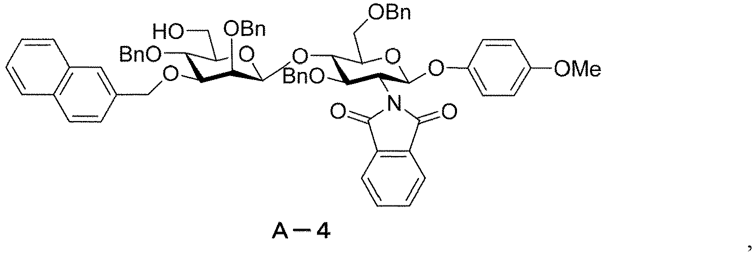 via α-1,6-glycosidic linkage to give a compound represented by Formula A-5: 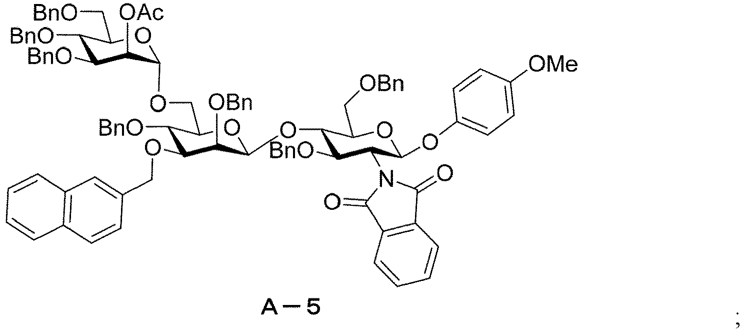
(Step I-2) producing a compound represented by Formula A-10: 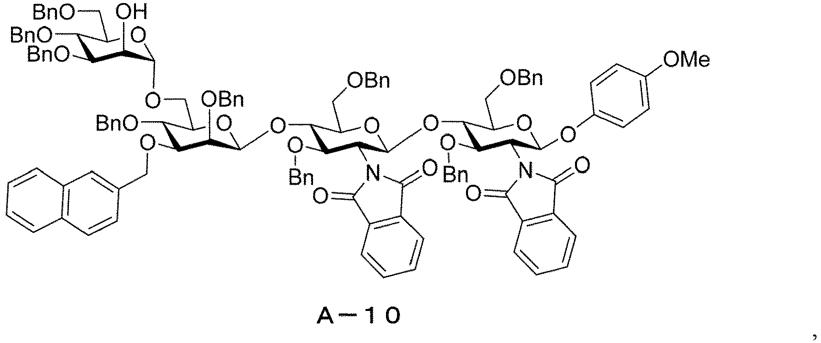 the step comprising a step of:
connecting the compound represented by Formula A-7 to a compound represented by Formula A-8: 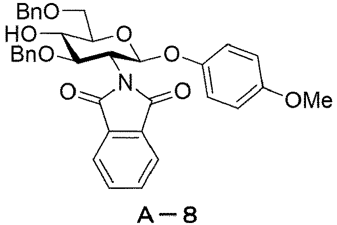 via β-1,4-glycosidic linkage to give a compound represented by Formula A-9: 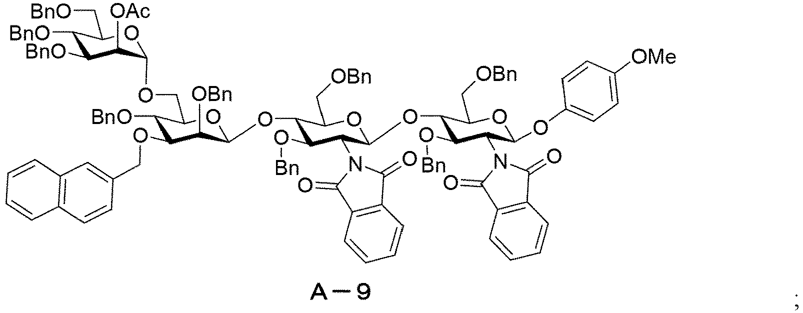 and
(Step I-3) producing the oligosaccharide represented by Formula A-13, wherein the step comprising a step of:
connecting the compound represented by Formula A-10 to a compound represented by Formula A-11: 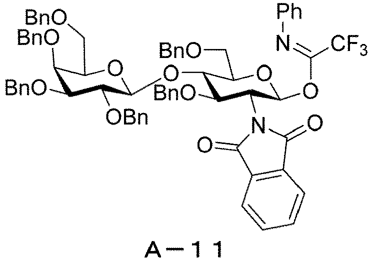 via β-1,2-glycosidic linkage to give a compound represented by Formula A-12:

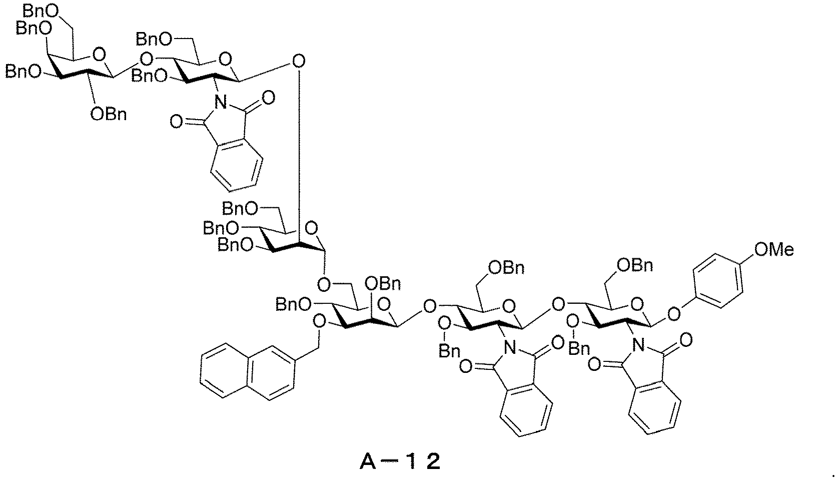

2. The method according to claim 1, wherein Step I-2 comprises reacting the compound represented by Formula A-9 with a strong base in the presence of an alkyl ester of perfluorocarboxylic acid to give the compound represented by Formula A-10.

3. The method according to claim 2, wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.

4. The method according to claim 2 or 3, wherein the strong base is selected from the group consisting of sodium, lithium, and potassium salts of metal amides; sodium, lithium, potassium, cesium, and barium salts of C1-C20 alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and a combination thereof.

5. The method according to claim 2 or 3, wherein the strong base is potassium tert-butoxide, sodium tert-butoxide, lithium tert-butoxide, or LHMDS (lithium hexamethyldisilazide).

6. The method according to any one of claims 2 to 5, wherein the reaction of Step I-2 is carried out in a C1-C10 alcohol solvent alone or in a mixture of a C1-C10 alcohol solvent and an amide-based solvent, an ether-based solvent, an ester-based solvent, an aromatic solvent, a halogen-based solvent, a hydrocarbon-based solvent, or a nitrile-based solvent.

7. The method according to any one of claims 1 to 6, wherein Step I-3 comprises reacting the compound represented by Formula A-12 with DDQ (2,3-dichloro-5,6-dicyano-p-benzoquinone) in a mixed solvent of fluorous alcohol and water to remove a 2-naphthylmethyl group in the compound represented by Formula A-12 to give the oligosaccharide represented by Formula A-13.

8. The method according to claim 7, wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and a combination thereof.

9. The method according to claim 7 or 8, wherein the reaction of Step I-3 is carried out at -35°C to 70°C.

10. The method according to claim 7 or 8, wherein the reaction of Step I-3 is carried out at -30°C to -10°C.

11. The method according to any one of claims 1 to 10, wherein the method comprises purifying the compound represented by Formula A-5 by
after stopping the reaction of the compound represented by Formula A-4 with the compound represented by Formula A-3 in Step I-1, adding a hydrophobic carrier and water to a water-soluble organic solvent containing the resulting compound represented by Formula A-5 and contaminants to adsorb the compound represented by Formula A-5 onto the hydrophobic carrier;
then filtering the resultant and washing the hydrophobic carrier with a mixture of the water-soluble organic solvent and the water to remove the contaminants;
and then eluting the compound represented by Formula A-5 from the hydrophobic carrier using an organic solvent.

12. The method according to any one of claims 1 to 11, wherein the method comprises purifying the compound represented by Formula A-9 by
after stopping the reaction of the compound represented by Formula A-7 with the compound represented by Formula A-8 in Step I-2, adding a hydrophobic carrier and water to a water-soluble organic solvent containing the resulting compound represented by Formula A-9 and contaminants to adsorb the compound represented by Formula A-9 onto the hydrophobic carrier;
then filtering the resultant and washing the hydrophobic carrier with a mixture of the water-soluble organic solvent and the water to remove the contaminants;
and then eluting the compound represented by Formula A-9 from the hydrophobic carrier using an organic solvent.

13. The method according to any one of claims 1 to 12, wherein the method comprises purifying the compound represented by Formula A-12 by
after stopping the reaction of the compound represented by Formula A-10 with the compound represented by Formula A-11 in Step I-3, adding a hydrophobic carrier and water to a water-soluble organic solvent containing the resulting compound represented by Formula A-12 and contaminants to adsorb the compound represented by Formula A-12 onto the hydrophobic carrier;
then filtering the resultant and washing the hydrophobic carrier with a mixture of the water-soluble organic solvent and the water to remove the contaminants;
and then eluting the compound represented by Formula A-12 from the hydrophobic carrier using an organic solvent.

14. The method according to claim 11, wherein the contaminants include a sugar compound other than the compound represented by Formula A-5, and/or a compound derived from a reaction reagent to obtain the compound to be purified.

15. The method according to claim 12, wherein the contaminants include a sugar compound other than the compound represented by Formula A-9, and/or a compound derived from a reaction reagent to obtain the compound to be purified.

16. The method according to claim 13, wherein the contaminants include a sugar compound other than the compound represented by Formula A-12, and/or a compound derived from a reaction reagent to obtain the compound to be purified.

17. The method according to any one of claims 11 to 16, wherein the hydrophobic carrier is a resin used for packing reversed-phase partition chromatography.

18. The method according to claim 17, wherein the resin packed for reversed-phase partition chromatography is selected from the group consisting of a poly(styrene/divinylbenzene) polymer gel resin, a polystyrene-divinylbenzene resin, a polyhydroxymethacrylate resin, a styrene-vinylbenzene copolymer resin, a polyvinyl alcohol resin, a polystyrene resin, a polymethacrylate resin, chemically bonded silica gel resin, and a combination thereof.

19. The method according to claim 18, wherein the chemically bonded silica gel resin is selected from the group consisting of (1) a resin obtained by reacting silica gel with a silane coupling agent, (2) a resin obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) a resin obtained by chemically bonding a docosyl or triacontyl group to silica gel, and (4) a combination of the resins (1) to (3).

20. The method according to claim 18, wherein the chemically bonded silica gel resin is an octadecyl group-bonded silica gel resin (ODS resin).

21. The method according to any one of claims 11 to 20, wherein the water-soluble organic solvent is a water-soluble alcohol-based solvent, a water-soluble nitrile-based solvent, a water-soluble ether-based solvent, a water-soluble ketone-based solvent, a water-soluble amide-based solvent, or a water-soluble sulfoxide-based solvent, or a mixed solvent containing at least one of the aforementioned water-soluble organic solvents.

22. The method according to claim 21, wherein the water-soluble nitrile-based solvent is acetonitrile.

23. The method according to any one of claims 11 to 22, wherein the organic solvent used for eluting the compound of interest from the hydrophobic carrier is a nitrile-based solvent, an ether-based solvent, an ester-based solvent, a ketone-based solvent, a halogen-based solvent, an aromatic solvent, or a mixed solvent containing at least one of the aforementioned solvents.

24. The method according to any one of claims 1 to 23, wherein the compound represented by Formula A-11 is produced by steps comprising:
(Step Y-1) a step of producing a compound represented by Formula B-4: 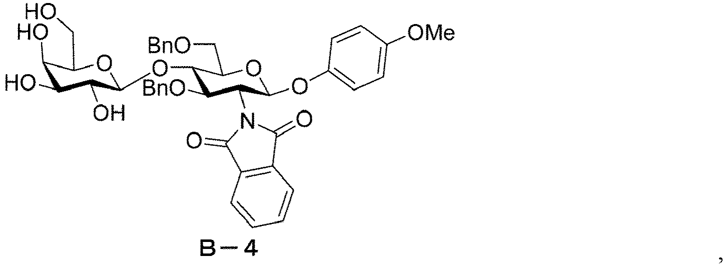 wherein the step comprising a step of:
connecting a compound represented by Formula B-1: 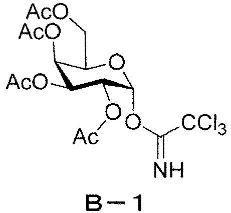 to a compound represented by Formula B-2: 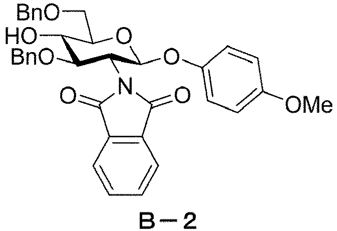 via β-1,4-glycosidic linkage to give a compound represented by Formula B-3: 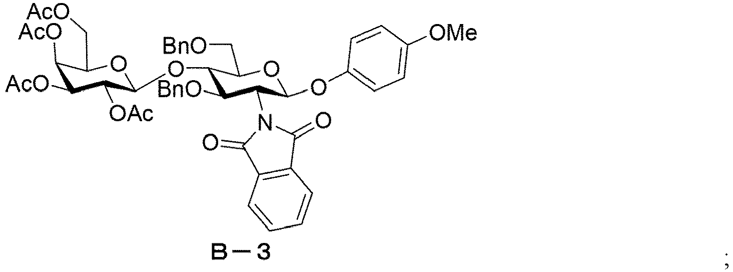 and
(Step Y-2) a step of adding lithium tert-butoxide or lithium tert-amoxide to a solvent containing the compound represented by Formula B-4 and benzyl halide or benzyl sulfonate to protect each hydroxyl group present in the compound represented by Formula B-4 by a benzyl group to give a compound represented by Formula B-5:

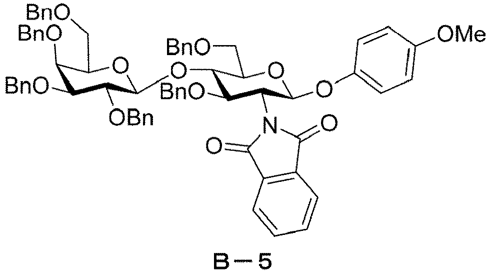

25. The method according to claim 24, wherein the solvent containing the compound represented by Formula B-4 and benzyl halide or benzyl sulfonate is an amide-based solvent, an ether-based solvent, an aromatic solvent, or a hydrocarbon-based solvent, a urea-based solvent, or a mixed solvent containing at least one of the aforementioned solvents.

26. The method according to claim 24 or 25, further comprising the step of purifying the compound represented by Formula B-5 by conducting ring-opening of a phthalimide group in the compound represented by Formula B-5, followed by reaction with cinchonidine to give a crystalline compound represented by Formula B-6: 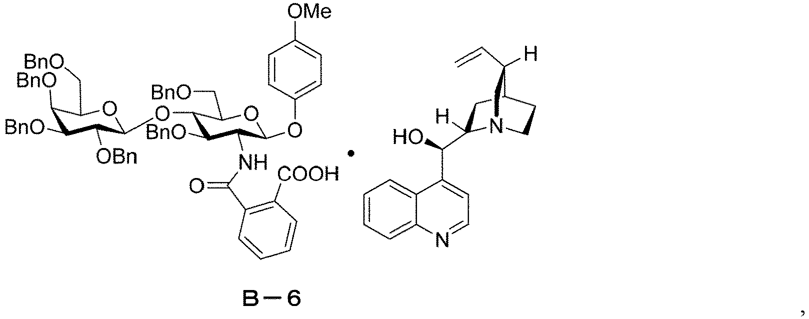 separating the crystalline compound represented by Formula B-6 from amorphous materials, then adding an acid aqueous solution and solvent to remove cinchonidine from the compound represented by Formula B-6 to give a compound represented by Formula B-7: 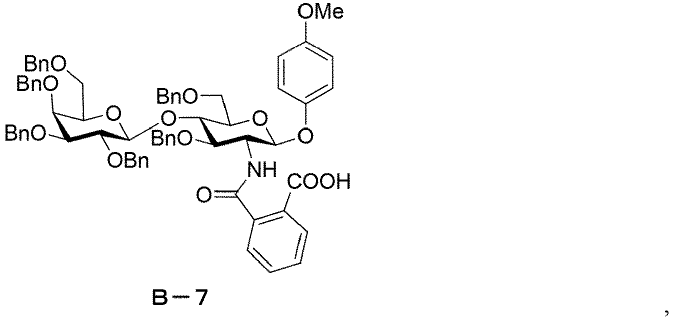 and then conducting ring-closing of the ring-opened phthalimide in the compound represented by Formula B-7.

27. The method according to any one of claims 1 to 26, further comprising the step of purifying the compound represented by Formula A-13 by conducting ring-opening of phthalimide groups in the compound represented by Formula A-13, followed by forming a salt with (R)-(+)-1-(1-naphthyl)ethylamine to give a crystalline compound represented by Formula A-14: 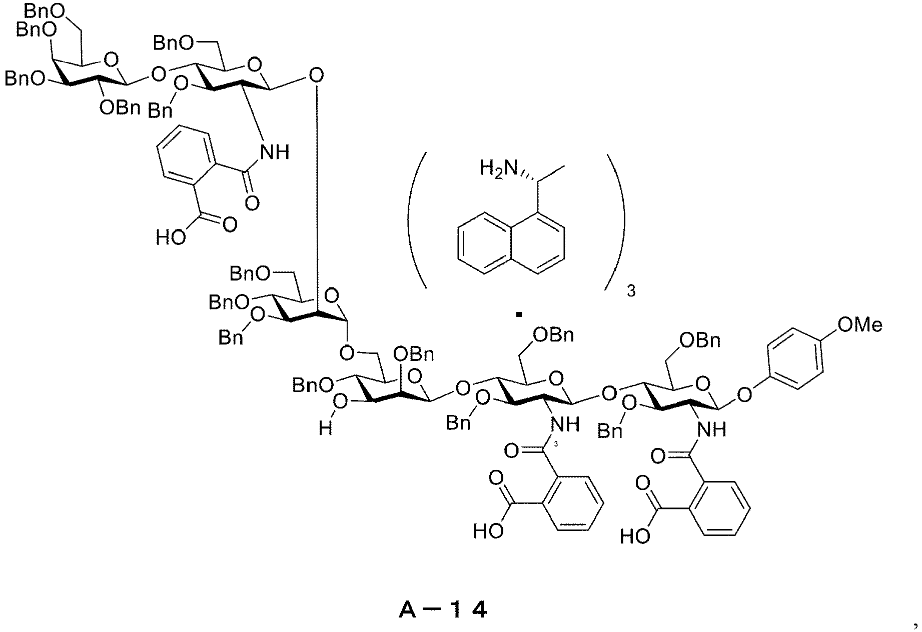 separating the crystalline compound represented by Formula A-14 from amorphous materials, then adding an acid aqueous solution and solvent to remove (R)-(+)-1-(1-naphthyl)ethylamine from the compound represented by Formula A-14 to give a compound represented by Formula A-15: 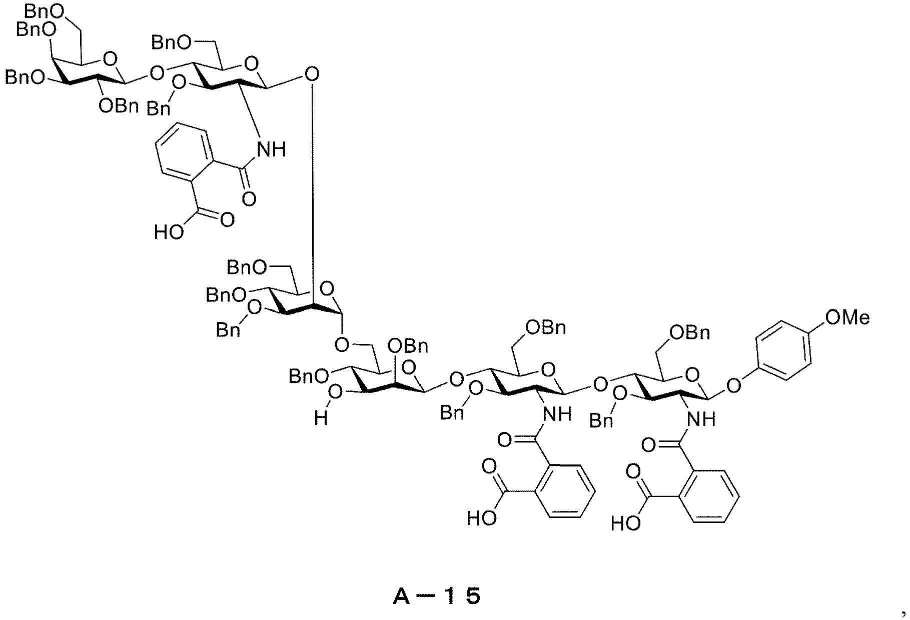 and then conducting ring-closing of the ring-opened phthalimides in the compound represented by Formula A-15.

28. A method for producing an oligosaccharide represented by Formula D-13: 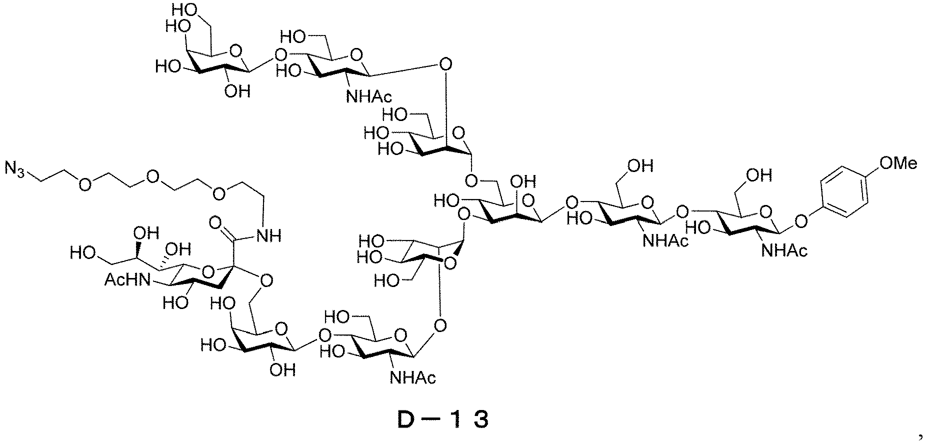 wherein the method comprises steps of:
(Step II-1) producing a compound represented by Formula D-2: 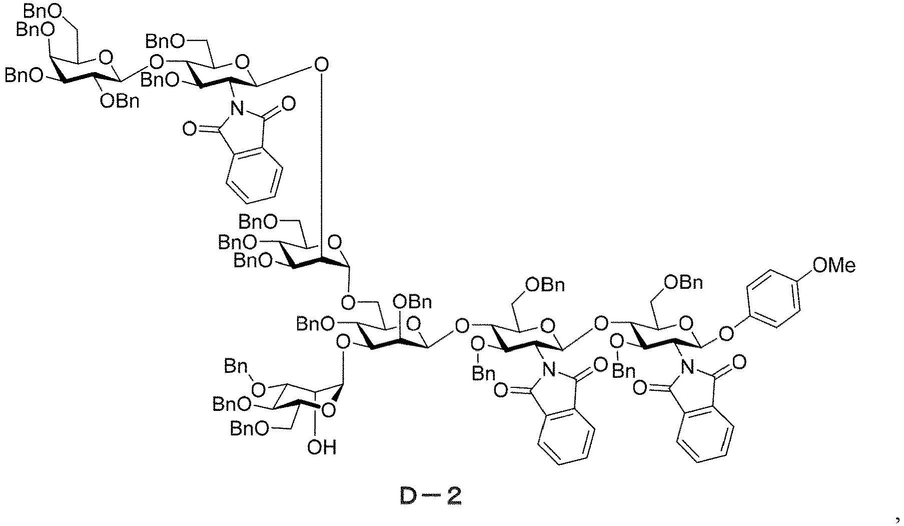 wherein the step comprising a step of:
connecting an oligosaccharide represented by Formula A-13: 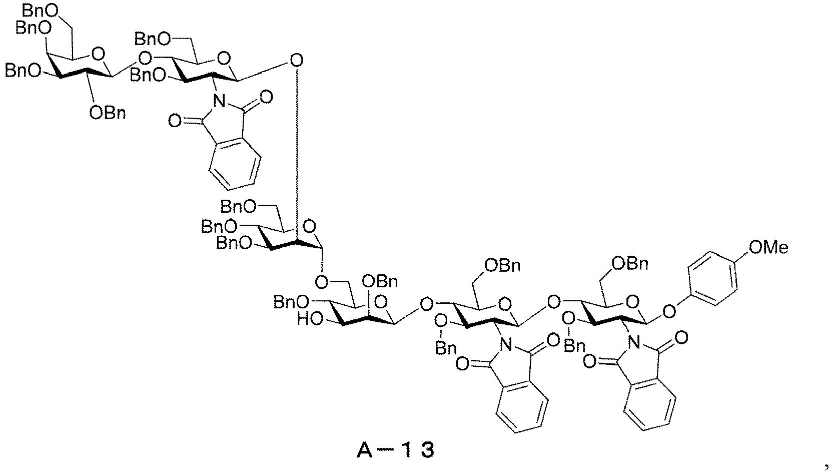 to a compound represented by Formula A-3: 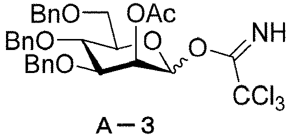 via α-1,3-glycosidic linkage to give a compound represented by Formula D-1: 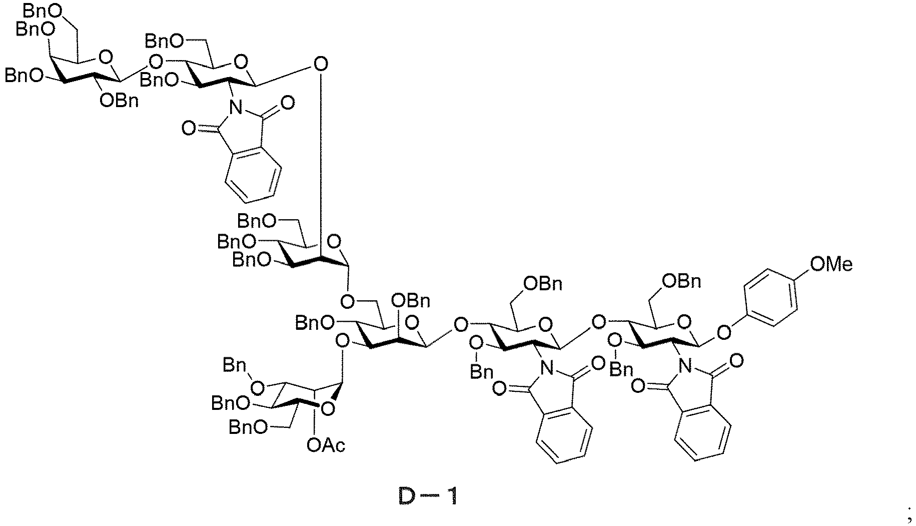
(Step II-2) producing a compound represented by Formula D-5: 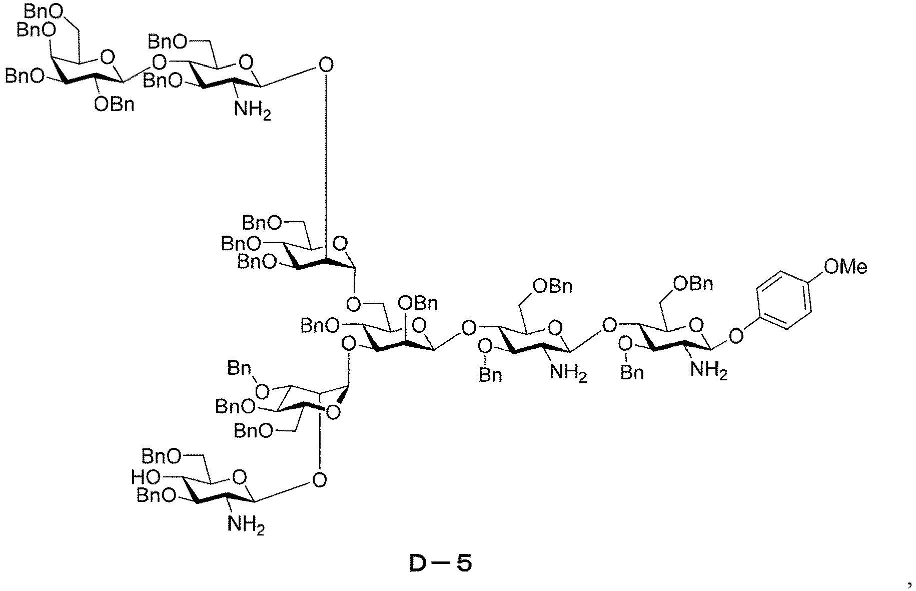 wherein the step comprising a step of
connecting the compound represented by Formula D-2 to a compound represented by Formula D-3: 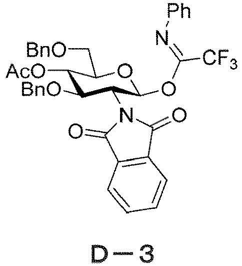 via β-1,2-glycosidic linkage to give a compound represented by Formula D-4: 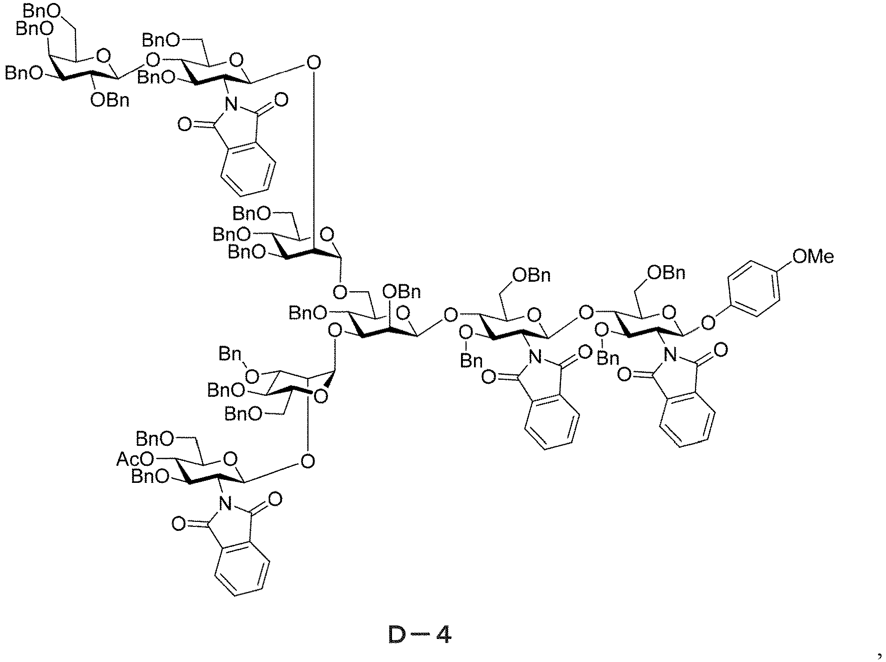 and then protecting the amino group in the compound represented by Formula D-5 with a protecting group selected from an aryloxycarbonyl (COOAr) group, an acetyl (Ac) group, a 2,2,2-trichloroethoxycarbonyl (Troc) group, or a phthalimide (Pht) group to give a compound represented by Formula D-6: 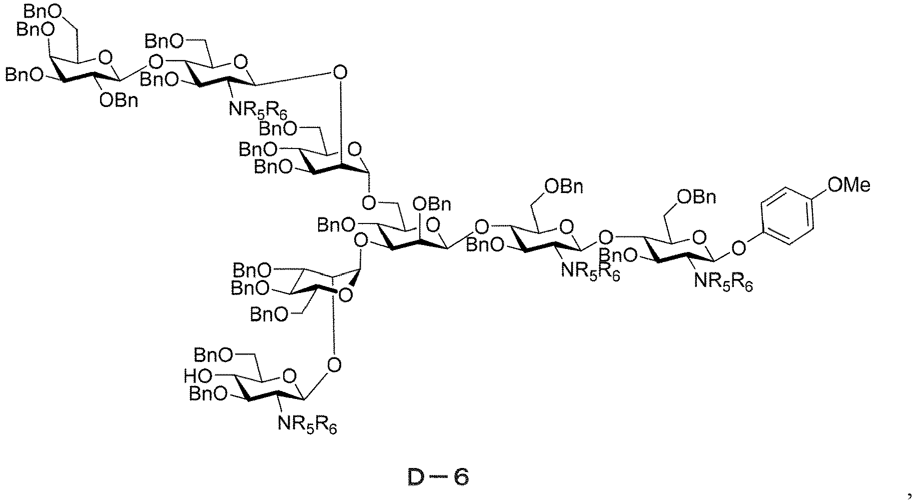
wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
or removing an acetyl (Ac) group on the compound represented by Formula D-4 to give the compound represented by Formula D-6 wherein R₅ and R₆ together with a nitrogen atom attached thereto form a phthalimide group;
(Step II-3) producing a compound represented by Formula D-11: 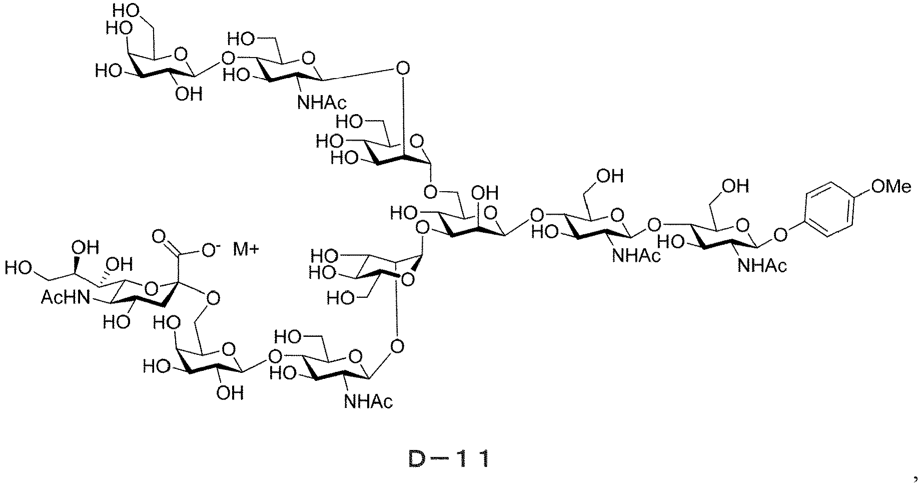
wherein M⁺ is a sodium ion, a lithium ion, a potassium ion, or a protonated triethylamine cation,
wherein the step comprising a step of
connecting the compound represented by Formula D-6 to a compound represented by Formula D-7: 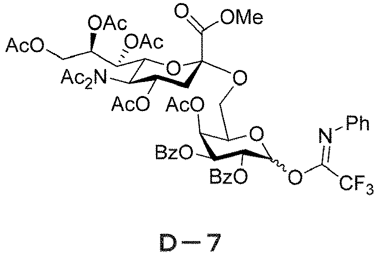 via β-1,4-glycosidic linkage to give a compound represented by Formula D-8: 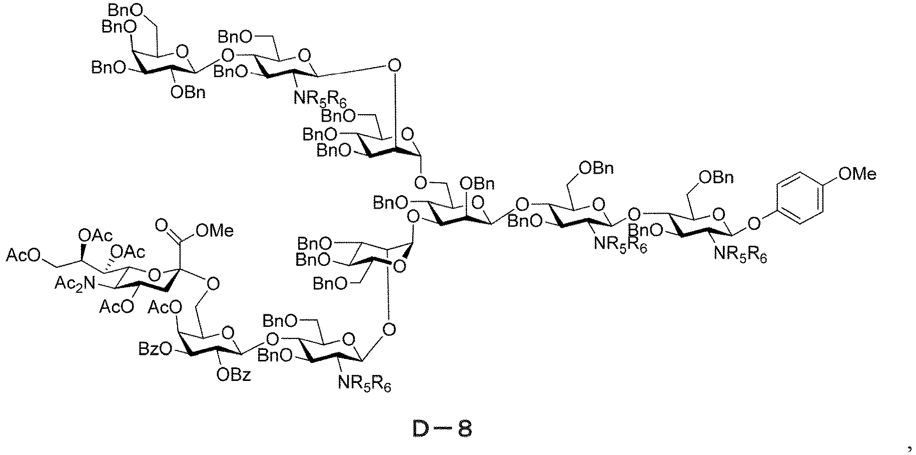
wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
and then removing each amino group-protecting group and alcohol acyl-based-protecting group on the compound represented by Formula D-8 to give a compound represented by Formula D-9: 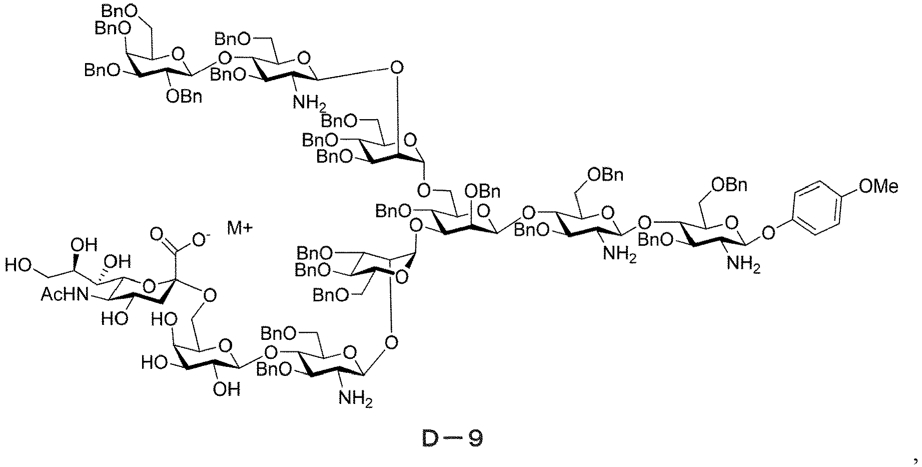
wherein M⁺ is a sodium ion, a lithium ion, a potassium ion, or a protonated triethylamine cation; and
(Step II-4) producing the oligosaccharide represented by Formula D-13 by reacting the compound represented by Formula D-11 with a compound represented by Formula D-12:

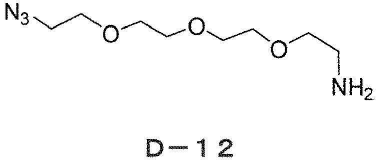

29. The method according to claim 28, wherein Step II-1 comprises reacting the compound represented by Formula D-1 with a strong base in the presence of an alkyl ester of perfluorocarboxylic acid to give the compound represented by Formula D-2.

30. The method according to claim 29, wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.

31. The method according to claim 29 or 30, wherein the strong base is selected from the group consisting of sodium, lithium, and potassium salts of metal amides; sodium, lithium, potassium, cesium, and barium salts of C1-C20 alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and a combination thereof.

32. The method according to claim 29 or 30, wherein the strong base is potassium tert-butoxide, sodium tert-butoxide, lithium tert-butoxide, or LHMDS (lithium hexamethyldisilazide).

33. The method according to any one of claims 29 to 32, wherein the step of reacting the compound represented by Formula D-1 with a strong base in the presence of a trifluoroacetate to give the compound represented by Formula D-2 is carried out in a C1-C10 alcohol solvent alone or in a mixture of a C1-C10 alcohol solvent and an amide-based solvent, an ether-based solvent, an ester-based solvent, an aromatic solvent, a halogen-based solvent, a hydrocarbon-based solvent, or a nitrile-based solvent.

34. The method according to any one of claims 28 to 33, wherein in Step II-3, each amino group in the compound represented by Formula D-5 is protected with an aryloxycarbonyl (COOAr) group to give the compound represented by Formula D-6.

35. The method according to any one of claims 28 to 34, wherein in Step II-3, the step of producing the compound represented by Formula D-6 from the compound represented by Formula D-5 is carried out in an aqueous solution containing sodium bicarbonate, potassium bicarbonate, disodium hydrogen phosphate, or dipotassium hydrogen phosphate.

36. The method according to any one of claims 28 to 35, wherein the compound represented by Formula D-12 is obtained by a method for purification comprising steps of:
adding, to a solution containing the crude compound represented by Formula D-12, a compound represented by Formula E-1: 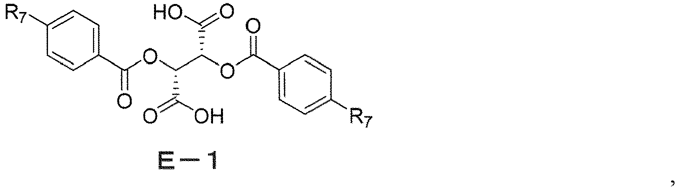
wherein R₇ is a hydrogen atom, a methyl group, or a methoxy group
to give a crystallin compound represented by Formula E-2: 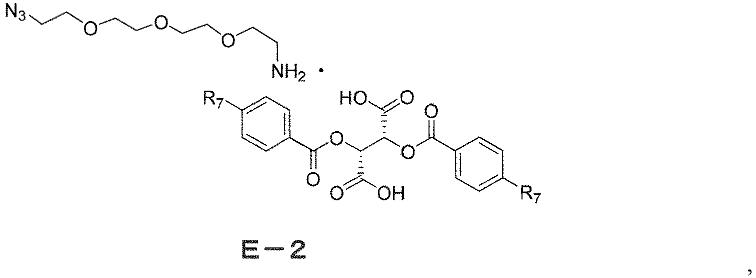
wherein R₇ is a hydrogen atom, a methyl group, or a methoxy group; and
isolating the crystalline compound and then extracting the compound represented by Formula D-12 from the crystalline compound isolated.

37. The method according to claim 36, wherein the purified compound represented by Formula D-12 has a purity of 95% or higher as measured by HPLC.

38. The method according to claim 37, wherein the purity is 98% or higher.

39. The method according to any one of claims 28 to 38, wherein the method comprises purifying the compound represented by Formula D-1 by
after stopping the reaction of the compound represented by Formula A-13 with the compound represented by Formula A-3 in Step II-1, adding a hydrophobic carrier and water to a water-soluble organic solvent containing the resulting compound represented by Formula D-1 and contaminants to adsorb the compound represented by Formula D-1 onto the hydrophobic carrier;
then filtering the resultant and washing the hydrophobic carrier with a mixture of the water-soluble organic solvent and the water to remove the contaminants;
and then eluting the compound represented by Formula D-1 from the hydrophobic carrier using an organic solvent.

40. The method according to any one of claims 28 to 39, wherein the method comprises purifying the compound represented by Formula D-5 by
after stopping the reaction of the compound represented by Formula D-3 with the compound represented by Formula D-4 in Step II-2, adding a hydrophobic carrier and water to a water-soluble organic solvent containing the resulting compound represented by Formula D-5 and contaminants to adsorb the compound represented by Formula D-5 onto the hydrophobic carrier;
then filtering the resultant and washing the hydrophobic carrier with a mixture of the water-soluble organic solvent and the water to remove the contaminants;
and then eluting the compound represented by Formula D-5 from the hydrophobic carrier using an organic solvent.

41. The method according to any one of claims 28 to 40, wherein the method comprises purifying the compound represented by Formula D-8 by
after stopping the reaction of the compound represented by Formula D-6 with the compound represented by Formula D-7 in Step II-3, adding a hydrophobic carrier and water to a water-soluble organic solvent containing the resulting compound represented by Formula D-8 and contaminants to adsorb the compound represented by Formula D-8 onto the hydrophobic carrier;
then filtering the resultant and washing the hydrophobic carrier with a mixture of the water-soluble organic solvent and the water to remove the contaminants;
and then eluting the compound represented by Formula D-8 from the hydrophobic carrier using an organic solvent.

42. The method according to any one of claims 28 to 41, wherein in Step II-3, the method comprises protecting each amino group on the Formula D-9 with an acetyl group to give a compound represented by Formula D-10: 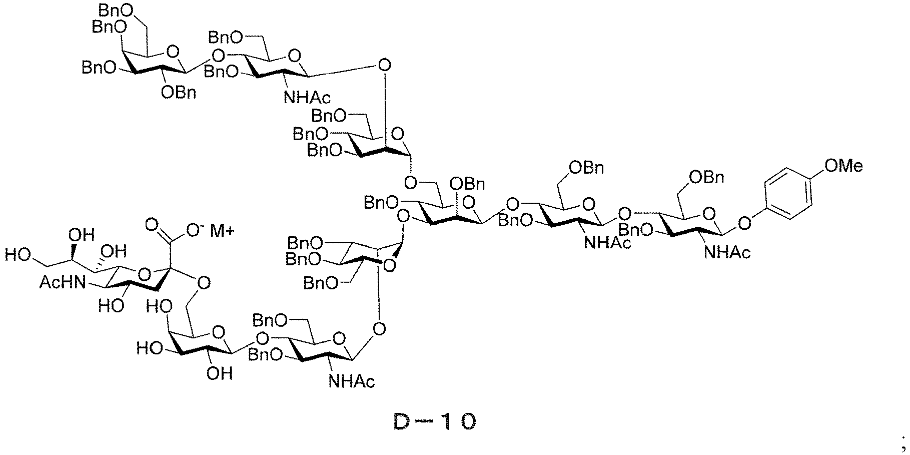 and purifying the compound represented by Formula D-10 by
adding a hydrophobic carrier and water to a water-soluble organic solvent containing the resulting compound represented by Formula D-10 and contaminants to adsorb the compound represented by Formula D-10 onto the hydrophobic carrier; then filtering the resultant and washing the hydrophobic carrier with a mixture of the water-soluble organic solvent and the water to remove the contaminants; and then eluting the compound represented by Formula D-10 from the hydrophobic carrier using an organic solvent.

43. The method according to claim 39, wherein the contaminants include a sugar compound other than the compound represented by Formula D-1, and/or a compound derived from a reaction reagent to obtain the compound to be purified.

44. The method according to claim 40, wherein the contaminants include a sugar compound other than the compound represented by Formula D-5, and/or a compound derived from a reaction reagent to obtain the compound to be purified.

45. The method according to claim 41, wherein the contaminants include a sugar compound other than the compound represented by Formula D-8, and/or a compound derived from a reaction reagent to obtain the compound to be purified.

46. The method according to claim 42, wherein the contaminants include a sugar compound other than the compound represented by Formula C-10, and/or a compound derived from a reaction reagent to obtain the compound to be purified.

47. The method according to any one of claims 39 to 46, wherein the hydrophobic carrier is a resin used for packing reversed-phase partition chromatography.

48. The method according to claim 47, wherein the resin packed for reversed-phase partition chromatography is selected from the group consisting of a poly(styrene/divinylbenzene) polymer gel resin, a polystyrene-divinylbenzene resin, polyhydroxymethacrylate resin, a styrene-vinylbenzene copolymer resin, a polyvinyl alcohol resin, polystyrene resin, a polymethacrylate resin, a chemically bonded silica gel resin, and a combination thereof.

49. The method according to claim 48, wherein the chemically bonded silica gel resin is selected from the group consisting of (1) a resin obtained by reacting silica gel with a silane coupling agent, (2) a resin obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) a resin obtained by chemically bonding a docosyl or triacontyl group to silica gel, and (4) a combination of the resins (1) to (3).

50. The method according to claim 48, wherein the chemically bonded silica gel resin is an octadecyl group-bonded silica gel resin (ODS resin).

51. The method according to any one of claims 39 to 50, wherein the water-soluble organic solvent is a water-soluble alcohol-based solvent, a water-soluble nitrile-based solvent, a water-soluble ether-based solvent, a water-soluble ketone-based solvent, a water-soluble amide-based solvent, or a water-soluble sulfoxide-based solvent, or a mixed solvent containing at least one of the aforementioned water-soluble organic solvents.

52. The method according to claim 51, wherein the water-soluble nitrile-based solvent is acetonitrile.

53. The method according to any one of claims 39 to 52, wherein the organic solvent used for eluting the compound of interest from the hydrophobic carrier is a nitrile-based solvent, an ether-based solvent, an ester-based solvent, a ketone-based solvent, a halogen-based solvent, an aromatic solvent, or a mixed solvent containing at least one of the aforementioned solvents.

54. The method according to any one of claims 28 to 53, further comprising the step of forming a salt of the compound represented by Formula D-5 with fumaric acid to give a crystalline compound represented by Formula D-5-FMA: 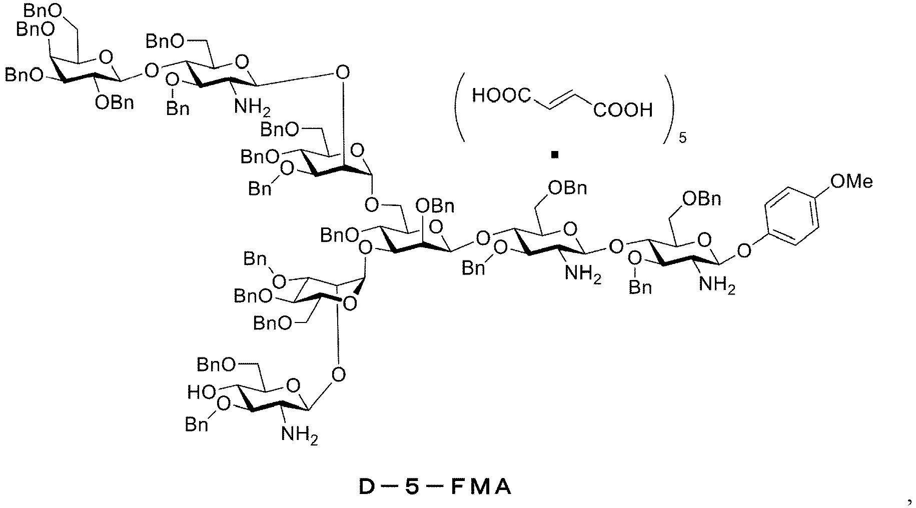 and then separating and purifying the crystalline compound represented by Formula D-5-FMA from non-crystalline materials.

55. A method for producing a compound represented by Formula B-5: 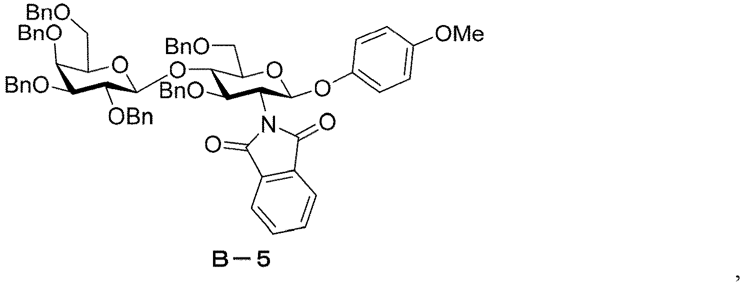 wherein the method comprises the step of adding lithium tert-butoxide or lithium tert-amoxide to a solvent containing a compound represented by Formula B-4: 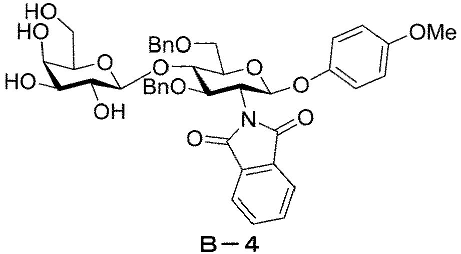 and benzyl halide or benzyl sulfonate to protect each hydroxyl group present in the compound represented by Formula B-4 with by a benzyl group.

56. The method according to claim 55, wherein the solvent is an amide-based solvent, an ether-based solvent, an aromatic solvent, a urea-based solvent, or a hydrocarbon-based solvent, or a mixed solvent containing at least one of the aforementioned solvents.

57. A method for producing a compound represented by Formula A-10; 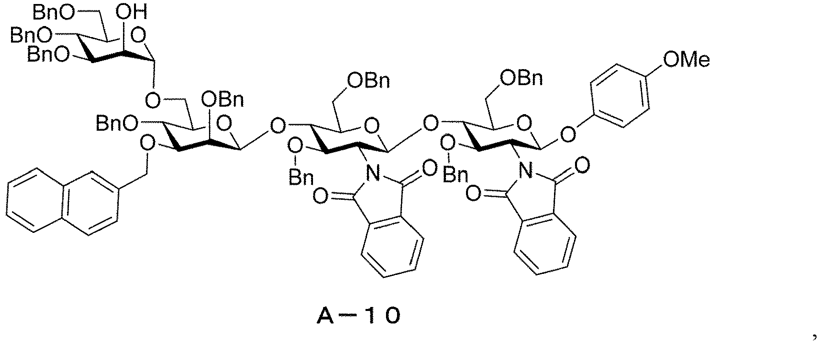 wherein the method comprises the step of reacting a compound represented by Formula A-9: 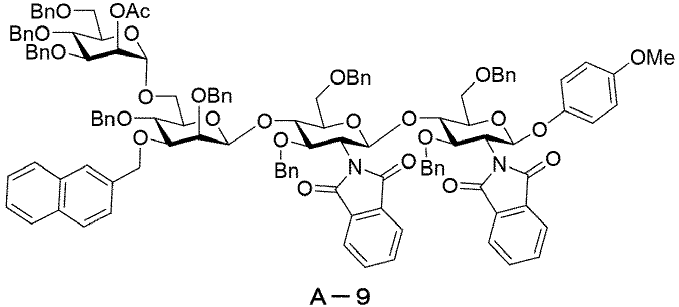 with a strong base in the presence of an alkyl ester of perfluorocarboxylic acid.

58. The method according to claim 57, wherein the alkyl ester of perfluorocarboxylic acid is methyl trifluoroacetate, ethyl trifluoroacetate, propyl trifluoroacetate, isopropyl trifluoroacetate, butyl trifluoroacetate, methyl pentafluoropropionate, ethyl pentafluoropropionate, propyl pentafluoropropionate, isopropyl pentafluoropropionate, methyl heptafluorobutyrate, ethyl heptafluorobutyrate, propyl heptafluorobutyrate, isopropyl heptafluorobutyrate, butyl heptafluorobutyrate, methyl nonafluorovalerate, ethyl nonafluorovalerate, propyl nonafluorovalerate, isopropyl nonafluorovalerate, butyl nonafluorovalerate, methyl undecafluorocaproate, ethyl undecafluorocaproate, propyl undecafluorocaproate, isopropyl undecafluorocaproate, or butyl undecafluorocaproate.

59. The method according to claim 57 or 58, wherein the strong base is selected from the group consisting of sodium salts, lithium salts, potassium salts of metal amides; sodium, lithium, potassium, cesium, and barium salts of C1-C20 alkoxides; sodium hydride, potassium hydride, lithium hydride, butyllithium, potassium carbonate, sodium carbonate, cesium carbonate, lithium carbonate, potassium phosphate, sodium phosphate, cesium phosphate, lithium phosphate, diazabicycloundecene (DBU), diazabicyclononene (DBN), and 1,1,3,3-tetramethylguanidine (TMG); and a combination thereof.

60. The method according to claim 57 or 58, wherein the strong base is potassium tert-butoxide, sodium tert-butoxide, lithium tert-butoxide, or LHMDS (lithium hexamethyldisilazide).

61. The method according to any one of claims 57 to 60, wherein the reaction is carried out in a C1-C10 alcohol solvent alone or in a mixture of a C1-C10 alcohol solvent and an amide-based solvent, an ether-based solvent, an ester-based solvent, an aromatic solvent, a halogen-based solvent, a hydrocarbon-based solvent, or a nitrile-based solvent.

62. A method for producing an oligosaccharide represented by Formula A-13: 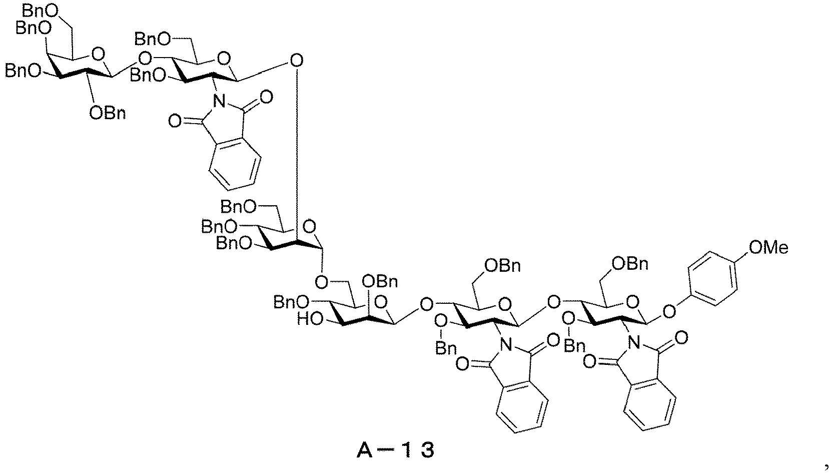 wherein the method comprises the step of reacting a compound represented by Formula A-12: 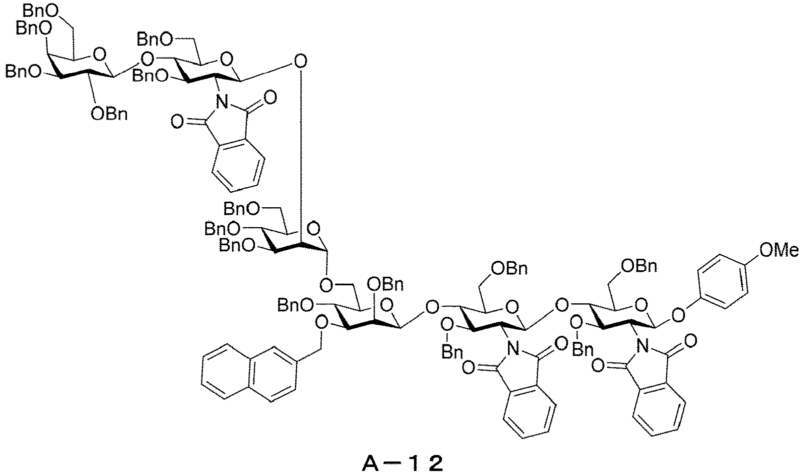 with DDQ (2,3-dichloro-5,6-dicyano-p-benzoquinone) in a mixed solvent of fluorous alcohol and water to remove a 2-naphthylmethyl group in the compound represented by Formula A-12.

63. The method according to claim 62, wherein the fluorous alcohol is selected from the group consisting of hexafluoro-2-propanol (HFIP), 2,2,2-trifluoroethanol (TFE), 2,2,3,3,4,4,5,5-octafluoro-1-pentanol, nonafluoro-tert-butyl alcohol, and a combination thereof.

64. The method according to claim 62 or 63, wherein the reaction is carried out at -35°C to 70°C.

65. The method according to claim 62 or 63, wherein the reaction is carried out at -30°C to -10°C.

66. A method of purifying a compound represented by Formula A-5: 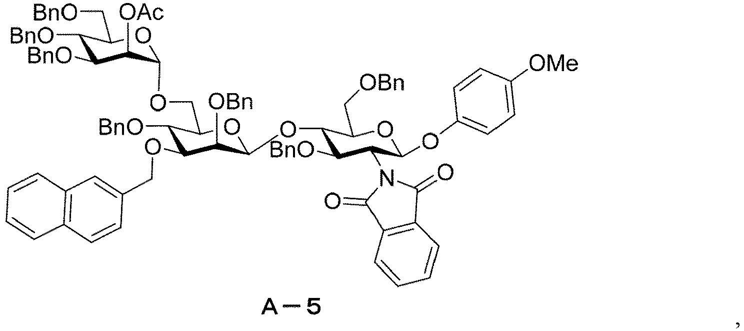 wherein the method comprises steps of:
adding a hydrophobic carrier and water to a water-soluble organic solvent containing the compound represented by Formula A-5 and contaminants to adsorb the compound represented by Formula A-5 onto the hydrophobic carrier;
then filtering the resultant and washing the hydrophobic carrier with a mixture of the water-soluble organic solvent and the water to remove the contaminants;
and then eluting the compound represented by Formula A-5 from the hydrophobic carrier using an organic solvent to purify the compound represented by Formula A-5.

67. A method of purifying a compound represented by Formula A-9: 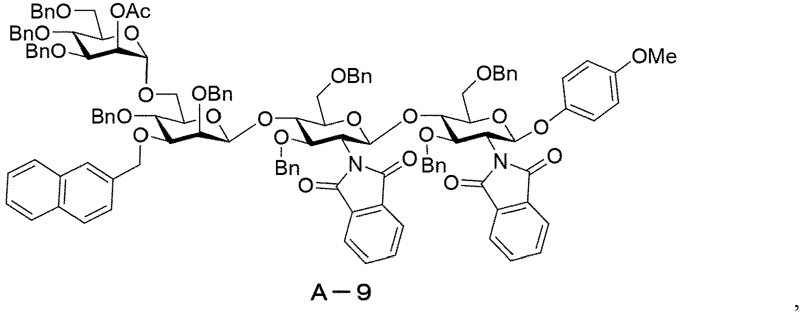 wherein the method comprises steps of:
adding a hydrophobic carrier and water to a water-soluble organic solvent containing the compound represented by Formula A-9 and contaminants to adsorb the compound represented by Formula A-9 onto the hydrophobic carrier;
then filtering the resultant and washing the hydrophobic carrier with a mixture of the water-soluble organic solvent and the water to remove the contaminants;
and then eluting the compound represented by Formula A-9 from the hydrophobic carrier using an organic solvent to purify the compound represented by Formula A-9.

68. A method of purifying a compound represented by Formula A-12: 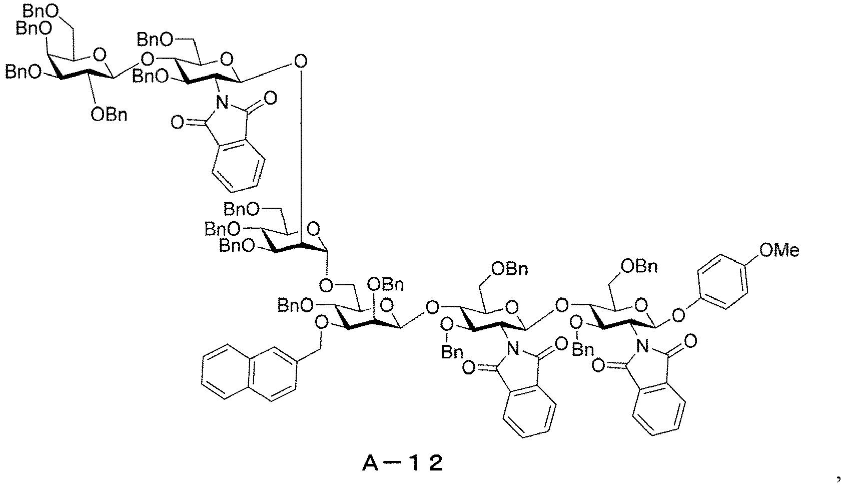 wherein the method comprises steps of:
adding a hydrophobic carrier and water to a water-soluble organic solvent containing the compound represented by Formula A-12 and contaminants to adsorb the compound represented by Formula A-12 onto the hydrophobic carrier;
then filtering the resultant and washing the hydrophobic carrier with a mixture of the water-soluble organic solvent and the water to remove the contaminants;
and then eluting the compound represented by Formula A-12 from the hydrophobic carrier using an organic solvent to purify the compound represented by Formula A-12.

69. A method of purifying a compound represented by Formula D-1: 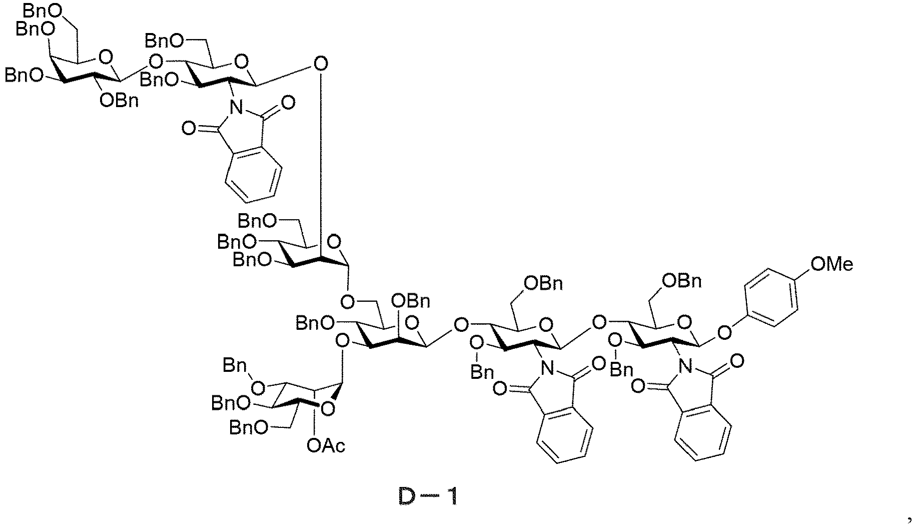
wherein the method comprises steps of: adding a hydrophobic carrier and water to a water-soluble organic solvent containing the compound represented by Formula D-1 and contaminants to adsorb the compound represented by Formula D-1 onto the hydrophobic carrier;
then filtering the resultant and washing the hydrophobic carrier with a mixture of the water-soluble organic solvent and the water to remove the contaminants;
and then eluting the compound represented by Formula D-1 from the hydrophobic carrier using an organic solvent to purify the compound represented by Formula D-1.

70. A method of purifying a compound represented by Formula D-5: 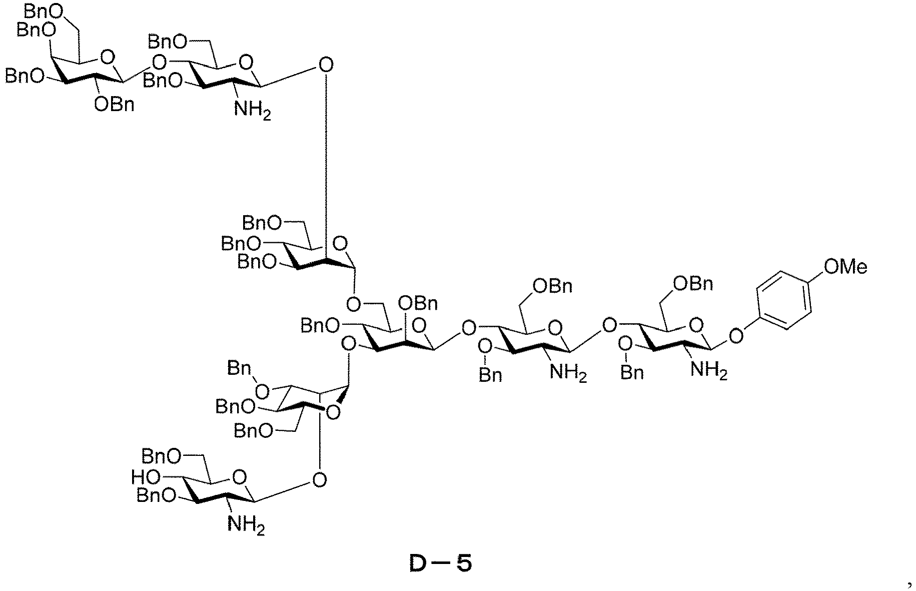 wherein the method comprises steps of:
adding a hydrophobic carrier and water to a water-soluble organic solvent containing the compound represented by Formula D-5 and contaminants to adsorb the compound represented by Formula D-5 onto the hydrophobic carrier;
then filtering the resultant and washing the hydrophobic carrier with a mixture of the water-soluble organic solvent and the water to remove the contaminants;
and then eluting the compound represented by Formula D-5 from the hydrophobic carrier using an organic solvent to purify the compound represented by Formula D-5.

71. A method of purifying a compound represented by Formula D-8: 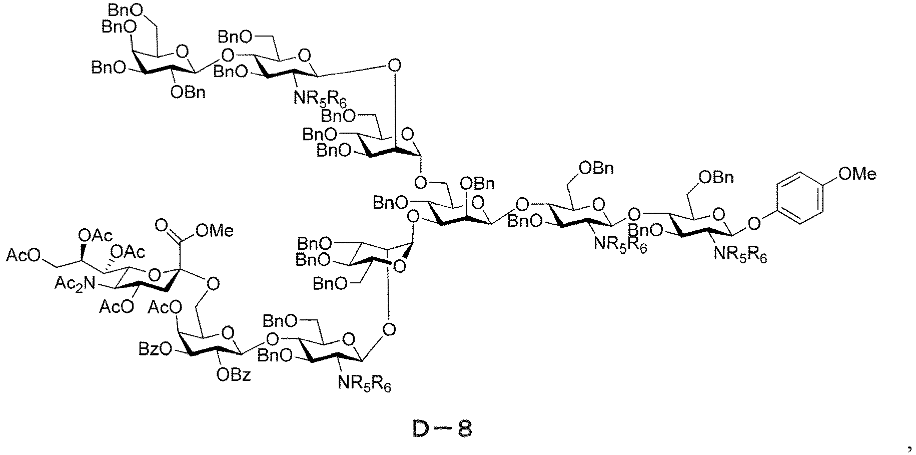
wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
wherein the method comprises steps of:
adding a hydrophobic carrier and water to a water-soluble organic solvent containing the compound represented by Formula D-8 and contaminants to adsorb the compound represented by Formula D-8 onto the hydrophobic carrier;
then filtering the resultant and washing the hydrophobic carrier with a mixture of the water-soluble organic solvent and the water to remove the contaminants;
and then eluting the compound represented by Formula D-8 from the hydrophobic carrier using an organic solvent to purify the compound represented by Formula D-8.

72. A method of purifying a compound represented by Formula D-10: 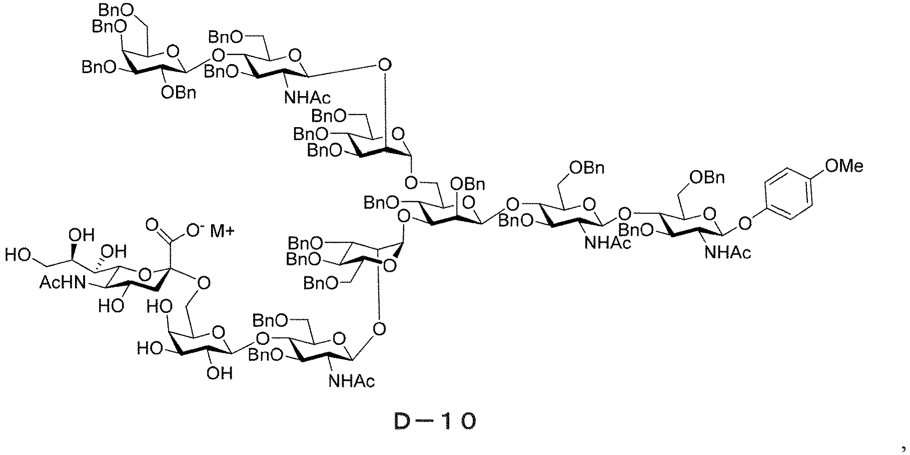 wherein the method comprises steps of:
adding a hydrophobic carrier and water to a water-soluble organic solvent containing the compound represented by Formula D-10 and contaminants to adsorb the compound represented by Formula D-10 onto the hydrophobic carrier;
then filtering the resultant and washing the hydrophobic carrier with a mixture of the water-soluble organic solvent and the water to remove the contaminants;
and then eluting the compound represented by Formula D-10 from the hydrophobic carrier using an organic solvent to purify the compound represented by Formula D-10.

73. The method according to claim 69, wherein the contaminants include a sugar compound other than the compound represented by Formula D-1, and/or a compound derived from a reaction reagent to obtain the compound to be purified.

74. The method according to claim 70, wherein the contaminants include a sugar compound other than the compound represented by Formula D-5, and/or a compound derived from a reaction reagent to obtain the compound to be purified.

75. The method according to claim 71, wherein the contaminants include a sugar compound other than the compound represented by Formula D-8, and/or a compound derived from a reaction reagent to obtain the compound to be purified.

76. The method according to claim 72, wherein the contaminants include a sugar compound other than the compound represented by Formula D-10, and/or a compound derived from a reaction reagent to obtain the compound to be purified.

77. The method according to any one of claims 69 to 76, wherein the hydrophobic carrier is a resin used for packing reversed-phase partition chromatography.

78. The method according to claim 77, wherein the resin packed for reversed-phase partition chromatography is selected from the group consisting of a poly(styrene/divinylbenzene) polymer gel resin, a polystyrene-divinylbenzene resin, a polyhydroxymethacrylate resin, a styrene-vinylbenzene copolymer resin, a polyvinyl alcohol resin, a polystyrene resin, a polymethacrylate resin, a chemically bonded silica gel resin, and a combination thereof.

79. The method according to claim 78, wherein the chemically bonded silica gel resin is selected from the group consisting of (1) a resin obtained by reacting silica gel with a silane coupling agent, (2) a resin obtained by chemically bonding a dimethyloctadecyl, octadecyl, trimethyloctadecyl, dimethyloctyl, octyl, butyl, ethyl, methyl, phenyl, cyanopropyl, or aminopropyl group to silica gel, and (3) a resin obtained by chemically bonding a docosyl or triacontyl group to silica gel, and (4) a combination of the resins (1) to (3).

80. The method according to claim 79, wherein the chemically bonded silica gel resin is an octadecyl group-bonded silica gel resin (ODS resin).

81. The method according to any one of claims 69 to 80, wherein the water-soluble organic solvent is a water-soluble alcohol-based solvent, a water-soluble nitrile-based solvent, a water-soluble ether-based solvent, a water-soluble ketone-based solvent, a water-soluble amide-based solvent, a water-soluble sulfoxide-based solvent, or a mixed solvent containing at least one of the aforementioned water-soluble organic solvents.

82. The method according to claim 81, wherein the water-soluble nitrile-based solvent is acetonitrile.

83. The method according to any one of claims 69 to 82, wherein the organic solvent used for eluting the compound of interest from the hydrophobic carrier is a nitrile-based solvent, an ether-based solvent, an ester-based solvent, a ketone-based solvent, a halogen-based solvent, an aromatic solvent, or a mixed solvent containing at least one of the aforementioned solvents.

84. A method for producing a compound represented by Formula D-8: 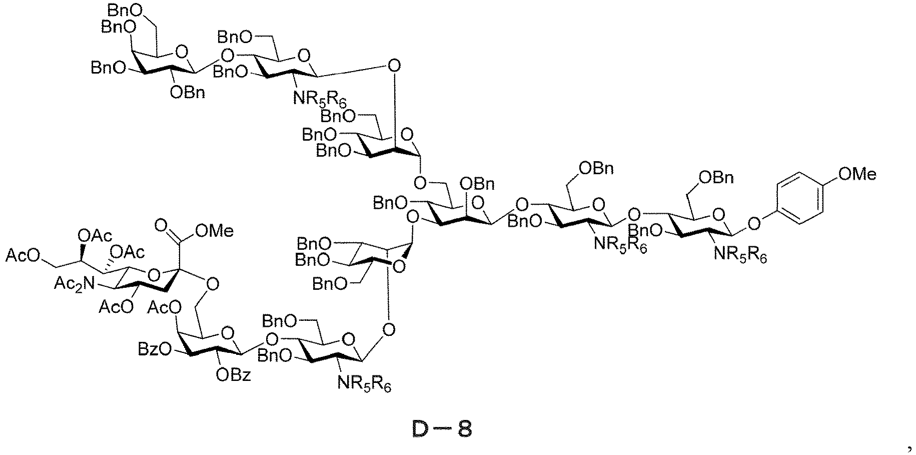
wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group,
wherein the method comprises steps of:
producing a compound represented by Formula D-6: 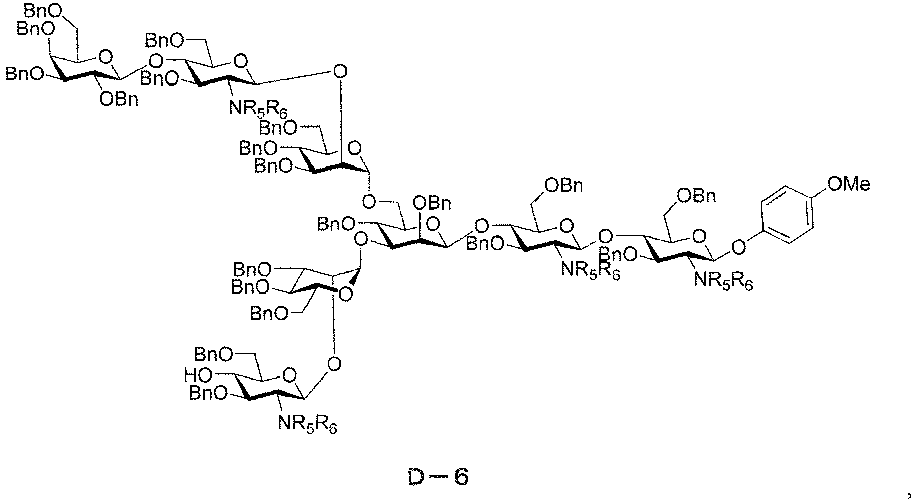
wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group;
and then connecting the compound represented by Formula D-6 to a compound represented by Formula D-7: 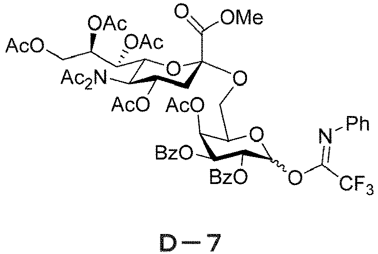 via β-1,4-glycosidic linkage to give the compound represented by Formula D-8.

85. The method according to claim 84, wherein R₅ is an aryloxycarbonyl (COOAr) group.

86. The method according to claim 84 or 85, wherein the method comprises a step of removing each amino group-protecting group and alcohol acyl-based-protecting group from the compound represented by Formula D-8 to give a compound represented by Formula D-9: 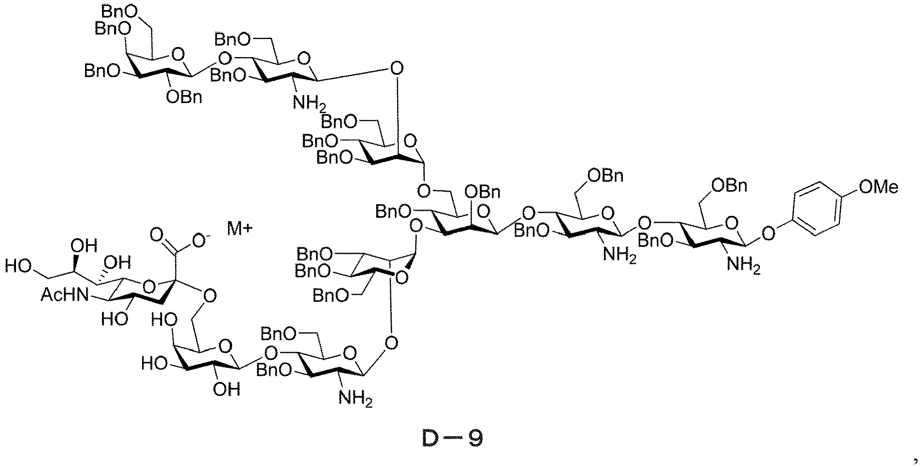 wherein M⁺ is a sodium ion, a lithium ion, a potassium ion, or a protonated triethylamine cation.

87. A method of purifying a compound represented by Formula D-12: 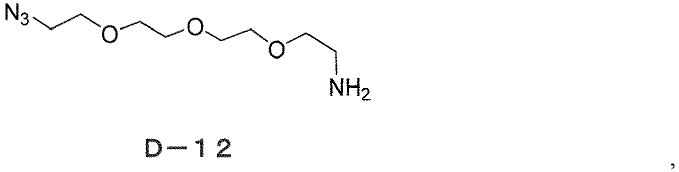 wherein the method comprises steps of:
adding, to a solution containing the crude compound represented by Formula D-12, a compound represented by Formula E-1: 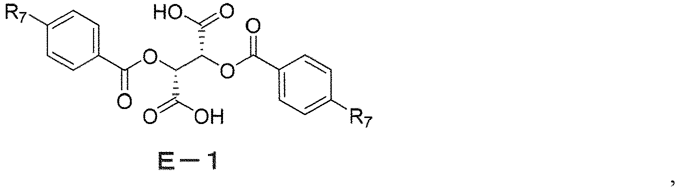 wherein R₇ is a hydrogen atom, a methyl group, or a methoxy group,
to give a crystallin compound represented by Formula E-2: 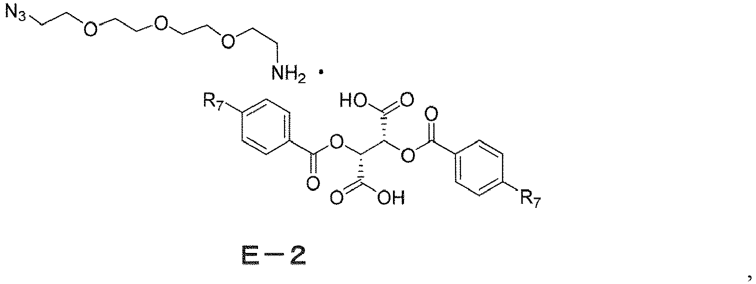 wherein R₇ is a hydrogen atom, a methyl group, or a methoxy group; and
isolating the crystalline compound and then extracting the compound represented by Formula D-12 from the crystalline compound isolated.

88. An oligosaccharide represented by Formula A-13:

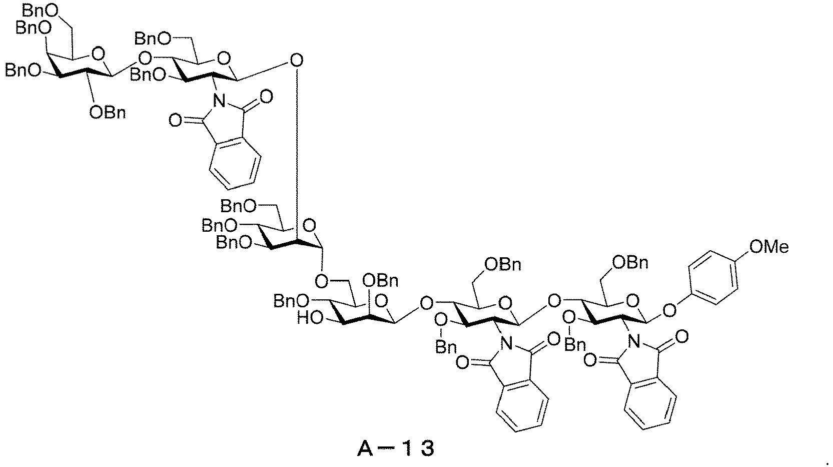

89. A compound represented by Formula A-5:

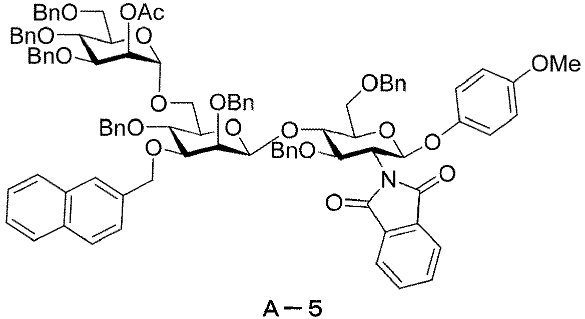

90. A compound represented by Formula A-6:

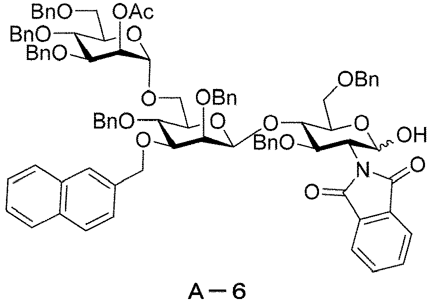

91. A compound represented by Formula A-7:

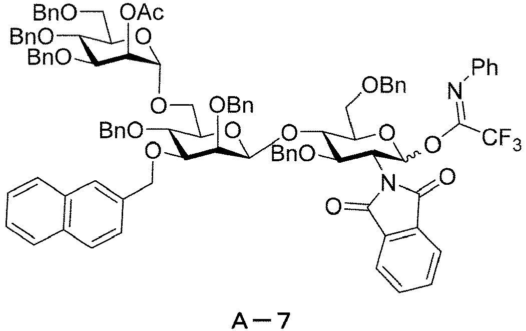

92. A compound represented by Formula A-9:

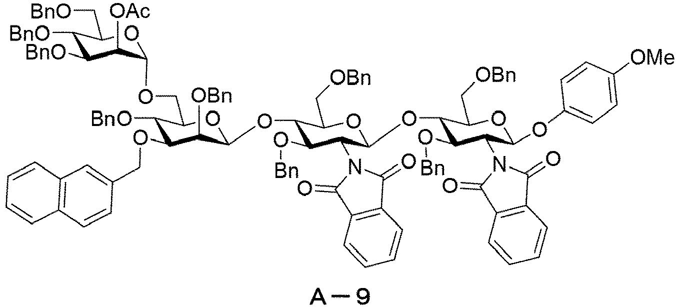

93. A compound represented by Formula A-10:

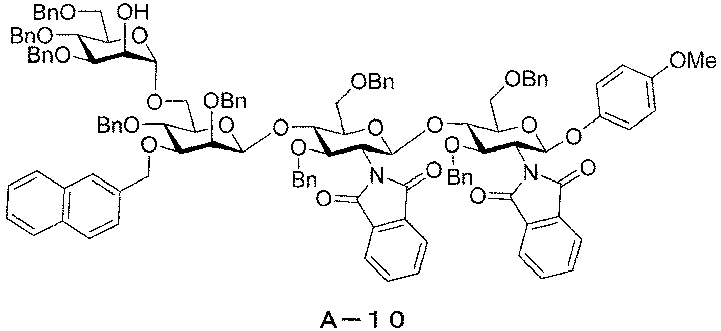

94. A compound represented by Formula A-11:

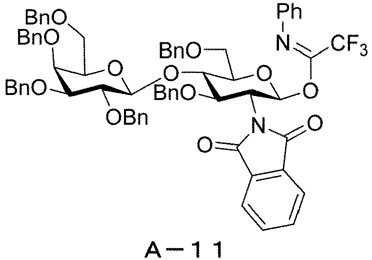

95. A compound represented by Formula A-12:

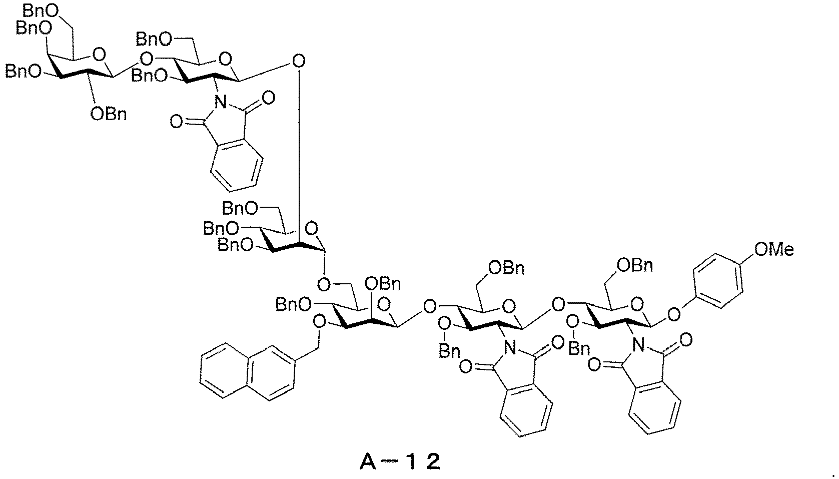

96. A compound represented by Formula A-14:

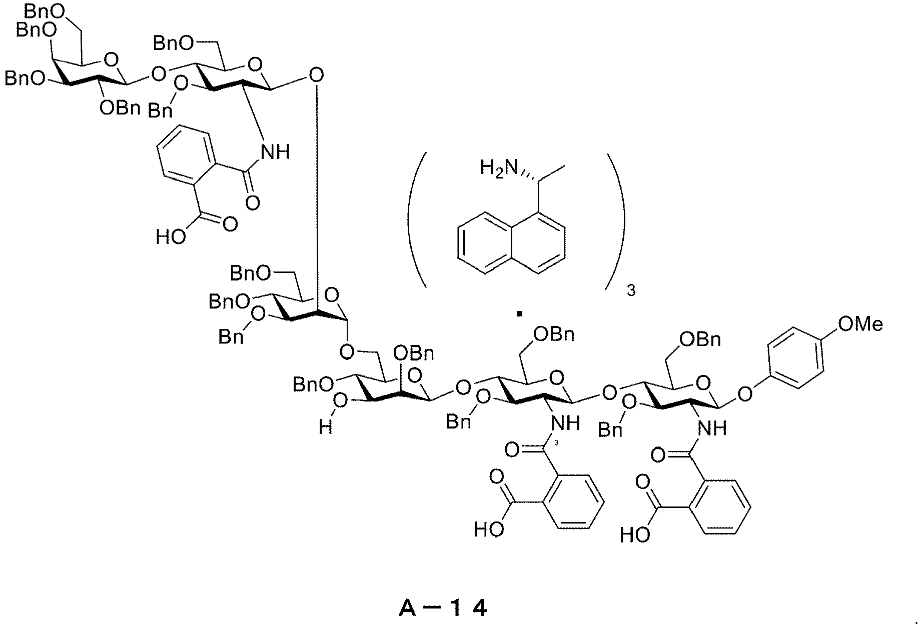

97. A compound represented by Formula A-15:

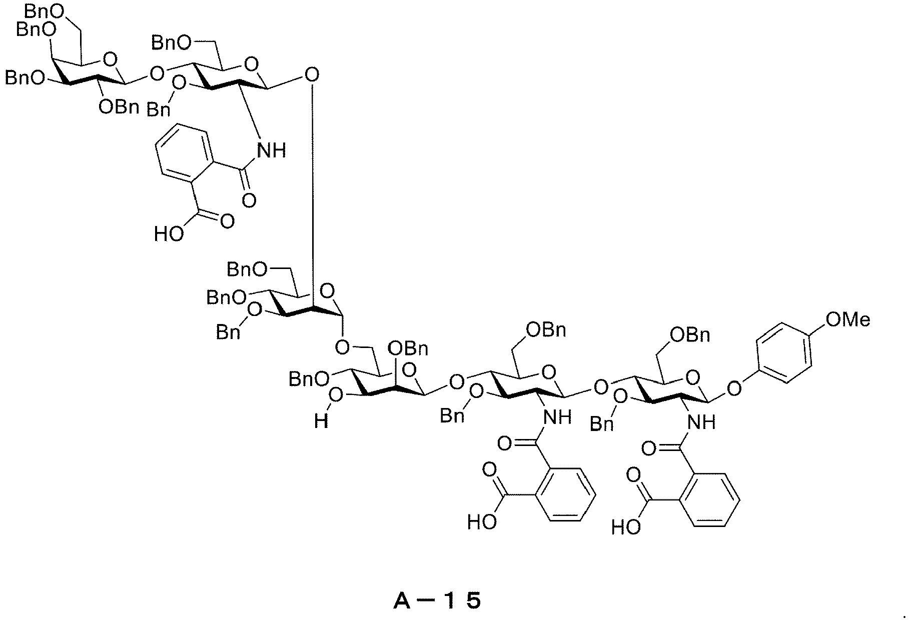

98. A compound represented by Formula B-4:

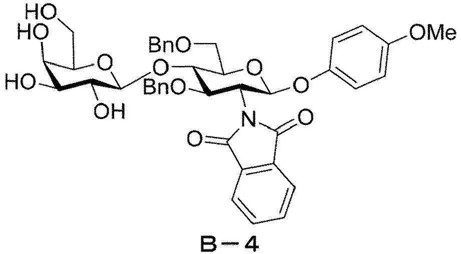

99. A compound represented by Formula B-5:

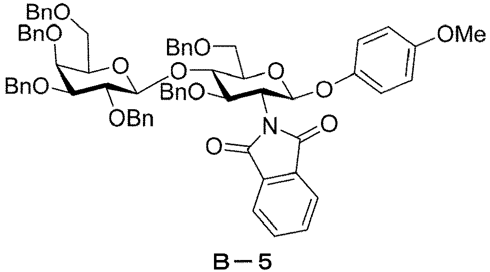

100. A compound represented by Formula B-6:

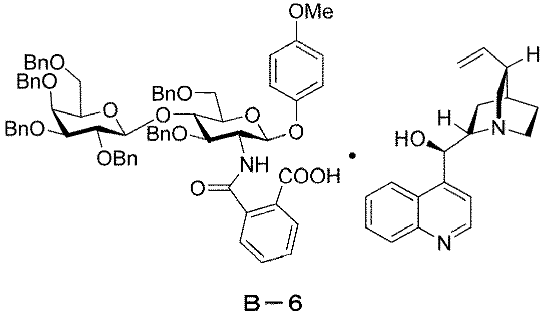

101. A compound represented by Formula B-7:

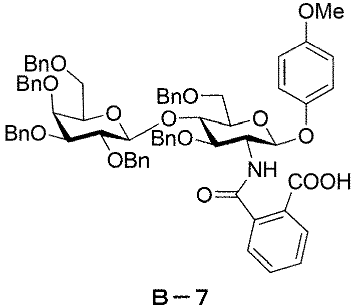

102. A compound represented by Formula B-8:

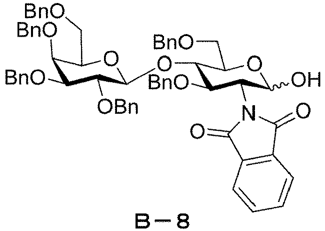

103. A compound represented by Formula D-1:

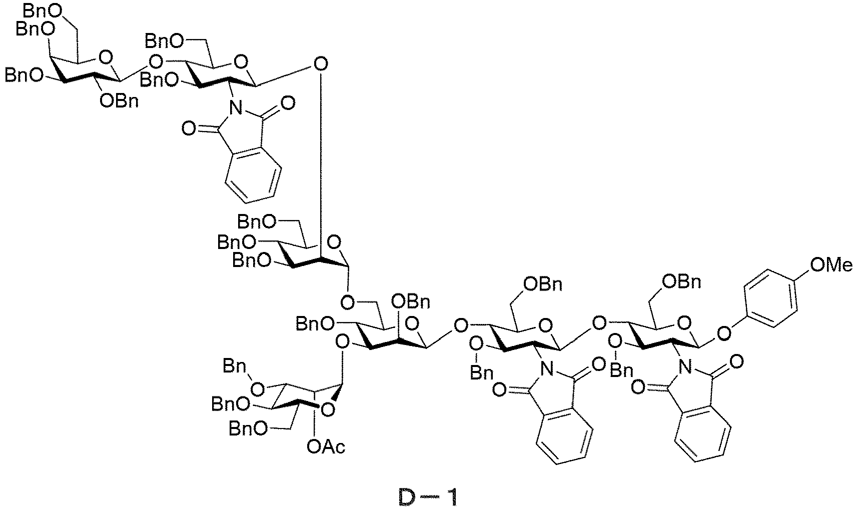

104. A compound represented by Formula D-2:

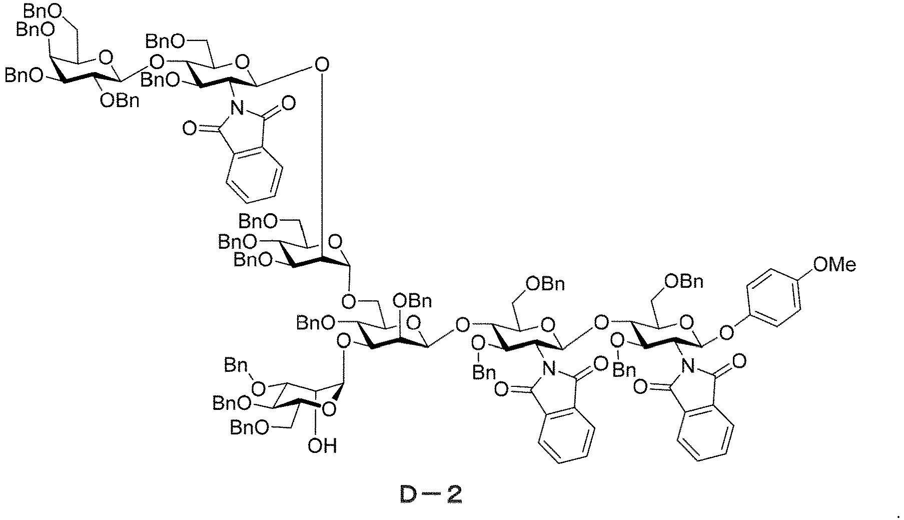

105. A compound represented by Formula D-4:

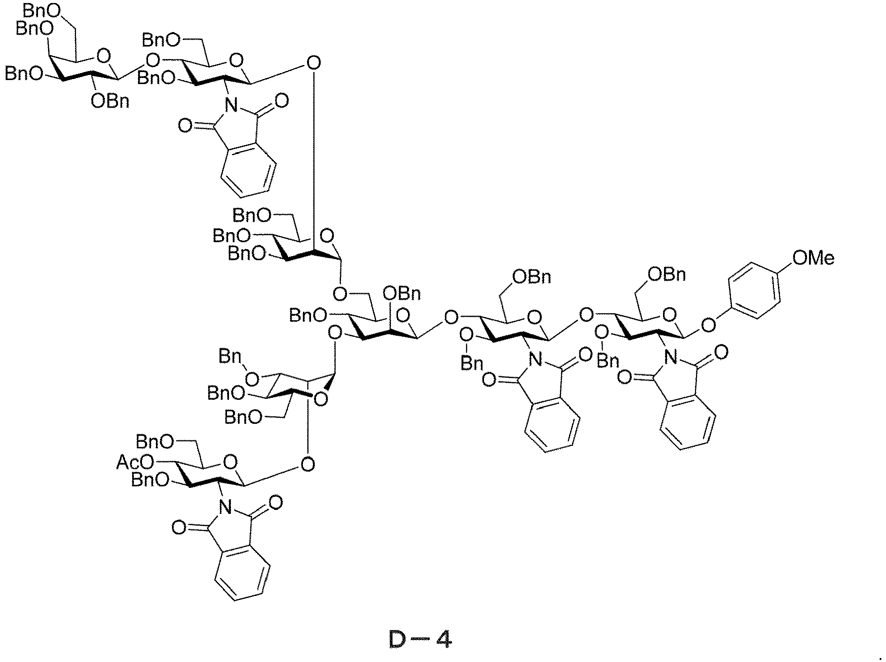

106. A compound represented by Formula D-5:

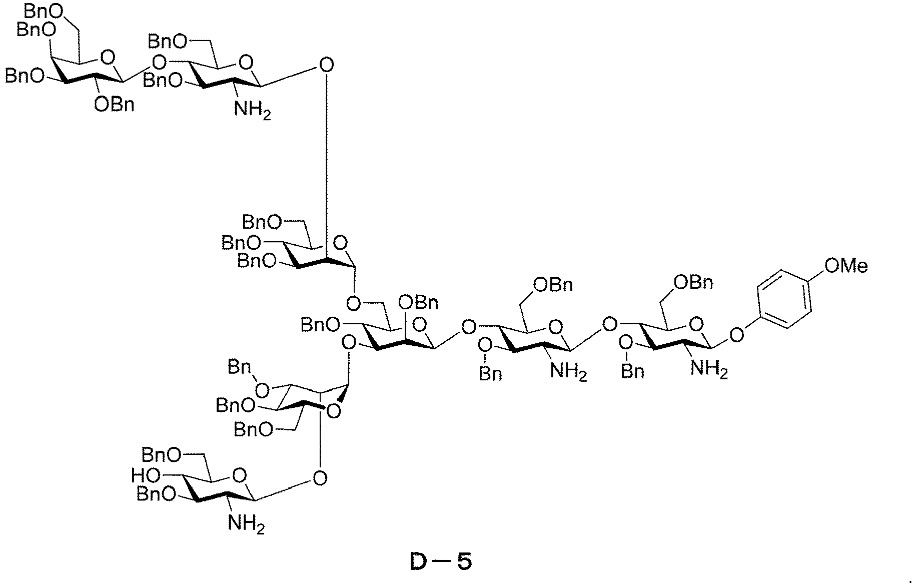

107. A compound represented by Formula D-5-FMA:

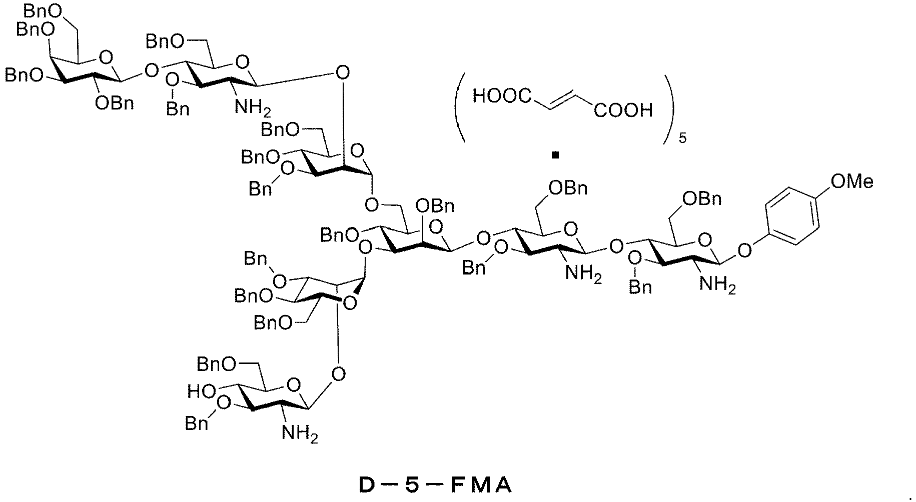

108. A compound represented by Formula D-6: 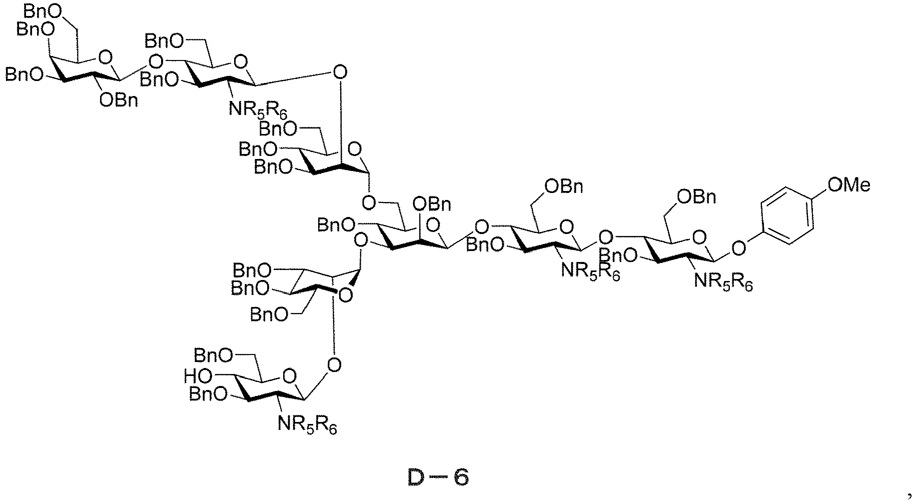 wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.

109. A compound represented by Formula D-8: 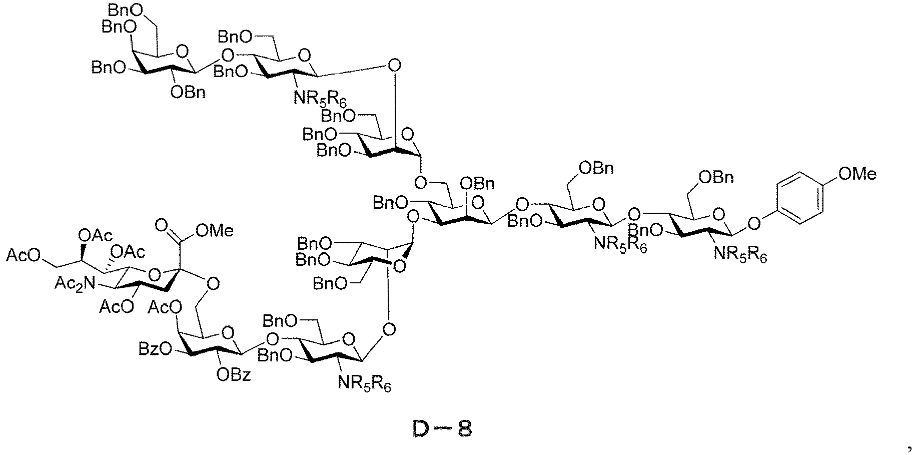 wherein R₅ is an aryloxycarbonyl (COOAr) group, an acetyl (Ac) group, or a 2,2,2-trichloroethoxycarbonyl (Troc) group and R₆ is a hydrogen atom, or R₅ and R₆ together with a nitrogen atom attached thereto form a phthalimide group.

110. A compound represented by Formula D-9: 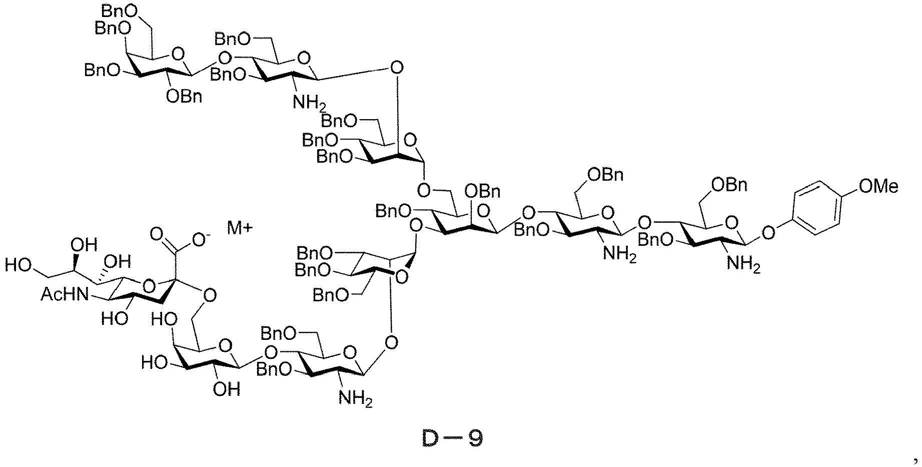 wherein M⁺ is a sodium ion, a lithium ion, a potassium ion, or a protonated triethylamine cation.

111. A compound represented by Formula D-10: 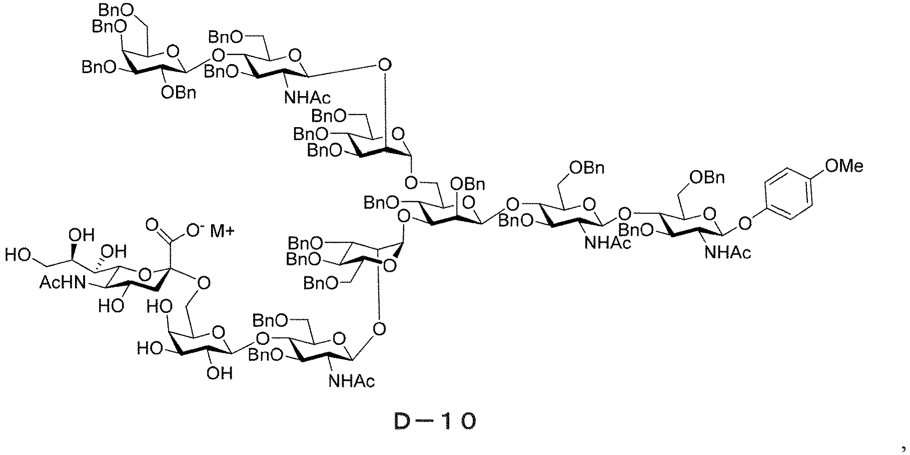 wherein M⁺ is a sodium ion, a lithium ion, a potassium ion, or a protonated triethylamine cation.

112. A compound represented by Formula D-11: 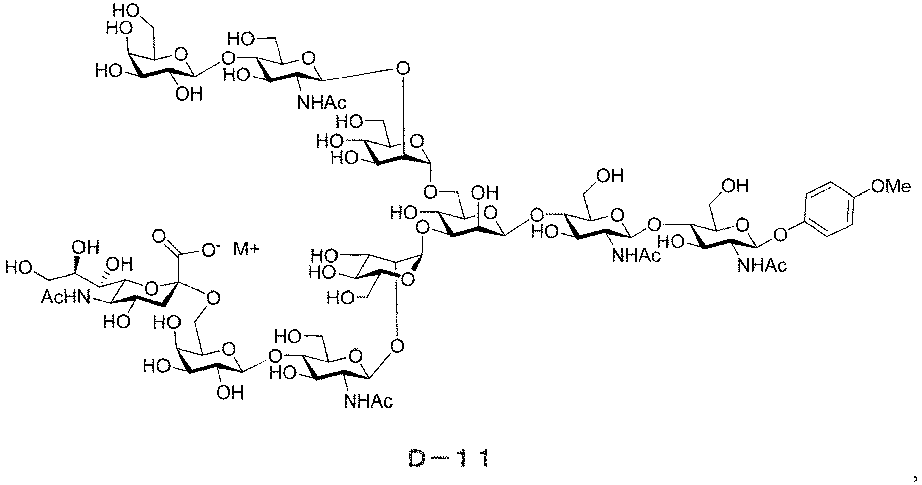 wherein M⁺ is a sodium ion, a lithium ion, a potassium ion, or a protonated triethylamine cation.

113. A crystalline compound represented by Formula E-2: 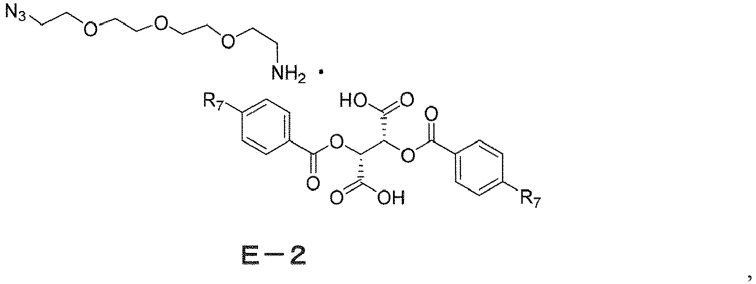 wherein R₇ is a hydrogen atom, a methyl group, or a methoxy group.

114. A compound represented by Formula D-12: 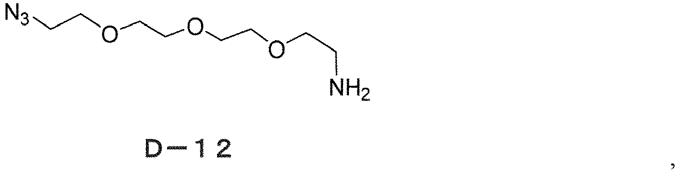 wherein the compound has a purity of 90% or higher as measured by HPLC.

115. The compound according to claim 109, wherein the compound has a purity of 95% or higher.

116. An oligosaccharide represented by Formula D-13:
